# EUROPÄISCHE PATENTSCHRIFT

(11) **EP 2 292 625 B1**
(45) Veröffentlichungstag und Bekanntmachung des Hinweises auf die Patenterteilung: **25.12.2013**
(21) Anmeldenummer: 10012043.5
(22) Anmeldetag: 17.05.2006
(51) Int. Cl.: C07D 498/10, A61K 31/438, A61P 29/00

(54) **SUBSTITUIERTE SPIRO-VERBINDUNGEN UND DEREN VERWENDUNG ZUR HERSTELLUNG VON ARZNEIMITTELN GEGEN SCHMERZ**
SUBSTITUTED SPIRO COMPOUNDS AND THEIR USE FOR PRODUCING PAIN-RELIEF MEDICAMENTS
COMPOSES SPIRO SUBSTITUES, ET LEUR UTILISATION POUR PRODUIRE DES MEDICAMENTS CONTRE LA DOULEUR

(30) Priorität: 19.05.2005 DE 102005023784
(43) Veröffentlichungstag der Anmeldung: 09.03.2011
(62) Teilanmeldung aus: 06742950.6
(73) Patentinhaber: Grünenthal GmbH, 52078 Aachen (DE)
(72) Erfinder: Schick, Hans, Prof. Dr., 13086 Berlin-Welssensee (DE); Frank, Robert, Dr., 52070 Aachen (DE); Reich, Melanie, Dr., 52072 Aachen (DE); Jostock, Ruth, Dr., 52223 Stolberg (DE); Bahrenberg, Gregor, Dr., 52156 Monschau-Konzen (DE); Theil, Fritz, Dr., 12247 Berlin (DE); Henkel, Brigitta, Dr., 12555 Berlin (DE)
(74) Vertreter: Brosch, Oliver

(56) Entgegenhaltungen:
- WO-A-97/33887
- WO-A-03/000699

## Beschreibung

Die vorllegende Erfindung betrifft substituierte Spiro-Verbindungen, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufrieden stellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz, insbesondere von neuropathischem Schmerz, stellt der Vanillold-Rezeptor vom Subtyp 1 (VR1/TRPV1) dar, der häufig auch als Capsalcin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Haminkontinenz.

WO 03/000699 offenbart substituierte 1-Oxa-2,8-Diaza -Spiro[4,5]dec-2-en-Derivate als Arzheimittel gegen Schmerz.

Eine Aufgabe der vorliegenden Erfindung bestand daher darin, neue Verbindungen zur Verfügung zu stellen, die sich insbesondere als pharmakologische Wirkstoffe In Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass sich substituierte Spiro-Verbindungen der nachstehend angegebenen allgemeinen Formel I zur Bekämpfung von Schmerz eignen und eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vaniloid-Rezeptoren 1 (VR1/TRPV1) vermittelt werden.

Ein Gegenstand der vorliegenden Erfindung sind daher substituierte Spiro-Verbindungen der allgemeinen Formel I, 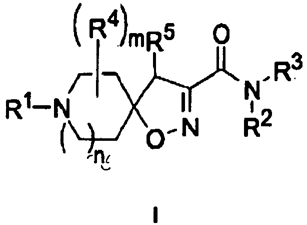 worin
- m: gleich 0, 1, 2, 3 oder 4 ist,
- n: gleich 1 ist,
- R¹: für eine -C(=S)-NR⁸R⁹-Gruppe steht;
oder sofern m ungleich 0 und/oder R⁵ ungleich H ist,
zusätzlich für eine -C(=O)-NR⁶R⁷-Gruppe stehen kann,
- R²: für einen linearen oder verzweigten, gesättigten oder ungesättigten, unsubstituierten oder wenigstens einfach substituierten, ggf. wenigstens ein Heteroatom als Kettenglied aufweisenden aliphatischen Rest;
wobei die Substituenten des aliphatischen Restes unabhängig voneinander aus der Gruppe bestehend aus F, Cl, Br, I, -CN, -NO₂, -OH, -SH und -NH₂ ausgewählt werden können;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
- R³: für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
wobei die Substituenten des aliphatischen Restes unabhängig voneinander aus der Gruppe bestehend aus F, Cl, Br, I, -CN, -NO₂, -OH, -SH und -NH₂ ausgewählt werden können;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
oder
- R² und R³: zusammen mit dem sie verbindenden Stickstoffatom als Ringglied einen unsubstituierten oder wenigstens einfach substituierten, ungesättigten oder gesättigten, ggf. wenigstens ein weiteres Heteroatom als Ringglied aufweisenden cycloaliphatischen Rest bilden, der mit einem unsubstituierten oder wenigstens einfach substituierten mono- oder polyzyklischen Ringsystem kondensiert sein kann;
- R⁴: für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R¹⁰-Gruppe, für eine -NR¹¹R¹²-Gruppe, für eine -C(=O)-R¹³-Gruppe, für eine -C(=O)-O-R¹⁴-Gruppe, für eine -O-C(=O)-R¹⁵-Gruppe, für eine -NH-C(=O)-R¹⁶-Gruppe, für eine -NR¹⁷-C(=O)-R¹⁸-Gruppe, für eine -C(=O)-NH₂-Gruppe, für eine -C(=O)-NH-R¹⁹-Gruppe, für eine -C(=O)-NR²⁰R²¹-Gruppe, für eine -O-R²²-Gruppe, für eine -S-R²³-Gruppe, oder für einen linearen oder verzweigten, gesättigten oder ungesättigten, unsubstituierten oder wenigstens einfach substituierten aliphatischen Rest steht;
- R⁵: für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R¹⁰-Gruppe, für eine -NR¹¹R¹²-Gruppe, für eine -C(=O)-R¹³-Gruppe, für eine -C(=O)-O-R¹⁴-Gruppe, für eine -O-C(=O)-R¹⁵-Gruppe, für eine -NH-C(=O)-R¹⁶-Gruppe, für eine -NR¹⁷-C(=O)-R¹⁸-Gruppe, für eine -C(=O)-NH₂-Gruppe, für eine -C(=O)-NH-R¹⁹-Gruppe, für eine -C(=O)-NR²⁰R²¹-Gruppe, für eine -O-R²²-Gruppe, für eine -S-R²³-Gruppe, für einen linearen oder verzweigten, gesättigten oder ungesättigten, unsubstituierten oder wenigstens einfach substituierten aliphatischen Rest oder für einen unsubstituierten oder wenigstens einfach substituierten Aryl- oder Heteroaryl-Rest, der über eine lineare oder verzweigte, unsubstituierte oder wenigstens einfach substituierten Alkylen-, Alkenylen- oder Alkinylen-Gruppe gebunden sein kann, steht;
- R⁶ und R⁸,: unabhängig voneinander, jeweils für

für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, für eine -C(=O)-R²⁴-Gruppe, die über eine lineare oder verzweigte, unsubstituierte oder wenigstens einfach substituierten Alkylen-Gruppe gebunden sein kann,
oder für eine -C(=O)-O-R²⁵-Gruppe, die über eine lineare oder verzweigte, unsubstituierte oder wenigstens einfach substituierten Alkylen-Gruppe gebunden sein kann, stehen;
- R⁷ und R⁹,: unabhängig voneinander, jeweils für
für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für eine -C(=O)-R²⁴-Gruppe, die über eine lineare oder verzweigte, unsubstituierte oder wenigstens einfach substituierten Alkylen-Gruppe gebunden sein kann,
oder für eine -C(=O)-O-R²⁵-Gruppe, die über eine lineare oder verzweigte, unsubstituierte oder wenigstens einfach substituierten Alkylen-Gruppe gebunden sein kann, stehen;
und
R¹⁰, R¹¹, R¹², R¹³, R¹⁴, R¹⁵, R¹⁶, R¹⁷, R¹⁸, R¹⁹, R²⁰, R²¹, R²², R²³, R²⁴ und R²⁵, unabhängig voneinander, jeweils für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden sein kann, stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt können die Substituenten der vorstehend genannten aliphatischen Reste in Position der Substituenten R¹ bis R²⁵ unabhängig voneinander aus der Gruppe bestehend aus F, Cl, Br, I, -CN, -NO₂, -OH, -SH und -NH₂ ausgewählt werden.

Bevorzugt können die Reste R⁵, R² und R³ Alkylen-, Alkenylen- oder Alkinylen-Gruppen aufweisen, die jeweils mit Substituenten unabhängig voneinander ausgewählt aus der Gruppe bestehend aus F, Cl, Br, -OH, -SH, -NH₂, -CN, -NO₂ und Phenyl substituiert sein können; wobei der Phenyl-Rest mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann.

Bevorzugt bilden dir Reste R² und R³ zusammen mit dem sie verbindenden Stickstoffatom einen heterocycloaliphatischen Rest, der mit Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -NH-C₁₋₅-Alkyl, -N(C₁₋₅-Alkyl)₂, -NH-C(=O)-O-C₁₋₅-Alkyl, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Aliphatische Reste umfassen im Sinne dieser Erfindung azyklische gesättigte oder ungesättigte Kohlenwasserstoffreste, die verzweigt oder geradkettig sowie unsubstituiert oder einfach substituiert oder mehrfach, beispielsweise 1-, 2-, 3-, 4-, 5-, 6-, 7-, 8- oder 9-fach, gleich oder verschieden substituiert sein können, mit 1 bis 20 (d.h. 1, 2, 3, 4, 5, 6, 7, 8, 9, 10, 11, 12, 13, 14, 15, 16, 17, 18, 19 oder 20), bevorzugt 1 bis 12 (d.h. 1, 2, 3, 4, 5, 6, 7, 8, 9, 10, 11 oder 12), besonders bevorzugt 1 bis 6 (d.h. 1, 2, 3, 4, 5 oder 6) Kohlenstoffatomen, d.h. C₁₋₂₀-, C₁₋₁₂-, C₁₋₆-Alkyle, C₂₋₂₀-, C₂-₁₂-, C₂₋₆-Alkenyle und C₂₋₂₀-, C₂₋₁₂-, C₂₋₆-Alkinyle. Dabei weisen Alkenyle mindestens eine C-C-Doppelbindung und Alkinyle mindestens eine C-C-Dreifachbindung auf. Beispielhaft seien aliphatische Reste ausgewählt aus der Gruppe bestehend aus Methyl, Ethyl, n-Propyl, Isopropyl, n-Butyl, Isobutyl, sec-Butyl, tert-Butyl, n-Pentyl, Isopentyl, neo-Pentyl, n-Hexyl, 2-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Ethenyl (Vinyl), Ethinyl, Propenyl (-CH₂CH=CH₂, -CH=CH-CH₃, -C(=CH₂)-CH₃), 2-Methyl-propenyl, Propinyl (-CH₂≡CCH, -C≡C-CH₃), Butenyl, Butinyl, Pentenyl, Pentinyl, Hexenyl, Hexinyl, Octenyl und Octinyl genannt.

Die vorstehend genannten aliphatischen Reste können bevorzugt 1, 2 oder 3 Heteroatome unabhängig voneinander ausgewählt aus der Gruppe umfassend Sauerstoff, Schwefel und Stickstoff, einschließlich -N(H)- und -N(C₁₋₆Alkyl), als Kettenglieder aufweisen.

Beispielhaft für aliphatische Reste, die 1, 2 oder 3 Heteroatome aufweisen, seien -(CH₂)-(CH₂)-O-CH₃, -(CH₂)-(CH₂)-(CH₂)-O-CH₃, -(CH₂)-(CH₂)-(CH₂)-N(C₂H₅)-(C₂H₅), -(CH₂)-(CH₂)-S-CH₃, -(CH₂)-(CH₂)-(CH₂)-S-CH₃, -(CH₂)-(CH₂)-(CH₂)-N(CH₃)-(CH₃) und -(CH₂)-O-CH₃ genannt.

Im Zusammenhang mit aliphatischen Resten versteht man unter dem Begriff "substituiert" - soweit nicht anders definiert - im Sinne der vorliegenden Erfindung die einfache oder mehrfache Substitution, bevorzugt die ein-, zwei-, drei-, vier-, fünf-, sechs-, sieben-, acht- oder neunfache Substitution, von einem oder mehreren Wasserstoffatomen durch beispielsweise F, Cl, Br, I, -CN, -NO₂, -OH, -SH und -NH₂, wobei die mehrfache Substitution entweder an verschiedenen oder an gleichen Atomen mehrfach, z.B. zwei- oder dreifach, erfolgt, beispielsweise dreifach am gleichen C-Atom wie im Falle von -CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Bevorzugte substituierte aliphatische Reste sind -CH₂-Cl, -CH₂-Br, -CH₂-CH₂-Cl, -CH₂-CH₂-Br, -CH₂-CH₂-CH₂-Br und -CH₂-CH₂-CH₂-Cl.

Cycloaliphatische Reste im Sinne der vorliegenden Erfindung sind zyklische gesättigte oder ungesättigte Kohlenwasserstoff-Reste mit 3, 4, 5, 6, 7, 8, 9 , 10, 11, 12, 13, 14, 15 oder 16, bevorzugt 3, 4, 5, 6, 7 oder 8 Kohlenstoffatomen, wobei jeder Rest unsubstituiert oder einfach oder mehrfach, beispielsweise 1-, 2-, 3-, 4-, 5-, 6-, 7-, 8- oder 9-fach, gleich oder verschieden substituiert sein kann. Cycloaliphatische Reste können bevorzugt 1, 2, 3, 4 oder 5 Heteroatome unabhängig voneinander ausgewählt aus der Gruppe bestehend aus Sauerstoff, Stickstoff (NH) und Schwefel als Ringglieder aufweisen.

Beispielhaft für cycloaliphatische Reste, die ggf. mit 1 oder 2 linearen oder verzweigten C₁₋₅-Alkylen-Gruppen überbrückt und mit einen mono- oder polyzyklischen Ringsystem kondensiert sein können, seien Cyclopropyl, Cyclobutyl, Cyclopentyl, Cyclohexyl, Cyclooctyl, Cyclononyl, Cyclodecyl, Cycloundecyl, Cyclododecyl, Cycloheptyl, Cyclopentenyl, Cyclohexenyl, Cycloheptenyl, [6,6]-Dimethyl-[3.1.1]-bicycloheptyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl, (1,2,3,4)-Tetrahydrochinazolinyl, (1,3,4,5)-Tetrahydropyrido[4,3-b]indolyl, (3,4)-Dihydro-1H-isochinolinyl, (1 ,3,4,9)-Tetrahydro-[b]-carbolinyl, Imidazolidinyl und (1,3)-Thiazolidinyl genannt.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind. Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien [1,3]-Benzodioxolyl, [1,4]-Benzodioxanyl, [1,2,3,4]-Tetrahydronaphthyl, [1,2,3,4]-Tetrahydrochinolinyl, [1,2,3,4]-Tetrahydrochinazolinyl und [3,4]-Dihydro-2H-1,4-benzoxazinyl genannt.

Im Zusammenhang mit cycloaliphatischen Resten und mono- oder polyzyklischen Ringsystemen versteht man unter dem Begriff "substituiert" - soweit nicht anders definiert - im Sinne dieser Erfindung die einfache oder mehrfache Substitution, bevorzugt die ein-, zwei-, drei-, vier-, fünf-, sechs-, sieben-, acht- oder neunfache Substitution, von einem oder mehreren Wasserstoffatomen durch beispielsweise Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann. Die mehrfache Substitution kann entweder an verschiedenen oder an gleichen Atomen mehrfach, z.B. zwei- oder dreifach, erfolgen. Die Mehrfachsubstitution kann mit dem gleichen oder mit verschiedenen Substituenten erfolgen.

Unter dem Ausdruck Aryl-Rest ist für die Zwecke der vorliegenden Erfindung bevorzugt ein Rest zu verstehen, der aus der Gruppe, die Phenyl, Naphthyl, Phenanthrenyl und Anthracenyl umfasst, ausgewählt ist und unsubstituiert oder einfach oder mehrfach gleich oder verschieden substituiert ist. Bevorzugt ist Aryl ein unsubstituiertes oder einfach substituiertes oder mehrfach, d. h. zwei-, drei- vier oder fünffach, gleich oder verschieden substituiertes Phenyl, 1-Naphthyl oder 2-Naphthyl.

Heteroaryl-Reste im Sinne der vorliegenden Erfindung sind solche Heterozyklen, die heteroaromatisch sind. Heteroaryl-Reste sind bevorzugt 5- bis 14-gliedrig, d. h. 5-, 6-, 7-, 8-, 9-, 10-, 11-, 12-, 13- oder 14-gliedrig und weisen bevorzugt 1, 2, 3, 4 oder 5 Heteroatome unabhängig voneinander ausgewählt aus der Gruppe umfassend Sauerstoff, Stickstoff und Schwefel auf. Jeder Heteroaryl-Rest kann unsubstituiert oder einfach substituiert oder mehrfach, d. h. zwei-, drei-, vier- oder fünffach, gleich oder verschieden substituiert vorliegen.

Beispielhaft für Heteroaryl-Reste im Sinne der vorliegenden Erfindung seien Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl, Benzothiazolyl, Benzo[2,1,3]thiadiazolyl, [1,2,3]-Benzothiadiazolyl, [2,1,3]-Benzoxadiazolyl und [1,2,3]-Benzoxadiazolyl genannt.

In Bezug auf Aryl- und Heteroaryl-Reste versteht man im Sinne dieser Erfindung unter "substituiert" die ein- oder mehrfache, z.B. zwei-, drei-, vier- oder fünffache, Substitution eines oder mehrerer Wasserstoffatome des Ringsystems durch geeignete Substituenten. Soweit die Bedeutung dieser geeigneten Substituenten im Zusammenhang mit Aryl- oder Heteroaryl-Resten nicht an anderer Stelle der Beschreibung oder in den Ansprüchen definiert ist, sind geeignete Substituenten F, Cl, Br, I, -CN, -CF₃, -SF₅, -OH, -O-C₁₋₅-Alkyl, -NH₂, -NO₂, -O-CF₃, -S-CF₃, -SH, -S-C₁-₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl, wobei jeweils der zyklische Teil der Reste Pyridinyl, Cyclopentyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.
Die Mehrfachsubstitution erfolgt dabei mit dem gleichen oder mit unterschiedlichen Substituenten.

Die vorstehend genannten linearen oder verzweigten Alkylen-, Alkenylen- oder Alkinylen-Gruppen weisen bevorzugt 1 bis 5 Kohlenstoffatome auf, d.h. es handelt sich um C₁₋₅-Alkylen, C₂₋₅-Alkenylen oder C₂₋₅-Alkinylen-Gruppen, die jeweils unsubstituiert oder mit 1, 2, 3, 4 oder 5 Substituenten unabhängig voneinander ausgewählt aus der Gruppe bestehend aus F, Cl, Br, -OH, -SH, -NH₂, -CN, NO₂ und Phenyl substituiert sein können.

Die vorstehend genannten Alkylen-, Alkenylen- oder Alkinylen-Gruppen weisen ggf. jeweils 1 oder 2 Heteroatom(e) ausgewählt aus der Gruppe bestehend aus Sauerstoff, Stickstoff, d. h. -N(H)- und -N(C₁₋₆-Alkyl)-, und Schwefel als Kettenglied(er) auf.

Bevorzugt können Alkylen-Gruppen ausgewählt werden aus der Gruppe bestehend aus -(CH₂)-, -(CH₂)₂-, -C(H)(CH₃)-, -C(CH₃)₂-, -(CH₂)₃-, -(CH₂)₄-, -(CH₂)₅-, -C(H)(CH₃)-(CH₂)-, -C(H)(C₂H₅)-(CH₂)-, -C(Phenyl)₂-, -C(H)(Phenyl)-, -(CH₂)-O-, -(CH₂)-N(CH₃)-, -(CH₂)-S-, -(CH₂)-(CH₂)-N(CH₃)- und -(CH₂)-(CH₂)-N(C₂H₅)-.

Der Fachmann versteht, dass sich für m gleich 0 die folgende allgemeine Formel Ic ergibt:

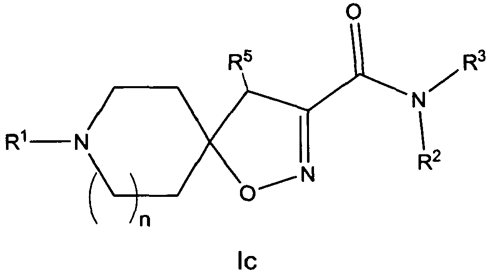

Ebenfalls bevorzugt sind substituierte Spiro-Verbindungen der vorstehend angegeben allgemeinen Formel I, worin
n gleich 1 ist;
und jeweils m und R¹ bis R⁴² die vorstehend genannte Bedeutung haben, jeweils ggf. in Form eines ihrer reinen Stereoisomeren, insbesondere Enantiomeren oder Diastereomeren, ihrer Racemate oder In Form einer Mischung von Stereoisomeren, insbesondere der Enantiomeren und/oder Diastereomeren, in einem beliebigen Mischungsverhältnis, oder jeweils in Form entsprechender Salze, oder jeweils In Form entsprechender Solvate.

Der Fachmann versteht, dass sich für n gleich 1 die folgende allgemeine Formel Id ergibt

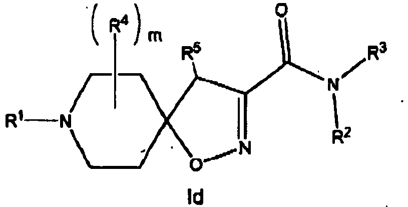

Besonders bevorzugt sind ferner substitulerte Spiro-Verbindungen der vorstehend angegeben allgemeinen Formel I, worin
- m: gleich 0, 1, 2, 3 oder 4 ist;
- n: gleich 1 ist;
- R¹: für eine -C(=S)-N⁸R⁹-Gruppe steht;
oder sofern m ungleich 0 und/oder R⁵ ungleich H ist,
zusätzlich für eine -C(=O)-NR⁶R⁷-Gruppe stehen kann,
- R²: für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl n-Hexyl und n-Heptyl steht;
für einen Rest ausgewählt aus der Gruppe bestehend aus Cyclopropyl, Cyclobutyl. Cyclopentyl, Cyclohexyl, Cycloheptyl. Cyclopentenyl, Cyclohexenyl, Cycloheptenyl, Imidazolidinyl, Tetrahydroturanyl, Tetrahydrothiophenyl, Pyrrolidinyl, Piperidinyl, Morpholinyl, Piperazinyl und Thiomorpholinyl steht, wobei der Rest jeweils mit 1 oder 2 Substituenten unabhängig voneinander ausgewählt aus der Gruppe bestehend aus Pyridinyl, Pyridazinyl, Phenyl und Benzyl substituiert sein kann, wobei jeweils der zyklische Tell der Reste Pyridinyl, Pyridazinyl, Phenyl und Benzyl mit 1, 2, 3, 4 oder 5 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
für einen Rest ausgewählt aus der Gruppe bestehend aus Phenyl, Naphthyl, Thiophenyl, Furanyl, Pyrrolyl, Pyrazolyl, Pyrazinyl und Pyridinyl steht, wobei der Rest ggf. jeweils mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -NH₂, -NO₂, -O-CF₃, -S-CF₃, -SH, -S-CH₃, - S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, - NH-S(=O)₂-CH₃, -NH-S(=O)₂-C₂H₅, -S(=O)₂-NH-CH₃ und -S(=O)₂-NH-C₂H₅ substituiert sein kann;
oder für -(CHR³⁰)-R³³; -(CHR³⁰)-(CHR³¹)-R³³ oder -(CHR³⁰)-(CHR³¹)-(CHR³²)-R³³ steht;
- R³: für einen Wasserstoff-Rest steht;
oder
- R² und R³: zusammen mit dem sie verbindenden Stickstoffatom als Ringglied einen Rest bilden ausgewählt aus der Gruppe bestehend aus Pyrrolidinyl, Piperidinyl, Piperazinyl, 3-Methyl-piperazinyl, 2-Methyl-piperazinyl, (3,5)-Dimethylpiperazinyl, (2,6)-Dimethylpiperazinyl, Morpholinyl und Thiomorpholinyl bilden, wobei der Rest jeweils mit 1 oder 2 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH)-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, Pyridinyl, Pyridazinyl, Phenyl und Benzyl substituiert sein kann, wobei jeweils der zyklische Teil der Reste Pyridinyl, -S(=O)₂-Phenyl, Pyridazinyl, Phenyl und Benzyl mit 1, 2, 3, 4 oder 5 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
- R⁴: für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht,
- R⁵: für einen Wasserstoff-Rest,
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht,
für einen Phenyl-Rest steht, der jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, - S-CH₃, -S-C₂H₅, Methyl, Ethyl, n-Propyl, Isopropyl, n-Butyl, sec-Butyl, Isobutyl und tert-Butyl substituiert sein kann;
oder für -(CH₂)-R³⁴ steht;
- R⁶ und R⁸,: unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen;
für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=OFC₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, - (CR³⁷R³⁸)-(CHR³⁹)-(CHR⁴⁰)-R⁴¹, -(CR³⁷R³⁸)-(CHR³⁹)-(CHR⁴⁰)-O-R⁴¹,-(CR³⁷R³⁸)-(CHR³⁹)-(CHR⁴⁰)-N(CH₃)-R⁴¹ oder -(CR³⁷R³⁸)-(CHR³⁹)-(CHR⁴⁰)-N(C₂H₅)-R⁴¹ stehen;
- R⁷ und R⁹: jeweils für einen Wasserstoff-Rest stehen;
- R²⁵: für einen Rest ausgewählt aus der Gruppe bestehend aus Methyl, Ethyl, n-Propyl, Isopropyl, n-Butyl, sec-Butyl, Isobutyl und tert-Butyl steht;
R²⁶, R²⁷, R²⁸, R³⁰, R³¹, R³², R³⁶, R³⁷ R³⁸, R³⁹ und R⁴⁰, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
- R²⁹ und R³³,: unabhängig voneinander, jeweils
für einen Rest ausgewählt aus der Gruppe bestehend aus Phenyl, Naphthyl, Thiophenyl, Furanyl, Pyrrolyl, Pyrazolyl, Pyrazinyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -NH₂, -NO₂, -O-CF₃, -S-CF₃, -SH, -S-CH₃, - S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=OFO-C₂H₅, -C(=O)-O-C(CH₃)₃, -O- C(=O)-CH₃, -O-C(=O)-C₂H₅, -O-C(=O)-C(CH₃)₃, -S(=O)₂-CH₃, -S(=O)₂-C₂H₅, - NH-S(=O)₂-CH₃, -NH-S(=O)₂-C₂H₅, -S(=O)₂-NH-CH₃ und -S(=O)₂-NH-C₂H₅ substituiert sein kann;
- R³⁴: für einen Phenyl-Rest steht, der mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -NH₂, -NO₂, -O-CF₃, -S-CF₃, -SH, -S-CH₃, - S-C₂H₅, Methyl, Ethyl, n-Propyl, Isopropyl, n-Butyl, sec-Butyl, Isobutyl und tert-Butyl substituiert sein kann;
und
- R⁴¹: für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, - S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅ und -C(=O)-C(CH₃)₃ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhättnis, oder jeweils In Form entsprechender Salze, oder jeweils In Form entsprechender Solvate.

Ganz besonders bevorzugt sind substituierte Spiro-Verbindungen der vorstehend angegeben allgemeinen Formel worin
m gleich 0 ist;
n gleich 1 ist;
- R¹: für eine -C(=S)-NR⁸R⁹-Gruppe steht;
- R²: für einen Rest ausgewählt aus der Gruppe bestehend aus Phenyl, Naphthyl und Pyridinyl steht, wobei der Rest jeweils mit 1, 2 oder 3 Substituenten unabhängig voneinander ausgewählt aus der Gruppe bestehend aus F, Cl, Br, -OH, -O-CH₃, -O-C₂H₅, -NH-S(=O)₂-CH₃ und -NH-S(=O)₂-C₂H₅ substituiert sein kann; für einen Piperazinyl-Rest steht, der an einem Stickstoffatom mit einem Substituenten ausgewählt aus der Gruppe bestehen aus Pyridinyl, Pyridazinyl, Phenyl und Benzyl substituiert sein kann, wobei jeweils der zyklische Teil der Substituenten Pyridinyl, Pyridazinyl, Phenyl und Benzyl mit 1, 2 oder 3 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl und tert-Butyl substituiert sein kann;
oder für -(CHR³⁰)-R³³; -(CHR³⁰)-(CHR³¹)-R³³ oder -(CHR³⁰)-(CHR³¹)-(CHR³²)-R³³ steht;
- R³: für einen Wasserstoff-Rest steht;
oder
- R² und R³: zusammen mit dem verbindenden Stickstoffatom als Ringglied einen Rest ausgewählt aus der Gruppe bestehend aus 3-Methyl-piperazinyl, 2-Methyl-piperazinyl, (3,5)-Dimethylpiperazinyl, (2,6)-Dimethylpiperazinyl und Piperazinyl bilden, der an einem Stickstoffatom mit einem Substituenten ausgewählt aus der Gruppe bestehen aus Pyridinyl, Pyridazinyl, Phenyl und Benzyl substituiert sein kann, wobei jeweils der zyklische Teil der Substituenten Pyridinyl, Pyridazinyl, Phenyl und Benzyl mit 1, 2 oder 3 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl und tert-Butyl substituiert sein kann;
- R⁵: für einen Wasserstoff-Rest,
- R⁶ und R⁸,: unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl und 2-Methyl-1-propenyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl und Adamantyl stehen;
für einen Rest ausgewählt aus der Gruppe bestehend aus Phenyl, Naphthyl und Pyridinyl stehen, wobei der Rest ggf. jeweils mit 1, 2, 3, 4 oder 5 Substituenten unabhängig voneinander ausgewählt aus der Gruppe bestehend aus -SF₅, F, Cl, Br, I, -CN, -CF₃, -O-CH₃, -O-C₂H₅, -NO₂, -O-CF₃, - S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann, wobei jeweils der zyklische Teil der Reste Cyclopentyl, Cyclohexyl, -O-Phenyl, -O-Benzyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, - (CR³⁷R³⁸)-(CHR³⁹)-(CHR⁴⁰)-R⁴¹, -(CR³⁷R³⁸)-(CHR³⁹)-(CHR⁴⁰)-O-R⁴¹, - (CR³⁷R³⁸)-(CHR³⁹)-(CHR⁴⁰)-N(CH₃)-R⁴¹ oder -(CR³⁷R³⁸)(CHR³⁹)-(CHR⁴¹)-N(C₂H₅)-R⁴¹ stehen;
- R⁷ und R⁹: jeweils für einen Wasserstoff-Rest stehen;
- R²⁵: für einen Rest ausgewählt aus der Gruppe bestehend aus Methyl, Ethyl, n-Propyl, Isopropyl, n-Butyl, sec-Butyl, Isobutyl und tert-Butyl steht;
- R³⁰, R³¹, R³², R³⁹ und R⁴⁰,: jeweils für einen Wasserstoff-Rest stehen;
- R³³: für einen Rest ausgewählt aus der Gruppe bestehend aus Phenyl, Naphthyl, Thiophenyl und Furanyl steht, wobei der Rest ggf. jeweils mit 1, 2, 3, 4 oder 5 Substituenten unabhängig voneinander ausgewählt aus der Gruppe bestehend aus -CF₃, F, Cl, Br, -OH, -O-CH₃, -O-C₂H₅, Methyl, Ethyl, n-Propyl, Isopropyl, n-Butyl, sec-Butyl, Isobutyl, tert-Butyl, -NH-S(=O)₂-CH₃ und -NH-S(=O)₂-C₂H₅ substituiert sein kann;
- R³⁶: für einen Wasserstoff-Rest
oder für einen Rest ausgewählt aus der Gruppe bestehend aus Methyl, Ethyl, n-Propyl, Isopropyl, n-Butyl, sec-Butyl, Isobutyl und tert-Butyl steht;
- R³⁷ und R³⁸,: unabhängig voneinander, jeweils für
einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
- R⁴¹: für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Tetrahydrofuranyl, Tetrahydrothiophenyl, Pyrrolidinyl, Morpholinyl, Piperidinyl und Piperazinyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, -OH, -O-CH₃, -O-C₂H₅, Methyl, Ethyl, n-Propyl, Isopropyl, n-Butyl, sec-Butyl, Isobutyl und tert-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der vorliegenden Erfindung sind substituierte Spiro-Verbindungen ausgewählt aus der Gruppe bestehend aus
[1] 3-[4-(3-Chlor-pyridin-2-yl)-piperazin-1-carbonyl]-1-oxa-2,8-diaza-spiro[4.5]dec-2-en-8-carbonsäure-(2-fluor-phenyl)-amid
[2] 1-Oxa-2,8-diaza-spiro[4.5]dec-2-en-3,8-dicarbonsäure-3-[(5-chlor-pyridin-2-yl)-amid]-8-[(2-fluor-phenyl)-amid]
[3] 1-Oxa-2,8-diaza-spiro[4.5]dec-2-en-3,8-dicarbonsäure-3-(4-chlor-benzylamid)-8-[(3-fluor-phenyl)-amid]
[4] 3-[4-(3-Chlor-pyridin-2-yl)-piperazin-1-carbonyl]-1-oxa-2,8-diaza-spiro[4.5]dec-2-en-8-carbonsäure-(3-fluor-phenyl)-amid
[5] 1-Oxa-2,8-diaza-spiro[4.5]dec-2-en-3,8-dicarbonsäure-3-[(5-chlor-pyridin-2-yl)-amid]-8-[(3-fluor-phenyl)-amid]
[6] 1-Oxa-2,8-diaza-spiro[4.5]dec-2-en-3,8-dicarbonsäure 8-[(2-chlor-phenyl)-amid]-3-(3,4-dimethoxy-benzylamid)
[7] 1-Oxa-2,8-diaza-spiro[4.5]dec-2-en-3,8-dicarbonsäure-3-(4-chlor-benzylamid)-8-[(2-chlor-phenyl)-amid]
[8] 3-[4-(3-Chlor-pyridin-2-yl)-piperazin-1-carbonyl]-1-oxa-2,8-diaza-spiro[4.5]dec-2-en-8-carbonsäure-(2-chlor-phenyl)-amid
[9] 1-Oxa-2,8-diaza-spiro[4.5]dec-2-en-3,8-dicarbonsäure 8-[(2-chlor-phenyl)-amid]-3-[(5-chlor-pyridin-2-yl)-amid]
[10] 1-Oxa-2,8-diaza-spiro[4.5]dec-2-en-3,8-dicarbonsäure 8-[(3-bromo-phenyl)-amid]-3-(3,4-dimethoxy-benzylamid)
[11] 3-[4-(3-Chlor-pyridin-2-yl)-piperazin-1-carbonyl]-1-oxa-2,8-diaza-spiro[4.5]dec-2-en-8-carbonsäure-(3-bromo-phenyl)-amid
[12] 1-Oxa-2,8-diaza-spiro[4.5]dec-2-en-3,8-dicarbonsäure 8-[(3-bromo-phenyl)-amid]-3-[(5-chlor-pyridin-2-yl)-amid]
[13] 1-Oxa-2,8-diaza-spiro[4.5]dec-2-en-3,8-dicarbonsäure-3-(3,4-dimethoxy-benzylamid)-8-[(2-methoxy-phenyl)-amid]
[14] 1-Oxa-2,8-diaza-spiro[4.5]dec-2-en-3,8-dicarbonsäure-3-(4-chlor-benzylamid)-8-[(2-methoxy-phenyl)-amid]
[15] 3-[4-(3-Chlor-pyridin-2-yl)-piperazin-1-carbonyl]-1-oxa-2,8-diaza-spiro[4.5]dec-2-en-8-carbonsäure-(2-methoxy-phenyl)-amid
[16] 1-Oxa-2,8-diaza-spiro[4.5]dec-2-en-3,8-dicarbonsäure-3-(3,4-dimethoxy-benzylamid)-8-[(3-methoxy-pheny)-amid]
[17] 3-[4-(3-Chlor-pyridin-2-yl)-piperazin-1-carbonyl]-1-oxa-2,8-diaza-spiro[4.5]dec-2-en-8-carbonsäure-(3-methoxy-phenyl)-amid
[18] 1-Oxa-2,8-diaza-spiro[4.5]dec-2-en-3,8-dicarbonsäure-3-[(5-chlor-pyridin-2-yl)-amid]-8-[(3-methoxy-phenyl)-amid]
[19] 3-[4-(3-Chlor-pyhdin-2-yl)-piperazin-1-carbonyl]-1-oxa-2,8-diaza-spiro[4.5]dec-2-en-8-carbonsäure-(4-phenoxy-phenyl)-amid
[20] 1-Oxa-2,8-diaza-spiro[4.5]dec-2-en-3,8-dicarbonsäure 8-[(2-chlor-5-trifluormethyl-phenyl)-amid]-3-(3,4-dimethoxy-benzylamid)
[21] 1-Oxa-2,8-diaza-spiro[4.5]dec-2-en-3,8-dicarbonsäure-3-(4-chlor-benzylamid)-8-[(2-chlor-5-trifluormethyl-phenyl)-amid]
[22] 3-[4-(3-Chlor-pyridin-2-yl)-piperazin-1-carbonyl]-1-oxa-2,8-diaza-spiro[4.5]dec-2-en-8-carbonsäure-(2-chlor-5-trifluormethyl-phenyl)-amid
[23] 1-Oxa-2,8-diaza-spiro[4.5]dec-2-en-3,8-dicarbonsäure-3-[(5-chlor-pyridin-2-yl)-amid]-8-[(2-chlor-5-trifluormethyl-phenyl)-amid]
[24] 1-Oxa-2,8-diaza-spiro[4.5]dec-2-en-3,8-dicarbonsäure 8-[(4-chlor-2-trifluormethyl-phenyl)-amid]-3-(3,4-dimethoxy-benzylamid)
[25] 3-[4-(3-Chlor-pyridin-2-yl)-piperazin-1-carbonyl]-1-oxa-2,8-diaza-spiro[4.5]dec-2-en-8-carbonsäure-(4-chlor-2-trifluormethyl-phenyl)-amid
[26] 1-Oxa-2,8-diaza-spiro[4.5]dec-2-en-3,8-dicarbonsäure-3-[(5-chlor-pyridin-2-yl)-amid]-8-[(4-chlor-2-trifluormethyl-phenyl)-amid]
[27] 1-Oxa-2,8-diaza-spiro[4.5]dec-2-en-3,8-dicarbonsäure 8-[(4-chlor-3-trifluormethyl-phenyl)-amid]-3-(3,4-dimethoxy-benzylamid)
[28] 3-[4-(3-Chlor-pyridin-2-yl)-piperazin-1-carbonyl]-1-oxa-2,8-diaza-spiro[4.5]dec-2-en-8-carbonsäure-(4-chlor-3-trifluormethyl-phenyl)-amid
[29] 1-Oxa-2,8-diaza-spiro[4.5]dec-2-en-3,8-dicarbonsäure-3-[(5-chlor-pyridin-2-yl)-amid]-8-[(4-chlor-3-trifluormethyl-phenyl)-amid]
[30] 1-Oxa-2,8-diaza-spiro[4.5]dec-2-en-3,8-dicarbonsäure-3-[(2-tert-butyl-6-methyl-phenyl)-amid]-8-(4-chlor-benzylamid)
[31] 3-[4-(3-Chlor-pyridin-2-yl)-piperazin-1-carbonyl]-1-oxa-2,8-diaza-spiro[4.5]dec-2-en-8-carbonsäure-(2-tert-butyl-6-methyl-phenyl)-amid
[32] 1-Oxa-2,8-diaza-spiro[4.5]dec-2-en-3,8-dicarbonsäure-3-(3,4-dimethoxy-benzylamid)-8-[(4-trifluormethoxy-phenyl)-amid]
[33] 1-Oxa-2,8-diaza-spiro[4.5]dec-2-en-3,8-dicarbonsäure-3-(4-chlor-benzylamid)-8-[(4-trifluormethoxy-phenyl)-amid]
[34] 3-[4-(3-Chlor-pyridin-2-yl)-piperazin-1-carbonyl]-1-oxa-2,8-diaza-spiro[4.5]dec-2-en-8-carbonsäure-(4-trifluormethoxy-phenyl)-amid
[35] 1-Oxa-2,8-diaza-spiro[4.5]dec-2-en-3,8-dicarbonsäure-3-[(5-chlor-pyridin-2-yl)-amid]-8-[(4-trifluormethoxy-phenyl)-amid]
[36] 1-Oxa-2,8-diaza-spiro[4.5]dec-2-en-3,8-dicarbonsäure-3-(3,4-dimethoxy-benzylamid)-8-(phenethyl-amid)
[37] 1-Oxa-2,8-diaza-spiro[4.5]dec-2-en-3,8-dicarbonsäure-3-(4-chlor-benzylamid)-8-(phenethyl-amid)
[38] 3-[4-(3-Chlor-pyridin-2-yl)-piperazin-1-carbonyl]-1-oxa-2,8-diaza-spiro[4.5]dec-2-en-8-carbonsäurephenethyl-amid
[39] 1-Oxa-2,8-diaza-spiro[4.5]dec-2-en-3,8-dicarbonsäure-3-(3,4-dimethoxy-benzylamid)-8-phenylamid
[40] 3-[4-(3-Chlor-pyridin-2-yl)-piperazin-1-carbonyl]-1-oxa-2,8-diaza-spiro[4.5]dec-2-en-8-carbonsäurephenylamid
[41] 1-Oxa-2,8-diaza-spiro[4.5]dec-2-en-3,8-dicarbonsäure-3-[(5-chlor-pyridin-2-yl)-amid]-8-phenylamid
[42] 1-Oxa-2,8-diaza-spiro[4.5]dec-2-en-3,8-dicarbonsäure-3-(4-chlor-benzylamid)-8-m-tolylamid
[43] 3-[4-(3-Chlor-pyridin-2-yl)-piperazin-1-carbonyl]-1-oxa-2,8-diaza-spiro[4.5]dec-2-en-8-carbonsäure-m-tolylamid
[44] 1-Oxa-2,8-diaza-spiro[4.5]dec-2-en-3,8-dicarbonsäure-3-(3,4-dimethoxy-benzylamid)-8-[(4-fluor-phenyl)-amid]
[45] 3-[4-(3-Chlor-pyridin-2-yl)-piperazin-1-carbonyl]-1-oxa-2,8-diaza-spiro[4.5]dec-2-en-8-carbonsäure-(4-fluor-phenyl)-amid
[46] 1-Oxa-2,8-diaza-spiro[4.5]dec-2-en-3,8-dicarbonsäure 8-[(3-chlor-phenyl)-amid]-3-(3,4-dimethoxy-benzylamid)
[47] 3-[4-(3-Chlor-pyridin-2-yl)-piperazin-1-carbonyl]-1-oxa-2,8-diaza-spiro[4.5]dec-2-en-8-carbonsäure-(3-chlor-phenyl)-amid
[48] 1-Oxa-2,8-diaza-spiro[4.5]dec-2-en-3,8-dicarbonsäure 8-[(3-chlor-phenyl)-amid]-3-[(5-chlor-pyridin-2-yl)-amid]
[49] 1-Oxa-2,8-diaza-spiro[4.5]dec-2-en-3,8-dicarbonsäure 8-[(4-chlor-phenyl)-amid]-3-(3,4-dimethoxy-benzylamid)
[50] 1-Oxa-2,8-diaza-spiro[4.5]dec-2-en-3,8-dicarbonsäure-3-(4-chlor-benzylamid)-8-[(4-chlor-phenyl)-amid]
[51] 3-[4-(3-Chlor-pyridin-2-yl)-piperazin-1-carbonyl]-1-oxa-2,8-diaza-spiro[4.5]dec-2-en-8-carbonsäure-(4-chlor-phenyl)-amid
[52] 3-[4-(3-Chlor-pyridin-2-yl)-piperazin-1-carbonyl]-1-oxa-2,8-diaza-spiro[4.5]dec-2-en-8-carbonsäure-(4-methoxy-phenyl)-amid
[53] 1-Oxa-2,8-diaza-spiro[4.5]dec-2-en-3,8-dicarbonsäure-3-(3,4-dimethoxy-benzylamid)-8-[(2-methylsulfanyl-phenyl)-amid]
[54] 3-[4-(3-Chlor-pyridin-2-yl)-piperazin-1-carbonyl]-1-oxa-2,8-diaza-spiro[4.5]dec-2-en-8-carbonsäure-(2-methylsulfanyl-phenyl)-amid
[55] 1-Oxa-2,8-diaza-spiro[4.5]dec-2-en-3,8-dicarbonsäure-3-(3,4-dimethoxy-benzylamid)-8-[(3-methylsulfanyl-phenyl)-amid]
[56] 3-[4-(3-Chlor-pyridin-2-yl)-piperazin-1-carbonyl]-1-oxa-2,8-diaza-spiro[4.5]dec-2-en-8-carbonsäure-(3-methylsulfanyl-phenyl)-amid
[57] 1-Oxa-2,8-diaza-spiro[4.5]dec-2-en-3,8-dicarbonsäure-3-(3,4-dimethoxy-benzylamid)-8-[(4-methylsulfanyl-phenyl)-amid]
[58] 3-[4-(3-Chlor-pyridin-2-yl)-piperazin-1-carbonyl]-1-oxa-2,8-diaza-spiro[4.5]dec-2-en-8-carbonsäure-(4-methylsulfanyl-phenyl)-amid
[59] 1-Oxa-2,8-diaza-spiro[4.5]dec-2-en-3,8-dicarbonsäure-3-[(5-chlor-pyridin-2-yl)-amid]-8-[(4-methylsulfanyl-phenyl)-amid]
[60] 1-Oxa-2,8-diaza-spiro[4.5]dec-2-en-3,8-dicarbonsäure-3-(3,4-dimethoxy-benzylamid)-8-[(2-isopropyl-phenyl)-amid]
[61] 3-[4-(3-Chlor-pyridin-2-yl)-piperazin-1-carbonyl]-1-oxa-2,8-diaza-spiro[4.5]dec-2-en-8-carbonsäure-(2-isopropyl-phenyl)-amid
[62] 1-Oxa-2,8-diaza-spiro[4.5]dec-2-en-3,8-dicarbonsäure-3-[(5-chlor-pyridin-2-yl)-amid]-8-[(2-isopropyl-phenyl)-amid]
[63] 3-[4-(3-Chlor-pyridin-2-yl)-piperazin-1-carbonyl]-1-oxa-2,8-diaza-spiro[4.5]dec-2-en-8-carbonsäure-(4-isopropyl-phenyl)-amid
[64] 1-Oxa-2,8-diaza-spiro[4.5]dec-2-en-3,8-dicarbonsäure-3-(3,4-dimethoxy-benzylamid)-8-[(2-trifluormethyl-phenyl)-amid]
[65] 3-[4-(3-Chlor-pyridin-2-yl)-piperazin-1-carbonyl]-1-oxa-2,8-diaza-spiro[4.5]dec-2-en-8-carbonsäure-(2-trifluormethyl-phenyl)-amid
[66] 1-Oxa-2,8-diaza-spiro[4.5]dec-2-en-3,8-dicarbonsäure-3-[(5-chlor-pyridin-2-yl)-amid]-8-[(2-trifluormethyl-phenyl)-amid]
[67] 1-Oxa-2,8-diaza-spiro[4.5]dec-2-en-3,8-dicarbonsäure-3-(3,4-dimethoxy-benzylamid)-8-[(3-trifluormethyl-phenyl)-amid]
[68] 3-[4-(3-Chlor-pyndin-2-yl)-piperazin-1-carbonyl]-1-oxa-2,8-diaza-spiro[4.5]dec-2-en-8-carbonsäure-(3-trifluormethyl-phenyl)-amid
[69] 1-Oxa-2,8-diaza-spiro[4.5]dec-2-en-3,8-dicarbonsäure-3-[(5-chlor-pyridin-2-yl)-amid]-8-[(3-trifluormethyl-phenyl)-amid]
[70] 1-Oxa-2,8-diaza-spiro[4.5]dec-2-en-3,8-dicarbonsäure-3-(3,4-dimethoxy-benzylamid)-8-[(4-trifluormethyl-phenyl)-amid]
[71] 3-[4-(3-Chlor-pyridin-2-yl)-piperazin-1-carbonyl]-1-oxa-2,8-diaza-spiro[4.5]dec-2-en-8-carbonsäure-(4-trifluormethyl-phenyl)-amid
[72] 1-Oxa-2,8-diaza-spiro[4.5]dec-2-en-3,8-dicarbonsäure-8-cyclohexylamid-3-(3,4-dimethoxy-benzylamid)
[73] 1-Oxa-2,8-diaza-spiro[4.5]dec-2-en-3,8-dicarbonsäure-3-(4-chlor-benzylamid)-8-cyclohexylamid
[74] 1-Oxa-2,8-diaza-spiro[4.5]dec-2-en-3,8-dicarbonsäure-8-benzylamid-3-(3,4-dimethoxy-benzylamid)
[75] 1-Oxa-2,8-diaza-spiro[4.5]dec-2-en-3,8-dicarbonsäure-3-(3,4-dimethoxy-benzylamid)-8-o-tolylamid
[76] 1-Oxa-2,8-diaza-spiro[4.5]dec-2-en-3,8-dicarbonsäure-3-(4-chlor-benzylamid)-8-o-tolylamid
[77] 1-Oxa-2,8-diaza-spiro[4.5]dec-2-en-3,8-dicarbonsäure-3-[(5-chlor-pyridin-2-yl)-amid]-8-o-tolylamid
[78] 1-Oxa-2,8-diaza-spiro[4.5]dec-2-en-3,8-dicarbonsäure-3-(3,4-dimethoxy-benzylamid)-8-[(2-ethyl-phenyl)-amid]
[79] 3-[4-(3-Chlor-pyridin-2-yl)-piperazin-1-carbonyl]-1-oxa-2,8-diaza-spiro[4.5]dec-2-en-8-carbonsäure-(2-ethyl-phenyl)-amid
[80] 1-Oxa-2,8-diaza-spiro[4.5]dec-2-en-3,8-dicarbonsäure-3-[(5-chlor-pyridin-2-yl)-amid]-8-[(2-ethyl-phenyl)-amid]
[81] 1-Oxa-2,8-diaza-spiro[4.5]dec-2-en-3,8-dicarbonsäure-3-(4-chlor-benzylamid)-8-[(4-ethyl-phenyl)-amid]
[82] 1-Oxa-2,8-diaza-spiro[4.5]dec-2-en-3,8-dicarbonsäure-3-[(5-chlor-pyridin-2-yl)-amid]-8-[(4-ethyl-phenyl)-amid]
[83] 1-Oxa-2,8-diaza-spiro[4.5]dec-2-en-3,8-dicarbonsäure-3-(3,4-dimethoxy-benzylamid)-8-[(2-fluor-phenyl)-amid]
[84] 1-Oxa-2,8-diaza-spiro[4.5]dec-2-en-3,8-dicarbonsäure-3-(3,4-dimethoxy-benzylamid)-8-p-tolylamid
[85] 1-Oxa-2,8-diaza-spiro[4.5]dec-2-en-3,8-dicarbonsäure-3-(4-chlor-benzylamid)-8-p-tolylamid
[86] 3-[4-(3-Chlor-pyridin-2-yl)-piperazin-1-carbonyl]-1-oxa-2,8-diaza-spiro[4.5]dec-2-en-8-carbonsäure-p-tolylamid
[87] 1-Oxa-2,8-diaza-spiro[4.5]dec-2-en-3,8-dicarbonsäure-3-[(5-chlor-pyridin-2-yl)-amid]-8-p-tolylamid
[88] 1-Oxa-2,8-diaza-spiro[4.5]dec-2-en-3,8-dicarbonsäure-3-(4-chlor-benzylamid)-8-[(3-ethyl-phenyl)-amid]
[89] 3-[4-(3-Chlor-pyridin-2-yl)-piperazin-1-carbonyl]-1-oxa-2,8-diaza-spiro[4.5]dec-2-en-8-carbonsäure-(3-ethyl-phenyl)-amid
[90] 1-Oxa-2,8-diaza-spiro[4.5]dec-2-en-3,8-dicarbonsäure-3-[(5-chlor-pyridin-2-yl)-amid]-8-[(3-ethyl-phenyl)-amid]
[91] 1-Oxa-2,8-diaza-spiro[4.5]dec-2-en-3,8-dicarbonsäure-3-(3,4-dimethoxy-benzylamid)-8-[(2-propyl-phenyl)-amid]
[92] 1-Oxa-2,8-diaza-spiro[4.5]dec-2-en-3,8-dicarbonsäure-3-(4-chlor-benzylamid)-8-[(2-propyl-phenyl)-amid]
[93] 1-Oxa-2,8-diaza-spiro[4.5]dec-2-en-3,8-dicarbonsäure-3-(4-chlor-benzylamid)-8-[(2-propyl-phenyl)-amid]
[94] 3-[4-(3-Chlor-pyridin-2-yl)-piperazin-1-carbonyl]-1-oxa-2,8-diaza-spiro[4.5]dec-2-en-8-carbonsäure-(2-propyl-phenyl)-amid
[95] 1-Oxa-2,8-diaza-spiro[4.5]dec-2-en-3,8-dicarbonsäure-3-[(5-chlor-pyridin-2-yl)-amid]-8-[(2-propyl-phenyl)-amid]
[96] 1-Oxa-2,8-diaza-spiro[4.5]dec-2-en-3,8-dicarbonsäure 8-[(2-bromo-phenyl)-amid]-3-(3,4-dimethoxy-benzylamid)
[97] 1-Oxa-2,8-diaza-spiro[4.5]dec-2-en-3,8-dicarbonsäure 8-[(2-bromo-phenyl)-amid]-3-(4-chlor-benzylamid)
[98] 3-[4-(3-Chlor-pyridin-2-yl)-piperazin-1-carbonyl]-1-oxa-2,8-diaza-spiro[4.5]dec-2-en-8-carbonsäure-(2-bromo-phenyl)-amid
[99] 3-[4-(3-Chlor-pyridin-2-yl)-piperazin-1-carbonyl]-1-oxa-2,8-diaza-spiro[4.5]dec-2-en-8-carbonsäure-(4-bromo-phenyl)-amid
[100] 3-[4-(3-Chlor-pyridin-2-yl)-piperazin-1-carbonyl]-1-oxa-2,8-diaza-spiro[4.5]dec-2-en-8-carbonsäure-biphenyl-4-ylamid
[101] 1-Oxa-2,8-diaza-spiro[4.5]dec-2-en-3,8-dicarbonsäure-3-(3,4-dimethoxy-benzylamid)-8-[(2-phenoxy-phenyl)-amid]
[102] 3-[4-(3-Chlor-pyridin-2-yl)-piperazin-1-carbonyl]-1-oxa-2,8-diaza-spiro[4.5]dec-2-en-8-carbonsäure-(2-phenoxy-phenyl)-amid
[103] 1-Oxa-2,8-diaza-spiro[4.5]dec-2-en-3,8-dicarbonsäure-3-[(5-chlor-pyridin-2-yl)-amid]-8-[(2-phenoxy-phenyl)-amid]
[104] 1-Oxa-2,8-diaza-spiro[4.5]dec-2-en-3,8-dicarbonsäure-3-(4-chlor-benzylamid)-8-[(2-trifluormethoxy-phenyl)-amid]
[105] 3-[4-(3-Chlor-pyridin-2-yl)-piperazin-1-carbonyl]-1-oxa-2,8-diaza-spiro[4.5]dec-2-en-8-carbonsäure-(2-trifluormethoxy-phenyl)-amid
[106] 1-Oxa-2,8-diaza-spiro[4.5]dec-2-en-3,8-dicarbonsäure-3-[(5-chlor-pyridin-2-yl)-amid]-8-[(2-trifluormethoxy-phenyl)-amid]
[107] 1-Oxa-2,8-diaza-spiro[4.5]dec-2-en-3,8-dicarbonsäure-3-(3,4-dimethoxy-benzylamid)-8-(4-methyl-benzylamid)
[108] 1-Oxa-2,8-diaza-spiro[4.5]dec-2-en-3,8-dicarbonsäure-3-(4-chlor-benzylamid)-8-(4-methyl-benzylamid)
[109] 3-[4-(3-Chlor-pyridin-2-yl)-piperazin-1-carbonyl]-1-oxa-2,8-diaza-spiro[4.5]dec-2-en-8-carbonsäure-4-methyl-benzylamid
[110] 1-Oxa-2,8-diaza-spiro[4.5]dec-2-en-3,8-dicarbonsäure-3-[(5-chlor-pyridin-2-yl)-amid]-8-(4-methyl-benzylamid)
[111] 1-Oxa-2,8-diaza-spiro[4.5]dec-2-en-3,8-dicarbonsäure-3-(3,4-dimethoxy-benzylamid)-8-(4-methoxy-benzylamid)
[112] 1-Oxa-2,8-diaza-spiro[4.5]dec-2-en-3,8-dicarbonsäure-3-(4-chlor-benzylamid)-8-(4-methoxy-benzylamid)
[113] 3-[4-(3-Chlor-pyridin-2-yl)-piperazin-1-carbonyl]-1-oxa-2,8-diaza-spiro[4.5]dec-2-en-8-carbonsäure-4-methoxy-benzylamid
[114] 1-Oxa-2,8-diaza-spiro[4.5]dec-2-en-3,8-dicarbonsäure 8-[(4-tert-butyl-phenyl)-amid]-3-(3,4-dimethoxy-benzylamid)
[115] 1-Oxa-2,8-diaza-spiro[4.5]dec-2-en-3,8-dicarbonsäure 8-[(4-tert-butyl-phenyl)-amid]-3-(4-chlor-benzylamid)
[116] 3-[4-(3-Chlor-pyridin-2-yl)-piperazin-1-carbonyl]-1-oxa-2,8-diaza-spiro[4.5]dec-2-en-8-carbonsäure-(4-tert-butyl-phenyl)-amid
[117] 1-Oxa-2,8-diaza-spiro[4.5]dec-2-en-3,8-dicarbonsäure-8-[(4-tert-butyl-phenyl)-amid]-3-[(5-chlor-pyridin-2-yl)-amid]
[118] 8-(2-Methoxy-phenylthiocarbamoyl)-1-oxa-2,8-diaza-spiro[4.5]dec-2-en-3-carbonsäure-3,4-dimethoxy-benzylamid
[119] 3-[4-(3-Chlor-pyridin-2-yl)-piperazin-1-carbonyl]-1-oxa-2,8-diaza-spiro[4.5]dec-2-en-8-carbonthiosäure-(2-methoxy-phenyl)-amid
[120] 8-(Cyclohexylmethyl-thiocarbamoyl)-1-oxa-2,8-diaza-spiro[4.5]dec-2-en-3-carbonsäure-3,4-dimethoxy-benzylamid
[121] 8-(Cyclohexylmethyl-thiocarbamoyl)-1-oxa-2,8-diaza-spiro[4.5]dec-2-en-3-carbonsäure-4-chlor-benzylamid
[122] 3-[4-(3-Chlor-pyridin-2-yl)-piperazin-1-carbonyl]-1-oxa-2,8-diaza-spiro[4.5]dec-2-en-8-carbonthiosäure cyclohexylmethyl-amid
[123] 8-Cyclooctylthiocarbamoyl-1-oxa-2,8-diaza-spiro[4.5]dec-2-en-3-carbonsäure-3,4-dimethoxy-benzylamid
[124] 8-Cyclooctylthiocarbamoyl-1-oxa-2,8-diaza-spiro[4.5]dec-2-en-3-carbonsäure-4-chlor-benzylamid
[125] 8-(3-Morpholin-4-yl-propylthiocarbamoyl)-1-oxa-2,8-diaza-spiro[4.5]dec-2-en-3-carbonsäure-4-chlor-benzylamid
[126] 8-(3-Morpholin-4-yl-propylthiocarbamoyl)-1-oxa-2,8-diaza-spiro[4.5]dec-2-en-3-carbonsäure-(5-chlor-pyridin-2-yl)-amid
[127] 8-p-Tolylthiocarbamoyl-1-oxa-2,8-diaza-spiro[4.5]dec-2-en-3-carbonsäure-4-chlor-benzylamid
[128] 8-Phenethylthiocarbamoyl-1-oxa-2,8-diaza-spiro[4.5]dec-2-en-3-carbonsäure-3,4-dimethoxy-benzylamid
[129] 8-Phenethylthiocarbamoyl-1-oxa-2,8-diaza-spiro[4.5]dec-2-en-3-carbonsäure-4-chlor-benzylamid
[130] 8-(3-Phenyl-propylthiocarbamoyl)-1-oxa-2,8-diaza-spiro[4.5]dec-2-en-3-carbonsäure-(5-chlor-pyridin-2-yl)-amid
[131] 8-(2-fluor-phenylthiocarbamoyl)-1-oxa-2,8-diaza-spiro[4.5]dec-2-en-3-carbonsäure-3,4-dimethoxy-benzylamid
[132] 8-(4-fluor-phenylthiocarbamoyl)-1-oxa-2,8-diaza-spiro[4.5]dec-2-en-3-carbonsäure-3,4-dimethoxy-benzylamid
[133] 8-(4-fluor-phenylthiocarbamoyl)-1-oxa-2,8-diaza-spiro[4.5]dec-2-en-3-carbonsäure-4-chlor-benzylamid
[134] 8-(2-chlor-phenylthiocarbamoyl)-1-oxa-2,8-diaza-spiro[4.5]dec-2-en-3-carbonsäure-3,4-dimethoxy-benzylamid
[135] 8-(2-chlor-phenylthiocarbamoyl)-1-oxa-2,8-diaza-spiro[4.5]dec-2-en-3-carbonsäure-4-chlor-benzylamid
[136] 8-(3-Trifluormethyl-phenylthiocarbamoyl)-1-oxa-2,8-diaza-spiro[4.5]dec-2-en-3-carbonsäure-4-chlor-benzylamid
[137] 8-(Naphthalen-1-ylthiocarbamoyl)-1-oxa-2,8-diaza-spiro[4.5]dec-2-en-3-carbonsäure-3,4-dimethoxy-benzylamid
[138] 8-(Naphthalen-1-ylthiocarbamoyl)-1-oxa-2,8-diaza-spiro[4.5]dec-2-en-3-carbonsäure-3,4-dimethoxy-benzylamid
[139] 8-Cyclopentylthiocarbamoyl-1-oxa-2,8-diaza-spiro[4.5]dec-2-en-3-carbonsäure-3,4-dimethoxy-benzylamid
[140] 3-[4-(3-Chlor-pyridin-2-yl)-piperazin-1-carbonyl]-1-oxa-2,8-diaza-spiro[4.5]dec-2-en-8-carbonthiosäure-cyclopentylamid
[141] 8-(2-Morpholin-4-yl-ethylthiocarbamoyl)-1-oxa-2,8-diaza-spiro[4.5]dec-2-en-3-carbonsäure-3,4-dimethoxy-benzylamid
[142] 8-(2-Morpholin-4-yl-ethylthiocarbamoyl)-1-oxa-2,8-diaza-spiro[4.5]dec-2-en-3-carbonsäure-4-chlor-benzylamid
[143] 8-(3-chlor-phenylthiocarbamoyl)-1-oxa-2,8-diaza-spiro[4.5]dec-2-en-3-carbonsäure-3,4-dimethoxy-benzylamid
[144] 3-[4-(3-Chlor-pyridin-2-yl)-piperazin-1-carbonyl]-1-oxa-2,8-diaza-spiro[4.5]dec-2-en-8-carbonthiosäure-(4-trifluormethyl-phenyl)-amid
[145] 8-(2-Methylsulfanyl-phenylthiocarbamoyl)-1-oxa-2,8-diaza-spiro[4.5]dec-2-en-3-carbonsäure-3,4-dimethoxy-benzylamid
[146] 8-(2-Methylsulfanyl-phenylthiocarbamoyl)-1-oxa-2,8-diaza-spiro[4.5]dec-2-en-3-carbonsäure-4-chlor-benzylamid
[147] 3-[4-(3-Chlor-pyridin-2-yl)-piperazin-1-carbonyl]-1-oxa-2,8-diaza-spiro[4.5]dec-2-en-8-carbonthiosäure (2-methylsulfanyl-phenyl)-amid
[148] 8-(4-Isopropyl-phenylthiocarbamoyl)-1-oxa-2,8-diaza-spiro[4.5]dec-2-en-3-carbonsäure-3,4-dimethoxy-benzylamid
[149] 8-(4-Isopropyl-phenylthiocarbamoyl)-1-oxa-2,8-diaza-spiro[4.5]dec-2-en-3-carbonsäure-4-chlor-benzylamid
[150] 8-(2-lodo-phenylthiocarbamoyl)-1-oxa-2,8-diaza-spiro[4.5]dec-2-en-3-carbonsäure-3,4-dimethoxy-benzylamid
[151] 8-(2-lodo-phenylthiocarbamoyl)-1-oxa-2,8-diaza-spiro[4.5]dec-2-en-3-carbonsäure-4-chlor-benzylamid
[152] 8-(2-Trifluormethyl-phenylthiocarbamoyl)-1-oxa-2,-diaza-spiro[4.5]dec-2-en-3-carbonsäure-3,4-dimethoxy-benzylamid
[153] 8-(2-Trifluormethyl-phenylthiocarbamoyl)-1-oxa-2,8-diaza-spiro[4.5]dec-2-en-3-carbonsäure-4-chlor-benzylamid
[154] 3-[4-(3-Chlor-pyridin-2-yl)-piperazin-1-carbonyl]-1-oxa-2,8-diaza-spiro[4.5]dec-2-en-8-carbonthiosäure-(2-trifluormethyl-phenyl)-amid
[155] 8-[(Benzo[1,3]dioxol-5-ylmethyl)-thiocarbamoyl]-1-oxa-2,8-diaza-spiro[4.5]dec-2-en-3-carbonsäure-3,4-dimethoxy-benzylamid
[156] 8-[(Benzo[1,3]dioxol-5-ylmethyl)-thiocarbamoyl]-1-oxa-2,8-diaza-spiro[4.5]dec-2-en-3-carbonsäure-4-chlor-benzylamid
[157] 3-[4-(3-Chlor-pyridin-2-yl)-piperazin-1-carbonyl]-1-oxa-2,8-diaza-spiro[4.5]dec-2-en-8-carbonthiosäure-(benzo[1,3]dioxol-5-ylmethyl)-amid
[158] 3-[4-(3-Chlor-pyridin-2-yl)-piperazin-1-carbonyl]-1-oxa-2,8-diaza-spiro[4.5]dec-2-en-8-carbonsäure-(3-cyano-phenyl)-amid
[159] 1-Oxa-2,8-diaza-spiro[4.5]dec-2-en-3,8-dicarbonsäure-3-(3,4-dimethoxy-benzylamid)-8-[(2,5-dimethoxy-phenyl)-amid]
[160] 1-Oxa-2,8-diaza-spiro[4.5]dec-2-en-3,8-dicarbonsäure-3-(4-chlor-benzylamid)-8-[(2,5-dimethoxy-phenyl)-amid]
[161] 3-[4-(3-Chlor-pyridin-2-yl)-piperazin-1-carbonyl]-1-oxa-2,8-diaza-spiro[4.5]dec-2-en-8-carbonsäure-(2,5-dimethoxy-phenyl)-amid
[162] 1-Oxa-2,8-diaza-spiro[4.5]dec-2-en-3,8-dicarbonsäure-3-(3,4-dimethoxy-benzylamid)-8-[(2,4-dimethyl-phenyl)-amid]
[163] 1-Oxa-2,8-diaza-spiro[4.5]dec-2-en-3,8-dicarbonsäure-3-(4-chlor-benzylamid)-8-[(2,4-dimethyl-phenyl)-amid]
[164] 3-[4-(3-Chlor-pyridin-2-yl)-piperazin-1-carbonyl]-1-oxa-2,8-diaza-spiro[4.5]dec-2-en-8-carbonsäure-(2,4-dimethyl-phenyl)-amid
[165] 1-Oxa-2,8-diaza-spiro[4.5]dec-2-en-3,8-dicarbonsäure-8-butylamid 3-(3,4-dimethoxy-benzylamid)
[166] 1-Oxa-2,8-diaza-spiro[4.5]dec-2-en-3,8-dicarbonsäure-8-butylamid 3-(4-chlorbenzylamid)
[167] 3-[4-(3-Chlor-pyridin-2-yl)-piperazin-1-carbonyl]-1-oxa-2,8-diaza-spiro[4.5]dec-2-en-8-carbonsäurebutylamid
[168] 1-Oxa-2,8-diaza-spiro[4.5]dec-2-en-3,8-dicarbonsäure-3-(3,4-dimethoxy-benzylamid)-8-pentylamid
[169] 1-Oxa-2,8-diaza-spiro[4.5]dec-2-en-3,8-dicarbonsäure-3-(4-chlor-benzylamid)-8-pentylamid
[170] 3-[4-(3-Chlor-pyridin-2-yl)-piperazin-1-carbonyl]-1-oxa-2,8-diaza-spiro[4.5]dec-2-en-8-carbonsäurepentylamid
[171] 1-Oxa-2,8-diaza-spiro[4.5]dec-2-en-3,8-dicarbonsäure-3-(3,4-dimethoxy-benzylamid)-8-[(2-ethoxy-phenyl)-amid]
[172] 1-Oxa-2,8-diaza-spiro[4.5]dec-2-en-3,8-dicarbonsäure-3-(4-chlor-benzylamid)-8-[(2-ethoxy-phenyl)-amid]
[173] 1-Oxa-2,8-diaza-spiro[4.5]dec-2-en-3,8-dicarbonsäure-3-(3,4-dimethoxy-benzylamid)-8-[(4-ethoxy-phenyl)-amid]
[174] 1-Oxa-2,8-diaza-spiro[4.5]dec-2-en-3,8-dicarbonsäure 8-[(2,4-difluor-phenyl)-amid]-3-(3,4-dimethoxy-benzylamid)
[175] 1-Oxa-2,8-diaza-spiro[4.5]dec-2-en-3,8-dicarbonsäure-3-(4-chlor-benzylamid)-8-[(2,4-difluor-phenyl)-amid]
[176] 1-Oxa-2,8-diaza-spiro[4.5]dec-2-en-3,8-dicarbonsäure 8-[(3-chlor-2-methyl-phenyl)-amid]-3-(3,4-dimethoxy-benzylamid)
[177] 1-Oxa-2,8-diaza-spiro[4.5]dec-2-en-3,8-dicarbonsäure-3-(4-chlor-benzylamid)8-[(3-chlor-2-methyl-phenyl)-amid]
[178] 1-Oxa-2,8-diaza-spiro[4.5]dec-2-en-3,8-dicarbonsäure 8-[(4-chlor-2-methylphenyl)-amid]-3-(3,4-dimethoxy-benzylamid)
[179] 1-Oxa-2,8-diaza-spiro[4.5]dec-2-en-3,8-dicarbonsäure-3-(4-chlor-benzylamid)-8-[(4-chlor-2-methyl-phenylfamid]
[180] 1-Oxa-2,8-diaza-spiro[4.5]dec-2-en-3,8-dicarbonsäure 8-[(3-chlor-4-methylphenyl)-amid]-3-(3,4-dimethoxy-benzylamid)
[181] 1-Oxa-2,8-diaza-spiro[4.5]dec-2-en-3,8-dicarbonsäure-3-(4-chlor-benzylamid)-8-[(3-chlor-4-methyl-phenyl)-amid]
[182] 1-Oxa-2,8-diaza-spiro[4.5]dec-2-en-3,8-dicarbonsäure 8-[(3-chlor-4-fluorphenyl)-amid]-3-(3,4-dimethoxy-benzylamid)
[183] 1-Oxa-2,8-diaza-spiro[4.5]dec-2-en-3,8-dicarbonsäure-3-(4-chlor-benzylamid)-8-[(3-chlor-4-fluor-phenyl)-amid]
[184] 1-Oxa-2,8-diaza-spiro[4.5]dec-2-en-3,8-dicarbonsäure 8-[(2,6-diisopropylphenyl)-amid]-3-(3,4-dimethoxy-benzylamid)
[185] 1-Oxa-2,8-diaza-spiro[4.5]dec-2-en-3,8-dicarbonsäure-3-(4-chlor-benzylamid)-8-[(2,6-diisopropyl-phenyl)-amid]
[186] 1-Oxa-2,8-diaza-spiro[4.5]dec-2-en-3,8-dicarbonsäure 8-[(5-chlor-2-methoxyphenyl)-amid]-3-(3,4-dimethoxy-benzylamid)
[187] 1-Oxa-2,8-diaza-spiro[4.5]dec-2-en-3,8-dicarbonsäure-3-(4-chlor-benzylamid)-8-[(5-chlor-2-methoxy-phenyl)-amid]
[188] 1-Oxa-2,8-diaza-spiro[4.5]dec-2-en-3,8-dicarbonsäure-3-(4-chlor-benzylamid)-8-[(3,4,5-trimethoxy-phenyl)-amid]
[189] 1-Oxa-2,8-diaza-spiro[4.5]dec-2-en-3,8-dicarbonsäure-3-(3,4-dimethoxy-benzylamid)-8-[(3,5-dimethyl-phenyl)-amid]
[190] 1-Oxa-2,8-diaza-spiro[4.5]dec-2-en-3,8-dicarbonsäure-3-(4-chlor-benzylamid)-8-[(3,5-dimethyl-phenyl)-amid]
[191] 1-Oxa-2,8-diaza-spiro[4.5]dec-2-en-3,8-dicarbonsäure-3-(3,4-dimethoxy-benzylamid)-8-[(2,6-dimethyl-phenyl)-amid]
[192] 1-Oxa-2,8-diaza-spiro[4.5]dec-2-en-3,8-dicarbonsäure-3-(4-chlor-benzylamid)-8-[(2,6-dimethyl-phenyl)-amid]
[193] 1-Oxa-2,8-diaza-spiro[4.5]dec-2-en-3,8-dicarbonsäure-3-(3,4-dimethoxy-benzylamid)-8-[(3,4-dimethyl-phenyl)-amid]
[194] 1-Oxa-2,8-diaza-spiro[4.5]dec-2-en-3,8-dicarbonsäure-3-(3,4-dimethoxy-benzylamid)-8-[(2,5-dimethyl-phenyl)-amid]
[195] 1-Oxa-2,8-diaza-spiro[4.5]dec-2-en-3,8-dicarbonsäure-3-(4-chlor-benzylamid)-8-[(2,5-dimethyl-phenyl)-amid]
[196] 1-Oxa-2,8-diaza-spiro[4.5]dec-2-en-3,8-dicarbonsäure-3-(3,4-dimethoxy-benzylamid)-8-[(2-ethyl-6-methyl-phenyl)-amid]
[197] 1-Oxa-2,8-diaza-spiro[4.5]dec-2-en-3,8-dicarbonsäure-3-(4-chlor-benzylamid)-8-[(2-ethyl-6-methyl-phenyl)-amid]
[198] 1-Oxa-2,8-diaza-spiro[4.5]dec-2-en-3,8-dicarbonsäure-3-(3,4-dimethoxy-benzylamid)-8-[(4-methoxy-2-methyl-phenyl)-amid]
[199] 1-Oxa-2,8-diaza-spiro[4.5]dec-2-en-3,8-dicarbonsäure-3-(4-chlor-benzylamid)-8-[(4-methoxy-2-methyl-phenyl)-amid]
[200] 1-Oxa-2,8-diaza-spiro[4.5]dec-2-en-3,8-dicarbonsäure-3-(3,4-dimethoxy-benzylamid)-8-[(2-methoxy-5-methyl-phenyl)-amid]
[201] 1-Oxa-2,8-diaza-spiro[4.5]dec-2-en-3,8-dicarbonsäure-3-(4-chlor-benzylamid)-8-[(2-methoxy-5-methyl-phenyl)-amid]
[202] 1-Oxa-2,8-diaza-spiro[4.5]dec-2-en-3,8-dicarbonsäure-3-(3,4-dimethoxy-benzylamid)-8-[(2,4,5-trimethyl-phenyl)-amid]
[203] 1-Oxa-2,8-diaza-spiro[4.5]dec-2-en-3,8-dicarbonsäure-3-(4-chlor-benzylamid)-8-[(2,4,5-trimethyl-phenyl)-amid]
[204] 1-Oxa-2,8-diaza-spiro[4.5]dec-2-en-3,8-dicarbonsäure-3-(3,4-dimethoxy-benzylamid)-8-[(2,4,6-trimethyl-phenyl)-amid]
[205] 1-Oxa-2,8-diaza-spiro[4.5]dec-2-en-3,8-dicarbonsäure-3-(4-chlor-benzylamid)-8-[(2,4,6-trimethyl-phenyl)-amid]
[206] 1-Oxa-2,8-diaza-spiro[4.5]dec-2-en-3,8-dicarbonsäure-8-[(2-bromo-ethyl)-amid]-3-(3,4-dimethoxy-benzylamid)
[207] 1-Oxa-2,8-diaza-spiro[4.5]dec-2-en-3,8-dicarbonsäure-8-[(2-bromo-ethyl)-amid]-3-(4-chlor-benzylamid)
[208] 1-Oxa-2,8-diaza-spiro[4.5]dec-2-en-3,8-dicarbonsäure-8-[(4-butyl-phenyl)-amid]-3-(3,4-dimethoxy-benzylamid)
[209] 1-Oxa-2,8-diaza-spiro[4.5]dec-2-en-3,8-dicarbonsäure-8-[(4-butyl-phenyl)-amid]-3-(4-chlor-benzylamid)
[210] 2-{[3-(4-Chlor-benzylcarbamoyl)-1-oxa-2,8-diaza-spiro[4.5]dec-2-en-8-carbonyl]-amino}-benzoesäuremethylester
[211] 1-Oxa-2,8-diaza-spiro[4.5]dec-2-en-3,8-dicarbonsäure-8-biphenyl-2-ylamid 3-(3,4-dimethoxy-benzylamid)
[212] 1-Oxa-2,8-diaza-spiro[4.5]dec-2-en-3,8-dicarbonsäure-8-biphenyl-2-ylamid 3-(4-chlor-benzylamid)
[213] 1-Oxa-2,8-diaza-spiro[4.5]dec-2-en-3,8-dicarbonsäure-8-[(2,6-dichlor-phenyl)-amid]-3-(3,4-dimethoxy-benzylamid)
[214] 1-Oxa-2,8-diaza-spiro[4.5]dec-2-en-3,8-dicarbonsäure-3-(4-chlor-benzylamid)-8-[(2,6-dichlor-phenyl)-amid]
[215] 3-{[3-(3,4-Dimethoxy-benzylcarbamoyl)-1-oxa-2,8-diaza-spiro[4.5]dec-2-en-8-carbonyl]-amino}-benzoesäureethylester
[216] 3-{[3-(4-Chlor-benzylcarbamoyl)-1-oxa-2,8-diaza-spiro[4.5]dec-2-en-8-carbonyl]-amino}-benzoesäureethylester
[217] 4-{[3-(3,4-Dimethoxy-benzy!carbamoyl)-1-oxa-2,8-diaza-spiro[4.5]dec-2-en-8-carbonyl]-amino}-benzoesäureethylester
[218] 4-{[3-(4-Chlor-benzylcarbamoyl)-1-oxa-2,8-diaza-spiro[4.5]dec-2-en-8-carbonyl]-amino}-benzoesäureethylester
[219] 1-Oxa-2,8-diaza-spiro[4.5]dec-2-en-3,8-dicarbonsäure-3-(4-chlor-benzylamid) 8-naphthalen-1-ylamid
[220] 3-[4-(3-Chlor-pyridin-2-yl)-piperazin-1-carbonyl]-1-oxa-2,8-diaza-spiro[4.5]dec-2-en-8-carbonthiosäure-(2-chlor-5-trifluormethyl-phenyl)-amid
[221] 8-(4-Chlor-3-trifluormethyl-phenylthiocarbamoyl)-1-oxa-2,8-diaza-spiro[4.5]dec-2-en-3-carbonsäure-3,4-dimethoxy-benzylamid
[222] 8-(3,5-Bis-trifluormethyl-phenylthiocarbamoyl)-1-oxa-2,8-diaza-spiro[4.5]dec-2-en-3-carbonsäure-3,4-dimethoxy-benzylamid
[223] 8-(3,5-Bis-trifluormethyl-phenylthiocarbamoyl)-1-oxa-2,8-diaza-spiro[4.5]dec-2-en-3-carbonsäure-4-chlor-benzylamid
[224] 3-[4-(3-Chlor-pyridin-2-yl)-piperazin-1-carbonyl]-1-oxa-2,8-diaza-spiro[4.5]dec-2-en-8-carbonthiosäure-(3,5-bis-trifluormethyl-phenylramid
[225] 8-Cyclododecylthiocarbamoyl-1-oxa-2,8-diaza-spiro[4.5]dec-2-en-3-carbonsäure-3,4-dimethoxy-benzylamid
[226] 8-Benzylthiocarbamoyl-1-oxa-2,8-diaza-spiro[4.5]dec-2-en-3-carbonsäure-3,4-dimethoxy-benzylamid
[227] 8-Benzylthiocarbamoyl-1-oxa-2,8-diaza-spiro[4.5]dec-2-en-3-carbonsäure-4-chlor-benzylamid
[228] 3-[4-(3-Trifluoromethyl-pyridin-2-yl)-piperazin-1-carbonylJ-1-oxa-2,8-diaza-spiro[4.5]dec-2-en-8-carbonsäure-(4-tert-butyl-phenyl)-amid
[229] 3-[4-(3-Chlor-pyridin-2-yl)-piperazin-1-carbonyl]-1-oxa-2,8-diaza-spiro[4.5]dec-2-en-8-carbonsäure-(4-pentafluorsulfanyl-phenyl)-amid
[230] N3-((5-Methylfuran-2-yl)methyl)-N8-(4-(trifluormethyl)phenyl)-1-oxa-2,8-diazaspiro[4.5]dec-2-en-3,8-dicarboxamid,
[231] N8-(4-Methoxyphenyl)-N3-((5-methylfuran-2-yl)methyl)-1-oxa-2,8-diazaspiro[4.5]dec-2-en-3,8-dicarboxamid,
[232] N8-(4-Chlorphenyl)-N3-((5-methylfuran-2-yl)methyl)-1-oxa-2,8-diazaspiro[4.5]dec-2-en-3,8-dicarboxamid,
[233] N3-(4-(3-Chlorpyridin-2-yl)piperazin-1-yl)-N8-(3,4-dichlorphenyl)-1-oxa-2,8-diazaspiro[4.5]dec-2-en-3,8-dicarboxamid,
[234] N3-(4-(3-Chlorpyridin-2-yl)piperazin-1-yl)-N8-(4-methoxyphenyl)-1-oxa-2,8-diazaspiro[4.5]dec-2-en-3,8-dicarboxamid,
[235] N8-(4-Chlorbenzyl)-N3-((5-methylfuran-2-yl)methyl)-1-oxa-2,8-diazaspiro[4.5]dec-2-en-3,8-dicarboxamid,
[236] N8-(3,4-Dichlorbenzyl)-N3-((5-methylfuran-2-yl)methyl)-1-oxa-2,8-diazaspiro[4.5]dec-2-en-3,8-dicarboxamid,
[237] N3-(4-Chlorbenzyl)-N8-(3,4-dichlorbenzyl)-1-oxa-2,8-diazaspiro[4.5]dec-2-en-3,8-dicarboxamid,
[238] N8-(3,4-Dichlorbenzyl)-N3-(4-hydroxy-3-methoxybenzyl)-1-oxa-2,8-diazaspiro[4.5]dec-2-en-3,8-dicarboxamid,
[239] N3-(4-tert-Butylbenzyl)-N8-(3,4-dichlorbenzyl)-1-oxa-2,8-diazaspiro[4.5]dec-2-en-3,8-dicarboxamid,
[240] N8-(3,4-Dichlorbenzyl)-N3-(4-(trifluormethyl)benzyl)-1-oxa-2,8-diazaspiro[4.5]dec-2-en-3,8-dicarboxamid,
[241] 3-(4-(3-Chlorpyridin-2-yl)piperazin-1-carbonyl)-N-(3,4-dichlorbenzyl)-1-oxa-2,8-diazaspiro[4.5]dec-2-en-8-carboxamid,
[242] N3-(4-(3-Chlorpyridin-2-yl)piperazin-1-yl)-N8-(3,4-dichlorbenzyl)-1-oxa-2,8-diazaspiro[4.5]dec-2-en-3,8-dicarboxamid,
[243] N8-(3,4-Dichlorphenyl)-N3-((5-methylfuran-2-yl)methyl)-1-oxa-2,8-diazaspiro[4.5]dec-2-en-3,8-dicarboxamid,
[244] N8-(4-Chlorphenyl)-N3-(4-hydroxy-3-methoxybenzyl)-1-oxa-2,8-diazaspiro[4.5]dec-2-en-3,8-dicarboxamid,
[245] N8-(3,4-Dichlorphenyl)-N3-(4-hydroxy-3-methoxybenzyl)-1-oxa-2,8-diazaspiro[4.5]dec-2-en-3,8-dicarboxamid,
[246] N3-(4-Chlorbenzyl)-N8-(3,4-dichlorphenyl)-1-oxa-2,8-diazaspiro[4.5]dec-2-en-3,8-dicarboxamid,
[247] N3-(4-Chlorbenzyl)-N8-(4-chlorphenyl)-1-oxa-2,8-diazaspiro[4.5]dec-2-en-3,8-dicarboxamid,
[248] N3-(4-Hydroxy-3-methoxybenzyl)-N8-(4-(trifluormethyl)phenyl)-1-oxa-2,8-diazaspiro[4.5]dec-2-en-3,8-dicarboxamid,
[249] N3-(4-Hydroxy-3-methoxybenzyl)-N8-(4-methoxyphenyl)-1-oxa-2,8-diazaspiro[4.5]dec-2-en-3,8-dicarboxamid,
[250] N3-(4-Chlorbenzyl)-N8-(4-(trifluormethyl)phenyl)-1-oxa-2,8-diazaspiro[4.5]dec-2-en-3,8-dicarboxamid,
[251] N8-(3,4-Dichlorbenzyl)-N3-(3,4-dimethoxybenzyl)-1-oxa-2,8-diazaspiro[4.5]dec-2-en-3,8-dicarboxamid,
[252] N3-(3,4-Dimethoxybenzyl)-N8-(4-(trifluormethyl)phenyl)-1-oxa-2,8-diazaspiro[4.5]dec-2-en-3,8-dicarboxamid,
[253] 3-[4-(3-Trifluormethyl-pyridin-2-yl)-piperazin-1-carbonyl]-1-oxa-2,8-diaza-spiro[4.5]dec-2-en-8-carbonsäure-(4-pentafluorsulfanyl-phenyl)-amid,
[254] N-(4-tert-Butylphenyl)-3-(4-(3-chlorpyridin-2-yl)-2-methylpiperazin-1-carbonyl)-1-oxa-2,8-diazaspiro[4.5]dec-2-en-8-carboxamid und
[255] N-(4-tert-Butylbenzyl)-3-(4-(3-chlorpyridin-2-yl)piperazin-1-carbonyl)-1-oxa-2,8-diazaspiro[4.5]dec-2-en-8-carboxamid.

Ferner können erfindungsgemäße substituierte Spiro-Verbindungen der allgemeinen Formel I bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM zeigen.

Dabei wird im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung von erfindungsgemäßen Verbindungen der vorstehend angegebenen allgemeinen Formel I gemäß dem wenigstens eine Verbindung der allgemeinen Formel II, 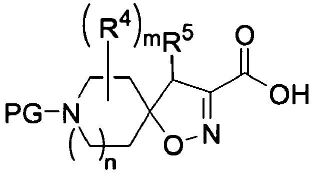 worin R⁴, R⁵, m und n die vorstehend genannte Bedeutung haben und PG für eine Schutzgruppe, bevorzugt für eine Benzyloxycarbonyl- oder tert-ButyloxycarbonylGruppe, steht, in einem Reaktionsmedium in Gegenwart wenigstens eines Kupplungsreagenzes ggf. in Gegenwart wenigstens einer Base mit einer Verbindung der allgemeinen Formel HNR²R³, worin R² und R³ die vorstehend genannte Bedeutung haben, zu wenigstens einer Verbindung der allgemeinen Formel III, 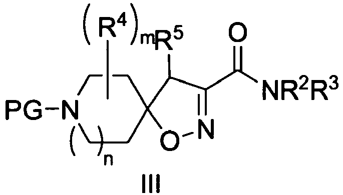 worin R², R³, R⁴, R⁵, m, n und PG die vorstehend genannte Bedeutung haben, umgesetzt wird und diese ggf. gereinigt und/oder isoliert wird
und
wenigstens eine Verbindung der allgemeinen Formel III in einem Reaktionsmedium in Gegenwart wenigstens einer Säure, vorzugsweise in Gegenwart wenigstens einer anorganischen oder organischen Säure ausgewählt aus der Gruppe bestehend aus Salzsäure, Schwefelsäure, Essigsäure und Trifluoressigsäure, oder in Gegenwart von Wasserstoff und eines Katalysators, vorzugsweise eines Katalysators basierend auf Palladium oder Platin, zu wenigstens einer Verbindung der allgemeinen Formel IV, 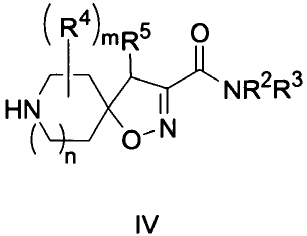 worin R², R³, R⁴, R⁵, m und n die vorstehend genannte Bedeutung haben, umgesetzt wird und diese ggf. gereinigt und/oder isoliert wird
und
wenigstens eine Verbindung der allgemeinen Formel IV in einem Reaktionsmedium mit wenigstens einem Isocyanat der allgemeinen Formel R⁶-N=C=O, worin R⁶ die vorstehend genannte Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Düsopropylethylamin, zu wenigstens einer Verbindung der allgemeinen Formel I umgesetzt wird, worin R², R³, R⁴, R⁵, m und n die vorstehend genannte Bedeutung haben und R¹ für -C(=O)-NR⁶R⁷ steht, wobei R⁶ die vorstehend genannte Bedeutung hat und R⁷ für einen Wasserstoff-Rest steht, und diese ggf. gereinigt und/oder isoliert wird
oder
wenigstens eine Verbindung der allgemeinen Formel IV in einem Reaktionsmedium mit wenigstens einem Isothiocyanat der allgemeinen Formel S=C=N-R⁸, worin R⁸ die vorstehend genannte Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R², R³, R⁴, R⁵, m und n die vorstehend genannte Bedeutung haben und R¹ für -C(=S)-N-R⁸R⁹ steht, wobei R⁸ die vorstehend genannte Bedeutung hat und R⁹ für einen Wasserstoff-Rest steht, und diese ggf. gereinigt und/oder isoliert wird
und ggf. wenigstens eine Verbindung der allgemeinen Formel I, worin R², R³, R⁴, R⁵, m und n die vorstehend genannte Bedeutung haben und R¹ für -C(=O)-NR⁶R⁷ oder - C(=S)-N-R⁸R⁹ steht, worin R⁷ und R⁹ jeweils für einen Wasserstoff-Rest stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kallumhydrid, Kallum-tert-butanolat Natrium-tert-butanolat, Kaliummethanolat, Natriummethanolat, Natriumethanolat und Kaliumethanolat, mit wenigstens einer Verbindung der allgemelnen Formel LG-R⁷ oder der allgemeinen Formel LG-R⁹, worin LG für eine Abgangsgruppe, bevorzugt für ein Halogen-Atom, besonders bevorzugt für ein Chloratom steht, und R⁷ und R⁹ die vorstehend genannte Bedeutung mit Ausnahme von Wasserstoff haben, zu wenigstens einer Verbindung der allgemeinen Formel **I** umgesetzt wird, worin R¹ bis R⁵, m und n die vorstehend genannte Bedeutung haben, und diese ggf. gereinigt und/oder isoliert wird,

Die erfindungsgemäßen Verfahren zur Herstellung substituierter Spiro-Verbindurtgen der vorstehend angegebenen allgemeinen Formel I send auch in den nachfolgenden Schemata 1 und 2 wiedergeben.

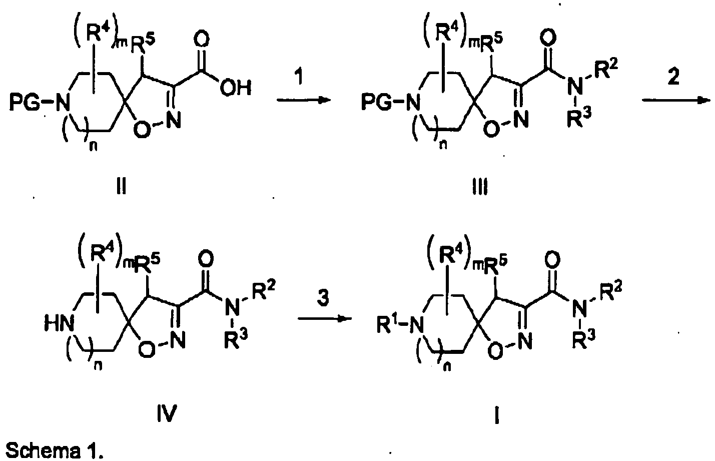

In Stufe 1 werden Verbindungen der vorstehend angegebenen allgemelnen Formel II mit Aminen der allgemeinen Formel HNR²R³, worin R³ und R² die vorstehend genannte Bedeutung haben, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC). N'(3-Dimethylaminopropyl)-N-ethylcarbodimid (EDCI), N-[(Dimethyamino)-1H-1, 2, 3-triazolo[4,5-b]pyridino-1-ylmethylen]-N-methomethanaminium hexafluorophosphat N-oxid (HATU), O-Benzotriazol-1-yl)-N,N,N',N'-tetramethyturonium hexafluorophosphat (HBTU), O-(Benzotriazol-1-yl)-N,N,N',N'-tetramethyluronium tetrafluoroborat (TBTU), 1-Hydroxybenzotriazol (HOBt) und 1-hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, N-Methylmorpholin, Pyridin, N,N-Dlmethylamlnopyridin und Diisopropylethylamin vorzugsweise bei Temperaturen von -70°C bis 100°C zu Verbindungen der allgemeinen Formel I umgesetzt.

In Stufe 2. werden Verbindungen der allgemeinen Formel III, worin PG für eine tert-Butyloxycarbonyl-Gruppe steht, in einem Reaktionsmedium vorzugsweise ausgewählt aus der Gruppe bestehend aus Methanol, Ethanol, Isopropanol, Wasser, Diethylether, Tetrahydrofuran sowie entsprechenden Mischungen in Gegenwart wenigstens einer Säure vorzugsweise ausgewählt aus der Gruppe bestehend aus Salzsäure, Schwefelsäure, Trifluoressigsäure und Essigsäure bei Temperaturen von vorzugsweise 20 bis 30 °C zu Verbindungen der allgemeinen Formel IV umgesetzt. Besonders bevorzugt erfolgt die Umsetzung der Verbindung der allgemeinen Formel III in einer 5 M Salzsäure-Lösung in Isopropanol bei einer Temperatur von vorzugsweise 20 bis 30 °C zu einer Verbindung der allgemeinen Formel IV in Form eines entsprechenden Hydrochlorids.

Alternativ werden Verbindungen der allgemeinen Formel III, worin PG für eine Benzyloxycarbonyl-Gruppe steht, in einem Reaktionsmedium vorzugsweise ausgewählt aus der Gruppe bestehend aus Diethylether, Tetrahydrofuran, Dioxan, Acetonitril, Toluol und entsprechenden Mischungen in Gegenwart von Wasserstoff und Palladium auf Kohle bei einer Temperatur von vorzugsweise 20 bis 80 °C zu einer Verbindung der allgemeinen Formel IV umgesetzt.

Sofern Verbindungen der allgemeinen Formel IV in Form eines entsprechenden Hydrochlorids vorliegen, werden diese in einem Reaktionsmedium, vorzugsweise ausgewählt aus der Gruppe bestehend aus Dioxan, Tetrahydrofuran, Diethylether, Methanol, Ethanol, Isopropanol, Wasser und entsprechenden Mischungen, in Gegenwart einer anorganischen Base, vorzugsweise unter Zusatz eines Metallhydroxids, beispielsweise Natriumhydroxid, Kaliumhydroxid oder Lithiumhydroxid, bei Temperaturen von vorzugsweise 0°C bis 30°C in die entsprechende Basen der allgemeinen Formel IV umgesetzt.

In Stufe 3 werden Verbindungen der allgemeinen Formel IV mit einem Isocyanat der allgemeinen Formel R⁶-N=C=O, worin R⁶ die vorstehend angegebene Bedeutung hat, oder mit einem Isothiocyanat der allgemeinen Formel R⁸-N=C=S, worin R⁸ die vorstehend angegebene Bedeutung hat, in einem Reaktionsmedium, vorzugsweise ausgewählt aus der Gruppe bestehend aus Acetonitril, Toluol, Dimethylformamid, Benzol, Ethanol, Methanol, Wasser und entsprechenden Mischungen, ggf. in Gegenwart wenigstens einer Base, bevorzugt In Gegenwart wenigstens einer Base ausgewähilt aus der Gruppe bestehend aus Triethylamin, N-Methylmorpholin, Pyridin, 4,4-Dimethylaminopyridin und Diisopropylethylamin, zu Verbindungen der allgemeinen Formel I umgesetzt, worin R¹ für -C(=O)-NR⁶R⁷ oder -C(=S)-NR⁸R⁹ steht, und R⁷ und R⁹ jeweils für einen Wasserstoff-Rest stehen.

Die Verbindungen der allgemeinen Formel II lassen sich wie in Schema 2. beschrieben erhalten.

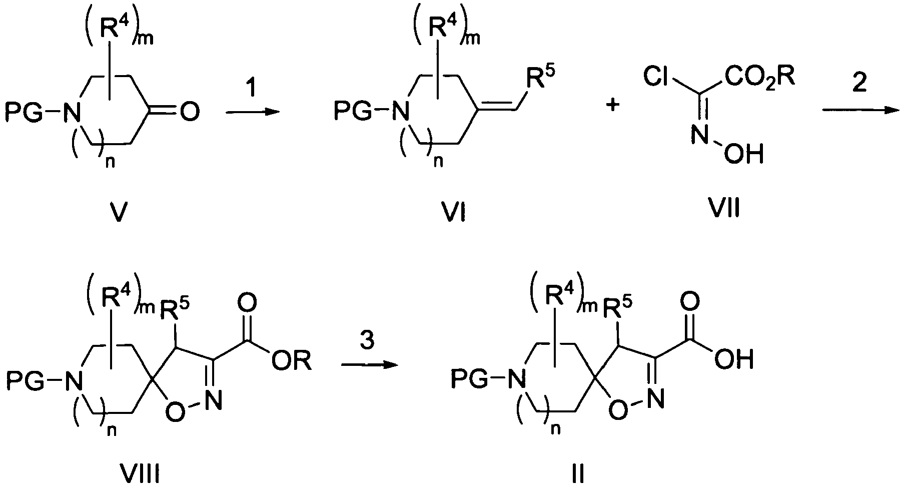

In Stufe 1 werden Verbindungen der allgemeinen Formel V, worin m, n, R⁴ und PG die vorstehend genannte Bedeutung haben, in einem Reaktionsmedium, vorzugsweise in einem Reaktionsmedium ausgewählt aus der Gruppe bestehend aus Tetrahydrofuran, Toluol, Diethylether und entsprechenden Mischungen, mit einem Reagenz zur Umwandlung von Carbonylgruppen in Doppelbindungen, bevorzugt mit einem Wittig-Reagenz der allgemeinen Formel R₃P(CH₂)R⁵X; worin R für einen Aryl-Rest steht, X für ein Halogenatom steht und R⁵ die vorstehend genannte Bedeutung hat; oder einem Wittig-Homer-Reagenz der allgemeinen Formel (RO)₂-P(=O)-(CH₂)-R⁵, worin R für einen Aryl-Rest steht und R⁵ die vorstehend genannte Bedeutung hat, besonders bevorzugt mit Methyltriphenylphosphoniumbromid, bei Temperaturen zwischen 0°C und 30 °C in Gegenwart einer Base, bevorzugt in Gegenwart eines Alkalimetallalkoholatsalzes, besonders bevorzugt in Gegenwart von Kalium-tert-butylat zu Verbindungen der allgemeinen Formel VI, worin m, n, R⁴, R⁵ und PG die vorstehend genannte Bedeutung haben, umgesetzt.

In Stufe 2 werden Verbindungen der allgemeinen Formel VI in einem Reaktionsmedium, bevorzugt in einem Reaktionsmedium ausgewählt aus der Gruppe bestehend aus Methanol, Tetrahydrofuran, Dichlormethan und entsprechenden Mischungen, in Gegenwart wenigstens einer Base, bevorzugt in Gegenwart von Natriumhydrogencarbonat, Lithiumhydroxid, Triethylamin oder N-Diisopropylethylamin, mit Verbindungen der allgemeinen Formel VII, worin R für einen linearen oder verzweigten C₁₋₆-Alkyl-Rest steht, besonders bevorzugt mit Verbindungen der allgemeinen Formel VII, worin R für einen Ethyl-Rest steht, d. h. mit Ethylchloroximidoacetat, bei Temperaturen zwischen 0 °C und 100 °C zu Verbindungen der allgemeinen Formel VIII, worin m, n, PG, R⁴, R⁵ und R die vorstehend genannte Bedeutung haben, umgesetzt.

In Stufe 3 werden Verbindungen der allgemeinen Formel VIII in einem Reaktionsmedium, bevorzugt in einem Reaktionsmedium ausgewählt aus der Gruppe bestehend aus Methanol, Ethanol, Wasser, Isopropanol und entsprechenden Mischungen, in Gegenwart wenigstens einer Base, bevorzugt in Gegenwart von Lithiumhydroxidmonohydrat, bei Temperaturen zwischen 0 °C und 50 °C zu Verbindungen der allgemeinen Formel II umgesetzt.

Die Verbindungen der vorstehend angegebenen Formeln R₃P(CH₂)R⁵X, (RO)₂-P(=O)-(CH₂)-R⁵, R¹-C(=O)-H, LG-R¹, LG-R⁷, LG-R⁹, HNR²R³, R⁶-N=C=O, R⁸-N=C=S, V und VII sind jeweils am Markt käuflich erhältlich und können auch nach üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten Spiro-Verbindungen der vorstehend genannten allgemeinen Formeln I, Ic und Id, im Folgenden nur als Spiro-Verbindungen der allgemeinen Formel I bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten Spiro-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bemsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen substituierten Spiro-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten Spiro-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Spiro-Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Spiro-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Spiro-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße Spiro-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, insbesondere zur Vanilloid-Rezeptor 1-(VR1/TRPV1)-Hemmung.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Haminkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Haminkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Haminkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Haminkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen substituierten Spiro-Verbindung, in der R¹ für eine - C(=O)NR⁶R⁷ und die übrigen Reste jeweils die vorstehend genannte Bedeutung haben, unter der Maßgabe, dass m gleich 0 und n gleich 1 und R⁵ gleich H ist, sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Bevorzugt ist die entsprechende Verwendung zur Herstellung eines Arzneimittels zur Prophylaxe und/oder Behandlung von Störungen oder Krankheiten, die zumindest teilweise durch Vanilloid-Rezeptoren 1 (VR1/TRPV1) vermittelt werden.

Besonders bevorzugt ist die entsprechende Verwendung zur Herstellung eines Arzneimittels zur Behandlung und/oder Prophylaxe von einer oder mehreren Erkrankungen ausgewählt aus der Gruppe bestehend aus akutem Schmerz; visceralem Schmerz; Gelenkschmerz; kognitiven Dysfunktionen, vorzugsweise kognitiven Mangelzuständen, besonders bevorzugt Gedächtnisstörungen; Lungenentzündung; Husten; einer überaktiven Blase (overactive bladder, OAB); Reizdarmsyndrom (irritable bowel syndrom); Augenreizungen; Hautreizungen; neurotischen Hauterkrankungen; Entzündungen des Darmes; Toleranzentwicklung gegenüber Medikamenten, vorzugsweise gegenüber natürlichen oder synthetischen Opioiden; und/oder zur Diurese oder zur Antinatriurese und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Das erfindungsgemäße Arzneimittel eignet sich zur Verabreichung an Erwachsene und Kinder einschließlich Kleinkindern und Säuglingen. Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten Spiro-Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen substituierten Spiro-Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige erfindungsgemäße substituierte Spiro-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen erfindungsgemäßen substituierten Spiro-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg, vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37 °C inkubiert. Anschließend werden die Platten 3 mal mit HBSS-Puffer gewaschen und nach einer weiteren Inkubation von 15 Minuten bei Raumtemperatur zur Ca²⁺⁻-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsazepin berechnet.

### III. Formalin-Test an der Maus

Die Untersuchung zur Bestimmung der antinociceptiven Wirkung der erfindungsgemäßen Verbindungen wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Verbindungen werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen substituierten Verbindungen vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lösung) vor Formalinapplikation erhalten. Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

### Abkürzungen:

- abs.: absolutiert
- aq.: wässrig
- Äq.: Stoffmengenäquivalent
- Boc: tert-Butoxycarbonyl
- BOP: 1-Benzotriazolyloxy-tris-(dimethylamino)-phosphonium hexafluorophosphat
- DCM: Dichlormethan
- DMF: Dimethylformamid
- EtOAc: Ethylacetat
- ges.: gesättigt
- MeOH: Methanol
- NMR: Kernresonanzspektroskopie
- RT: Raumtemperatur

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Lösungsmitteln, Laufmitteln oder für chromatographische Untersuchungen sind stets in Volumen/Volumen angegeben.
Die Analytik erfolgte durch Massenspektroskopie und NMR.

### Herstellung erfindungsgemäßer substituierter 1-Oxa-2,8-diaza-spiro[4.5]dec-2-en-Derivate

Die Synthese von 1-Oxa-2,8-diaza-spiro[4.5]dec-2-en-3,8-dicarbonsäure 8-tert-butylester ist in Schema 3 verdeutlicht.

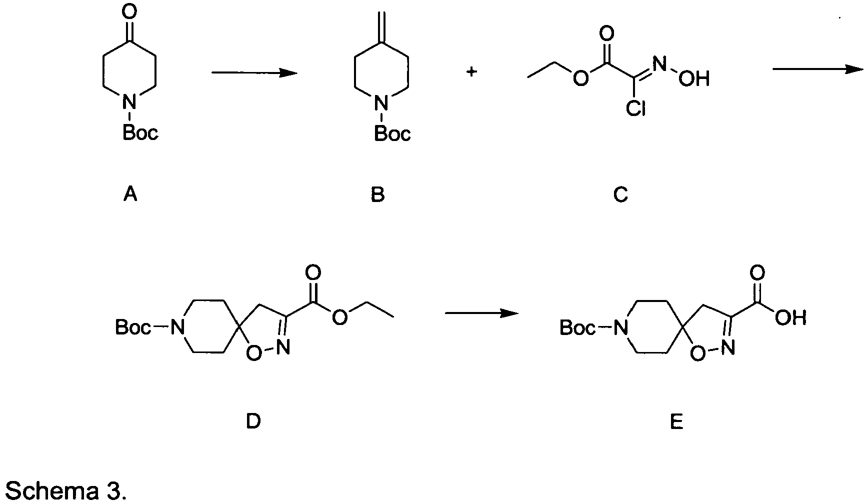

### Synthese von 4-Methylen-piperidin-1-carbonsäure-tert-butylester (B)

Zu einer Suspension von 5,34 g (15 mmol) Methyltriphenylphosphoniumbromid in 50 ml Diethylether wurden unter Rühren bei 0°C (Eisbad) 1,6 g (14 mmol) Kalium-tert-butylat zugegeben. Nach 15 min Rühren wurde eine Lösung von 2,00 g (10 mmol) 1-Boc-4-Piperidon (A) in 15 ml Diethylether langsam zugegeben. Man ließ die Suspension weitere 30 min bei 0°C rühren. Nach Zugabe von 60 ml 10%-iger aq. NH₄Cl-Lösung wurde die organische Phase abgetrennt, über Magnesiumsulfat getrocknet und im Vakuum vom Lösungsmittel befreit. Nach Chromatographie an Kieselgel (Hexan:EtOAc = 5:1) erhielt man 1,71 g (89%) 4-Methylen-piperidin-1-carbonsäure-tert-butylester (B) als farblose Flüssigkeit.
¹H-NMR-Spektrum (d₆-DMSO): δ = 1,47 ppm (s, 9H, C(CH₃)₃); 2,16-2.19 ppm (m, 4H, CH₂); 3,40-3,44 ppm (m, 4H, CH₂); 4,74 (s, 2H, C=CH₂).

### Synthese von 1-Oxa-2,8-diaza-spiro[4.5]dec-2-en-3,8-dicarbonsäure 8-tert-butylester 3-ethylester (D)

Zu einer Mischung von 0,50 g (2,6 mmol) 4-Methylen-piperidin-1-carbonsäure-tert-butylester (B) und 0,60 g (3,9 mmol) 2-Chlor-2-hydroxyiminoessigsäureethylester (C) in 10 ml DCM wurden bei 0°C (Eisbad) 0,55 ml (3,9 mmol) an zuvor frisch destilliertem Triethylamin langsam hinzu gegeben. Nach 12 h Rühren bei RT wurden erneut 0.79 g (5,1 mmol) an 2-Chlor-2-hydroxyiminoessigsäureethylester und 0,72 ml (5,1 mmol) an Triethylamin bei 0°C hinzu gegeben und das Reaktionsgemisch wurde für weitere 24 h gerührt. Nach Waschen mit 10%-iger wäßriger Zitronensäure und ges. aq. NaCl-Lösung erhielt man nach Trocknen der organischen Phase (MgSO₄) und Entfernen des Lösungsmittels im Vakuum ein gelbes Öl. Nach Säulenchromatographie an Kieselgel (Hexan:Diethylether = 4:1) erhielt man 320 mg (39%) an 1-Oxa-2,8-diaza-spiro[4.5]dec-2-en-3,8-dicarbonsäure-8-tert-butylester 3-ethylester (D) in Form eines leicht gelblich gefärbten Öles.
¹H-NMR-Spektrum (d₆-DMSO): δ = 1,37 ppm (t, *J* = 6.0 Hz, 3H, CH₃); 1,46 ppm (s, 9H, C(CH₃)₃); 1,67-1,75 ppm (m, 2H, CH₂); 1,85-1,92 ppm (m, 2H, CH₂); 2,96 ppm (s, 2H, CH₂); 3.39-3,49 ppm (m, 2H, CH₂); 3,60-3,70 ppm (m, 2H, CH₂); 4,35 (q, *J* = 6.0 Hz, 2H, CH₂).

### Synthese von 1-Oxa-2,8-diaza-spiro[4.5]dec-2-en-3,8-dicarbonsäure-8-tert-butylester (E)

Eine Mischung von 320 mg (1 mmol) 4-Methylen-piperidin-1-carbonsäure-tert-butylester-3-ethylester (D) in 2 ml MeOH und 70 mg (1,5 mmol) Lithiumhydroxidmonohydrat in 1.3 ml H₂O wurde 1.5 h bei RT gerührt. Nach Entfernen des Lösungsmittels im Vakuum wurde der Rückstand in Wasser und EtOAc aufgenommen und getrennt, wobei die aq. Phase mit Zitronensäure auf pH = 4 eingestellt wurde. Die organische Phase wurde getrocknet (MgSO₄) und im Vakuum vom Lösungsmittel befreit. Man erhielt 280 mg (98%) der freien Säure 1-Oxa-2,8-diaza-spiro[4.5]dec-2-en-3,8-dicarbonsäure-8-tert-butylester (E) in Form eines farblosen Feststoffes.

### Herstellung erfindungsgemäßer substituierter Spiro-Verbindungen

Die Synthese von erfindungsgemäßen substituierten Spiro-Verbindungen ist in Schema 4 verdeutlicht.

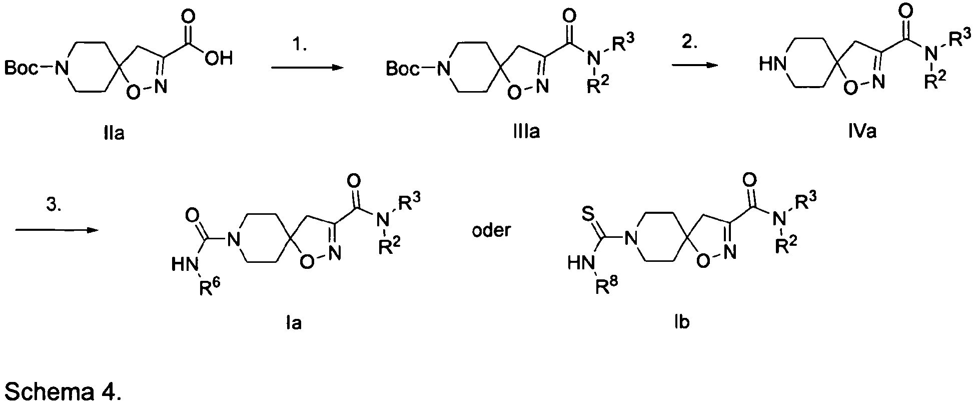

### Stufe 1: Allgemeine Vorschrift zur Umsetzung von 1-Oxa-2,8-diaza-spiro[4.5]dec-2-en-3,8-dicarbonsäure-8-tert-butylester der allgemeinen Formel IIa mit primären oder sekundären Aminen der allgemeinen Formel HNR²R³

Eine Mischung von 1 Äquivalent an 1-Oxa-2,8-diaza-spiro[4.5]dec-2-en-3,8-dicarbonsäure-8-tert-butylester (E), 1 Äquivalent des jeweiligen Amins HNR²R³, 2.7 Äquivalenten an N-Methylmorpholin und 1.8 Äquivalenten BOP in DMF wurde 12 h bei RT rühren gelassen. Nach Entfernen des DMF im Vakuum wurde der Rückstand mit H₂O und EtOAc versetzt und getrennt. Die organische Phase wurde mit H₂O, 10%-iger aq. Zitronensäure-Lösung, ges. aq. Na₂CO₃-Lösung und ges. aq. NaCl-Lösung gewaschen, getrocknet (MgSO₄) und im Vakuum vom Lösungsmittel befreit. Nach Säulenchromatographie (Kieselgel, Diethylether:Hexan = 10:1) erhielt man die jeweiligen Kupplungsprodukte.

In einigen Fällen wurde die Kupplung mit 1 Äquivalent an 1-Oxa-2,8-diaza-spiro[4.5]dec-2-en-3,8-dicarbonsäure-8-tert-butylester (E), 1.05 Äquivalent des jeweiligen Amins und 1.05 Äquivalent N,N'-Carbonyldiimidazol in THF bei RT durchgeführt. Das Reaktionsgemisch wurde dazu erst 90 Minuten in Gegenwart der Säure und von N,N'-Carbonyldiimidazol und nach Zugabe des Amins weitere 21 Stunden gerührt. Zur Aufarbeitung wurde das Lösungsmittel entfernt, der Rückstand in EtOAc aufgenommen und 10%-iger aq. Zitronensäure-Lösung versetzt. Die organische Phase wurde abgetrennt, mit 10%-iger Zitronensäure-Lösung extrahiert, mit ges. aq. NaHCO₃-Lösung und mit ges. aq. NaCl-Lösung gewaschen. Die organische Phase wurde getrocknet und das Lösungsmittel im Vakuum entfernt.

### Synthese von 3-(3,4-Dimethoxybenzylcarbamoyl)-1-oxa-2,8-diazaspiro[4.5]dec-2-en-8-carbonsäure-tert-butylester

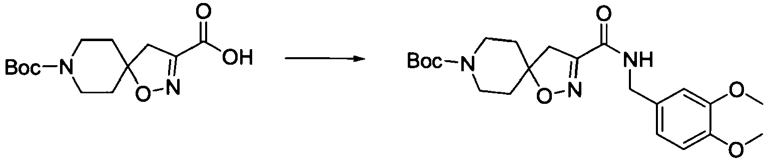

Zu einer Lösung von 1-Oxa-2,8-diaza-spiro[4.5]dec-2-en-3,8-dicarbonsäure-8-tert-butylester (7 g, 24.6 mmol) in abs. THF (100 ml) wurde *N,N'*-Carbonyldiimidazol (4,2 g, 26.06 mmol) gegeben und 1.5 h bei RT gerührt. Nach Zugabe von 3,4-Dimethoxy-benzylamin (4.35 g, 3.92 ml, 26.06 mmol) wurde weitere 21 h gerührt. Zur Aufarbeitung wurde der Ansatz eingeengt, in EtOAc (70 ml) aufgenommen und mit 10-%iger aq. Zitronensäure-Lösung (40 ml) versetzt. Die organische Phase wurde abgetrennt, mit 10-%iger aq. Zitronensäure-Lösung (40 ml) extrahiert, mit ges. aq. NaHCO₃-Lösung (2 x 40 ml) und mit ges. aq. NaCl-Lösung (40 ml) gewaschen. Die organische Phase wurde getrocknet und das Lösungsmittel im Vakuum entfernt. Das Amid 3-(3,4-Dimethoxybenzylcarbamoyl)-1-oxa-2,8-diazaspiro[4.5]dec-2-en-8-carbonsäure-tert-butylester wurde als beigefarbener Feststoff in einer Ausbeute von 80 % (8.49 g) mit einem Schmelzpunkt von 118-120 °C isoliert.

### Synthese von 3-(4-tert-Butyl-benzylcarbamoyl)-1-oxa-2,8-diazaspiro[4.5]dec-2-en-8-carbonsäure-tert-butylester

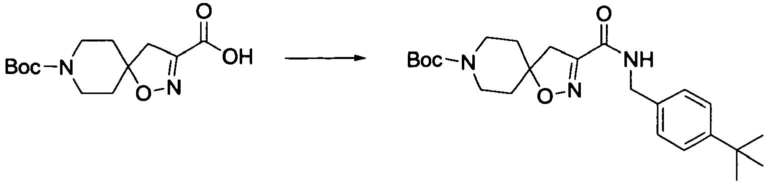

Zu einer Lösung von 1-Oxa-2,8-diaza-spiro[4.5]dec-2-en-3,8-dicarbonsäure-8-tert-butylester (1 g, 3.5 mmol) in abs. DMF (20 mL) wurden 4-Methylmorpholin (1.16 mL), 10.5 mmol) und 4-(tert-Butyl)-benzylamin (0.574 g, 3.5 mmol) gegeben und die Reaktionsmischung gerührt. Es wurde BOP (2.02 g, 4.57 mmol) zugegeben und über Nacht gerührt. Zur Aufarbeitung wurde der Ansatz eingeengt, in EtOAc (70 ml) aufgenommen ges. aq. NaHCO₃-Lösung (2 x 40 ml) aufgenommen. Die Phasen wurden getrennt und die wäßrige Phase mit Essigester gewaschen. Die vereinigten organischen Phasen wurden mit ges. aq. NaCl-Lösung (40 ml) gewaschen, getrocknet und das Lösungsmittel im Vakuum entfernt. Das Amid 3-(4-tert-Butyl-benzylcarbamoyl)-1-oxa-2,8-diazaspiro[4.5]dec-2-en-8-carbonsäure-tert-butylester wurde nach Säulenchromatographie (SiO₂, EE/Hexan 1:2) als Feststoff in einer Ausbeute von 84 % (1.27 g) isoliert.

### Synthese von 3-(4-triflourmethyl-benzylcarbamoyl)-1-oxa-2,8-diazaspiro[4.5]dec-2-en-8-carbonsäure-tert-butylester

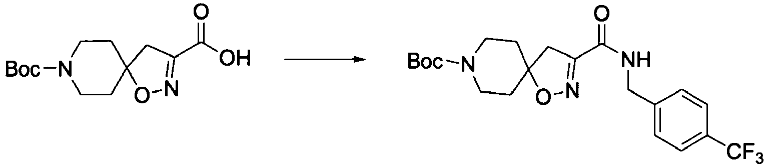

Zu einer Lösung von 1-Oxa-2,8-diaza-spiro[4.5]dec-2-en-3,8-dicarbonsäure-8-tert-butylester (1 g, 3.5 mmol) in abs. DMF (20 mL) wurden 4-Methylmorpholin (1.16 mL), 10.5 mmol) und 4-(Triflourmethyl)-benzylamin (0.574 g, 3.5 mmol) gegeben und die Reaktionsmischung gerührt. Es wurde BOP (2.02 g, 4.57 mmol) zugegeben und über Nacht gerührt. Zur Aufarbeitung wurde der Ansatz eingeengt, in EtOAc (70 ml) und ges. aq. NaHCO₃-Lösung (2 x 40 ml) aufgenommen. Die Phasen wurden getrennt und die wäßrige Phase mit Essigester gewaschen. Die vereinigten organischen Phasen wurden mit ges. aq. NaCl-Lösung (40 ml) gewaschen, getrocknet und das Lösungsmittel im Vakuum entfernt. Das Amid 3-(4-Triflourmethyl-benzylcarbamoyl)-1-oxa-2,8-diazaspiro[4.5]dec-2-en-8-carbonsäure-tert-butylester wurde nach Säulenchromatographie (SiO₂, Ether/Hexan 10:1) als Feststoff in einer Ausbeute von 84 % (1.31 g) isoliert.

### Synthese von 3-(5-Chlorpyridin-2-ylcarbamoyl)-1-oxa-2,8-diazaspiro[4.5]dec-2-en-8-carbonsäure-tert-butylester

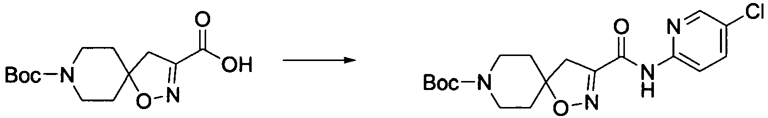

Zu einer Lösung von 1-Oxa-2,8-diaza-spiro[4.5]dec-2-en-3,8-dicarbonsäure-8-tert-butylester (7 g, 24.6 mmol) in abs. THF (100 ml) wurde *N,N'*-Carbonyldiimidazol (5.8 g, 36.9 mmol) gegeben und 2 h bei RT gerührt. Nach Zugabe von 2-Amino-5-chloropyridin (4.74 g, 36.9 mmol) wurde die Reaktionsmischung 45 h gerührt. Zur Aufarbeitung wurde der Ansatz eingeengt, in EtOAc (120 ml) aufgenommen und mit 10%-iger aq. Zitronensäure-Lösung (100 ml) versetzt. Zwischen den Phasen blieb ein Nebenprodukt ungelöst zurück, das durch Filtration abgetrennt wurde. Die organische Phase des Filtrats wurde abgetrennt, mit 10%-iger aq. Zitronensäure-Lösung (50 ml) extrahiert, mit ges. aq. NaHCO₃-Lösung (2 × 50 ml) und ges. aq. NaCl-Lösung (50 ml) gewaschen. Die organische Phase wurde getrocknet und das Lösungsmittel im Vakuum entfernt. Das Amid 3-(5-Chlorpyridin-2-ylcarbamoyl)-1-oxa-2,8-diazaspiro[4.5]dec-2-en-8-carbonsäure-tert-butylester wurde als braunes festes Rohprodukt (7.62 g) isoliert. Nach chromatographischer Reinigung [Kieselgel 60 (150 g); EtOAc/Cyclohexan 1 : 3 (1.8 I)] wurde das Amid in einer Ausbeute von 70 % (6.8 g) mit einem Schmelzpunkt von 171-172 °C isoliert.

### Synthese von 3-[4-(3-Chlorpyridin-2-yl)piperazin-1-carbonyl]-1-oxa-2,8-diazaspiro[4.5]dec-2-en-8-carbonsäure-tert-butylester

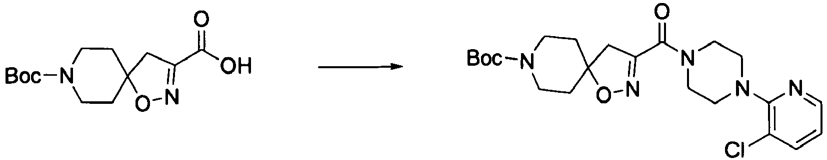

Zu einer Lösung von 1-Oxa-2,8-diaza-spiro[4.5]dec-2-en-3,8-dicarbonsäure-8-tert-butylester (7 g, 24.6 mmol) in abs. THF (80 ml) wurde *N,N'*-Carbonyldiimidazol (4.39 g, 27.1 mmol) hinzugefügt. Das Gemisch wurde 1.5 h bei RT gerührt. Nach Zugabe von 1-(3-Chloro-2-pyridinyl)piperazin (5.36 g, 27.1 mmol) in abs. THF (20 ml) wurde 6 Tage bei RT gerührt. Zur Aufarbeitung wurde das Lösungsmittel im Vakuum entfernt. Der braune ölige Rückstand wurde in EtOAc (80 ml) aufgenommen und mit 10%-iger aq. Zitronensäure-Lösung (80 ml) 20 min gerührt. Die Phasen wurden getrennt. Die organische Phase wurde mit ges. aq. NaHCO₃-Lösung (2 × 40 ml) und ges. aq. NaCl-Lösung (40 ml) gewaschen, getrocknet und das Lösungsmittel im Vakuum entfernt. Das Amid 3-[4-(3-Chlorpyridin-2-yl)piperazin-1-carbonyl]-1-oxa-2,8-diazaspiro[4.5]dec-2-en-8-carbonsäure-tert-butylester wurde als zähes braunes Öl in einer Ausbeute von 72 % (8.25 g) erhalten.

### Synthese von 3-[4-(3-Chlorpyridin-2-yl)-2-methyl-piperazin-1-carbonyl]-1-oxa-2,8-diazaspiro[4.5]dec-2-en-8-carbonsäure-tert-butylester

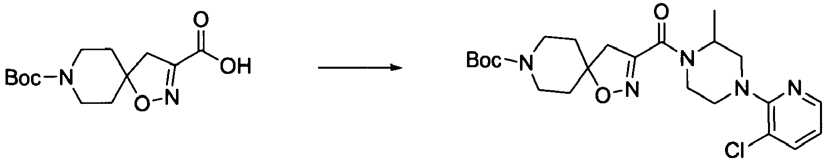

Zu einer Lösung von 1-Oxa-2,8-diaza-spiro[4.5]dec-2-en-3,8-dicarbonsäure-8-tert-butylester (2.5 g, 8.8 mmol) in abs. THF (67 ml) wurden TBTU (2.8 g, 8.8 mmol), HOBt (1.18 g, 8.8 mmol), Diisopropylethylamin (5 mL, 26.4 mmol) und 1-(3-Triflourmethyl-2-pyridinyl)-3-methyl-piperazin (1.86 g, 8.8 mmol) gegeben. Die Reaktionsmischung wurde über Nacht bei RT gerührt, DMF (20 mL) hinzu gegeben und weitere 6 Stunden gerührt. Zur Aufarbeitung wurde das Lösungsmittel im Vakuum entfernt. Der braune ölige Rückstand wurde in EtOAc (200 mL) aufgenommen und mit ges. aq. NaHCO₃-Lösung (200 mL), ges. aq. NaCl-Lösung (200 ml), ges. aq. NaCl-Lösung (200 mL) und ges. aq. NH₄HSO₄-Lösung (200 mL) gewaschen, getrocknet und das Lösungsmittel im Vakuum entfernt. Das Amid 3-[4-(3-Chlorpyridin-2-yl)-2-methyl-piperazin-1-carbonyl]-1-oxa-2,8-diazaspiro[4.5]dec-2-en-8-carbonsäure-tert-butylester wurde in einer Ausbeute von 60 % (2.5 g) erhalten.

### Synthese von 3-[(5-Methylfuran-2-ylmethyl)carbamoyl]-1-oxa-2,8-diazaspiro[4.5]dec-2-en-8-carbonsäure-tert-butylester

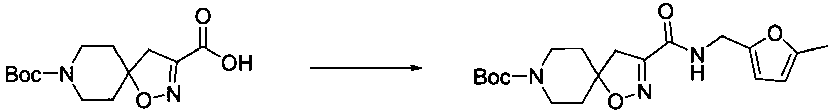

Zu einer Lösung von 1-Oxa-2,8-diaza-spiro[4.5]dec-2-en-3,8-dicarbonsäure-8-tert-butylester (7 g, 24.6 mmol) in abs. THF (80 ml) wurde *N,N'*-Carbonyldiimidazol (4.39 g, 27.1 mmol) gegeben und 2.5 h bei RT gerührt. Nach Zugabe von 5-Methyl-furanyl-2-methylamin (3.01 g, 27.1 mmol) in abs. THF (20 ml) wurde die Reaktionsmischung 4 Tage bei RT gerührt. Zur Aufarbeitung wurde das Lösungsmittel im Vakuum entfernt. Der hellbraune ölige Rückstand wurde in EtOAc (80 ml) aufgenommen und mit 10%-iger aq. Zitronensäure-Lösung (80 ml) 20 min gerührt. Die Phasen wurden getrennt. Die organische Phase wurde mit ges. aq. NaHCO₃-Lösung (2 × 40 ml) und ges. aq. NaCl-Lösung (40 ml) gewaschen, getrocknet und das Lösungsmittel im Vakuum entfernt. Das Amid 3-[(5-Methylfuran-2-ylmethyl)carbamoyl]-1-oxa-2,8-diazaspiro[4.5]dec-2-en-8-carbonsäure-tert-butylester wurde als hellgelber Feststoff in einer Ausbeute von 98 % (9.1 g) erhalten.

### Synthese von 3-(4-Chlorbenzylcarbamoyl)-1-oxa-2,8-diazaspiro[4.5]dec-2-en-8-car-bonsäure-tert-butylester

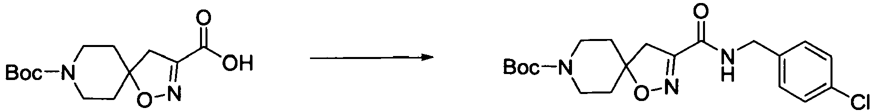

Zu einer Lösung von 1-Oxa-2,8-diaza-spiro[4.5]dec-2-en-3,8-dicarbonsäure-8-tert-butylester (7 g, 24.6 mmol) in abs. THF (100 ml) wurde *N,N'*-Carbonyldiimidazol (4.39 g, 27.1 mmol) gegeben. Das Reaktionsgemisch wurde 1.5 h bei RT gerührt. Nach Zugabe von 4-Chlorbenzylamin (4,22 g, 29,8 mmol) wurde die Reaktionsmischung 3 Tage bei RT gerührt. Zur Aufarbeitung wurde das Lösungsmittel im Vakuum entfernt. Der braune ölige Rückstand wurde in EtOAc (80 ml) aufgenommen und mit 10%-iger aq. Zitronensäure-Lösung (80 ml) 20 min gerührt. Die Phasen wurden getrennt. Die organische Phase wurde mit ges. aq. NaHCO₃-Lösung (2 × 40 ml) und ges. aq. NaCl-Lösung (40 ml) gewaschen, getrocknet und das Lösungsmittel im Vakuum entfernt. Das Amid 3-(4-Chlorbenzylcarbamoyl)-1-oxa-2,8-diazaspiro[4.5]dec-2-en-8-car-bonsäure-tert-butylester wurde als hellbrauner Feststoff in einer Ausbeute von 97 % (9.74 g) mit einem Schmelzpunkt von 136-138 °C erhalten.

### Synthese von 3-(4-Hydroxy-3-methoxybenzylcarbamoyl)-1-oxa-2,8-diazaspiro[4.5]dec-2-en-8-carbonsäure-tert-butylester

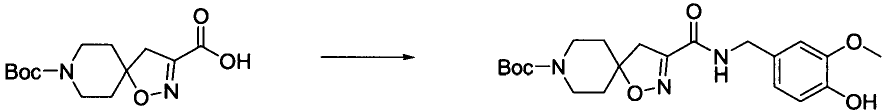

Zu einer von 1-Oxa-2,8-diaza-spiro[4.5]dec-2-en-3,8-dicarbonsäure-8-tert-butylester (5.8 g, 20.4 mmol) in wasserfreiem DMF (60 ml) wurden BOP (11.72 g, 26.53 mmol) und *N*-Methylmorpholin (6.73 g, 61.23 mmol) gegeben. Das Gemisch wurde 1.5 h bei RT unter Argon gerührt. Nach Zugabe von 4-Hydroxy-3-methoxy-benzylamin (4.69 g, 30.6 mmol) wurde die Reaktionsmischung 22.5 h bei RT gerührt. Zur Aufarbeitung wurde das Lösungsmittel im Vakuum entfernt. Der braune ölige Rückstand wurde in Wasser (100 ml), ges. aq. NaHCO₃-Lösung (60 ml) und DCM (100 ml) aufgenommen. Die Phasen wurden getrennt und die wässrige Phase mit DCM extrahiert (3 × 100 ml). Die organische Phase wurde getrocknet und das Lösungsmittel im Vakuum entfernt. Der braune ölige Rückstand wurde noch einmal in DCM (70 ml) aufgenommen und mit Wasser (2 × 35 ml) gewaschen. Die Phasen wurden getrennt. Die organische Phase wurde getrocknet und das Lösungsmittel im Vakuum entfernt. Anschließend wurde der Rückstand in Wasser (150 ml) aufgenommen und gerührt und der ausfallende Feststoff abgesaugt. Das Amid 3-(4-Hydroxy-3-methoxybenzylcarbamoyl)-1-oxa-2,8-diazaspiro[4.5]dec-2-en-8-carbonsäure-tert-butylester wurde als hellbrauner Feststoff in einer Ausbeute von 99 % (8,47 g) mit einem Schmelzpunkt von 151-157 °C erhalten.

### Synthese von 3-[4-(3-Trifluormethyl-pyridin-2-yl)piperazin-1-carbonyl]-1-oxa-2,8-diazaspiro[4.5]dec-2-en-8-carbonsäure-tert-butylester

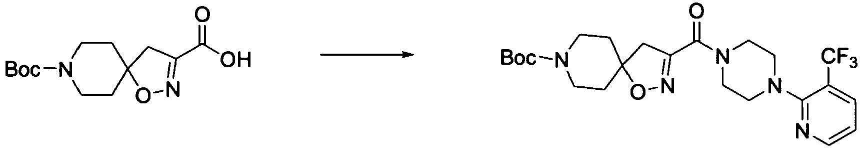

Zu einer Lösung von 1-Oxa-2,8-diaza-spiro[4.5]dec-2-en-3,8-dicarbonsäure-8-tert-butylester (1.5 g, 5.2 mmol) in abs. DMF (25 ml) wurden 4-Methylmorpholin (1.75 mL, 15. 81 mmol), BOP (3.03 g, 6.9 mmol) und 1-(3-Trifluormethyl-2-pyridinyl)piperazin hinzugefügt wurde und die Reaktionsmischung wurde über Nacht bei RT gerührt. Zur Aufarbeitung wurde das Lösungsmittel im Vakuum entfernt. Der braune ölige Rückstand wurde in EtOAc (40 ml) und ges. aq. NaHCO₃-Lösung (2 × 20 ml) aufgenommen. Die Phasen wurden getrennt. Die wäßrige Phase wurde mit EtOAc gewaschen und die vereinigten organischen Phase wurde mit ges. aq. NH₄Cl-Lösung (2 × 20 ml) und ges. aq. NaCl-Lösung (20 ml) gewaschen, getrocknet und das Lösungsmittel im Vakuum entfernt. Der Rückstand wurde in Essigsester aufgenommen und säulenchromatographisch (SiO₂, EE/ Hexan 1:1) gereinigt. Es wurde das gewünschte Produkt in einer Ausbeute von 2.18 g erhalten.

### Stufe 2: Allgemeine Vorschrift zur Abspaltung der Boc-Gruppe aus Verbindungen der allgemeinen Formel IIIa

Das entsprechende *N*-Boc-Piperidin der allgemeinen Formel IIIa in MeOH wurde mit einem Überschuss einer 5 N Lösung von HCl in Isopropanol bei RT versetzt und gerührt. Nach vollständiger Reaktion wurde die Lösung bis zur ersten Trübung eingeengt, dann mit Diethylether versetzt und zur Vervollständigung der Fällung des gewünschten Produkts als Hydrochlorid bei 4°C über Nacht gelagert. Der ausgefallene Feststoff wurde abfiltriert, mit kleinen Portionen an Diethylether gewaschen und im Vakuum getrocknet.

### Synthese von 1-Oxa-2,8-diazaspiro[4.5]dec-2-en-3-carbonsäure-3,4-dimethoxy-benzylamid

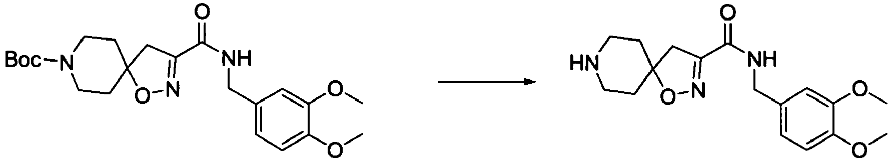

Eine Lösung von 3-(3,4-Dimethoxybenzylcarbamoyl)-1-oxa-2,8-diazaspiro[4.5]dec-2-en-8-carbonsäure-tert-butylester (8.4 g, 19.38 mmol) in MeOH (300 ml) wurde mit 5 N HCl (80 ml, 400 mmol) in Isopropanol versetzt und 3 d bei RT gerührt. Dabei fiel ein Teil des Hydrochlorids des Amins 1-Oxa-2,8-diazaspiro[4.5]dec-2-en-3-carbonsäure-3,4-dimethoxy-benzylamid als farbloser Feststoff aus (4.2 g). Das Filtrat wurde auf 80 ml eingeengt und weiteres Hydrochlorid isoliert (2.29 g). Die beiden Fraktionen wurden vereinigt und das Hydrochlorid in einer Ausbeute von 92 % (Schmp. 132-136 °C) erhalten. Zur Freisetzung der Base wurde das Hydrochlorid in DCM (100 ml) und ges. aq. NaHCO₃-Lösung aufgenommen und 1 h bei RT gerührt. Die wässrige Phase wurde abgetrennt und mit DCM (4 × 50 ml) extrahiert. Die vereinigten organischen Phasen wurden getrocknet und das Lösungsmittel im Vakuum entfernt. Das Amin 1-Oxa-2,8-diazaspiro[4.5]dec-2-en-3-carbonsäure-3,4-dimethoxy-benzylamid wurde als farbloser Feststoff mit einem Schmelzpunkt von 121-123 °C in einer Ausbeute von 89 % (5.68 g) isoliert.

### Synthese von 1-Oxa-2,8-diazaspiro[4.5]dec-2-en-3-carbonsäure-4-triflourmethyl-benzylamid Hydrochlorid

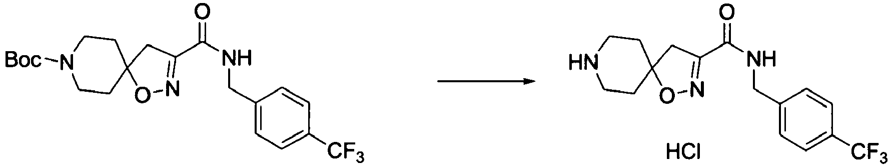

Eine Lösung von 3-(4-Triflourmethyl-benzylcarbamoyl)-1-oxa-2,8-diazaspiro[4.5]dec-2-en-8-carbonsäure-tert-butylester (1 g, 3.6 mmol) in methanolischer HCl (4 N, 5 mL) wurde bei RT gerührt. Es wurde zweimal mit jeweils konz. aq. HCl (1 mL) und einmal mit MeOH (5 mL) nachdosiert. Das Lösungsmittel wurde im Vakuum entfernt und der Rückstand in Ether aufgenommen. Dabei fiel das Hydrochlorid des Amins 1-Oxa-2,8-diazaspiro[4.5]dec-2-en-3-carbonsäure-4-triflourmethyl-benzylamid als farbloser Feststoff aus (1.31 g, 85 %).

### Synthese von 1-Oxa-2,8-diazaspiro[4.5]dec-2-en-3-carbonsäure-4-tert-butyl-benzylamid Hydrochlorid

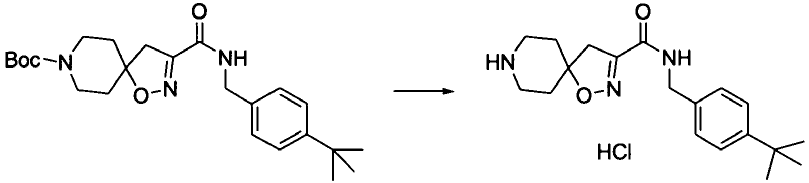

Eine Lösung von 3-(4-tert-Butyl-benzylcarbamoyl)-1-oxa-2,8-diazaspiro[4.5]dec-2-en-8-carbonsäure-tert-butylester (0.55 g, 1.28 mmol) in Methanol (10 mL) wurde nach Zugabe von konz. aq. HCl (2.2 mL) bei RT für 48 Stunden gerührt. Das Lösungsmittel wurde im Vakuum entfernt und der Rückstand in Ether aufgenommen. Dabei fiel das Hydrochlorid des Amins 1-Oxa-2,8-diazaspiro[4.5]dec-2-en-3-carbonsäure-4-tert-butyl-benzylamid als farbloser Feststoff aus (0.32 g, 69 %).

### Synthese von 1-Oxa-2,8-diazaspiro[4.5]dec-2-en-3-carbonsäure-4-chlorbenzylamid-hydrochlorid und N-(5-Chlorpyridin-2-yl)-1-oxa-2,8-diazaspiro[4.5]dec-2-en-3-imidsäure-methylester-dihydrochlorid

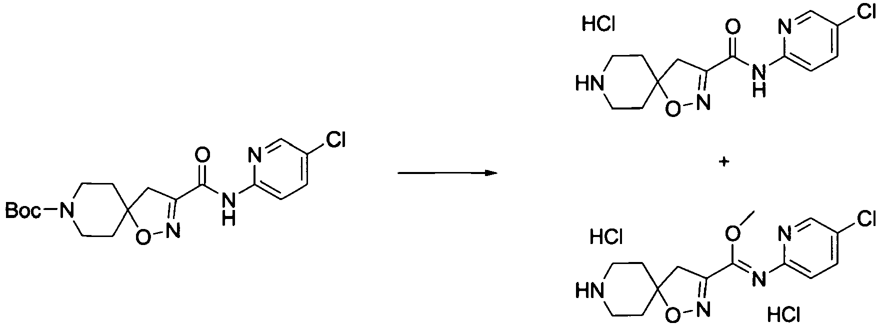

Eine Lösung von 3-(5-Chlorpyridin-2-ylcarbamoyl)-1-oxa-2,8-diazaspiro[4.5]dec-2-en8-carbonsäure-tert-butylester (6.8 g, 17.2 mmol) in abs. MeOH (600 ml) wurde mit 5 N Salzsäure (69 ml, 344 mmol) in Isopropanol versetzt und 3 d bei RT gerührt. Der Ansatz wurde auf 50 ml eingeengt, wobei 1-Oxa-2,8-diazaspiro[4.5]dec-2-en-3carbonsäure-4-chlorbenzylamid-hydrochlorid als farbloser Feststoff (1,9 g, 33 %, Schmp. 280-284 °C) ausfiel. Das Filtrat wurde eingeengt und der ölige Rückstand in MeOH (30 ml) aufgenommen. Nach Zugabe von Diethylether (200 ml) wurde die Mischung 16 h bei RT gerührt. Der Imidoester *N*-(5-Chlorpyridin-2-yl)-1-oxa-2,8diaza-spiro[4.5]dec-2-en-3-imidsäure-methylester-dihydrochlorid fiel als farbloser Feststoff aus und konnte in einer Ausbeute von 63 % (4.12 g) erhalten werden.

### Synthese von 1-Oxa-2,8-diazaspiro[4.5]dec-2-en-3-carbonsäure-4-chlorbenzylamid-trifluoroacetat

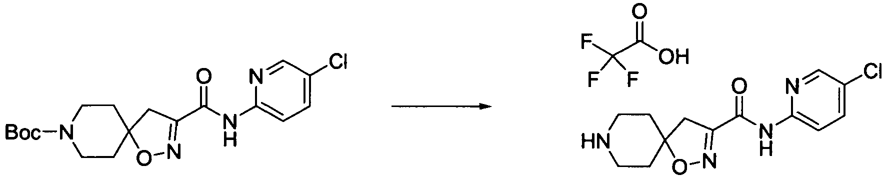

Zu einer Lösung 3-(5-Chlorpyridin-2-ylcarbamoyl)-1-oxa-2,8-diazaspiro[4.5]dec-2-en8-carbonsäure-tert-butylester (4.59 g, 11.6 mmol) in DCM (30 ml) wurde langsam Trifluoressigsäure (20 ml) gegeben. Die Reaktionslösung wurde 3 d bei RT gerührt. Der Ansatz wurde eingeengt, der ölige Rückstand mit Diethylether (100 ml) versetzt und 2 h gerührt. 1-Oxa-2,8-diazaspiro[4.5]dec-2-en-3-carbonsäure-4-chlorbenzylamid-trifluoroacetat wurde als farbloser Feststoff in einer Ausbeute von 99 % (4.65 g) mit einem Schmelzpunkt von 232-234 °C isoliert.

### Synthese von [4-(3-Chlorpyridin-2-yl)piperazin-1-yl]-(1-oxa-2,8-diazaspiro[4.5]dec-2-en-3-yl)methanon-hydrochlorid

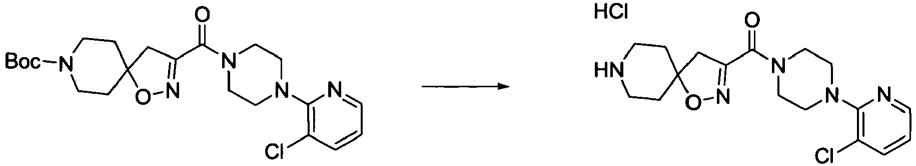

Eine Lösung von 3-[4-(3-Chlorpyridin-2-yl)piperazin-1-carbonyl]-1-oxa-2,8-diazaspiro[4.5]dec-2-en-8-carbonsäure-tert-butylester (8.25 g, 17.8 mmol) in abs. MeOH (300 ml) wurde mit 5.5 N HCl in Isopropanol (64.7 ml, 356 mmol) versetzt und 22 h bei RT gerührt. Der Ansatz wurde auf 10 ml das Lösungsmittel im Vakuum entfernt. Zur erkalteten Lösung wurde langsam Diethylether (10 ml) bis zur Trübung gegeben. Die Lösung wurde 1.5 h bei RT gerührt, wobei das [4-(3-Chlorpyridin-2-yl)piperazin-1-yl]-(1-oxa-2,8-diazaspiro[4.5]dec-2-en-3-yl)methanon-hydrochlorid als farbloser Feststoff (6.22 g, 88 %, Smp. 135-138 °C) ausfiel.

### Synthese von [4-(3-Chlorpyridin-2-yl)--piperazin-1-yl]-(1-oxa-2,8-diazaspiro[4.5]dec-2-en-3-yl)methanon-hydrochlorid

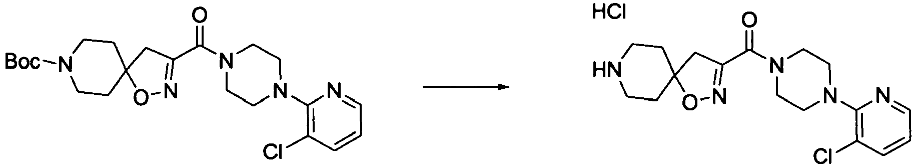

Eine Lösung von 3-[4-(3-Chlor-pyridin-2-yl)-piperazin-1-carbonyl]-1-oxa-2,8-diazaspiro[4.5]dec-2-en-8-carbonsäure-tert-butylester (2.5 g, 5.23 mmol) in abs. MeOH (50 mL) wurde unter Eiskühlung mit 1.25 N HCl in Isopropanol (34.3 ml, 43 mmol) versetzt und 5 h bei RT gerührt. Das Lösungsmittel wurde im Vakuum entfernt. Zur erkalteten Lösung wurde langsam Diethylether (10 ml) bis zur Trübung gegeben. Die Lösung wurde 1.5 h bei RT gerührt, wobei das [4-(3-Chlor-pyridin-2-yl)--piperazin-1-yl]-(1-oxa-2,8-diazaspiro[4.5]dec-2-en-3-yl)methanon-hydrochlorid als farbloser Feststoff (2.4 g) ausfiel.

### Synthese von [4-(3-Trifluormethyl-pyridin-2-yl)piperazin-1-yl]-(1-oxa-2,8-diazaspiro[4.5]dec-2-en-3-yl)methanon-hydrochlorid

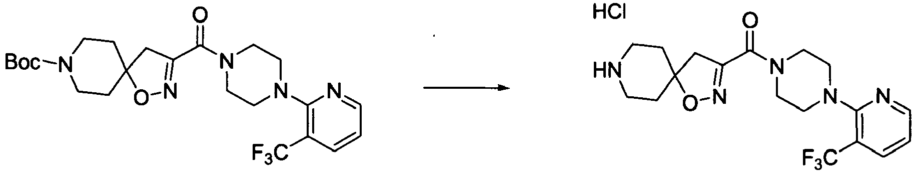

Eine Lösung von 3-[4-(3-Trifluormethyl-pyridin-2-yl)piperazin-1-carbonyl]-1-oxa-2,8-diazaspiro[4.5]dec-2-en-8-carbonsäure-tert-butylester (2.18 g, 4.3 mmol) in abs. MeOH (40 ml) wurde mit 1.25 N HCl in Isopropanol (29 mL, 36 mmol) unter Eiskühlung versetzt und über Nacht bei RT gerührt. Das Lösungsmittel wurde im Vakuum entfernt, der Rückstand in Ether (80 mL) aufgenommen und 20 min bei RT gerührt, wobei das [4-(3-Triflourmethyl-pyridin-2-yl)piperazin-1-yl]-(1-oxa-2,8-diazaspiro[4.5]dec-2-en-3-yl)methanon-hydrochlorid als farbloser Feststoff (1.8 g, 97 %) ausfiel.

### Synthese von 1-Oxa-2,8-diazaspiro[4.5]dec-2-en-3-carbonsäure-(5-methylfuran-2-ylmethyl)amid als Hydrochloridsalz

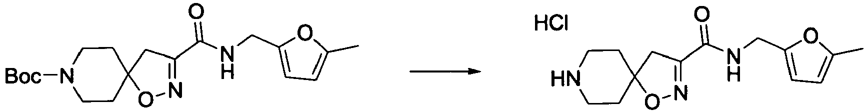

Eine Lösung von 3-[(5-Methylfuran-2-ylmethyl)carbamoyl]-1-oxa-2,8-diazaspiro[4.5]dec-2-en-8-carbonsäure-tert-butylester (9.2 g, 24.3 mmol) in abs. MeOH (300 ml) wurde mit 5.5 N HCl in Isopropanol (88.6 ml, 487.5 mmol) versetzt und 24 h bei RT gerührt. Der Ansatz wurde auf die Hälfte eingeengt, wobei ein farbloser Feststoff ausfiel. Die Suspension wurde 30 min bei RT nachgerührt und der entstandene Feststoff dann abgesaugt. Das Hydrochlorid von 1-Oxa-2,8-diazaspiro[4.5]dec-2-en-3-carbonsäure-(5-methylfuran-2-ylmethyl)amid wurde als farbloser Feststoff (5,3 g, 70 %) mit einem Schmelzpunkt von 218-220 °C erhalten.

### Synthese von 1-Oxa-2,8-diazaspiro[4.5]dec-2-en-3-carbonsäure-4-chlorbenzylamid als Hydrochloridsalz

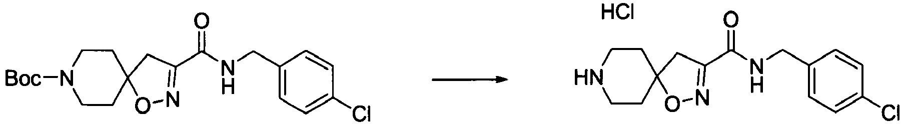

Eine Lösung von 3-(4-Chlorbenzylcarbamoyl)-1-oxa-2,8-diazaspiro[4.5]dec-2-en-8-car-bonsäure-tert-butylester (9,7 g, 23,7 mmol) in abs. MeOH (350 ml) wurde mit 5,5 N HCl in Isopropanol (86.2 ml, 474 mmol) versetzt und 20 h bei RT gerührt. Der Ansatz wurde auf die Hälfte eingeengt, wobei ein farbloser Feststoff ausfiel. Die Suspension wurde 30 min bei RT nachgerührt und dann abgesaugt. Das Hydrochlorid von 1-Oxa-2,8-diazaspiro[4.5]dec-2-en-3-carbonsäure-4-chlorbenzylamid konnte als farbloser Feststoff (5,95 g, 73 %) mit einem Schmelzpunkt von 269-273 °C erhalten werden.

### Synthese von 1-Oxa-2,8-diazaspiro[4.5]dec-2-en-3-carbonsäure-4-hydroxy-3-methoxy-benzylamid als Hydrochloridsalz

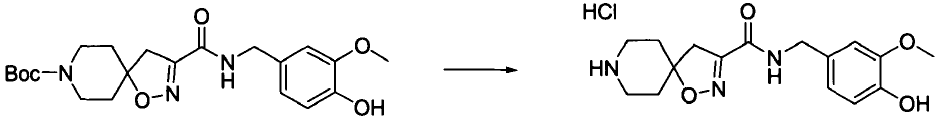

Eine Lösung von 3-(4-Hydroxy-3-methoxybenzylcarbamoyl)-1-oxa-2,8-diazaspiro[4.5]dec-2-en-8-carbonsäure-tert-butylester (8.27 g, 19.7 mmol) in abs. MeOH (650 ml) wurde mit 5.5 N HCl in Isopropanol (71.7 ml, 394.3 mmol) versetzt und 18 h bei RT gerührt. Ein farbloser Feststoff fiel aus und wurde abgesaugt. Der Ansatz wurde eingeengt, wobei nochmals ein farbloser Feststoff ausfiel. Das Hydrochlorid von 1-Oxa-2,8-diazaspiro[4.5]dec-2-en-3-carbonsäure-4-hydroxy-3-methoxy-benzylamid wurde in einer Ausbeute von 68 % (4.75 g) mit einem Schmelzpunkt von 264-269 °C erhalten.

### Stufe 3: Allgemeine Vorschrift zur Umsetzung von Aminen der allgemeinen Formel IVa mit Isocyanaten bzw. Thioisocyanaten der allgemeinen Formel R⁶-N=C=O bzw. R⁸-N=C=S

### a. Manuelle Synthese

Die Amine der allgemeinen Formel IVa (1 Äquivalent) wurden in Toluol (150 Äquivalente) oder DMF (25 Äquivalente) gelöst. Das Isocyanat bzw. Thioisocyanat der allgemeinen Formel R⁶-N=C=O bzw. R⁸-N=C=S (1 Äquivalent) und Triethylamin (1 Äquivalent) wurden hinzu gegeben und die Reaktionsmischung wurde zum Sieden erhitzt. Nach 3 Stunden wurden EtOAc und ges. aq. NaHCO₃-Lösung hinzu gegeben und die Phasen getrennt. Die wässrige Phase wurde mehrfach mit EtOAc extrahiert. Die vereinigten organischen Phasen wurden mit Wasser und ges. aq. NaCl-Lösung gewaschen, über Magnesiumsulfat getrocknet und das Lösungsmittel im Vakuum entfernt. Der Rückstand wurde säulenchromatographisch (Kieselgel, Hexan/EtOAc) getrennt.

Die folgende erfindungsgemäße substituierte Spiro-Verbindung wurde wie unter 3a. beschrieben hergestellt.

### Darstellung von 3-[4-(3-Chlor-pyridin-2-yl)-piperazin-1-carbonyl]-1-oxa-2,8-diaza-spiro[4.5]dec-2-en-8-carbonsäure-(4-tert-butyl-phenyl)-amid

Die Verbindung [4-(3-Chlorpyridin-2-yl)piperazin-1-yl]-(1-oxa-2,8-diazaspiro[4.5]dec-2-en-3-yl)methanon-hydrochlorid (1 g, 2.50 mmol) wurde in Toluol (40 ml) gelöst und mit tert-Butylphenylisocyanat (438 mg, 2.50 mmol) und Triethylamin (350 µl) versetzt. Die Reaktionsmischung wurde 3 Stunden zum Sieden erhitzt. Der ausgefallene Niederschlag wurde abgesaugt, getrocknet und in EtOAc und ges. aq. NaHCO₃-Lösung aufgenommen. Die Phasen wurden getrennt und die wässrige Phase wurde mit EtOAc extrahiert. Die vereinigten organischen Phasen wurden über Magnesiumsuflat getrocknet und das Lösungsmittel im Vakuum entfernt. Der Rückstand wurde getrocknet und säulenchromatographisch (Kieselgel, Hexan/EtOAc 1:1) gereinigt.

### b. Automatisierte Synthese

Es wurden zunächst folgende Stammlösungen erzeugt:
- Lösung I:: 0.05 M Lösung des Amins der allgemeinen Formel IVa in Toluol
- Lösung II:: 0.1 M Lösung des Isocyanats der allgemeinen Formel R⁶-N=C=O bzw. des Isothiocyanats der allgemeinen Formel R⁸-N=C=S in Toluol

Lösung I (2 mL) wurde in einem trockenen Gewindeglas mit Septumkappe bei RT vorgelegt und mit Lösung II (1 mL) versetzt. Das Reaktionsgemisch wurde 6 h im Trefferreaktor unter Rückfluß gerührt. Die Reaktionsmischung wird in Vials überführt und das Lösungsmittel in der GeneVac entfernt.

Die folgenden Isocyanate der allgemeinen Formel R⁶-N=C=O wurden zur Synthese verwendet: Phenylisocyanat, 2-Methylphenylisocyanat, m-Tolylisocyanat, p-Tolylisocyanat, 2-Ethylphenylisocyanat, 3-Ethylphenylisocyanat, 4-Ethylphenylisocyanat, 2-Propylphenylisocyanat, 2-Fluorphenylisocyanat, 3-Fluorphenylisocyanat, 4-Fluorphenylisocyanat, 2-Chlorphenylisocyanat, 3-Chlorphenylisocyanat, 4-Chlorphenylisocyanat, 2-Bromphenylisocyanat, 3-Bromphenylisocyanat,4-Bromphenylisocyanat, 3-lodphenylisocyanat, 4-lodphenylisocyanat, 2-Methoxy-äphenylisocyanat, 3-Methoxyphenylisocyanat, 4-Methoxyphenylisocyanat, 2-Ethoxyphenylisocyanat, 4-Ethoxyphenylisocyanat, 2-(Methylthio)-phenylisocyanat, 3-(Methylthio)-phenylisocyanat, 4-(Methylthio)-phenylisocyanat, 2-Isopropyl-phenylisocyanat, 4-Isopropylphenylisocyanat, 4-Butylphenylisocyanat, 3-Cyanphenylisocyanat, 2-Methoxycarbonylphenylisocyanat, 3-Methoxycarbonylphenylisocyanat, Ethyl-2-isocyanatbenzoat, 3-Ethoxycarbonylphenylisocyanat, 4-Ethoxycarbonylphenylisocyanat, 2-(Trifluormethyl)-phenylisocyanat, 3-(Trifluormethyl)-phenylisocyanat, 4-(Trifluormethyl)-phenylisocyanat.1-Naphthyl-isocyanat, 2-Biphenylisocyanat, 4-Biphenylisocyanat, 2-Phenoxyphenylisocyanat, 4-Phenoxyphenylisocyanat, 4-Benzyloxyphenylisocyanat, 4-(Dimethylamino)-phenylisocyanat, 2,6-Difluorphenylisocyanat, 2,5-Difluorphenylisocyanat, 2,4-Difluorphenylisocyanat, 3,4-Difluorphenylisocyanat, 2,6-Dichlorphenylisocyanat, 2,3-Dichlorphenylisocyanat, 2,5-Dichlorphenylisocyanat, 3,5-Dichlorphenylisocyanat, 2,4-Dichlorphenylisocyanat, 3,4-Dichlorphenylisocyanat, 2,4-Dibromphenylisocyanat, 2-Chlor-5-(trifluormethyl)-phenylisocyanat, 4-Chlor-2-(trifluormethyl)-phenylisocyanat, 4-Chlor-3-(trifluormethyl)-phenylisocyanat, 4-Brom-2-(trifluormethyl)-phenylisocyanat, 3,5-Bis-(trifluormethyl)-phenylisocyanat, 2-(Trifluormethoxy)-phenylisocyanat, 2,4-Dimethoxyphenylisocyanat, 2,5-Dimethoxyphenylisocyanat, 3,5-Dimethoxyphenylisocyanat, 2-Fluor-5-methylphenylisocyanat, 3-Fluor-4-methylphenylisocyanat, 3-Chlor-2-methylphenylisocyanat, 4-Chlor-2-methylphenylisocyanat, 5-Chlor-2-methylphenylisocyanat, 3-Chlor-4-methylphenylisocyanat, 4-Brom-2-methylphenylisocyanat, 3-Chlor-4-fluorphenylisocyanat, 4-Brom-2-fluorphenylisocyanat, 3,5-Dimethylphenylisocyanat, 2,6-Dimethylphenylisocyanat, 3,4-Dimethylphenylisocyanat, 2,5-Dimethylphenylisocyanat, 2,4-Dimethylphenylisocyanat, 2-Ethyl-6-methylphenylisocyanat, 2-Isopropyl-6-methylphenylisocyanat, 2-tert.-Butyl-6-methylphenylisocyanat, 2,6-Diethylphenylisocyanat, 2-Ethyl-6-isopropylphenylisocyanat, 2,6-Diisopropylphenylisocyanat, 4-Methoxy-2-methylphenylisocyanat, 2-Methoxy-5-methylphenylisocyanat, 5-Chlor-2-methoxyphenylisocyanat, 4-Brom-2,6-dimethylphenylisocyanat, 2,4,5-Trichlorphenyl-isocyanat, 2,4-Dibrom-6-fluorphenylisocyanat, 2-Brom-4,6-difluorphenylisocyanat, 5-Chlor-2,4-dimethoxyphenylisocyanat, 3,4,5-Trimethoxyphenylisocyanat, 2,4,5-Trimethylphenylisocyanat, 2,4,6-Trimethylphenylisocyanat, 4-Trifluormethoxyphenylisocyanat, n-Propylisocyanat, n-Butylisocyanat, Cyclohexylisocyanat, 2-Chlorethylisocyanat, 3-Chlorpropylisocyanat, 2-Bromethylisocyanat, Benzylisocyanat, Phenylethylisocyanat, 3-Methylbenzylisocyanat, 4-Methylbenzylisocyanat, 2-Ethylbenzylisocyanat, 3-Ethylbenzylisocyanat, 4-Ethylbenzylisocyanat, 4-Fluorbenzylisocyanat, 2-Chlorbenzylisocyanat, 1-(4-Bromphenyl)ethylisocyanat, 2,4-Dichlorbenzylisocyanat, 3,4-Dichlorbenzylisocyanat, 4-Methoxybenzylisocyanat, 1-(1-Naphthyl)ethylisocyanat, 2-Methylbenzylisocyanat, n-Pentylisocyanat, 4-tert-Butylphenylisocyanat.

Die folgenden Isothiocyanate der allgemeinen Formel R⁸-N=C=S wurden zur Synthese verwendet: Methylisothiocyanat, Ethylisothiocyanat, n-Propylisothiocyanat, n-Butylisothiocyanat, n-Pentylisothiocyanat, n-Hexylisothiocyanat, n-Heptylisothiocyanat, n-Octylisothiocyanat, n-Nonylisothiocyanat, n-Decylisothiocyanat, n-Dodecylisothiocyanat, n-Tetradecylisothiocyanat, n-Octadecylisothiocyanat, Isopropylisothiocyanat, Isobutylisothiocyanat, tert-Butylisothiocyanat, tert.-Amylisothiocyanat, Allylisothiocyanat, Methallylisothiocyanat, Chlormethylthiocyanat, 2-Chlorethylisothiocyanat, 2-Methoxyethylisothiocyanat, 3-Ethoxypropylisothiocyanat, Isothiocyanatoessigsäuremethylester, 2-Isothiocyanatopropionsäuremethylester, Ethyl-3-isothiocyanatopropionat, Ethyl-2-isothiocyanatopropionat, Ethyl-3-isothiocyanatobutyrat, 3-(Diethylamino)propyl-isothiocyanat, Cyclopropylisothiocyanat, Cyclopentylisothiocyanat, Cyclohexylisothiocyanat, Cyclohexylmethyllisothiocyanat, Cyclooctylisothiocyanat, Cyclododecylisothiocyanat, 1-Adamtylisothiocyanat, 2-(Isothiocyanatomethyl)-tetrahydrofuran, 2-(4-Morpholino)ethylisothiocyanat, 3-(4-Morpholino)-propylisothiocyanat, 2-Furylmethylisothiocyanat, Phenylisothiocyanat, 2-Methylphenylisothiocyanat, 3-Methylphenylisothiocyanat, p-Tolylisothiocyanat, 2-Ethylphenylisothiocyanat, Benzylisothiocyanat, 2-Phenylethylisothiocyanat, Alpha-methylbenzylisothiocyanat, 3-Phenylpropylisothiocyanat, 2-Isopropylphenyl-isothiocyanat, 4-Isopropylphenylisothiocyanat, 4-Butylphenylisothiocyanat, 4-Pentafluorsulfanylisothiocyanat, 2-Fluorphenylisothiocyanat, 3-Fluorphenylisothiocyanat, 4-Fluorphenylisothiocyanat, 2-Chlorphenylisothiocyanat, 3-Chlorphenylisothiocyanat, 4-Chlorphenylisothiocyanat, 2-Bromphenylisothiocyanat, 3-Bromphenylisothiocyanat, 4-Bromphenylisothiocyanat, 4-Pentafluorsulfanylisocyanat, 2-lodphenylisothiocyanat, 4-lodphenylisothiocyanat, 4-Fluorbenzylisothiocyanat, 1-(4-Fluorphenyl)ethylisothiocyanat, 2-Chlorbenzylisothiocyanat, 4-Chlorbenzyl-isothiocyanat, 2-(4-Chlorphenyl)ethylisothiocyanat, 2-(Trifluormethyl)-phenylisothiocyanat, 3-(Trifluormethyl)-phenylisothiocyanat, 4-(Trifluormethyl)-phenylisothiocyanat, 2-(Methylthio)phenylisothiocyanat, 3-(Methylthio)phenyl-isothiocyanat, 4-(Methylthio)phenylisothiocyanat, 2-Methoxyphenylisothiocyanat, 3-Methoxyphenylisothiocyanat, 4-Methoxyphenylisothiocyanat, 4-Methoxybenzylisothiocyanat, 2-Nitrophenylisothiocyanat, 3-Nitrophenylisothiocyanat, 4-Nitrophenylisothiocyanat, 3-Pyridylisothiocyanat, 4-Cyanophenylisothiocyanat, 3-Cyanophenylisothiocyanat, (4-Isothiocyanato-phenyl)-dimethylamin, 4-Diethylaminophenylisothiocyanat, 4-Acetylphenylisothiocyanat, 3-Carbonylphenylisothiocyanat, 4-Ethoxyphenylisothiocyanat, 4-Isothiocyanatophenylacetat, 4-Benzyloxyphenylisothiocyanat, 2-Methoxycarbonylphenylisothiocyanat, 3-Methoxycarbonylphenylisothiocyanat, 4-Methoxycarbonylphenylisothiocyanat, Ethyl-2-isothiocyanatobenzoat, 4-Ethoxycarbonylphenylisothiocyanat, 2,4-Dimethylphenylisothiocyanat, 2,6-Dimethylphenylisothiocyanat, 3,5-Dimethylphenylisothiocyanat, 2-Ethyl-6-methylphenylisothiocyanat, 2-Ethyl-6-isopropylphenylisothiocyanat, 2,6-Diethylphenylisothiocyanat, 2,6-Diisopropylphenylisothiocyanat, 2-Chlor-6-methylphenylisothiocyanat, 5-Chlor-2-methylphenylisothiocyanat, 3-Chlor-4-methylphenylisothiocyanat, 4-Chlor-2-methylphenylisothiocyanat, 4-Brom-2-methylphenylisothiocyanat, 2-Brom-4-methylphenylisothiocyanat, 2,4-Difluorphenylisothiocyanat, 2,6-Difluorphenylisothiocyanat, 2,5-Difluorphenylisothiocyanat, 2,3-Dichlorphenylisothiocyanat, 2,6-Dichlorphenylisothiocyanat, 2,5-Dichlorphenylisothiocyanat, 3,4-Dichlorphenylisothiocyanat, 2,4-Dichlorphenylisothiocyanat, 3,5-Dichlorphenylisothiocyanat, 3,4-Dichlorbenzylisothiocyanat, 4-Brom-2-chlorphenylisothiocyanat, 4-Chlor-3-nitrophenylisothiocyanat, 2-Chlor-4-nitrophenylisothiocyanat, 5-Chlor-2-methoxyphenylisothiocyanat, 2-Chloro-5-(trifluormethyl)phenyl-isothiocyanat, 4-Chloro-3-(trifluormethyl)phenylisothiocyanat, 4-Bromo-2-(trifluormethyl)isothiocyanat, 3,5-Bis-(trifluormethyl)-phenylisothiocyanat, 2-Methoxy-5-methylphenylisothiocyanat, 2,5-Dimethoxyphenylisothiocyanat, 2,4-Dimethoxy-phenylisothiocyanat, 3,5-Dimethoxyphenylisothiocyanat, 3,4-Dimethoxy-phenylisothiocyanat, 4-Methoxy-2-nitrophenylisothiocyanat, 2-Methoxy-4-nitrophenylisothiocyanat, 4-Methyl-2-nitrophenylisothiocyanat, (2-Methoxy-5-phenyl)phenylisothiocyanat, 2,4,6-Trifluorphenylisothiocyanat, 2,4,5-Trichlorphenyl-isothiocyanat, 2,4,6-Trichlorphenylisothiocyanat, 2,3,4-Trichlorphenylisothiocyanat, 2,4,6-Tribromphenylisothiocyanat, 2,4,6-Trimethylphenylisothocyanat, 4-Brom-2,6-dimethylphenylisothiocyanat, 3,4,5-Trimethoxyphenylisothiocyanat, 2,3,5,6-Tetrafluorphenylisothiocyanat, 2,3,4,5-Tetrachlorphenylisothiocyanat, Pentaflour-phenylisothiocyanat, 1-Naphthylisothiocyanat, 3,4-Methylendioxobenzylisothiocyanat, Triphenylmethylisothiocyanat, 4-(trans-4-propylcyclohexyl)phenylisothiocyanat, 4-tert.Butylphenylisothiocyanat.

Die folgenden erfindungsgemäßen substituierten Spiro-Verbindungen wurden wie unter 3b. beschrieben hergestellt.

| | Name | [M+H] |
|---|---|---|
| 1 | 3-[4-(3-Chlor-pyridin-2-yl)-piperazin-1-carbonyl]-1-oxa-2,8-diaza-spiro[4.5]dec-2-en-8-carbonsäure-(2-fluor-phenyl)-amid | 502,0 |
| 2 | 1-Oxa-2,8-diaza-spiro[4.5]dec-2-en-3,8-dicarbonsäure-3-[(5-chlor-pyridin-2-yl)-amid]-8-[(2-fluor-phenyl)-amid] | 432,9 |
| 3 | 1-Oxa-2,8-diaza-spiro[4.5]dec-2-en-3,8-dicarbonsäure-3-(4-chlor-benzylamid)-8-[(3-fluor-phenyl)-amid] | 445,9 |
| 4 | 3-[4-(3-Chlor-pyridin-2-yl)-piperazin-1-carbonyl]-1-oxa-2,8-diaza-spiro[4.5]dec-2-en-8-carbonsäure-(3-fluor-phenyl)-amid | 502,0 |
| 5 | 1-Oxa-2,8-diaza-spiro[4.5]dec-2-en-3,8-dicarbonsäure-3-[(5-chlor-pyridin-2-yl)-amid]-8-[(3-fluor-phenyl)-amid] | 432,9 |
| 6 | 1-Oxa-2,8-diaza-spiro[4.5]dec-2-en-3,8-dicarbonsäure 8-[(2-chlor-phenyl)-amid]-3-(3,4-dimethoxy-benzylamid) | 488,0 |
| 7 | 1-Oxa-2,8-diaza-spiro[4.5]dec-2-en-3,8-dicarbonsäure-3-(4-chlor-benzylamid)-8-[(2-chlor-phenyl)-amid] | 462, 3 |
| 8 | 3-[4-(3-Chlor-pyridin-2-yl)-piperazin-1-carbonyl]-1-oxa-2,8-diaza-spiro[4.5]dec-2-en-8-carbonsäure-(2-chlor-phenyl)-amid | 518,4 |
| 9 | 1-Oxa-2,8-diaza-spiro[4.5]dec-2-en-3,8-dicarbonsäure 8-[(2-chlor-phenyl)-amid]-3-[(5-chlor-pyridin-2-yl)-amid] | 449,3 |
| 10 | 1-Oxa-2,8-diaza-spiro[4.5]dec-2-en-3,8-dicarbonsäure 8-[(3-bromo-phenyl)-amid]-3-(3,4-dimethoxy-benzylamid) | 532,4 |
| 11 | 3-[4-(3-Chlor-pyridin-2-yl)-piperazin-1-carbonyl]-1-oxa-2,8-diaza-spiro[4.5]dec-2-en-8-carbonsäure-(3-bromo-phenyl)-amid | 562,9 |
| 12 | 1-Oxa-2,8-diaza-spiro[4.5]dec-2-en-3,8-dicarbonsäure 8-[(3-bromo-phenyl)-amid]-3-[(5-chlor-pyridin-2-yl)-amid] | 493,8 |
| 13 | 1-Oxa-2,8-diaza-spiro[4.5]dec-2-en-3,8-dicarbonsäure-3-(3,4-dimethoxy-benzylamid)-8-[(2-methoxy-phenyl)-amid] | 483,5 |
| 14 | 1-Oxa-2,8-diaza-spiro[4.5]dec-2-en-3,8-dicarbonsäure-3-(4-chlor-benzylamid)-8-[(2-methoxy-phenyl)-amid] | 457,9 |
| 15 | 3-[4-(3-Chlor-pyridin-2-yl)-piperazin-1-carbonyl]-1-oxa-2,8-diaza-spiro[4.5]dec-2-en-8-carbonsäure-(2-methoxy-phenyl)-amid | 514,0 |
| 16 | 1-Oxa-2,8-diaza-spiro[4.5]dec-2-en-3,8-dicarbonsäure-3-(3,4-dimethoxy-benzylamid)-8-[(3-methoxy-phenyl)-amid] | 483, 5 |
| 17 | 3-[4-(3-Chlor-pyridin-2-yl)-piperazin-1-carbonyl]-1-oxa-2,8-diaza-spiro[4.5]dec-2-en-8-carbonsäure-(3-methoxy-phenyl)-amid | 514,0 |
| 18 | 1-Oxa-2,8-diaza-spiro[4.5]dec-2-en-3,8-dicarbonsäure-3-[(5-chlor-pyridin-2-yl)-amid]-8-[(3-methoxy-phenyl)-amid] | 444,9 |
| 19 | 3-[4-(3-Chlor-pyridin-2-yl)-piperazin-1-carbonyl]-1-oxa-2,8-diaza-spiro[4.5]dec-2-en-8-carbonsäure-(4-phenoxy-phenyl)-amid | 576,1 |
| 20 | 1-Oxa-2,8-diaza-spiro[4.5]dec-2-en-3,8-dicarbonsäure 8-[(2-chlor-5-trifluormethyl-phenyl)-amid]-3-(3,4-dimethoxy-benzylamid) | 556,0 |
| 21 | 1-Oxa-2,8-diaza-spiro[4.5]dec-2-en-3,8-dicarbonsäure-3-(4-chlor-benzylamid)-8-[(2-chlor-5-trifluormethyl-phenyl)-amid] | 530,3 |
| 22 | 3-[4-(3-Chlor-pyridin-2-yl)-piperazin-1-carbonyl]-1-oxa-2,8-diaza-spiro[4.5]dec-2-en-8-carbonsäure-(2-chlor-5-trifluormethyl-phenyl)-amid | 586,4 |
| 23 | 1-Oxa-2,8-diaza-spiro[4.5]dec-2-en-3,8-dicarbonsäure-3-[(5-chlor-pyridin-2-yl)-amid]-8-[(2-chlor-5-trifluormethyl-phenyl)-amid] | 517,3 |
| 24 | 1-Oxa-2,8-diaza-spiro[4.5]dec-2-en-3,8-dicarbonsäure 8-[(4-chlor-2-trifluormethyl-phenyl)-amid]-3-(3,4-dimethoxy-benzylamid) | 556,0 |
| 25 | 3-[4-(3-Chlor-pyridin-2-yl)-piperazin-1-carbonyl]-1-oxa-2,8-diaza-spiro[4.5]dec-2-en-8-carbonsäure-(4-chlor-2-trifluormethyl-phenyl)-amid | 586,4 |
| 26 | 1-Oxa-2,8-diaza-spiro[4.5]dec-2-en-3,8-dicarbonsäure-3-[(5-chlor-pyridin-2-yl)-amid]-8-[(4-chlor-2-trifluormethyl-phenyl)-amid] | 517,3 |
| 27 | 1-Oxa-2,8-diaza-spiro[4.5]dec-2-en-3,8-dicarbonsäure 8-[(4-chlor-3-trifluormethyl-phenyl)-amid]-3-(3,4-dimethoxy-benzylamid) | 556,0 |
| 28 | 3-[4-(3-Chlor-pyridin-2-yl)-piperazin-1-carbonyl]-1-oxa-2,8-diaza-spiro[4.5]dec-2-en-8-carbonsäure-(4-chlor-3-trifluormethyl-phenyl)-amid | 586,4 |
| 29 | 1-Oxa-2,8-diaza-spiro[4.5]dec-2-en-3,8-dicarbonsäure-3-[(5-chlor-pyridin-2-yl)-amid]-8-[(4-chlor-3-trifluormethyl-phenyl)-amid] | 517,3 |
| 30 | 1-Oxa-2,8-diaza-spiro[4.5]dec-2-en-3,8-dicarbonsäure-3-[(2-tert-butyl-6-methyl-phenyl)-amid]-8-(4-chlor-benzylamid) | 498,0 |
| 31 | 3-[4-(3-Chlor-pyridin-2-yl)-piperazin-1-carbonyl]-1-oxa-2,8-diaza-spiro[4.5]dec-2-en-8-carbonsäure-(2-tert-butyl-6-methyl-phenyl)-amid | 554,1 |
| 32 | 1-Oxa-2,8-diaza-spiro[4.5]dec-2-en-3,8-dicarbonsäure-3-(3,4-dimethoxy-benzylamid)-8-[(4-trifluormethoxy-phenyl)-amid] | 537,5 |
| 33 | 1-Oxa-2,8-diaza-spiro[4.5]dec-2-en-3,8-dicarbonsäure-3-(4-chlor-benzylamid)-8-[(4-trifluormethoxy-phenyl)-amid] | 511,9 |
| 34 | 3-[4-(3-Chlor-pyridin-2-yl)-piperazin-1-carbonyl]-1-oxa-2,8-diaza-spiro[4.5]dec-2-en-8-carbonsäure-(4-trifluormethoxy-phenyl)-amid | 568,0 |
| 35 | 1-Oxa-2,8-diaza-spiro[4.5]dec-2-en-3,8-dicarbonsäure-3-[(5-chlor-pyridin-2-yl)-amid]-8-[(4-trifluormethoxy-phenyl)-amid] | 498,9 |
| 36 | 1-Oxa-2,8-diaza-spiro[4.5]dec-2-en-3,8-dicarbonsäure-3-(3,4-dimethoxy-benzylamid)-8-(phenethyl-amid) | 481,6 |
| 37 | 1-Oxa-2,8-diaza-spiro[4.5]dec-2-en-3,8-dicarbonsäure-3-(4-chlor-benzylamid)-8-(phenethyl-amid) | 456,0 |
| 38 | 3-[4-(3-Chlor-pyridin-2-yl)-piperazin-1-carbonyl]-1-oxa-2,8-diaza-spiro[4.5]dec-2-en-8-carbonsäurephenethyl-amid | 512,0 |
| 39 | 1-Oxa-2,8-diaza-spiro[4.5]dec-2-en-3,8-dicarbonsäure-3-(3,4-dimethoxy-benzylamid)-8-phenylamid | 453,5 |
| 40 | 3-[4-(3-Chlor-pyridin-2-yl)-piperazin-1-carbonyl]-1-oxa-2,8-diaza-spiro[4.5]dec-2-en-8-carbonsäurephenylamid | 484,0 |
| 41 | 1-Oxa-2,8-diaza-spiro[4.5]dec-2-en-3,8-dicarbonsäure-3-[(5-chlor-pyridin-2-yl)-amid]-8-phenylamid | 414,9 |
| 42 | 1-Oxa-2,8-diaza-spiro[4.5]dec-2-en-3,8-dicarbonsäure-3-(4-chlor-benzylamid)-8-m-tolylamid | 441,9 |
| 43 | 3-[4-(3-Chlor-pyridin-2-yl)-piperazin-1-carbonyl]-1-oxa-2,8-diaza-spiro[4.5]dec-2-en-8-carbonsäure-m-tolylamid | 498,0 |
| 44 | 1-Oxa-2,8-diaza-spiro[4.5]dec-2-en-3,8-dicarbonsäure-3-(3,4-dimethoxy-benzylamid)-8-[(4-fluor-phenyl)-amid] | 471,5 |
| 45 | 3-[4-(3-Chlor-pyridin-2-yl)-piperazin-1-carbonyl]-1-oxa-2,8-diaza-spiro[4.5]dec-2-en-8-carbonsäure-(4-fluor-phenyl)-amid | 502,0 |
| 46 | 1-Oxa-2,8-diaza-spiro[4.5]dec-2-en-3,8-dicarbonsäure 8-[(3-chlor-phenyl)-amid]-3-(3,4-dimethoxy-benzylamid) | 488,0 |
| 47 | 3-[4-(3-Chlor-pyridin-2-yl)-piperazin-1-carbonyl]-1-oxa-2,8-diaza-spiro[4.5]dec-2-en-8-carbonsäure-(3-chlor-phenyl)-amid | 518,4 |
| 48 | 1-Oxa-2,8-diaza-spiro[4.5]dec-2-en-3,8-dicarbonsäure 8-[(3-chlor-phenyl)-amid]-3-[(5-chlor-pyridin-2-yl)-amid] | 449,3 |
| 49 | 1-Oxa-2,8-diaza-spiro[4.5]dec-2-en-3,8-dicarbonsäure 8-[(4-chlor-phenyl)-amid]-3-(3,4-dimethoxy-benzylamid) | 488,0 |
| 50 | 1-Oxa-2,8-diaza-spiro[4.5]dec-2-en-3,8-dicarbonsäure-3-(4-chlor-benzylamid)-8-[(4-chlor-phenyl)-amid] | 462,3 |
| 51 | 3-[4-(3-Chlor-pyridin-2-yl)-piperazin-1-carbonyl]-1-oxa-2,8-diaza-spiro[4.5]dec-2-en-8-carbonsäure-(4-chlor-phenyl)-amid | 518,4 |
| 52 | 3-[4-(3-Chlor-pyridin-2-yl)-piperazin-1-carbonyl]-1-oxa-2,8-diaza-spiro[4.5]dec-2-en-8-carbonsäure-(4-methoxy-phenyl)-amid | 514,0 |
| 53 | 1-Oxa-2,8-diaza-spiro[4.5]dec-2-en-3,8-dicarbonsäure-3-(3,4-dimethoxy-benzylamid)-8-[(2-methylsulfanyl-phenyl)-amid] | 499,6 |
| 54 | 3-[4-(3-Chlor-pyridin-2-yl)-piperazin-1-carbonyl]-1-oxa-2,8-diaza-spiro[4.5]dec-2-en-8-carbonsäure-(2-methylsulfanyl-phenyl)-amid | 530,1 |
| 55 | 1-Oxa-2,8-diaza-spiro[4.5]dec-2-en-3,8-dicarbonsäure-3-(3,4-dimethoxy-benzylamid)-8-[(3-methylsulfanyl-phenyl)-amid] | 499,6 |
| 56 | 3-[4-(3-Chlor-pyridin-2-yl)-piperazin-1-carbonyl]-1-oxa-2,8-diaza-spiro[4.5]dec-2-en-8-carbonsäure-(3-methylsulfanyl-phenyl)-amid | 530,1 |
| 57 | 1-Oxa-2,8-diaza-spiro[4.5]dec-2-en-3,8-dicarbonsäure-3-(3,4-dimethoxy-benzylamid)-8-[(4-methylsulfanyl-phenyl)-amid] | 499,6 |
| 58 | 3-[4-(3-Chlor-pyridin-2-yl)-piperazin-1-carbonyl]-1-oxa-2,8-diaza-spiro[4.5]dec-2-en-8-carbonsäure-(4-methylsulfanyl-phenyl)-amid | 530,1 |
| 59 | 1-Oxa-2,8-diaza-spiro[4.5]dec-2-en-3,8-dicarbonsäure-3-[(5-chlor-pyridin-2-yl)-amid]-8-[(4-methylsulfanyl-phenyl)-amid] | 461,0 |
| 60 | 1-Oxa-2,8-diaza-spiro[4.5]dec-2-en-3,8-dicarbonsäure-3-(3,4-dimethoxy-benzylamid)-8-[(2-isopropyl-phenyl)-amid] | 495,6 |
| 61 | 3-[4(3-Chlor-pyridin-2-yl)-piperazin-1-carbonyl]-1-oxa-2,8-diaza-spiro[4.5]dec-2-en-8-carbonsäure-(2-isopropyl-phenyl)-amid | 526,1 |
| 62 | 1-Oxa-2,8-diaza-spiro[4.5]dec-2-en-3,8-dicarbonsäure-3-[(5-chlor-pyridin-2-yl)-amid]-8-[(2-isopropyl-phenyl)-amid] | 456,9 |
| 63 | 3-[4-(3-Chlor-pyridin-2-yl)-piperazin-1-carbonyl]-1-oxa-2,8-diaza-spiro[4.5]dec-2-en-8-carbonsäure-(4-isopropyl-phenyl)-amid | 526,1 |
| 64 | 1-Oxa-2,8-diaza-spiro[4.5]dec-2-en-3,8-dicarbonsäure-3-(3,4-dimethoxy-benzylamid)-8-[(2-trifluormethyl-phenyl)-amid] | 521,5 |
| 65 | 3-[4-(3-Chlor-pyridin-2-yl)-piperazin-1-carbonyl]-1-oxa-2,8-diaza-spiro[4.5]dec-2-en-8-carbonsäure-(2-trifluormethyl-phenyl)-amid | 552,0 |
| 66 | 1-Oxa-2,8-diaza-spiro[4.5]dec-2-en-3,8-dicarbonsäure-3-[(5-chlor-pyridin-2-yl)-amid]-8-[(2-trifluormethyl-phenyl)-amid] | 482,9 |
| 67 | 1-Oxa-2,8-diaza-spiro[4.5]dec-2-en-3,8-dicarbonsäure-3-(3,4-dimethoxy-benzylamid)-8-[(3-trifluormethyl-phenyl)-amid] | 521,5 |
| 68 | 3-[4-(3-Chlor-pyridin-2-yl)-piperazin-1-carbonyl]-1-oxa-2,8-diaza-spiro[4.5]dec-2-en-8-carbonsäure-(3-trifluormethyl-phenyl)-amid | 552,0 |
| 69 | 1-Oxa-2,8-diaza-spiro[4.5]dec-2-en-3,8-dicarbonsäure-3-[(5-chlor-pyridin-2-yl)-amid]-8-[(3-trifluormethyl-phenyl)-amid] | 482,9 |
| 70 | 1-Oxa-2,8-diaza-spiro[4.5]dec-2-en-3,8-dicarbonsäure-3-(3,4-dimethoxy-benzylamid)-8-[(4-trifluormethyl-phenyl)-amid] | 521,5 |
| 71 | 3-[4-(3-Chlor-pyridin-2-yl)-piperazin-1-carbonyl]-1-oxa-2,8-diaza-spiro[4.5]dec-2-en-8-carbonsäure-(4-trifluormethyl-phenyl)-amid | 552,0 |
| 72 | 1-Oxa-2,8-diaza-spiro[4.5]dec-2-en-3,8-dicarbonsäure-8-cyclohexylamid-3-(3,4-dimethoxy-benzylamid) | 459,6 |
| 73 | 1-Oxa-2,8-diaza-spiro[4.5]dec-2-en-3,8-dicarbonsäure-3-(4-chlor-benzylamid)-8-cyclohexylamid | 434,0 |
| 74 | 1-Oxa-2,8-diaza-spiro[4.5]dec-2-en-3,8-dicarbonsäure-8-benzylamid-3-(3,4-dimethoxy-benzylamid) | 467,5 |
| 75 | 1-Oxa-2,8-diaza-spiro[4.5]dec-2-en-3,8-dicarbonsäure-3-(3,4-dimethoxy-benzylamid)-8-o-tolylamid | 467,5 |
| 76 | 1-Oxa-2,8-diaza-spiro[4.5]dec-2-en-3,8-dicarbonsäure-3-(4-chlor-benzylamid)-8-o-tolylamid | 441,9 |
| 77 | 1-Oxa-2,8-diaza-spiro[4.5]dec-2-en-3,8-dicarbonsäure-3-[(5-chlor-pyridin-2-yl)-amid]-8-o-tolylamid | 428,9 |
| 78 | 1-Oxa-2,8-diaza-spiro[4.5]dec-2-en-3,8-dicarbonsäure-3-(3,4-dimethoxy-benzylamid)-8-[(2-ethyl-phenyl)-amid] | 481,6 |
| 79 | 3-[4-(3-Chlor-pyridin-2-yl)-piperazin-1-carbonyl]-1-oxa-2,8-diaza-spiro[4.5]dec-2-en-8-carbonsäure-(2-ethyl-phenyl)-amid | 512,0 |
| 80 | 1-Oxa-2,8-diaza-spiro[4.5]dec-2-en-3,8-dicarbonsäure-3-[(5-chlor-pyridin-2-yl)-amid]-8-[(2-ethyl-phenyl)-amid] | 442,9 |
| 81 | 1-Oxa-2,8-diaza-spiro[4.5]dec-2-en-3,8-dicarbonsäure-3-(4-chlor-benzylamid)-8-[(4-ethyl-phenyl)-amid] | 456,0 |
| 82 | 1-Oxa-2,8-diaza-spiro[4.5]dec-2-en-3,8-dicarbonsäure-3-[(5-chlor-pyridin-2-yl)-amid]-8-[(4-ethyl-phenyl)-amid] | 442,9 |
| 83 | 1-Oxa-2,8-diaza-spiro[4.5]dec-2-en-3,8-dicarbonsäure-3-(3,4-dimethoxy-benzylamid)-8-[(2-fluor-phenyl)-amid] | 471,5 |
| 84 | 1-Oxa-2,8-diaza-spiro[4.5]dec-2-en-3,8-dicarbonsäure-3-(3,4-dimethoxy-benzylamid)-8-p-tolylamid | 467,5 |
| 85 | 1-Oxa-2,8-diaza-spiro[4.5]dec-2-en-3,8-dicarbonsäure-3-(4-chlor-benzylamid)-8-p-tolylamid | 441,9 |
| 86 | 3-[4-(3-Chlor-pyridin-2-yl)-piperazin-1-carbonyl]-1-oxa-2,8-diaza-spiro[4.5]dec-2-en-8-carbonsäure-p-tolylamid | 498,0 |
| 87 | 1-Oxa-2,8-diaza-spiro[4.5]dec-2-en-3,8-dicarbonsäure-3-[(5-chlor-pyridin-2-yl)-amid]-8-p-tolylamid | 428,9 |
| 88 | 1-Oxa-2,8-diaza-spiro[4.5]dec-2-en-3,8-dicarbonsäure-3-(4-chlor-benzylamid)-8-[(3-ethyl-phenyl)-amid] | 456,0 |
| 89 | 3-[4-(3-Chlor-pyridin-2-yl)-piperazin-1-carbonyl]-1-oxa-2,8-diaza-spiro[4.5]dec-2-en-8-carbonsäure-(3-ethyl-phenyl)-amid | 512,0 |
| 90 | 1-Oxa-2,8-diaza-spiro[4.5]dec-2-en-3,8-dicarbonsäure-3-[(5-chlor-pyridin-2-yl)-amid]-8-[(3-ethyl-phenyl)-amid] | 442,9 |
| 91 | 1-Oxa-2,8-diaza-spiro[4.5]dec-2-en-3,8-dicarbonsäure-3-(3,4-dimethoxy-benzylamid)-8-[(2-propyl-phenyl)-amid] | 495,6 |
| 92 | 1-Oxa-2,8-diaza-spiro[4.5]dec-2-en-3,8-dicarbonsäure-3-(4-chlor-benzylamid)-8-[(2-propyl-phenyl)-amid] | 470,0 |
| 93 | 1-Oxa-2,8-diaza-spiro[4.5]dec-2-en-3,8-dicarbonsäure-3-(4-chlor-benzylamid)-8-[(2-propyl-phenyl)-amid] | 470,0 |
| 94 | 3-[4-(3-Chlor-pyridin-2-yl)-piperazin-1-carbonyl]-1-oxa-2,8-diaza-spiro[4.5]dec-2-en-8-carbonsäure-(2-propyl-phenyl)-amid | 526,1 |
| 95 | 1-Oxa-2,8-diaza-spiro[4.5]dec-2-en-3,8-dicarbonsäure-3-[(5-chlor-pyridin-2-yl)-amid]-8-[(2-propyl-phenyl)-amid] | 456,9 |
| 96 | 1-Oxa-2,8-diaza-spiro[4.5]dec-2-en-3,8-dicarbonsäure 8-[(2-bromo-phenyl)-amid]-3-(3,4-dimethoxy-benzylamid) | 532,4 |
| 97 | 1-Oxa-2,8-diaza-spiro[4.5]dec-2-en-3,8-dicarbonsäure 8-[(2-bromo-phenyl)-amid]-3-(4-chlor-benzylamid) | 506,8 |
| 98 | 3-[4-(3-Chlor-pyridin-2-yl)-piperazin-1-carbonyl]-1-oxa-2,8-diaza-spiro[4.5]dec-2-en-8-carbonsäure-(2-bromo-phenyl)-amid | 562,9 |
| 99 | 3-[4-(3-Chlor-pyridin-2-yl)-piperazin-1-carbonyl]-1-oxa-2,8-diaza-spiro[4.5]dec-2-en-8-carbonsäure-(4-bromo-phenyl)-amid | 562,9 |
| 100 | 3-[4-(3-Chlor-pyridin-2-yl)-piperazin-1-carbonyl]-1-oxa-2,8-diaza-spiro[4.5]dec-2-en-8-carbonsäure-biphenyl-4-ylamid | 560,1 |
| 101 | 1-Oxa-2,8-diaza-spiro[4.5]dec-2-en-3,8-dicarbonsäure-3-(3,4-dimethoxy-benzylamid)-8-[(2-phenoxy-phenyl)-amid] | 545,6 |
| 102 | 3-[4-(3-Chlor-pyridin-2-yl)-piperazin-1-carbonyl]-1-oxa-2,8-diaza-spiro[4.5]dec-2-en-8-carbonsäure-(2-phenoxy-phenyl)-amid | 576,1 |
| 103 | 1-Oxa-2,8-diaza-spiro[4.5]dec-2-en-3,8-dicarbonsäure-3-[(5-chlor-pyridin-2-yl)-amid]-8-[(2-phenoxy-phenyl)-amid] | 507,0 |
| 104 | 1-Oxa-2,8-diaza-spiro[4.5]dec-2-en-3,8-dicarbonsäure-3-(4-chlor-benzylamid)-8-[(2-trifluormethoxy-phenyl)-amid] | 511,9 |
| 105 | 3-[4-(3-Chlor-pyridin-2-yl)-piperazin-1-carbonyl]-1-oxa-2,8-diaza-spiro[4.5]dec-2-en-8-carbonsäure-(2-trifluormethoxy-phenyl)-amid | 568,0 |
| 106 | 1-Oxa-2,8-diaza-spiro[4.5]dec-2-en-3,8-dicarbonsäure-3-[(5-chlor-pyridin-2-yl)-amid]-8-[(2-trifluormethoxy-phenyl)-amid] | 498,9 |
| 107 | 1-Oxa-2,8-diaza-spiro[4.5]dec-2-en-3,8-dicarbonsäure-3-(3,4-dimethoxy-benzylamid)-8-(4-methyl-benzylamid) | 481,6 |
| 108 | 1-Oxa-2,8-diaza-spiro[4.5]dec-2-en-3,8-dicarbonsäure-3-(4-chlor-benzylamid)-8-(4-methyl-benzylamid) | 456,0 |
| 109 | 3-[4-(3-Chlor-pyridin-2-yl)-piperazin-1-carbonyl]-1-oxa-2,8-diaza-spiro[4.5]dec-2-en-8-carbonsäure-4-methyl-benzylamid | 512,0 |
| 110 | 1-Oxa-2,8-diaza-spiro[4.5]dec-2-en-3,8-dicarbonsäure-3-[(5-chlor-pyridin-2-yl)-amid]-8-(4-methyl-benzylamid) | 442,9 |
| 111 | 1-Oxa-2,8-diaza-spiro[4.5]dec-2-en-3,8-dicarbonsäure-3-(3,4-dimethoxy-benzylamid)-8-(4-methoxy-benzylamid) | 497,6 |
| 112 | 1-Oxa-2,8-diaza-spiro[4.5]dec-2-en-3,8-dicarbonsäure-3-(4-chlor-benzylamid)-8-(4-methoxy-benzylamid) | 472,0 |
| 113 | 3-[4-(3-Chlor-pyridin-2-yl)-piperazin-l-carbonyl]-1-oxa-2,8-diaza-spiro[4.5]dec-2-en-8-carbonsäure-4-methoxy-benzylamid | 528,0 |
| 114 | 1-Oxa-2,8-diaza-spiro[4.5]dec-2-en-3,8-dicarbonsäure 8-[(4-tert-butyl-phenyl)-amid]-3-(3,4-dimethoxy-benzylamid) | 509,6 |
| 115 | 1-Oxa-2,8-diaza-spiro[4.5]dec-2-en-3,8-dicarbonsäure 8-[(4-tert-butyl-phenyl)-amid]-3-(4-chlor-benzylamid) | 484,0 |
| 116 | 3-[4-(3-Chlor-pyridin-2-yl)-piperazin-1-carbonyl]-1-oxa-2,8-diaza-spiro[4.5]dec-2-en-8-carbonsäure-(4-tert-butyl-phenyl)-amid | 540,1 |
| 117 | 1-Oxa-2,8-diaza-spiro[4.5]dec-2-en-3,8-dicarbonsäure-8-[(4-tert-butyl-phenyl)-amid]-3-[(5-chlor-pyridin-2-yl)-amid] | 471,0 |
| 118 | 8-(2-Methoxy-phenylthiocarbamoyl)-1-oxa-2,8-diaza-spiro[4.5]dec-2-en-3-carbonsäure-3,4-dimethoxy-benzylamid | 499,6 |
| 119 | 3-[4-(3-Chlor-pyridin-2-yl)-piperazin-1-carbonyl]-1-oxa-2,8-diaza-spiro[4.5]dec-2-en-8-carbonthiosäure-(2-methoxy-phenyl)-amid | 530,1 |
| 120 | 8-(Cyclohexylmethyl-thiocarbamoyl)-1-oxa-2,8-diaza-spiro[4.5]dec-2-en-3-carbonsäure-3,4-dimethoxy-benzylamid | 489, 7 |
| 121 | 8-(Cyclohexylmethyl-thiocarbamoyl)-1-oxa-2,8-diaza-spiro[4.5]dec-2-en-3-carbonsäure-4-chlor-benzylamid | 464,0 |
| 122 | 3-[4-(3-Chlor-pyridin-2-yl)-piperazin-1-carbonyl]-1-oxa-2,8-diaza-spiro[4.5]dec-2-en-8-carbonthiosäure cyclohexylmethyl-amid | 520,1 |
| 123 | 8-Cyclooctylthiocarbamoyl-1-oxa-2,8-diaza-spiro[4.5]dec-2-en-3-carbonsäure-3,4-dimethoxy-benzylamid | 503,7 |
| 124 | 8-Cyclooctylthiocarbamoyl-1-oxa-2,8-diaza-spiro[4.5]dec-2-en-3-carbonsäure-4-chlor-benzylamid | 478,1 |
| 125 | 8-(3-Morpholin-4-yl-propylthiocarbamoyl)-1-oxa-2,8-diaza-spiro[4.5]dec-2-en-3-carbonsäure-4-chlor-benzylamid | 495,1 |
| 126 | 8-(3-Morpholin-4-yl-propylthiocarbamoyl)-1-oxa-2,8-diaza-spiro[4.5]dec-2-en-3-carbonsäure-(5-chlor-pyridin-2-yl)-amid | 482,0 |
| 127 | 8-p-Tolylthiocarbamoyl-1-oxa-2,8-diaza-spiro[4.5]dec-2-en-3-carbonsäure-4-chlor-benzylamid | 458,0 |
| 128 | 8-Phenethylthiocarbamoyl-1-oxa-2,8-diaza-spiro[4.5]dec-2-en-3-carbonsäure-3,4-dimethoxy-benzylamid | 497,6 |
| 129 | 8-Phenethylthiocarbamoyl-1-oxa-2,8-diaza-spiro[4.5]dec-2-en-3-carbonsäure-4-chlor-benzylamid | 472,0 |
| 130 | 8-(3-Phenyl-propylthiocarbamoyl)-1-oxa-2,8-diaza-spiro[4.5]dec-2-en-3-carbonsäure-(5-chlor-pyrid in-2-yl)-amid | 473,0 |
| 131 | 8-(2-fluor-phenylthiocarbamoyl)-1-oxa-2,8-diaza-spiro[4.5]dec-2-en-3-carbonsäure-3,4-dimethoxy-benzylamid | 487,6 |
| 132 | 8-(4-fluor-phenylthiocarbamoyl)-1-oxa-2,8-diaza-spiro[4.5]dec-2-en-3-carbonsäure-3,4-dimethoxy-benzylamid | 487,6 |
| 133 | 8-(4-fluor-phenylthiocarbamoyl)-1-oxa-2,8-diaza-spiro[4.5]dec-2-en-3-carbonsäure-4-chlor-benzylamid | 462,0 |
| 134 | 8-(2-chlor-phenylthiocarbamoyl)-1-oxa-2,8-diaza-spiro[4.5]dec-2-en-3-carbonsäure-3,4-dimethoxy-benzylamid | 504,0 |
| 135 | 8-(2-chlor-phenylthiocarbamoyl)-1-oxa-2,8-diaza-spiro[4.5]dec-2-en-3-carbonsäure-4-chlor-benzylamid | 478,4 |
| 136 | 8-(3-Trifluormethyl-phenylthiocarbamoyl)-1-oxa-2,8-diaza-spiro[4.5]dec-2-en-3-carbonsäure-4-chlor-benzylamid | 512,0 |
| 137 | 8-(Naphthalen-1-ylthiocarbamoyl)-1-oxa-2,8-diaza-spiro[4.5]dec-2-en-3-carbonsäure-3,4-dimethoxy-benzylamid | 519,6 |
| 138 | 8-(Naphthalen-1-ylthiocarbamoyl)-1-oxa-2,8-diaza-spiro[4.5]dec-2-en-3-carbonsäure-3,4-dimethoxy-benzylamid | 519,6 |
| 139 | 8-Cyclopentylthiocarbamoyl-1-oxa-2,8-diaza-spiro[4.5]dec-2-en-3-carbonsäure-3,4-dimethoxy-benzylamid | 461,6 |
| 140 | 3-[4-(3-Chlor-pyridin-2-yl)-piperazin-1-carbonyl]-1-oxa-2,8-diaza-spiro[4.5]dec-2-en-8-carbonthiosäure-cyclopentylamid | 492,1 |
| 141 | 8-(2-Morpholin-4-yl-ethylthiocarbamoyl)-1-oxa-2,8-diaza-spiro[4.5]dec-2-en-3-carbonsäure-3,4-dimethoxy-benzylamid | 506,6 |
| 142 | 8-(2-Morpholin-4-yl-ethylthiocarbamoyl)-1-oxa-2,8-diaza-spiro[4.5]dec-2-en-3-carbonsäure-4-chlor-benzylamid | 481,0 |
| 143 | 8-(3-chlor-phenylthiocarbamoyl)-1-oxa-2,8-diaza-spiro[4.5]dec-2-en-3-carbonsäure-3,4-dimethoxy-benzylamid | 504,0 |
| 144 | 3-[4-(3-Chlor-pyridin-2-yl)-piperazin-1-carbonyl]-1-oxa-2,8-diaza-spiro[4.5]dec-2-en-8-carbonthiosäure-(4-trifluormethyl-phenyl)-amid | 568,0 |
| 145 | 8-(2-Methylsulfanyl-phenylthiocarbamoyl)-1-oxa-2,8-diaza-spiro[4.5]dec-2-en-3-carbonsäure-3,4-dimethoxy-benzylamid | 515, 7 |
| 146 | 8-(2-Methylsulfanyl-phenylthiocarbamoyl)-1-oxa-2,8-diaza-spiro[4.5]dec-2-en-3-carbonsäure-4-chlor-benzylamid | 490,1 |
| 147 | 3-[4-(3-Chlor-pyridin-2-yl)-piperazin-1-carbonyl]-1-oxa-2,8-diaza-spiro[4.5]dec-2-en-8-carbonthiosäure (2-methylsulfanyl-phenyl)-amid | 546,1 |
| 148 | 8-(4-Isopropyl-phenylthiocarbamoyl)-1-oxa-2,8-diaza-spiro[4.5]dec-2-en-3-carbonsäure-3,4-dimethoxy-benzylamid | 511,7 |
| 149 | 8-(4-Isopropyl-phenylthiocarbamoyl)-1-oxa-2,8-diaza-spiro[4.5]dec-2-en-3-carbonsäure-4-chlor-benzylamid | 486,0 |
| 150 | 8-(2-Iodo-phenylthiocarbamoyl)-1-oxa-2,8-diaza-spiro[4.5]dec-2-en-3-carbonsäure-3,4-dimethoxy-benzylamid | 595,5 |
| 151 | 8-(2-Iodo-phenylthiocarbamoyl)-1-oxa-2,8-diaza-spiro[4.5]dec-2-en-3-carbonsäure-4-chlor-benzylamid | 569,9 |
| 152 | 8-(2-Trifluormethyl-phenylthiocarbamoyl)-1-oxa-2,8-diaza-spiro[4.5]dec-2-en-3-carbonsäure-3,4-dimethoxy-benzylamid | 537,6 |
| 153 | 8-(2-Trifluormethyl-phenylthiocarbamoyl)-1-oxa-2,8-diaza-spiro[4.5]dec-2-en-3-carbonsäure-4-chlor-benzylamid | 512,0 |
| 154 | 3-[4-(3-Chlor-pyridin-2-yl)-piperazin-1-carbonyl]-1-oxa-2,8-diaza-spiro[4.5]dec-2-en-8-carbonthiosäure-(2-trifluormethyl-phenyl)-amid | 568,0 |
| 155 | 8-[(Benzo[1,3]dioxol-5-ylmethyl)-thiocarbamoyl]-1-oxa-2,8-diaza-spiro[4.5]dec-2-en-3-carbonsäure-3,4-dimethoxy-benzylamid | 527,6 |
| 156 | 8-[(Benzo[1,3]dioxol-5-ylmethyl)-thiocarbamoyl]-1-oxa-2,8-diaza-spiro[4.5]dec-2-en-3-carbonsäure-4-chlor-benzylamid | 502,0 |
| 157 | 3-[4-(3-Chlor-pyridin-2-yl)-piperazin-1-carbonyl]-1-oxa-2,8-diaza-spiro[4.5]dec-2-en-8-carbonthiosäure-(benzo[1,3]dioxol-5-ylmethyl)-amid | 558,1 |
| 158 | 3-[4-(3-Chlor-pyridin-2-yl)-piperazin-1-carbonyl]-1-oxa-2,8-diaza-spiro[4.5]dec-2-en-8-carbonsäure-(3-cyano-phenyl)-amid | 509,0 |
| 159 | 1-Oxa-2,8-diaza-spiro[4.5]dec-2-en-3,8-dicarbonsäure-3-(3,4-dimethoxy-benzylamid)-8-[(2,5-dimethoxy-phenyl)-amid] | 513,6 |
| 160 | 1-Oxa-2,8-diaza-spiro[4.5]dec-2-en-3,8-dicarbonsäure-3-(4-chlor-benzylamid)-8-[(2,5-dimethoxy-phenyl)-amid] | 488,0 |
| 161 | 3-[4-(3-Chlor-pyridin-2-yl)-piperazin-1-carbonyl]-1-oxa-2,8-diaza-spiro[4.5]dec-2-en-8-carbonsäure-(2, 5-dimethoxy-phenyl)-amid | 544,0 |
| 162 | 1-Oxa-2,8-diaza-spiro[4.5]dec-2-en-3,8-dicarbonsäure-3-(3,4-dimethoxy-benzylamid)-8-[(2,4-dimethyl-phenyl)-amid] | 481,6 |
| 163 | 1-Oxa-2,8-diaza-spiro[4.5]dec-2-en-3,8-dicarbonsäure-3-(4-chlor-benzylamid)-8-[(2,4-dimethyl-phenyl)-amid] | 456,0 |
| 164 | 3-[4-(3-Chlor-pyridin-2-yl)-piperazin-1-carbonyl]-1-oxa-2,8-diaza-spiro[4.5]dec-2-en-8-carbonsäure-(2,4-dimethyl-phenyl)-amid | 512,0 |
| 165 | 1-Oxa-2,8-diaza-spiro[4.5]dec-2-en-3,8-dicarbonsäure-8-butylamid 3-(3,4-dimethoxy-benzylamid) | 433, 5 |
| 166 | 1-Oxa-2,8-diaza-spiro[4.5]dec-2-en-3,8-dicarbonsäure-8-butylamid 3-(4-chlor-benzylamid) | 407,9 |
| 167 | 3-[4-(3-Chlor-pyridin-2-yl)-piperazin-1-carbonyl]-1-oxa-2,8-diaza-spiro[4.5]dec-2-en-8-carbonsäurebutylamid | 464,0 |
| 168 | 1-Oxa-2,8-diaza-spiro[4.5]dec-2-en-3,8-dicarbonsäure-3-(3,4-dimethoxy-benzylamid)-8-pentylamid | 447,5 |
| 169 | 1-Oxa-2,8-diaza-spiro[4.5]dec-2-en-3,8-dicarbonsäure-3-(4-chlor-benzylamid)-8-pentylamid | 421,9 |
| 170 | 3-[4-(3-Chlor-pyridin-2-yl)-piperazin-1-carbonyl]-1-oxa-2,8-diaza-spiro[4.5]dec-2-en-8-carbonsäurepentylamid | 478,0 |
| 171 | 1-Oxa-2,8-diaza-spiro[4.5]dec-2-en-3,8-dicarbonsäure-3-(3,4-dimethoxy-benzylamid)-8-[(2-ethoxy-phenyl)-amid] | 497,6 |
| 172 | 1-Oxa-2,8-diaza-spiro[4.5]dec-2-en-3,8-dicarbonsäure-3-(4-chlor-benzylamid)-8-[(2-ethoxy-phenyl)-amid] | 472,0 |
| 173 | 1-Oxa-2,8-diaza-spiro[4.5]dec-2-en-3,8-dicarbonsäure-3-(3,4-dimethoxy-benzylamid)-8-[(4-ethoxy-phenyl)-amid] | 497,6 |
| 174 | 1-Oxa-2,8-diaza-spiro[4.5]dec-2-en-3,8-dicarbonsäure 8-[(2,4-difluor-phenyl)-amid]-3-(3,4-dimethoxy-benzylamid) | 489, 5 |
| 175 | 1-Oxa-2,8-diaza-spiro[4.5]dec-2-en-3,8-dicarbonsäure-3-(4-chlor-benzylamid)-8-[(2,4-difluor-phenyl)-amid] | 463,9 |
| 176 | 1-Oxa-2,8-diaza-spiro[4.5]dec-2-en-3,8-dicarbonsäure 8-[(3-chlor-2-methyl-phenyl)-amid]-3-(3,4-dimethoxy-benzylamid) | 502,0 |
| 177 | 1-Oxa-2,8-diaza-spiro[4.5]dec-2-en-3,8-dicarbonsäure-3-(4-chlor-benzylamid)-8-[(3-chlor-2-methyl-phenyl)-amid] | 476,4 |
| 178 | 1-Oxa-2,8-diaza-spiro[4.5]dec-2-en-3,8-dicarbonsäure 8-[(4-chlor-2-methyl-phenyl)-amid]-3-(3,4-dimethoxy-benzylamid) | 502,0 |
| 179 | 1-Oxa-2,8-diaza-spiro[4.5]dec-2-en-3,8-dicarbonsäure-3-(4-chlor-benzylamid)-8-[(4-chlor-2-methyl-phenyl)-amid] | 476,4 |
| 180 | 1-Oxa-2,8-diaza-spiro[4.5]dec-2-en-3,8-dicarbonsäure 8-[(3-chlor-4-methyl-phenyl)-amid]-3-(3,4-dimethoxy-benzylamid) | 502,0 |
| 181 | 1-Oxa-2,8-diaza-spiro[4.5]dec-2-en-3,8-dicarbonsäure-3-(4-chlor-benzylamid)-8-[(3-chlor-4-methyl-phenyl)-amid] | 476,4 |
| 182 | 1-Oxa-2,8-diaza-spiro[4.5]dec-2-en-3,8-dicarbonsäure 8-[(3-chlor-4-fluor-phenyl)-amid]-3-(3,4-dimethoxy-benzylamid) | 505,9 |
| 183 | 1-Oxa-2,8-diaza-spiro[4.5]dec-2-en-3,8-dicarbonsäure-3-(4-chlor-benzylamid)-8-[(3-chlor-4-fluor-phenyl)-amid] | 480,3 |
| 184 | 1-Oxa-2,8-diaza-spiro[4.5]dec-2-en-3,8-dicarbonsäure 8-[(2,6-diisopropyl-phenyl)-amid]-3-(3,4-dimethoxy-benzylamid) | 537, 7 |
| 185 | 1-Oxa-2,8-diaza-spiro[4.5]dec-2-en-3,8-dicarbonsäure-3-(4-chlor-benzylamid)-8-[(2,6-diisopropyl-phenyl)-amid] | 512,1 |
| 186 | 1-Oxa-2,8-diaza-spiro[4.5]dec-2-en-3,8-dicarbonsäure 8-[(5-chlor-2-methoxy-phenyl)-amid]-3-(3,4-dimethoxy-benzylamid) | 518,0 |
| 187 | 1-Oxa-2,8-diaza-spiro[4.5]dec-2-en-3,8-dicarbonsäure-3-(4-chlor-benzylamid)-8-[(5-chlor-2-methoxy-phenyl)-amid] | 492,4 |
| 188 | 1-Oxa-2,8-diaza-spiro[4.5]dec-2-en-3,8-dicarbonsäure-3-(4-chlor-benzylamid)-8-[(3,4,5-trimethoxy-phenyl)-amid] | 518,0 |
| 189 | 1-Oxa-2,8-diaza-spiro[4.5]dec-2-en-3,8-dicarbonsäure-3-(3,4-dimethoxy-benzylamid)-8-[(3,5-dimethyl-phenyl)-amid] | 481,6 |
| 190 | 1-Oxa-2,8-diaza-spiro[4.5]dec-2-en-3,8-dicarbonsäure-3-(4-chlor-benzylamid)-8-[(3,5-dimethyl-phenyl)-amid] | 456,0 |
| 191 | 1-Oxa-2,8-diaza-spiro[4.5]dec-2-en-3,8-dicarbonsäure-3-(3,4-dimethoxy-benzylamid)-8-[(2,6-dimethyl-phenyl)-amid] | 481,6 |
| 192 | 1-Oxa-2,8-diaza-spiro[4.5]dec-2-en-3,8-dicarbonsäure-3-(4-chlor-benzylamid)-8-[(2,6-dimethyl-phenyl)-amid] | 456,0 |
| 193 | 1-Oxa-2,8-diaza-spiro[4.5]dec-2-en-3,8-dicarbonsäure-3-(3,4-dimethoxy-benzylamid)-8-[(3,4-dimethyl-phenyl)-amid] | 481,6 |
| 194 | 1-Oxa-2,8-diaza-spiro[4.5]dec-2-en-3,8-dicarbonsäure-3-(3,4-dimethoxy-benzylamid)-8-[(2,5-dimethyl-phenyl)-amid] | 481,6 |
| 195 | 1-Oxa-2,8-diaza-spiro[4.5]dec-2-en-3,8-dicarbonsäure-3-(4-chlor-benzylamid)-8-[(2,5-dimethyl-phenyl)-amid] | 456,0 |
| 196 | 1-Oxa-2,8-diaza-spiro[4.5]dec-2-en-3,8-dicarbonsäure-3-(3,4-dimethoxy-benzylamid)-8-[(2-ethyl-6-methyl-phenyl)-amid] | 495,6 |
| 197 | 1-Oxa-2,8-diaza-spiro[4.5]dec-2-en-3,8-dicarbonsäure-3-(4-chlor-benzylamid)-8-[(2-ethyl-6-methyl-phenyl)-amid] | 470,0 |
| 198 | 1-Oxa-2,8-diaza-spiro[4.5]dec-2-en-3,8-dicarbonsäure-3-(3,4-dimethoxy-benzylamid)-8-[(4-methoxy-2-methyl-phenyl)-amid] | 497,6 |
| 199 | 1-Oxa-2,8-diaza-spiro[4.5]dec-2-en-3,8-dicarbonsäure-3-(4-chlor-benzylamid)-8-[(4-methoxy-2-methyl-phenyl)-amid] | 472,0 |
| 200 | 1-Oxa-2,8-diaza-spiro[4.5]dec-2-en-3,8-dicarbonsäure-3-(3,4-dimethoxy-benzylamid)-8-[(2-methoxy-5-methyl-phenyl)-amid] | 497,6 |
| 201 | 1-Oxa-2,8-diaza-spiro[4.5]dec-2-en-3,8-dicarbonsäure-3-(4-chlor-benzylamid)-8-[(2-methoxy-5-methyl-phenyl)-amid] | 472,0 |
| 202 | 1-Oxa-2,8-diaza-spiro[4.5]dec-2-en-3,8-dicarbonsäure-3-(3,4-dimethoxy-benzylamid)-8-[(2,4,5-trimethyl-phenyl)-amid] | 495,6 |
| 203 | 1-Oxa-2,8-diaza-spiro[4.5]dec-2-en-3,8-dicarbonsäure-3-(4-chlor-benzylamid)-8-[(2,4,5-trimethyl-phenyl)-amid] | 470,0 |
| 204 | 1-Oxa-2,8-diaza-spiro[4.5]dec-2-en-3,8-dicarbonsäure-3-(3,4-dimethoxy-benzylamid)-8-[(2,4,6-trimethyl-phenyl)-amid] | 495,6 |
| 205 | 1-Oxa-2,8-diaza-spiro[4.5]dec-2-en-3,8-dicarbonsäure-3-(4-chlor-benzylamid)-8-[(2,4,6-trimethyl-phenyl)-amid] | 470,0 |
| 206 | 1-Oxa-2,8-diaza-spiro[4.5]dec-2-en-3,8-dicarbonsäure-8-[(2-bromo-ethyl)-amid]-3-(3,4-dimethoxy-benzylamid) | 484,4 |
| 207 | 1-Oxa-2,8-diaza-spiro[4.5]dec-2-en-3,8-dicarbonsäure-8-[(2-bromo-ethyl)-amid]-3-(4-chlor-benzylamid) | 458,8 |
| 208 | 1-Oxa-2,8-diaza-spiro[4.5]dec-2-en-3,8-dicarbonsäure-8-[(4-butyl-phenyl)-amid]-3-(3,4-dimethoxy-benzylamid) | 509,6 |
| 209 | 1-Oxa-2,8-diaza-spiro[4.5]dec-2-en-3,8-dicarbonsäure-8-[(4-butyl-phenyl)-amid]-3-(4-chlor-benzylamid) | 484,0 |
| 210 | 2-{[3-(4-Chlor-benzylcarbamoyl)-1-oxa-2,8-diaza-spiro[4.5]dec-2-en-8-carbonyl]-amino}-benzoesäuremethylester | 486,1 |
| 211 | 1-Oxa-2,8-diaza-spiro[4.5]dec-2-en-3,8-dicarbonsäure-8-biphenyl-2-ylamid 3-(3,4-dimethoxy-benzylamid) | 529,6 |
| 212 | 1-Oxa-2,8-diaza-spiro[4.5]dec-2-en-3,8-dicarbonsäure-8-biphenyl-2-ylamid 3-(4-chlor-benzylamid) | 504,0 |
| 213 | 1-Oxa-2,8-diaza-spiro[4.5]dec-2-en-3,8-dicarbonsäure-8-[(2,6-dichlor-phenyl)-amid]-3-(3,4-dimethoxy-benzylamid) | 522,4 |
| 214 | 1-Oxa-2,8-diaza-spiro[4.5]dec-2-en-3,8-dicarbonsäure-3-(4-chlor-benzylamid)-8-[(2,6-dichlor-phenyl)-amid] | 496,8 |
| 215 | 3-{[3-(3,4-Dimethoxy-benzylcarbamoyl)-1-oxa-2,8-diaza-spiro[4.5]dec-2-en-8-carbonyl]-amino}-benzoesäureethylester | 525,6 |
| 216 | 3-{[3-(4-Chlor-benzylcarbamoyl)-1-oxa-2,8-diaza-spiro[4.5]dec-2-en-8-carbonyl]-amino}-benzoesäureethylester | 500,0 |
| 217 | 4-{[3-(3,4-Dimethoxy-benzylcarbamoyl)-1-oxa-2,8-diaza-spiro[4.5]dec-2-en-8-carbonyl]-amino}-benzoesäureethylester | 525,6 |
| 218 | 4-{[3-(4-Chlor-benzylcarbamoyl)-1-oxa-2,8-diaza-spiro[4.5]dec-2-en-8-carbonyl]-amino}-benzoesäureethylester | 500,0 |
| 219 | 3-[4-(3-Chlor-pyridin-2-yl)-piperazin-1-carbonyl]-1-oxa-2,8-diaza-spiro[4.5]dec-2-en-8-carbonsäure-(2-fluor-phenyl)-amid | 478,0 |
| 220 | 1-Oxa-2,8-diaza-spiro[4.5]dec-2-en-3,8-dicarbonsäure-3-[(5-chlor-pyridin-2-yl)-amid]-8-[(2-fluor-phenyl)-amid] | 602,5 |
| 221 | 1-Oxa-2,8-diaza-spiro[4.5]dec-2-en-3,8-dicarbonsäure-3-(4-chlor-benzylamid)-8-[(3-fluor-phenyl)-amid] | 572,0 |
| 222 | 3-[4-(3-Chlor-pyridin-2-yl)-piperazin-1-carbonyl]-1-oxa-2,8-diaza-spiro[4.5]dec-2-en-8-carbonsäure-(3-fluor-phenyl)-amid | 605,6 |
| 223 | 1-Oxa-2,8-diaza-spiro[4.5]dec-2-en-3,8-dicarbonsäure-3-[(5-chlor-pyridin-2-yl)-amid]-8-[(3-fluor-phenyl)-amid] | 580,0 |
| 224 | 1-Oxa-2,8-diaza-spiro[4.5]dec-2-en-3,8-dicarbonsäure 8-[(2-chlor-phenyl)-amid]-3-(3,4-dimethoxy-benzylamid) | 636,0 |
| 225 | 1-Oxa-2,8-diaza-spiro[4.5]dec-2-en-3,8-dicarbonsäure-3-(4-chlor-benzylamid)-8-[(2-chlor-phenyl)-amid] | 559,8 |
| 226 | 3-[4-(3-Chlor-pyridin-2-yl)-piperazin-1-carbonyl]-1-oxa-2,8-diaza-spiro[4.5]dec-2-en-8-carbonsäure-(2-chlor-phenyl)-amid | 483,6 |
| 227 | 1-Oxa-2,8-diaza-spiro[4.5]dec-2-en-3,8-dicarbonsäure 8-[(2-chlor-phenyl)-amid]-3-[(5-chlor-pyridin-2-yl)-amid] | 458,0 |
| 228 | 1-Oxa-2,8-diaza-spiro[4.5]dec-2-en-3,8-dicarbonsäure 8-[(3-bromo-phenyl)-amid]-3-(3,4-dimethoxy-benzylamid) | 573,6 |
| 229 | 3-[4-(3-Chlor-pyridin-2-yl)-piperazin-1-carbonyl]-1-oxa-2,8-diaza-spiro[4.5]dec-2-en-8-carbonsäure-(4-pentafluorsulfanyl-phenyl)-amid | |
| 230 | N3-((5-methylfuran-2-yl)methyl)-N8-(4-(trifluormethyl)phenyl)-1-oxa-2,8-diazaspiro[4.5]dec-2-en-3,8-dicarboxamid | 465,1 |
| 231 | N8-(4-methoxyphenyl)-N3-((5-methylfuran-2-yl)methyl)-1-oxa-2,8-diazaspiro[4.5]dec-2-en-3,8-dicarboxamid | 427,7 |
| 232 | N8-(4-chlorphenyl)-N3-((5-methylfuran-2-yl)methyl)-1-oxa-2,8-diazaspiro[4.5]dec-2-en-3,8-dicarboxamid | 431,2 |
| 233 | N3-(4-(3-chlorpyridin-2-yl)piperazin-1-yl)-N8-(3,4-dichlorphenyl)-1-oxa-2,8-diazaspiro[4.5]dec-2-en-3,8-dicarboxamid | 566,1 |
| 234 | N3-(4-(3-chlorpyridin-2-yl)piperazin-1-yl)-N8-(4-methoxyphenyl)-1-oxa-2,8-diazaspiro[4.5]dec-2-en-3,8-dicarboxamid | 528,4 |
| 235 | N8-(4-chlorbenzyl)-N3-((5-methylfuran-2-yl)methyl)-1-oxa-2,8-diazaspiro[4.5]dec-2-en-3,8-dicarboxamid | 445,2 |
| 236 | N8-(3,4-dichlorbenzyl)-N3-((5-methylfuran-2-yl)methyl)-1-oxa-2,8-diazaspiro[4.5]dec-2-en-3,8-dicarboxamid | 479,2 |
| 237 | N3-(4-chlorbenzyl)-N8-(3,4-dichlorbenzyl)-1-oxa-2,8-diazaspiro[4.5]dec-2-en-3,8-dicarboxamid | 509, 3 |
| 238 | N8-(3,4-dichlorbenzyl)-N3-(4-hydroxy-3-methoxybenzyl)-1-oxa-2,8-diazaspiro[4.5]dec-2-en-3,8-dicarboxamid | 521,3 |
| 239 | N3-(4-tert-butylbenzyl)-N8-(3,4-dichlorbenzyl)-1-oxa-2,8-diazaspiro[4.5]dec-2-en-3,8-dicarboxamid | 531,2 |
| 240 | N8-(3,4-dichlorbenzyl)-N3-(4-(trifluormethyl)benzyl)-1-oxa-2,8-diazaspiro[4.5]dec-2-en-3,8-dicarboxamid | 543,1 |
| 241 | 3-(4-(3-chlorpyridin-2-yl)piperazin-1-carbonyl)-N-(3,4-dichlorbenzyl)-1-oxa-2,8-diazaspiro[4.5]dec-2-en-8-carboxamid | 565,4 |
| 242 | N3-(4-(3-chlorpyridin-2-yl)piperazin-1-yl)-N8-(3,4-dichlorbenzyl)-1-oxa-2,8-diazaspiro[4.5]dec-2-en-3,8-dicarboxamid | 580,9 |
| 243 | N8-(3,4-dichlorphenyl)-N3-((5-methylfuran-2-yl)methyl)-1-oxa-2,8-diazaspiro[4.5]dec-2-en-3,8-dicarboxamid | 465,2 |
| 244 | N8-(4-chlorphenyl)-N3-(4-hydroxy-3-methoxybenzyl)-1-oxa-2,8-diazaspiro[4.5]dec-2-en-3,8-dicarboxamid | 473, 5 |
| 245 | N8-(3,4-dichlorphenyl)-N3-(4-hydroxy-3-methoxybenzyl)-1-oxa-2,8-diazaspiro[4.5]dec-2-en-3,8-dicarboxamid | 507,6 |
| 246 | N3-(4-chlorbenzyl)-N8-(3,4-dichlorphenyl)-oxa-2,8-diazaspiro[4.5]dec-2-en-3,8-dicarboxamid | 495,6 |
| 247 | N3-(4-chlorbenzyl)-N8-(4-chlorphenyl)-1-oxa-2,8-diazaspiro[4.5]dec-2-en-3,8-dicarboxamid | 461,2 |
| 248 | N3-(4-hydroxy-3-methoxybenzyl)-N8-(4-(trifluormethyl)phenyl)-1-oxa-2,8-diazaspiro[4.5]dec-2-en-3,8-dicarboxamid | 507,3 |
| 249 | N3-(4-hydroxy-3-methoxybenzyl)-N8-(4-methoxyphenyl)-1-oxa-2,8-diazaspiro[4.5]dec-2-en-3,8-dicarboxamid | 469,8 |
| 250 | N3-(4-chlorbenzyl)-N8-(4-(trifluormethyl)phenyl)-1-oxa-2,8-diazaspiro[4.5]dec-2-en-3,8-dicarboxamid | 495,3 |
| 251 | N8-(3,4-dichlorbenzyl)-N3-(3,4-dimethoxybenzyl)-1-oxa-2,8-diazaspiro[4.5]dec-2-en-3,8-dicarboxamid | 535,6 |
| 252 | N3-(3,4-dimethoxybenzyl)-N8-(4-(trifluormethyl)phenyl)-1-oxa-2,8-diazaspiro[4.5]dec-2-en-3,8-dicarboxamid | 521,3 |
| 253 | 3-[4-(3-Trifluormethyl-pyridin-2-yl)-piperazin-1-carbonyl]-1-oxa-2,8-diaza-spiro[4.5]dec-2-en-8-carbonsäure-(4-pentafluorsulfanyl-phenyl)-amid | 643, 3 |
| 254 | N-(4-tert-butylphenyl)-3-(4-(3-chlorpyridin-2-yl)-2-methylpiperazin-1-carbonyl)-1-oxa-2,8-diazaspiro[4.5]dec-2-en-8-carboxamid | 553,2 |
| 255 | N-(4-tert-butylbenzyl)-3-(4-(3-chlorpyridin-2-yl)piperazin-1-carbonyl)-1-oxa-2,8-diazaspiro[4.5]dec-2-en-8-carboxamid | 553,4 |

### Stufe 3: Allgemeine Vorschrift zur Umsetzung von Aminen der allgemeinen Formel IVa mit Aldehyden der allgemeinen Formel R¹-C(=O)-H

Zu einer Lösung der Verbindung der allgemeinen Formel IVa (120 µmol in 0.5 mL MeOH) wurde unter Rühren bei RT der jeweilige Aldehyd der allgemeinen Formel R¹-C(=O)-H (120 µmol in 0.5 mL MeOH) und anschließend Boran-Pyridin-Komplex (BH₃•C₅H₅N, 100 µmol in 0.5 mL MeOH) gegeben. Die Reaktionsmischung wurde wenigstens 16 Stunden bei 64 °C gerührt und anschließend unter Rühren mit 5%-iger (Gewichtsprozent) Salzsäure-Lösung in Wasser (0.5 mL) versetzt. Zu der Reaktionsmischung wurde 10%-ige (Gewichtsprozent) Natriumhydroxid-Lösung in Wasser (1 mL) gegeben und die Mischung wurde dreimal mit DCM (jeweils 2 mL) extrahiert. Die vereinigten organischen Phasen wurden über MgSO₄-Kartuschen getrocknet, das Lösungsmittel im Vakuum entfernt und der Rückstand mittels präparativer HPLC gereinigt, um das gewünschte Produkt der allgemeinen Formel I zu erhalten.

### Pharmakologische Daten

Die Affinität der erfindungsgemäßen Spiro-Verbindungen für den Vanilloid-Rezeptor 1 (VR1/TRPV1-Rezeptor) wurde wie vorstehend beschrieben bestimmt.

| **Verbindung gemäß Beispiel** | **VR1 (Mensch) (% Stimulation im Vgl. zu 10 µM CP)** | **VR1 (Mensch) (% Hemmung im Vgl. zu 10 µM CP)** | **VR1 (Ratte) (% Stimulation im Vgl. zu 10 µM CP)** | **VR1 (Ratte) (% Hemmung im Vgl. zu 10 µM CP)** |
|---|---|---|---|---|
| 1 | 4,85 | 33,56 | 6,37 | 34,23 |
| 9 | -0,48 | 26,23 | 0,23 | 45,63 |
| 11 | -0,15 | 37,28 | 11,45 | 41,27 |
| 16 | 0,40 | 0,77 | 0,00 | 8,65 |
| 17 | 19,54 | 53,69 | 19,83 | 46,89 |
| 18 | 0,67 | 11,17 | 4,68 | 34,24 |
| 22 | -0,66 | 8,37 | 2,79 | 43,32 |
| 28 | 0,08 | 27,56 | 0,63 | 45,91 |
| 34 | -0,13 | 51,91 | 6,21 | 86,21 |
| 51 | 13,15 | 63,38 | 18,63 | 98,80 |
| 52 | 47,48 | 45,14 | 38,53 | 55,84 |
| 56 | 5,26 | 41,94 | 30,25 | 63,33 |
| 58 | 72,92 | 84,90 | 19,54 | 72,54 |
| 61 | 0,08 | -15,08 | 0,47 | 47,05 |
| 63 | 2,59 | 15,81 | 24,47 | 78,90 |
| 68 | 16,35 | 24,87 | 6,55 | 58,09 |
| 70 | -0,18 | -0,43 | 0,35 | 67,41 |
| 71 | 34,68 | 42,03 | 31,24 | 57,20 |
| 116 | 42 | 77 | 59 | 95 |
| 228 | 7,07 | 48,14 | 17,25 | 100,28 |
| 229 | 10 | 37 | 45 | 94 |
| 259 | 2 | 14 | 8 | 94 |
| 261 | 19 | 66 | 70 | 94 |

## Patentansprüche

1. Substituierte Spiro-Verbindungen der allgemeinen Formel I, 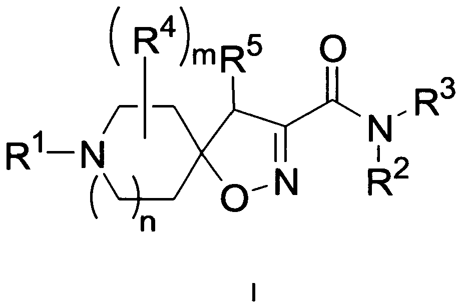 worin
m gleich 0, 1, 2, 3 oder 4 ist,
n gleich 1 ist,
R¹ für eine -C(=S)-NR⁸R⁹-Gruppe steht, oder
sofern m ungleich 0 und/oder R⁵ ungleich H ist, zusätzlich für eine -C(=O)-NR⁶R⁷-Gruppe stehen kann,
R² für einen linearen oder verzweigten, gesättigten oder ungesättigten, unsubstituierten oder wenigstens einfach substituierten, ggf. wenigstens ein Heteroatom als Kettenglied aufweisenden aliphatischen Rest;
wobei die Substituenten des aliphatischen Restes unabhängig voneinander aus der Gruppe bestehend aus F, Cl, Br, I, -CN, -NO₂, - OH, -SH und -NH₂ ausgewählt werden können;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
R³ für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
wobei die Substituenten des aliphatischen Restes unabhängig voneinander aus der Gruppe bestehend aus F, Cl, Br, I, -CN, -NO₂, - OH, -SH und -NH₂ ausgewählt werden können;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
oder
R² und R³ zusammen mit dem sie verbindenden Stickstoffatom einen unsubstituierten oder wenigstens einfach substituierten, ungesättigten oder gesättigten, ggf. wenigstens ein weiteres Heteroatom als Ringglied aufweisenden cycloaliphatischen Rest bilden, der mit einem unsubstituierten oder wenigstens einfach substituierten mono- oder polyzyklischen Ringsystem kondensiert sein kann;
R⁴ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R¹⁰-Gruppe, für eine -NR¹¹R¹²-Gruppe, für eine -C(=O)-R¹³ -Gruppe, für eine -C(=O)-O-R¹⁴-Gruppe, für eine -O-C(=O)-R¹⁵-Gruppe, für eine -NH-C(=O)-R¹⁶-Gruppe, für eine -NR¹⁷-C(=O)-R¹⁸-Gruppe, für eine -C(=O)-NH₂-Gruppe, für eine -C(=O)-NH-R¹⁹-Gruppe, für eine -C(=O)-NR²⁰R²¹-Gruppe, für eine -O-R²²-Gruppe, für eine -S-R²³-Gruppe, oder für einen linearen oder verzweigten, gesättigten oder ungesättigten, unsubstituierten oder wenigstens einfach substituierten aliphatischen Rest steht;
R⁵ für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R¹⁰-Gruppe, für eine -NR¹¹R¹²-Gruppe, für eine -C(=O)-R¹³-Gruppe, für eine -C(=O)-O-R¹⁴-Gruppe, für eine -O-C(=O)-R¹⁵-Gruppe, für eine -NH-C(=O)-R¹⁶-Gruppe, für eine -NR¹⁷-C(=O)-R¹⁸-Gruppe, für eine -C(=O)-NH₂-Gruppe, für eine -C(=O)-NH-R¹⁹-Gruppe, für eine -C(=O)-NR²⁰R²¹-Gruppe, für eine -O-R²²-Gruppe, für eine -S-R²³-Gruppe, für einen linearen oder verzweigten, gesättigten oder ungesättigten, unsubstituierten oder wenigstens einfach substituierten aliphatischen Rest
oder für einen unsubstituierten oder wenigstens einfach substituierten Aryl- oder Heteroaryl-Rest, der über eine lineare oder verzweigte, unsubstituierte oder wenigstens einfach substituierten Alkylen-, Alkenylen- oder Alkinylen-Gruppe gebunden sein kann, steht;
R⁶ und R⁸, unabhängig voneinander, jeweils für
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für eine -C(=O)-R²⁴-Gruppe, die über eine lineare oder verzweigte, unsubstituierte oder wenigstens einfach substituierten Alkylen-Gruppe gebunden sein kann, oder für eine -C(=O)-O-R²⁵-Gruppe, die über eine lineare oder verzweigte, unsubstituierte oder wenigstens einfach substituierten Alkylen-Gruppe gebunden sein kann, stehen;
R⁷ und R⁹, unabhängig voneinander, jeweils für
für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für eine -C(=O)-R²⁴-Gruppe, die über eine lineare oder verzweigte, unsubstituierte oder wenigstens einfach substituierten Alkylen-Gruppe gebunden sein kann,
oder für eine -C(=O)-O-R²⁵-Gruppe, die über eine lineare oder verzweigte, unsubstituierte oder wenigstens einfach substituierten Alkylen-Gruppe gebunden sein kann, stehen;
und
R¹⁰ bis R²⁵, unabhängig voneinander, jeweils für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden sein kann, stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass**
m gleich 0, 1, 2, 3 oder 4 ist,
n gleich 1 ist,
R¹ für eine -C(=S)-NR⁸R⁹-Gruppe, oder
sofern m ungleich 0 und/oder R⁵ ungleich H ist, zusätzlich für eine -C(=O)-NR⁶R⁷-Gruppe stehen kann,
R² für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CHR³⁰)-Xᵥ-(CHR³¹)_{w}-Yₓ-(CHR³²)_{y}-Z_{z}-R³³ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, steht;
R³ für einen Wasserstoff-Rest;
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CHR³⁰)-Xᵥ-(CHR³¹)_{w}-Yₓ-(CHR³²)_{y}-Z_{z}-R³³ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, steht;
oder
R² und R³ zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden, der mit einem gesättigten, ungesättigten oder aromatischen, ggf. substituierten mono- oder polyzyklischen Ringsystem kondensiert sein kann,
R⁴ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R¹⁰-Gruppe, für eine -NR¹¹R¹²-Gruppe, für eine -C(=O)-R¹³ -Gruppe, für eine -C(=O)-O-R¹⁴-Gruppe, für eine -O-C(=O)-R¹⁵-Gruppe, für eine -NH-C(=O)-R¹⁶-Gruppe, für eine -NR¹⁷-C(=O)-R¹⁸-Gruppe, für eine -C(=O)-NH₂-Gruppe, für eine -C(=O)-NH-R¹⁹-Gruppe, für eine -C(=O)-NR²⁰R²¹-Gruppe, für eine -O-R²²-Gruppe, für eine -S-R²³-Gruppe, oder für einen linearen oder verzweigten, gesättigten oder ungesättigten, ggf. substituierten C₁₋₁₀ aliphatischen Rest steht;
R⁵ für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R¹⁰-Gruppe, für eine -NR¹¹R¹²-Gruppe, für eine -C(=O)-R¹³ -Gruppe, für eine -C(=O)-O-R¹⁴-Gruppe, für eine -O-C(=O)-R¹⁵-Gruppe, für eine -NH-C(=O)-R¹⁶-Gruppe, für eine -NR¹⁷-C(=O)-R¹⁸-Gruppe, für eine -C(=O)-NH₂-Gruppe, für eine -C(=O)-NH-R¹⁹-Gruppe, für eine -C(=O)-NR²⁰R²¹-Gruppe, für eine -O-R²²-Gruppe, für eine -S-R²³-Gruppe, für einen linearen oder verzweigten, gesättigten oder ungesättigten, ggf. substituierten C₁₋₁₀ aliphatischen Rest steht;
für einen ggf. substituierten 5- bis 14-gliedrigen Aryl- oder Heteroaryl-Rest
oder für -(CH₂)ₐₐ-R³⁴ mit aa = 1, 2, 3 oder 4 steht;
R⁶ und R⁸, unabhängig voneinander, jeweils
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)_{cc}-C(=O)-R²⁴ mit bb = 0 oder 1 und cc = 0 oder 1;
für -(CHR³⁶)-(CH₂)_{dd}-(CH₂)ₑₑ-C(=O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ⱼⱼ-Zₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
R⁷ und R⁹, unabhängig voneinander, jeweils
für einen Wasserstoff-Rest;
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)_{cc}-C(=O)-R²⁴ mit bb = 0 oder 1 und cc = 0 oder 1;
für -(CHR³⁶)-(CH₂)_{dd}-(CH₂)ₑₑ-C(=O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ⱼⱼ-Zₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
R¹⁰ bis R²⁵, unabhängig voneinander, jeweils
für einen linearen oder verzweigten, gesättigten oder ungesättigten, ggf. substituierten C₁-₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CH₂)ₘₘ-R⁴² mit mm gleich 1, 2 oder 3 stehen;
R²⁶, R²⁷, R²⁸, R³⁰, R³¹, R³², R³⁵, R³⁶, R³⁷, R³⁸, R³⁹ und R⁴⁰, unabhängig voneinander, jeweils
für einen Wasserstoff-Rest;
für einen linearen oder verzweigten, gesättigten oder ungesättigten, ggf. substituierten C₁-₁₀ aliphatischen Rest,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
R²⁹, R³³ und R⁴¹, unabhängig voneinander, jeweils
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit 1, 2, 3, 4 oder 5 linearen oder verzweigten, ggf. substituierten C₁₋₅-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
und
R³⁴ und R⁴², unabhängig voneinander, jeweils
für einen ggf. substituierten 5- bis 14-gliedrigen Aryl- oder Heteroaryl-Rest stehen;
wobei
die vorstehend genannten C₁₋₁₀ aliphatischen Reste und C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, - NO₂, -OH, -SH und -NH₂ substituiert sein können;
die vorstehend genannten cycloaliphatischen Reste und heterocycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, - S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, - O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -C₁₋₅-Alkyl, -O-C₁₋₅-Alkyl, -O-CF₃, -S-CF₃, Phenyl und -0-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die vorstehend genannten heterocycloaliphatischen Reste jeweils ggf. 1, 2 oder 3 zusätzliche(s) Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein können, wobei jeweils der zyklische Teil der Reste Pyridinyl, Cyclopentyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₉-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, - NO₂, -O-CF₃, -S-CF₃, -SH, -S-C₁₋₅-Alkyl, -C₁₋₅-Alkyl, -C(=O)-OH, -C(=O)-O-C₁₅-Alkyl, -O-C(=OFC₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, - S(=O)2-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und
die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, dass**
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl steht, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O=O-C(CH₃)₃, -0-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅. -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, lsoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für -(CHR³⁰)-R³³; -(CHR³⁰)-(CHR³¹)-R³³; -(CHR³⁰)-(CHR³¹)-O-R³³; - (CHR³⁰)-(CHR³¹)-(CHR³²)-R³³; -(CHR³⁰)-(CHR³¹)-S-(CHR³²)-R³³; -(CHR³⁰)-(CHR³¹)-(CHR³²)-N(CH₃)-R³³ steht.

4. Verbindungen gemäß einem oder mehreren der Ansprüche 1 bis 3, **dadurch gekennzeichnet, dass**
R³ für einen Wasserstoff-Rest steht.

5. Verbindungen gemäß einem oder mehreren der Ansprüche 1 bis 4, **dadurch gekennzeichnet, dass**
R² und R³ zusammen mit dem sie verbindenden Stickstoffatom als Ringglied einen Rest bilden ausgewählt aus der Gruppe bestehend aus Pyrrolidinyl, Piperidinyl, (1,3,4,5)-Tetrahydropyrido[4,3-b]indolyl, (3,4)-Dihydro-1 H-isochinolinyl, (1,3,4,9)-Tetrahydro-[b]-carbolinyl, Imidazolidinyl, (1,3)-Thiazolidinyl, Piperazinyl, Morpholinyl, Azepanyl, Diazepanyl und Thiomorpholinyl bilden, wobei der Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann.

6. Verbindungen gemäß einem oder mehreren der Ansprüche 1 bis 5, **dadurch gekennzeichnet, dass**
R⁴ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann.

7. Verbindungen gemäß einem oder mehreren der Ansprüche 1 bis 6, **dadurch gekennzeichnet, dass**
R⁵ für einen Wasserstoff-Rest steht,
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-H, -C(=O)-CH₃, - C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂ und -C(=O)-N-(C₂H₅)₂ substituiert sein kann;
oder für -(CH₂)-R³⁴, -(CH₂)-(CH₂)-R³⁴ oder -(CH₂)-(CH₂)-(CH₂)-R³⁴ steht.

8. Verbindungen gemäß einem oder mehreren der Ansprüche 1 bis 7, **dadurch gekennzeichnet, dass**
R⁶ und R⁸, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, - NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CHR³⁵)-C(=O)-R²⁴ oder -(CHR³⁵)-(CH₂)-C(=O)-R²⁴ stehen;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, (CR³⁷R³⁸)-(CHR³⁹)-(CHR⁴⁰)-R⁴¹, -(CR³⁷R³⁸)-(CHR³⁹)-(CHR⁴⁰)-O-R⁴¹,-(CR³⁷R³⁸)-(CHR³⁹)-(CHR⁴⁰)-N(CH₃)-R⁴¹ oder -(CR³⁷R³⁸)-(CHR³⁹)-(CHR⁴⁰)-N(C₂H₅)-R⁴¹ stehen.

9. Verbindungen gemäß einem oder mehreren der Ansprüche 1 bis 8, **dadurch gekennzeichnet, dass**
R⁷ und R⁹ jeweils für einen Wasserstoff-Rest stehen.

10. Verbindungen gemäß einem oder mehreren der Ansprüche 1 bis 9, **dadurch gekennzeichnet, dass**
R¹⁰ bis R²⁵, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen,
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest jeweils mit 1 oder 2 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, Pyridinyl, Phenyl und Benzyl substituiert sein kann, wobei jeweils der zyklische Teil der Reste Pyridinyl, -S(=O)₂-Phenyl, Phenyl und Benzyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
für einen Rest ausgewählt aus der Gruppe bestehend aus Phenyl, Naphthyl , Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann,
oder für -(CH₂)-R⁴² oder -(CH₂)-(CH₂)-R⁴² stehen.

11. Verbindungen gemäß einem oder mehreren der Ansprüche 1 bis 10, **dadurch gekennzeichnet, dass**
R²⁶, R²⁷, R²⁸, R³⁰, R³¹, R³², R³⁵, R³⁶, R³⁷, R³⁸, R³⁹ und R⁴⁰, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, - O-C(=O)-C(CH₃)₃, -N(CH₃)₂, -N(C₂H₅)₂, -NH-CH₃, -NH-C₂H₅, -C(=O)-CH₃, - C(=O)-C₂H₅, -C(=O)-C(CH₃)₃, Cyclohexyl, Cyclopentyl, -O-Phenyl, -O-Benzyl und Phenyl substituiert sein kann.

12. Verbindungen gemäß einem oder mehreren der Ansprüche 1 bis 11, **dadurch gekennzeichnet, dass**
R²⁹, R³³ und R⁴¹, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl stehen, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-O_{H}, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann.

13. Verbindungen gemäß einem oder mehreren der Ansprüche 1 bis 12, **dadurch gekennzeichnet, dass**
R³⁴ und R⁴², unabhängig voneinander, jeweils für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, - C(=O)-C(CH₃)₃ substituiert sein kann.

14. Verbindungen gemäß einem oder mehreren der Ansprüche 1 bis 13, **dadurch gekennzeichnet, dass**
m gleich 0 ist.

15. Verbindungen gemäß einem oder mehreren der Ansprüche 1-14, **dadurch gekennzeichnet, dass**
m gleich 0, 1, 2, 3 oder 4 ist;
n gleich 1 ist;
R¹ für eine -C(=S)-NR⁸R⁹-Gruppe steht; oder
sofern m ungleich 0 und/oder n ungleich 1 und/oder R⁵ ungleich H ist, zusätzlich für eine -C(=O)-NR⁶R⁷-Gruppe stehen kann,
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl n-Hexyl und n-Heptyl steht; für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht, wobei der Rest jeweils mit 1 oder 2 Substituenten unabhängig voneinander ausgewählt aus der Gruppe bestehend aus Pyridinyl, Pyridazinyl, Phenyl und Benzyl substituiert sein kann, wobei jeweils der zyklische Teil der Reste Pyridinyl, Pyridazinyl, Phenyl und Benzyl mit 1, 2, 3, 4 oder 5 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
für einen Rest ausgewählt aus der Gruppe bestehend aus Phenyl, Naphthyl, Thiophenyl, Furanyl, Pyrrolyl, Pyrazolyl, Pyrazinyl und Pyridinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ und -S(=O)₂-NH-C₂H₅ substituiert sein kann;
oder für -(CHR³⁰)-R³³; -(CHR³⁰)-(CHR³¹)-R³³ oder -(CHR³⁰)-(CHR³¹)-(CHR³²)-R³³ steht;
R³ für einen Wasserstoff-Rest steht;
oder
R² und R³ zusammen mit dem sie verbindenden Stickstoffatom als Ringglied einen Rest bilden ausgewählt aus der Gruppe bestehend aus Pyrrolidinyl, Piperidinyl, Piperazinyl, 3-Methyl-piperazinyl, 2-Methyl-piperazinyl, (3,5)-Dimethylpiperazinyl, (2,6)-Dimethylpiperazinyl, Morpholinyl und Thiomorpholinyl bilden, wobei der Rest jeweils mit 1 oder 2 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, - S(=O)₂-Phenyl, Pyridinyl, Pyridazinyl, Phenyl und Benzyl substituiert sein kann, wobei jeweils der zyklische Teil der Reste Pyridinyl, -S(=O)₂-Phenyl, Pyridazinyl, Phenyl und Benzyl mit 1, 2, 3, 4 oder 5 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
R⁴ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, R⁵ für einen Wasserstoff-Rest,
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht,
für einen Phenyl-Rest steht, der jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl und tert-Butyl substituiert sein kann;
oder für -(CH₂)-R³⁴ steht;
R⁶ und R⁸, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen;
für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, - S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ oder -(CR³⁷R³⁸)-(CHR³⁹)-(CHR⁴⁰)-N(C₂H₅)-R⁴¹ stehen;
R⁷ und R⁹ jeweils für einen Wasserstoff-Rest stehen;
R²⁵ für einen Rest ausgewählt aus der Gruppe bestehend aus Methyl, Ethyl, n-Propyl, Isopropyl, n-Butyl, sec-Butyl, Isobutyl und tert-Butyl steht;
R²⁶, R²⁷, R²⁸, R³⁰, R³¹, R³², R³⁶, R³⁷, R³⁸, R³⁹ und R⁴⁰, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
R²⁹ und R³³, unabhängig voneinander, jeweils
für einen Rest ausgewählt aus der Gruppe bestehend aus Phenyl, Naphthyl, Thiophenyl, Furanyl, Pyrrolyl, Pyrazolyl, Pyrazinyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ und -S(=O)₂-NH-C₂H₅ substituiert sein kann;
R³⁴ für einen Phenyl-Rest steht, der mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, - S-CF₃, -SH, -S-CH₃, -S-C₂H₅, Methyl, Ethyl, n-Propyl, Isopropyl, n-Butyl, sec-Butyl, Isobutyl und tert-Butyl substituiert sein kann;
und
R⁴¹ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, - C(=O)-H, -C(=O)-CH₃, -C(=O)-C₂H₅ und -C(=O)-C(CH₃)₃ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

16. Verbindungen gemäß einem oder mehreren der Ansprüche 1-15, **dadurch gekennzeichnet, dass**
m gleich 0 ist;
n gleich 1 ist;
R¹ für eine -C(=S)-NR⁸R⁹-Gruppe steht;
R² für einen Rest ausgewählt aus der Gruppe bestehend aus Phenyl, Naphthyl und Pyridinyl steht, wobei der Rest jeweils mit 1, 2 oder 3 Substituenten unabhängig voneinander ausgewählt aus der Gruppe bestehend aus F, Cl, Br, -OH, -O-CH₃, -O-C₂H₅, -NH-S(=O)₂-CH₃ und - NH-S(=O)₂-C₂H₅ substituiert sein kann;
für einen Piperazinyl-Rest stehen, der an einem Stickstoffatom mit einem Substituenten ausgewählt aus der Gruppe bestehen aus Pyridinyl, Pyridazinyl, Phenyl und Benzyl substituiert sein kann, wobei jeweils der zyklische Teil der Substituenten Pyridinyl, Pyridazinyl, Phenyl und Benzyl mit 1, 2 oder 3 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl und tert-Butyl substituiert sein kann; oder für -(CHR³⁰)-R³³; -(CHR³⁰)-(CHR³¹)-R³³ oder -(CHR³⁰)-(CHR³¹)-(CHR³²)-R³³ steht;
R³ für einen Wasserstoff-Rest steht;
oder
R² und R³ zusammen mit dem verbindenden Stickstoffatom als Ringglied einen Rest ausgewählt aus der Gruppe bestehend aus 3-Methyl-piperazinyl, 2-Methyl-piperazinyl, (3,5)-Dimethylpiperazinyl, (2,6)-Dimethylpiperazinyl und Piperazinyl bilden, der an einem Stickstoffatom mit einem Substituenten ausgewählt aus der Gruppe bestehen aus Pyridinyl, Pyridazinyl, Phenyl und Benzyl substituiert sein kann, wobei jeweils der zyklische Teil der Substituenten Pyridinyl, Pyridazinyl, Phenyl und Benzyl mit 1, 2 oder 3 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl und tert-Butyl substituiert sein kann;
R⁵ für einen Wasserstoff-Rest,
R⁶ und R⁸, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl und 2-Methyl-1-propenyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl und Adamantyl stehen;
für einen Rest ausgewählt aus der Gruppe bestehend aus Phenyl, Naphthyl und Pyridinyl stehen, wobei der Rest ggf. jeweils mit 1, 2, 3, 4 oder 5 Substituenten unabhängig voneinander ausgewählt aus der Gruppe bestehend aus -SF₅, F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, - C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, - C(=O)-C₂H₅, -C(=O)-C(CH₃)₃, Cyclohexyl, Cyclopentyl, -O-Phenyl, -O-Benzyl und Phenyl substituiert sein kann, wobei jeweils der zyklische Teil der Reste Cyclopentyl, Cyclohexyl, -O-Phenyl, -O-Benzyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ oder -(CR³⁷R³⁸)-(CHR³⁹)-(CHR⁴⁰)-N(C₂H₅)-R⁴¹ stehen;
R⁷ und R⁹ jeweils für einen Wasserstoff-Rest stehen;
R²⁵ für einen Rest ausgewählt aus der Gruppe bestehend aus Methyl, Ethyl, n-Propyl, Isopropyl, n-Butyl, sec-Butyl, Isobutyl und tert-Butyl steht;
R³⁰, R³¹, R³², R³⁹ und R⁴⁰, jeweils für einen Wasserstoff-Rest stehen;
R³³ für einen Rest ausgewählt aus der Gruppe bestehend aus Phenyl, Naphthyl, Thiophenyl und Furanyl steht, wobei der Rest ggf. jeweils mit 1, 2, 3, 4 oder 5 Substituenten unabhängig voneinander ausgewählt aus der Gruppe bestehend aus -CF₃, F, Cl, Br, -OH, -O-CH₃, -O-C₂H₅, Methyl, Ethyl, n-Propyl, Isopropyl, n-Butyl, sec-Butyl, Isobutyl, tert-Butyl, -NH-S(=O)₂-CH₃ und -NH-S(=O)₂-C₂H₅ substituiert sein kann;
R³⁶ für einen Wasserstoff-Rest
oder für einen Rest ausgewählt aus der Gruppe bestehend aus Methyl, Ethyl, n-Propyl, Isopropyl, n-Butyl, sec-Butyl, Isobutyl und tert-Butyl steht;
R³⁷ und R³⁸, unabhängig voneinander, jeweils für
einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
R⁴¹ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Tetrahydrofuranyl, Tetrahydrothiophenyl, Pyrrolidinyl, Morpholinyl, Piperidinyl und Piperazinyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, -OH, -O-CH₃, -O-C₂H₅, Methyl, Ethyl, n-Propyl, Isopropyl, n-Butyl, sec-Butyl, Isobutyl und tert-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

17. Verbindungen gemäß einem oder mehreren der Ansprüche 1 bis 16, **dadurch gekennzeichnet, dass** die Verbindungen im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

18. Verfahren zur Herstellung von substituierten Spiro-Verbindungen der allgemeinen Formel I gemäß einem oder mehreren der Ansprüche 1 bis 17, **dadurch gekennzeichnet, dass** wenigstens eine Verbindung der allgemeinen Formel II, 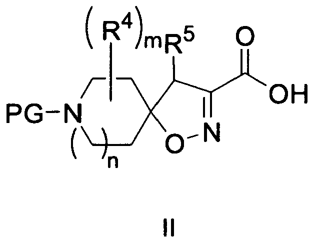 worin R⁴, R⁵, m und n die Bedeutung gemäß einem oder mehreren der Ansprüche 1 bis 17 haben und PG für eine Schutzgruppe, bevorzugt für eine Benzyloxycarbonyl- oder tert-Butyloxycarbonyl-Gruppe, steht, in einem Reaktionsmedium in Gegenwart wenigstens eines Kupplungsreagenzes ggf. in Gegenwart wenigstens einer Base mit einer Verbindung der allgemeinen Formel HNR²R³, worin R² und R³ die Bedeutung gemäß einem oder mehreren der Ansprüche 1 bis 17 haben, zu wenigstens einer Verbindung der allgemeinen Formel III, 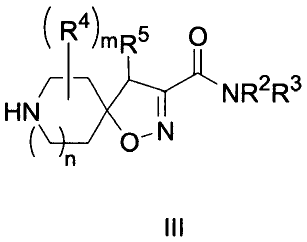 worin R², R³, R⁴, R⁵, m, n und PG die vorstehend genannte Bedeutung haben, umgesetzt wird und diese ggf. gereinigt und/oder isoliert wird
und
wenigstens eine Verbindung der allgemeinen Formel III in einem Reaktionsmedium in Gegenwart wenigstens einer Säure, vorzugsweise in Gegenwart wenigstens einer anorganischen oder organischen Säure ausgewählt aus der Gruppe bestehend aus Salzsäure, Schwefelsäure, Essigsäure und Trifluoressigsäure, oder in Gegenwart von Wasserstoff und eines Katalysators, vorzugsweise eines Katalysators basierend auf Palladium oder Platin, zu wenigstens einer Verbindung der allgemeinen Formel IV, 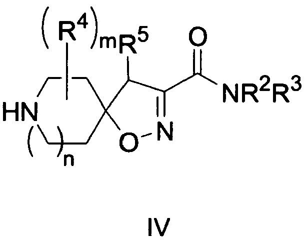 worin R², R³, R⁴, R⁵, m und n die vorstehend genannte Bedeutung haben, umgesetzt wird und diese ggf. gereinigt und/oder isoliert wird
und
wenigstens eine Verbindung der allgemeinen Formel IV in einem Reaktionsmedium mit wenigstens einem Isocyanat der allgemeinen Formel R⁶-N=C=O, worin R⁶ die Bedeutung gemäß einem oder mehreren der Ansprüche 1 bis 17 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R², R³, R⁴, R⁵, m und n die vorstehend genannte Bedeutung haben und R¹ für -C(=O)-NR⁶R⁷ steht, wobei R⁶ die vorstehend genannte Bedeutung hat und R⁷ für einen Wasserstoff-Rest steht, und diese ggf. gereinigt und/oder isoliert wird
oder
wenigstens eine Verbindung der allgemeinen Formel IV in einem Reaktionsmedium mit wenigstens einem Isothiocyanat der allgemeinen Formel S=C=N-R³, worin R⁸ die Bedeutung gemäß einem oder mehreren der Ansprüche 1 bis 17 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R², R³, R⁴, R⁵, m und n die vorstehend genannte Bedeutung haben und R¹ für -C(=S)-N-R⁸R⁹ steht, wobei R⁸ die vorstehend genannte Bedeutung hat und R⁹ für einen Wasserstoff-Rest steht, und diese ggf. gereinigt und/oder isoliert wird
und ggf. wenigstens eine Verbindung der allgemeinen Formel I, worin R², R³, R⁴, R⁵, m und n die vorstehend genannte Bedeutung haben und R¹ für - C(=O)-NR⁶R⁷ oder -C(=S)-N-R⁸R⁹ steht, worin R⁷ und R⁹ jeweils für einen Wasserstoff-Rest stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁷ oder der allgemeinen Formel LG-R⁹, worin LG für eine Abgangsgruppe, bevorzugt für ein HalogenAtom, besonders bevorzugt für ein Chloratom steht, und R⁷ und R⁹ die vorstehend genannte Bedeutung mit Ausnahme von Wasserstoff haben, zu wenigstens einer Verbindung der allgemeinen Formel I umgesetzt wird, worin R¹ bis R⁵, m und n die vorstehend genannte Bedeutung haben, und diese ggf. gereinigt und/oder isoliert wird.

19. Arzneimittel enthaltend wenigstens eine Verbindung gemäß einem oder mehreren der Ansprüche 1 bis 17 und ggf. einen oder mehrere physiologisch verträgliche Hilfsstoffe.

20. Arzneimittel gemäß Anspruch 19 zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

21. Arzneimittel gemäß Anspruch 20 zur zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

22. Verwendung wenigstens einer Verbindung gemäß einem oder mehreren der Ansprüche 1 bis 17 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

23. Verwendung wenigstens einer Verbindung gemäß einem oder mehreren der Ansprüche 1 bis 17 zur Herstellung eines Arzneimittels zur Prophylaxe und/oder Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

24. Verwendung wenigstens einer substituierten Spiro-Verbindungen der allgemeinen Formel I, 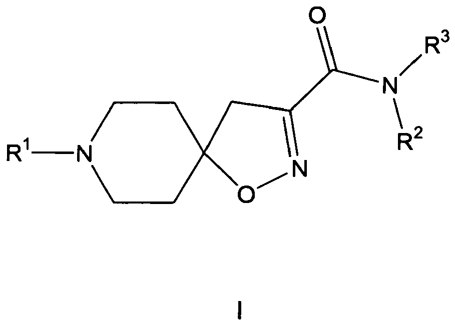 worin
R¹ für eine -C(=O)-NR⁶R⁷-Gruppe steht,
R² für einen linearen oder verzweigten, gesättigten oder ungesättigten, unsubstituierten oder wenigstens einfach substituierten, ggf. wenigstens ein Heteroatom als Kettenglied aufweisenden aliphatischen Rest;
wobei die Substituenten des aliphatischen Restes unabhängig voneinander aus der Gruppe bestehend aus F, Cl, Br, I, -CN, -NO₂, - OH, -SH und -NH₂ ausgewählt werden können;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
R³ für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
wobei die Substituenten des aliphatischen Restes unabhängig voneinander aus der Gruppe bestehend aus F, Cl, Br, I, -CN, -NO₂, - OH, -SH und -NH₂ ausgewählt werden können;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
oder
R² und R³ zusammen mit dem sie verbindenden Stickstoffatom einen unsubstituierten oder wenigstens einfach substituierten, ungesättigten oder gesättigten, ggf. wenigstens ein weiteres Heteroatom als Ringglied aufweisenden cycloaliphatischen Rest bilden, der mit einem unsubstituierten oder wenigstens einfach substituierten mono- oder polyzyklischen Ringsystem kondensiert sein kann;
R⁶ für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für eine -C(=O)-R²⁴-Gruppe, die über eine lineare oder verzweigte, unsubstituierte oder wenigstens einfach substituierten Alkylen-Gruppe gebunden sein kann,
oder für eine -C(=O)-O-R²⁵-Gruppe, die über eine lineare oder verzweigte, unsubstituierte oder wenigstens einfach substituierten Alkylen-Gruppe gebunden sein kann, stehen;
R⁷ für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für eine -C(=O)-R²⁴-Gruppe, die über eine lineare oder verzweigte, unsubstituierte oder wenigstens einfach substituierten Alkylen-Gruppe gebunden sein kann,
oder für eine -C(=O)-O-R²⁵-Gruppe, die über eine lineare oder verzweigte, unsubstituierte oder wenigstens einfach substituierten Alkylen-Gruppe gebunden sein kann, stehen;
und
R²⁴ und R²⁵, unabhängig voneinander, jeweils
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden sein kann, stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate,
zur Herstellung eines Arzneimittels zur Prophylaxe und/oder Behandlung von Störungen oder Krankheiten, die zumindest teilweise durch Vanilloid-Rezeptoren 1 (VR1 /TRPV1) vermittelt werden.

25. Verwendung gemäß Anspruch 24 zur Behandlung und/oder Prophylaxe von einer oder mehreren Erkrankungen ausgewählt aus der Gruppe bestehend aus akutem Schmerz; visceralem Schmerz; Gelenkschmerz; kognitiven Dysfunktionen, vorzugsweise kognitiven Mangelzuständen, besonders bevorzugt Gedächtnisstörungen; Lungenentzündung; Husten; einer überaktiven Blase (overactive bladder, OAB); Reizdarmsyndrom (irritable bowel syndrom); Augenreizungen; Hautreizungen; neurotischen Hauterkrankungen; Entzündungen des Darmes; Toleranzentwicklung gegenüber Medikamenten, vorzugsweise gegenüber natürlichen oder synthetischen Opioiden; und/oder zur Diurese oder zur Antinatriurese und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

26. Verwendung gemäß Anspruch 24 oder 25, **dadurch gekennzeichnet, dass**
R¹ für eine -C(=O)-NR⁶R⁷-Gruppe steht,
R² für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann,
oder für -(CHR³⁰)-Xᵥ-(CHR³¹)_{w}-Yₓ-(CHR³²)_{y}-Z_{z}-R³³ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, steht;
R³ für einen Wasserstoff-Rest;
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CHR³⁰)-Xᵥ-(CHR³¹)_{w}-Yₓ-(CHR³²)_{y}-Z_{z}-R³³ mit v = 0 oder 1, w = 0 oder 1, x =0 oder 1, y =0 oder 1, z =0 oder 1, worin X,Y und Z, unabhängig voneinander, jeweils für O, S, NH, N(CH₃), N(C₂H₅) oder N[CH(CH₃)₂] stehen, steht;
oder
R² und R³ zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden, der mit einem gesättigten, ungesättigten oder aromatischen, ggf. substituierten mono- oder polyzyklischen Ringsystem kondensiert sein kann,
R⁶ für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)_{cc}-C(=O)-R²⁴ mit bb = 0 oder 1 und cc = 0 oder 1;
für -(CHR³⁶)-(CH₂)_{dd}-(CH_{Z})ₑₑ-C(=O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ₖZₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
R⁷ für einen Wasserstoff-Rest;
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)_{cc}-C(=O)-R²⁴ mit bb = 0 oder 1 und cc = 0 oder 1;
für -(CHR³⁶)-(CH₂)_{dd}-(CH₂)ₑₑ-C(=O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ⱼⱼ-Zₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
R²⁴ und R²⁵, unabhängig voneinander, jeweils
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CH₂)ₘₘ-R⁴² mit mm gleich 1, 2 oder 3 stehen;
R³⁰, R³¹, R³², R³⁵, R³⁶, R³⁷, R³⁸, R³⁹ und R⁴⁰, unabhängig voneinander, jeweils
für einen Wasserstoff-Rest;
für einen linearen oder verzweigten, gesättigten oder ungesättigten, ggf. substituierten C₁₋₁₀ aliphatischen Rest,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
R³³ und R⁴¹, unabhängig voneinander, jeweils
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit 1, 2, 3, 4 oder 5 linearen oder verzweigten, ggf. substituierten C₁₋₅-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
und
R³⁴ und R⁴², unabhängig voneinander, jeweils
für einen ggf. substituierten 5- bis 14-gliedrigen Aryl- oder Heteroaryl-Rest stehen;
wobei
die vorstehend genannten C₁₋₁₀ aliphatischen Reste und C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, - NO₂, -OH, -SH und -NH₂ substituiert sein können;
die vorstehend genannten cycloaliphatischen Reste und heterocycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, - S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, - O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -C₁₋₅-Alkyl, -O-C₁₋₅-Alkyl, -O-CF₃, -S-CF₃, Phenyl und -0-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die vorstehend genannten heterocycloaliphatischen Reste jeweils ggf. 1, 2 oder 3 zusätzliche(s) Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein können, wobei jeweils der zyklische Teil der Reste Pyridinyl, Cyclopentyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, - NO₂, -O-CF₃, -S-CF₃, -SH, -S-C₁₋₅-Alkyl, -C₁₋₅-Alkyl, -C(=O)-OH, -C(=O)-O-C₁₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, - S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und
die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

27. Verwendung gemäß einem oder mehreren der Ansprüche 24-26, **dadurch gekennzeichnet, dass**
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl steht, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für -(CHR³⁰)-R³³; -(CHR³⁰)-(CHR³¹)-R³³; -(CHR³⁰)-(CHR³¹)-O-R³³; - (CHR³⁰)-(CHR³¹)-(CHR³²)-R³³; -(CHR³⁰)-(CHR³¹)-S-(CHR³²)-R³³; -(CHR³⁰)-(CHR³¹)-(CHR³²)-N(CH₃)-R³³ steht.

28. Verwendung gemäß einem oder mehreren der Ansprüche 24 bis 27, **dadurch gekennzeichnet, dass**
R³ für einen Wasserstoff-Rest steht.

29. Verwendung gemäß einem oder mehreren der Ansprüche 24 bis 28, **dadurch gekennzeichnet, dass**
R² und R³ zusammen mit dem sie verbindenden Stickstoffatom als Ringglied einen Rest bilden ausgewählt aus der Gruppe bestehend aus Pyrrolidinyl, Piperidinyl, (1,3,4,5)-Tetrahydropyrido[4,3-b]indolyl, (3,4)-Dihydro-1 H-isochinolinyl, (1,3,4,9)-Tetrahydro-[b]-carbolinyl, Imidazolidinyl, (1,3)-Thiazolidinyl, Piperazinyl, Morpholinyl, Azepanyl, Diazepanyl und Thiomorpholinyl bilden, wobei der Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann.

30. Verwendung gemäß einem oder mehreren der Ansprüche 24 bis 29, **dadurch gekennzeichnet, dass**
R⁶ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CHR³⁵)-C(=O)-R²⁴ oder -(CHR³⁵)-(CH₂)-C(=O)-R²⁴ stehen;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, - (CR³⁷R³⁸)-(CHR³⁹)-(CHR⁴⁰)-R⁴¹, -(CR³⁷R³⁸)-(CHR³⁹)-(CHR⁴⁰)-O-R⁴¹, - (CR³⁷R³⁸)-(CHR³⁹)-(CHR⁴⁰)-N(CH₃)-R⁴¹ oder -(CR³⁷R³⁸)-(CHR³⁹)-(CHR⁴⁰)-N(C₂H₅)-R⁴¹ stehen.

31. Verwendung gemäß einem oder mehreren der Ansprüche 24 bis 30, **dadurch gekennzeichnet, dass**
R⁷ für einen Wasserstoff-Rest steht.

32. Verwendung gemäß einem oder mehreren der Ansprüche 24 bis 31, **dadurch gekennzeichnet, dass**
R²⁴ und R²⁵, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen,
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest jeweils mit 1 oder 2 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, Pyridinyl, Phenyl und Benzyl substituiert sein kann, wobei jeweils der zyklische Teil der Reste Pyridinyl, -S(=O)₂-Phenyl, Phenyl und Benzyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
für einen Rest ausgewählt aus der Gruppe bestehend aus Phenyl, Naphthyl , Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann,
oder für -(CH₂)-R⁴² oder -(CH₂)-(CH₂)-R⁴² stehen.

33. Verwendung gemäß einem oder mehreren der Ansprüche 24 bis 32, **dadurch gekennzeichnet, dass**
R³⁰, R³¹, R³², R³⁵, R³⁶, R³⁷, R³⁸, R³⁹ und R⁴⁰, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, - O-C(=O)-C(CH₃)₃, -N(CH₃)₂, -N(C₂H₅)₂, -NH-CH₃, -NH-C₂H₅, -C(=O)-CH₃, - C(=O)-C₂H₅, -C(=O)-C(CH₃)₃, Cyclohexyl, Cyclopentyl, -O-Phenyl, -O-Benzyl und Phenyl substituiert sein kann.

34. Verwendung gemäß einem oder mehreren der Ansprüche 24 bis 33, **dadurch gekennzeichnet, dass**
R³³ und R⁴¹, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl stehen, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann.

35. Verwendung gemäß einem oder mehreren der Ansprüche 24 bis 34, **dadurch gekennzeichnet, dass**
R³⁴ und R⁴², unabhängig voneinander, jeweils für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, - C(=O)-C(CH₃)₃ substituiert sein kann.

36. Verwendung gemäß einem oder mehreren der Ansprüche 24 bis 35, **dadurch gekennzeichnet, dass**
R¹ für eine -C(=O)-NR⁶R⁷-Gruppe steht;
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht, wobei der Rest jeweils mit 1 oder 2 Substituenten unabhängig voneinander ausgewählt aus der Gruppe bestehend aus Pyridinyl, Pyridazinyl, Phenyl und Benzyl substituiert sein kann, wobei jeweils der zyklische Teil der Reste Pyridinyl, Pyridazinyl, Phenyl und Benzyl mit 1, 2, 3, 4 oder 5 Substituenten unabhängig voneinander ausgewählt aus der Gruppe bestehend aus - CF₃, F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
für einen Rest ausgewählt aus der Gruppe bestehend aus Phenyl, Naphthyl, Thiophenyl, Furanyl, Pyrrolyl, Pyrazolyl, Pyrazinyl und Pyridinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ und -S(=O)₂-NH-C₂H₅ substituiert sein kann;
oder für -(CHR³⁰)-R³³; -(CHR³⁰)-(CHR³¹)-R³³ oder -(CHR³⁰)-(CHR³¹)-(CHR³²)-R³³ steht;
R³ für einen Wasserstoff-Rest steht;
oder
R² und R³ zusammen mit dem sie verbindenden Stickstoffatom als Ringglied einen Rest bilden ausgewählt aus der Gruppe bestehend aus Pyrrolidinyl, Piperidinyl, Piperazinyl, 3-Methylpiperazinyl, 2-Methyl-piperazinyl, (3,5)-Dimethylpiperazinyl, (2,6)-Dimethylpiperazinyl, Morpholinyl und Thiomorpholinyl bilden, wobei der Rest jeweils mit 1 oder 2 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, - S(=O)₂-Phenyl, Pyridinyl, Pyridazinyl, Phenyl und Benzyl substituiert sein kann, wobei jeweils der zyklische Teil der Reste Pyridinyl, -S(=O)₂-Phenyl, Pyridazinyl, Phenyl und Benzyl mit 1, 2, 3, 4 oder 5 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
R⁶ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen;
für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, - S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ oder -(CR³⁷R³⁸)-(CHR³⁹)-(CHR⁴⁰)-N(C₂H₅)-R⁴¹ stehen;
R⁷ für einen Wasserstoff-Rest steht;
R²⁵ für einen Rest ausgewählt aus der Gruppe bestehend aus Methyl, Ethyl, n-Propyl, Isopropyl, n-Butyl, sec-Butyl, Isobutyl und tert-Butyl steht;
R³⁰, R³¹, R³², R³⁶, R³⁷, R³⁸, R³⁹ und R⁴⁰, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
R³³ für einen Rest ausgewählt aus der Gruppe bestehend aus Phenyl, Naphthyl, Thiophenyl, Furanyl, Pyrrolyl, Pyrazolyl, Pyrazinyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, **-**O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ und -S(=O)₂-NH-C₂H₅ substituiert sein kann;
und
R⁴¹ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, - C(=O)-H, -C(=O)-CH₃, -C(=O)-C₂H₅ und -C(=O)-C(CH₃)₃ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

37. Verwendung gemäß einem oder mehreren der Ansprüche 24 bis 36, **dadurch gekennzeichnet, dass**
R¹ für eine -C(=O)-NR⁶R⁷-Gruppe steht;
R² für einen Rest ausgewählt aus der Gruppe bestehend aus Phenyl, Naphthyl und Pyridinyl steht, wobei der Rest jeweils mit 1, 2 oder 3 Substituenten unabhängig voneinander ausgewählt aus der Gruppe bestehend aus F, Cl, Br, -OH, -O-CH₃, -O-C₂H₅, -NH-S(=O)₂-CH₃ und - NH-S(=O)₂-C₂H₅ substituiert sein kann;
für einen Piperazinyl-Rest steht, der an einem Stickstoffatom mit einem Substituenten ausgewählt aus der Gruppe bestehen aus Pyridinyl, Pyridazinyl, Phenyl und Benzyl substituiert sein kann, wobei jeweils der zyklische Teil der Substituenten Pyridinyl, Pyridazinyl, Phenyl und Benzyl mit 1, 2 oder 3 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl und tert-Butyl substituiert sein kann;
oder für -(CHR³ )-R³³; -(CHR³⁰)-(CHR³¹)-R³³ oder -(CHR³⁰)-(CHR³¹)-(CHR³²)-R³³ steht;
R³ für einen Wasserstoff-Rest steht;
oder
R² und R³ zusammen mit dem verbindenden Stickstoffatom als Ringglied einen Rest ausgewählt aus der Gruppe bestehend aus 3-Methylpiperazinyl, 2-Methyl-piperazinyl, (3,5)-Dimethylpiperazinyl, (2,6)-Dimethylpiperazinyl und Piperazinyl bilden, der an einem Stickstoffatom mit einem Substituenten ausgewählt aus der Gruppe bestehen aus Pyridinyl, Pyridazinyl, Phenyl und Benzyl substituiert sein kann, wobei jeweils der zyklische Teil der Substituenten Pyridinyl, Pyridazinyl, Phenyl und Benzyl mit 1, 2 oder 3 Substituenten unabhängig voneinander ausgewählt aus der Gruppe bestehend aus -CF₃, F, Cl, Br, Methyl, Ethyl, n-Propyl, Isopropyl, n-Butyl, sec-Butyl, Isobutyl und tert-Butyl substituiert sein kann;
R⁶ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl und 2-Methyl-1-propenyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl und Adamantyl stehen;
für einen Rest ausgewählt aus der Gruppe bestehend aus Phenyl, Naphthyl und Pyridinyl stehen, wobei der Rest ggf. jeweils mit 1, 2, 3, 4 oder 5 Substituenten unabhängig voneinander ausgewählt aus der Gruppe bestehend aus -SF₅, F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, - C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, - C(=O)-C₂H₅, -C(=O)-C(CH₃)₃, Cyclohexyl, Cyclopentyl, -O-Phenyl, -O-Benzyl und Phenyl substituiert sein kann, wobei jeweils der zyklische Teil der Reste Cyclopentyl, Cyclohexyl, -O-Phenyl, -O-Benzyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ oder -(CR³⁷R³⁸)-(CHR³⁹)-(CHR⁴⁰)-N(C₂H₅)-R⁴¹ stehen;
R⁷ für einen Wasserstoff-Rest steht;
R²⁵ für einen Rest ausgewählt aus der Gruppe bestehend aus Methyl, Ethyl, n-Propyl, Isopropyl, n-Butyl, sec-Butyl, Isobutyl und tert-Butyl steht;
R³⁰, R³¹, R³², R³⁹ und R⁴⁰, jeweils für einen Wasserstoff-Rest stehen;
R³³ für einen Rest ausgewählt aus der Gruppe bestehend aus Phenyl, Naphthyl, Thiophenyl und Furanyl steht, wobei der Rest ggf. jeweils mit 1, 2, 3, 4 oder 5 Substituenten unabhängig voneinander ausgewählt aus der Gruppe bestehend aus -CF₃, F, Cl, Br, -OH, -O-CH₃, -O-C₂H₅, Methyl, Ethyl, n-Propyl, Isopropyl, n-Butyl, sec-Butyl, Isobutyl, tert-Butyl, -NH-S(=O)₂-CH₃ und -NH-S(=O)₂-C₂H₅ substituiert sein kann;
R³⁶ für einen Wasserstoff-Rest
oder für einen Rest ausgewählt aus der Gruppe bestehend aus Methyl, Ethyl, n-Propyl, Isopropyl, n-Butyl, sec-Butyl, Isobutyl und tert-Butyl steht;
R³⁷ und R³⁸, unabhängig voneinander, jeweils für
einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
R⁴¹ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Tetrahydrofuranyl, Tetrahydrothiophenyl, Pyrrolidinyl, Morpholinyl, Piperidinyl und Piperazinyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, -OH, -O-CH₃, -O-C₂H₅, Methyl, Ethyl, n-Propyl, Isopropyl, n-Butyl, sec-Butyl, Isobutyl und tert-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

38. Verwendung gemäß einem oder mehreren der Ansprüche 24 bis 37, **dadurch gekennzeichnet, dass** die Spiro-Verbindung ausgewählt ist aus der Gruppe bestehend aus
[1] 3-[4-(3-Chlor-pyridin-2-yl)-piperazin-1-carbonyl]-1-oxa-2,8-diaza-spiro[4.5]dec-2-en-8-carbonsäure-(2-fluor-phenyl)-amid
[2] 1-Oxa-2,8-diaza-spiro[4.5]dec-2-en-3,8-dicarbonsäure-3-[(5-chlor-pyridin-2-yl)-amid]-8-[(2-fluor-phenyl)-amid]
[3] 1-Oxa-2,8-diaza-spiro[4.5]dec-2-en-3,8-dicarbonsäure-3-(4-chlor-benzylamid)-8-[(3-fluor-phenyl)-amid]
[4] 3-[4-(3-Chlor-pyridin-2-yl)-piperazin-1-carbonyl]-1-oxa-2,8-diaza-spiro[4.5]dec-2-en-8-carbonsäure-(3-fluor-phenyl)-amid
[5] 1-Oxa-2,8-diaza-spiro[4.5]dec-2-en-3,8-dicarbonsäure-3-[(5-chlor-pyridin-2-yl)-amid]-8-[(3-fluor-phenyl)-amid]
[6] 1-Oxa-2,8-diaza-spiro[4.5]dec-2-en-3,8-dicarbonsäure 8-[(2-chlor-phenyl)-amid]-3-(3,4-dimethoxy-benzylamid)
[7] 1-Oxa-2,8-diaza-spiro[4.5]dec-2-en-3,8-dicarbonsäure-3-(4-chlor-benzylamid)-8-[(2-chlor-phenyl)-amid]
[8] 3-[4-(3-Chlor-pyridin-2-yl)-piperazin-1-carbonyl]-1-oxa-2,8-diaza-spiro[4.5]dec-2-en-8-carbonsäure-(2-chlor-phenyl)-amid
[9] 1-Oxa-2,8-diaza-spiro[4.5]dec-2-en-3,8-dicarbonsäure 8-[(2-chlor-phenyl)-amid]-3-[(5-chlor-pyridin-2-yl)-amid]
[10] 1-Oxa-2,8-diaza-spiro[4.5]dec-2-en-3,8-dicarbonsäure 8-[(3-bromo-phenyl)-amid]-3-(3,4-dimethoxy-benzylamid)
[11] 3-[4-(3-Chlor-pyridin-2-yl)-piperazin-1-carbonyl]-1-oxa-2,8-diaza-spiro[4.5]dec-2-en-8-carbonsäure-(3-bromo-phenyl)-amid
[12] 1-Oxa-2,8-diaza-spiro[4.5]dec-2-en-3,8-dicarbonsäure 8-[(3-bromo-phenyl)-amid]-3-[(5-chlor-pyridin-2-yl)-amid]
[13] 1-Oxa-2,8-diaza-spiro[4.5]dec-2-en-3,8-dicarbonsäure-3-(3,4-dimethoxy-benzylamid)-8-[(2-methoxy-phenyl)-amid]
[14] 1-Oxa-2,8-diaza-spiro[4.5]dec-2-en-3,8-dicarbonsäure-3-(4-chlor-benzylamid)-8-[(2-methoxy-phenyl)-amid]
[15] 3-[4-(3-Chlor-pyridin-2-yl)-piperazin-1-carbonyl]-1-oxa-2,8-diaza-spiro[4.5]dec-2-en-8-carbonsäure-(2-methoxy-phenyl)-amid
[16] 1-Oxa-2,8-diaza-spiro[4.5]dec-2-en-3,8-dicarbonsäure-3-(3,4-dimethoxy-benzylamid)-8-[(3-methoxy-phenyl)-amid]
[17] 3-[4-(3-Chlor-pyridin-2-yl)-piperazin-1-carbonyl]-1-oxa-2,8-diaza-spiro[4.5]dec-2-en-8-carbonsäure-(3-methoxy-phenyl)-amid
[18] 1-Oxa-2,8-diaza-spiro[4.5]dec-2-en-3,8-dicarbonsäure-3-[(5-chlor-pyridin-2-yl)-amid]-8-[(3-methoxy-phenyl)-amid]
[19] 3-[4-(3-Chlor-pyridin-2-yl)-piperazin-1-carbonyl]-1-oxa-2,8-diaza-spiro[4.5]dec-2-en-8-carbonsäure-(4-phenoxy-phenyl)-amid
[20] 1-Oxa-2,8-diaza-spiro[4.5]dec-2-en-3,8-dicarbonsäure 8-[(2-chlor-5-trifluormethyl-phenyl)-amid]-3-(3,4-dimethoxy-benzylamid)
[21] 1-Oxa-2,8-diaza-spiro[4.5]dec-2-en-3,8-dicarbonsäure-3-(4-chlor-benzylamid)-8-[(2-chlor-5-trifluormethyl-phenyl)-amid]
[22] 3-[4-(3-Chlor-pyridin-2-yl)-piperazin-1-carbonyl]-1-oxa-2,8-diaza-spiro[4.5]dec-2-en-8-carbonsäure-(2-chlor-5-trifluormethyl-phenyl)-amid
[23] 1-Oxa-2,8-diaza-spiro[4.5]dec-2-en-3,8-dicarbonsäure-3-[(5-chlor-pyridin-2-yl)-amid]-8-[(2-chlor-5-trifluormethyl-phenyl)-amid]
[24] 1-Oxa-2,8-diaza-spiro[4.5]dec-2-en-3,8-dicarbonsäure 8-[(4-chlor-2-trifluormethyl-phenyl)-amid]-3-(3,4-dimethoxy-benzylamid)
[25] 3-[4-(3-Chlor-pyridin-2-yl)-piperazin-1-carbonyl]-1-oxa-2,8-diaza-spiro[4.5]dec-2-en-8-carbonsäure-(4-chlor-2-trifluormethyl-phenyl)-amid
[26] 1-Oxa-2,8-diaza-spiro[4.5]dec-2-en-3,8-dicarbonsäure-3-[(5-chlor-pyridin-2-yl)-amid]-8-[(4-chlor-2-trifluormethyl-phenyl)-amid]
[27] 1-Oxa-2,8-diaza-spiro[4.5]dec-2-en-3,8-dicarbonsäure 8-[(4-chlor-3-trifluormethyl-phenyl)-amid]-3-(3,4-dimethoxy-benzylamid)
[28] 3-[4-(3-Chlor-pyridin-2-yl)-piperazin-1-carbonyl]-1-oxa-2,8-diaza-spiro[4.5]dec-2-en-8-carbonsäure-(4-chlor-3-trifluormethyl-phenyl)-amid
[29] 1-Oxa-2,8-diaza-spiro[4.5]dec-2-en-3,8-dicarbonsäure-3-[(5-chlor-pyridin-2-yl)-amid]-8-[(4-chlor-3-trifluormethyl-phenyl)-amid]
[30] 1-Oxa-2,8-diaza-spiro[4.5]dec-2-en-3,8-dicarbonsäure-3-[(2-tert-butyl-6-methyl-phenyl)-amid]-8-(4-chlor-benzylamid)
[31] 3-[4-(3-Chlor-pyridin-2-yl)-piperazin-1-carbonyl]-1-oxa-2,8-diaza-spiro[4.5]dec-2-en-8-carbonsäure-(2-tert-butyl-6-methyl-phenyl)-amid
[32] 1-Oxa-2,8-diaza-spiro[4.5]dec-2-en-3,8-dicarbonsäure-3-(3,4-dimethoxy-benzylamid)-8-[(4-trifluormethoxy-phenyl)-amid]
[33] 1-Oxa-2,8-diaza-spiro[4.5]dec-2-en-3,8-dicarbonsäure-3-(4-chlor-benzylamid)-8-[(4-trifluormethoxy-phenyl)-amid]
[34] 3-[4-(3-Chlor-pyridin-2-yl)-piperazin-1-carbonyl]-1-oxa-2,8-diaza-spiro[4.5]dec-2-en-8-carbonsäure-(4-trifluormethoxy-phenyl)-amid
[35] 1-Oxa-2,8-diaza-spiro[4.5]dec-2-en-3,8-dicarbonsäure-3-[(5-chlor-pyridin-2-yl)-amid]-8-[(4-trifluormethoxy-phenyl)-amid]
[36] 1-Oxa-2,8-diaza-spiro[4.5]dec-2-en-3,8-dicarbonsäure-3-(3,4-dimethoxy-benzylamid)-8-(phenethyl-amid)
[37] 1-Oxa-2,8-diaza-spiro[4.5]dec-2-en-3,8-dicarbonsäure-3-(4-chlor-benzylamid)-8-(phenethyl-amid)
[38] 3-[4-(3-Chlor-pyridin-2-yl)-piperazin-1-carbonyl]-1-oxa-2,8-diaza-spiro[4.5]dec-2-en-8-carbonsäurephenethyl-amid
[39] 1-Oxa-2,8-diaza-spiro[4.5]dec-2-en-3,8-dicarbonsäure-3-(3,4-dimethoxy-benzylamid)-8-phenylamid
[40] 3-[4-(3-Chlor-pyridin-2-yl)-piperazin-1-carbonyl]-1-oxa-2,8-diaza-spiro[4.5]dec-2-en-8-carbonsäurephenylamid
[41] 1-Oxa-2,8-diaza-spiro[4.5]dec-2-en-3,8-dicarbonsäure-3-[(5-chlor-pyridin-2-yl)-amid]-8-phenylamid
[42] 1-Oxa-2,8-diaza-spiro[4.5]dec-2-en-3,8-dicarbonsäure-3-(4-chlor-benzylamid)-8-m-tolylamid
[43] 3-[4-(3-Chlor-pyridin-2-yl)-piperazin-1-carbonyl]-1-oxa-2,8-diaza-spiro[4.5]dec-2-en-8-carbonsäure-m-tolylamid
[44] 1-Oxa-2,8-diaza-spiro[4.5]dec-2-en-3,8-dicarbonsäure-3-(3,4-dimethoxy-benzylamid)-8-[(4-fluor-phenyl)-amid]
[45] 3-[4-(3-Chlor-pyridin-2-yl)-piperazin-1-carbonyl]-1-oxa-2,8-diaza-spiro[4.5]dec-2-en-8-carbonsäure-(4-fluor-phenyl)-amid
[46] 1-Oxa-2,8-diaza-spiro[4.5]dec-2-en-3,8-dicarbonsäure 8-[(3-chlorphenyl)-amid]-3-(3,4-dimethoxy-benzylamid)
[47] 3-[4-(3-Chlor-pyridin-2-yl)-piperazin-1-carbonyl]-1-oxa-2,8-diaza-spiro[4.5]dec-2-en-8-carbonsäure-(3-chlor-phenyl)-amid
[48] 1-Oxa-2,8-diaza-spiro[4.5]dec-2-en-3,8-dicarbonsäure 8-[(3-chlor-phenyl)-amid]-3-[(5-chlor-pyridin-2-yl)-amid]
[49] 1-Oxa-2,8-diaza-spiro[4.5]dec-2-en-3,8-dicarbonsäure 8-[(4-chlor-phenyl)-amid]-3-(3,4-dimethoxy-benzylamid)
[50] 1-Oxa-2,8-diaza-spiro[4.5]dec-2-en-3,8-dicarbonsäure-3-(4-chlor-benzylamid)-8-[(4-chlor-phenyl)-amid]
[51] 3-[4-(3-Chlor-pyridin-2-yl)-piperazin-1-carbonyl]-1-oxa-2,8-diaza-spiro[4.5]dec-2-en-8-carbonsäure-(4-chlor-phenyl)-amid
[52] 3-[4-(3-Chlor-pyridin-2-yl)-piperazin-1-carbonyl]-1-oxa-2,8-diaza-spiro[4.5]dec-2-en-8-carbonsäure-(4-methoxy-phenyl)-amid
[53] 1-Oxa-2,8-diaza-spiro[4.5]dec-2-en-3,8-dicarbonsäure-3-(3,4-dimethoxy-benzylamid)-8-[(2-methylsulfanyl-phenyl)-amid]
[54] 3-[4-(3-Chlor-pyridin-2-yl)-piperazin-1-carbonyl]-1-oxa-2,8-diaza-spiro[4.5]dec-2-en-8-carbonsäure-(2-methylsulfanyl-phenyl)-amid
[55] 1-Oxa-2,8-diaza-spiro[4.5]dec-2-en-3,8-dicarbonsäure-3-(3,4-dimethoxy-benzylamid)-8-[(3-methylsulfanyl-phenyl)-amid]
[56] 3-[4-(3-Chlor-pyridin-2-yl)-piperazin-1-carbonyl]-1-oxa-2,8-diaza-spiro[4.5]dec-2-en-8-carbonsäure-(3-methylsulfanyl-phenyl)-amid
[57] 1-Oxa-2,8-diaza-spiro[4.5]dec-2-en-3,8-dicarbonsäure-3-(3,4-dimethoxy-benzylamid)-8-[(4-methylsulfanyl-phenyl)-amid]
[58] 3-[4-(3-Chlor-pyridin-2-yl)-piperazin-1-carbonyl]-1-oxa-2,8-diaza-spiro[4.5]dec-2-en-8-carbonsäure-(4-methylsulfanyl-phenyl)-amid
[59] 1-Oxa-2,8-diaza-spiro[4.5]dec-2-en-3,8-dicarbonsäure-3-[(5-chlor-pyridin-2-yl)-amid]-8-[(4-methylsulfanyl-phenyl)-amid]
[60] 1-Oxa-2,8-diaza-spiro[4.5]dec-2-en-3,8-dicarbonsäure-3-(3,4-dimethoxy-benzylamid)-8-[(2-isopropyl-phenyl)-amid]
[61] 3-[4-(3-Chlor-pyridin-2-yl)-piperazin-1-carbonyl]-1-oxa-2,8-diaza-spiro[4.5]dec-2-en-8-carbonsäure-(2-isopropyl-phenyl)-amid
[62] 1-Oxa-2,8-diaza-spiro[4.5]dec-2-en-3,8-dicarbonsäure-3-[(5-chlor-pyridin-2-yl)-amid]-8-[(2-isopropyl-phenyl)-amid]
[63] 3-[4-(3-Chlor-pyridin-2-yl)-piperazin-1-carbonyl]-1-oxa-2,8-diaza-spiro[4.5]dec-2-en-8-carbonsäure-(4-isopropyl-phenyl)-amid
[64] 1-Oxa-2,8-diaza-spiro[4.5]dec-2-en-3,8-dicarbonsäure-3-(3,4-dimethoxy-benzylamid)-8-[(2-trifluormethyl-phenyl)-amid]
[65] 3-[4-(3-Chlor-pyridin-2-yl)-piperazin-1-carbonyl]-1-oxa-2,8-diaza-spiro[4.5]dec-2-en-8-carbonsäure-(2-trifluormethyl-phenyl)-amid
[66] 1-Oxa-2,8-diaza-spiro[4.5]dec-2-en-3,8-dicarbonsäure-3-[(5-chlor-pyridin-2-yl)-amid]-8-[(2-trifluormethyl-phenyl)-amid]
[67] 1-Oxa-2,8-diaza-spiro[4.5]dec-2-en-3,8-dicarbonsäure-3-(3,4-dimethoxy-benzylamid)-8-[(3-trifluormethyl-phenyl)-amid]
[68] 3-[4-(3-Chlor-pyridin-2-yl)-piperazin-1-carbonyl]-1-oxa-2,8-diaza-spiro[4.5]dec-2-en-8-carbonsäure-(3-trifluormethyl-phenyl)-amid
[69] 1-Oxa-2,8-diaza-spiro[4.5]dec-2-en-3,8-dicarbonsäure-3-[(5-chlor-pyridin-2-yl)-amid]-8-[(3-trifluormethyl-phenyl)-amid]
[70] 1-Oxa-2,8-diaza-spiro[4.5]dec-2-en-3,8-dicarbonsäure-3-(3,4-dimethoxy-benzylamid)-8-[(4-trifluormethyl-phenyl)-amid]
[71] 3-[4-(3-Chlor-pyridin-2-yl)-piperazin-1-carbonyl]-1-oxa-2,8-diaza-spiro[4.5]dec-2-en-8-carbonsäure-(4-trifluormethyl-phenyl)-amid
[72] 1-Oxa-2,8-diaza-spiro[4.5]dec-2-en-3,8-dicarbonsäure-8-cyclohexylamid-3-(3,4-dimethoxy-benzylamid)
[73] 1-Oxa-2,8-diaza-spiro[4.5]dec-2-en-3,8-dicarbonsäure-3-(4-chlor-benzylamid)-8-cyclohexylamid
[74] 1-Oxa-2,8-diaza-spiro[4.5]dec-2-en-3,8-dicarbonsäure-8-benzylamid-3-(3,4-dimethoxy-benzylamid)
[75] 1-Oxa-2,8-diaza-spiro[4.5]dec-2-en-3,8-dicarbonsäure-3-(3,4-dimethoxy-benzylamid)-8-o-tolylamid
[76] 1-Oxa-2,8-diaza-spiro[4.5]dec-2-en-3,8-dicarbonsäure-3-(4-chlor-benzylamid)-8-o-tolylamid
[77] 1-Oxa-2,8-diaza-spiro[4.5]dec-2-en-3,8-dicarbonsäure-3-[(5-chlor-pyridin-2-yl)-amid]-8-o-tolylamid
[78] 1-Oxa-2,8-diaza-spiro[4.5]dec-2-en-3,8-dicarbonsäure-3-(3,4-dimethoxy-benzylamid)-8-[(2-ethyl-phenyl)-amid]
[79] 3-[4-(3-Chlor-pyridin-2-yl)-piperazin-1-carbonyl]-1-oxa-2,8-diaza-spiro[4.5]dec-2-en-8-carbonsäure-(2-ethyl-phenyl)-amid
[80] 1-Oxa-2,8-diaza-spiro[4.5]dec-2-en-3,8-dicarbonsäure-3-[(5-chlor-pyridin-2-yl)-amid]-8-[(2-ethyl-phenyl)-amid]
[81] 1-Oxa-2,8-diaza-spiro[4.5]dec-2-en-3,8-dicarbonsäure-3-(4-chlor-benzylamid)-8-[(4-ethyl-phenyl)-amid]
[82] 1-Oxa-2,8-diaza-spiro[4.5]dec-2-en-3,8-dicarbonsäure-3-[(5-chlor-pyridin-2-yl)-amid]-8-[(4-ethyl-phenyl)-amid]
[83] 1-Oxa-2,8-diaza-spiro[4.5]dec-2-en-3,8-dicarbonsäure-3-(3,4-dimethoxy-benzylamid)-8-[(2-fluor-phenyl)-amid]
[84] 1-Oxa-2,8-diaza-spiro[4.5]dec-2-en-3,8-dicarbonsäure-3-(3,4-dimethoxy-benzylamid)-8-p-tolylamid
[85] 1-Oxa-2,8-diaza-spiro[4.5]dec-2-en-3,8-dicarbonsäure-3-(4-chlor-benzylamid)-8-p-tolylamid
[86] 3-[4-(3-Chlor-pyridin-2-yl)-piperazin-1-carbonyl]-1-oxa-2,8-diaza-spiro[4.5]dec-2-en-8-carbonsäure-p-tolylamid
[87] 1-Oxa-2,8-diaza-spiro[4.5]dec-2-en-3,8-dicarbonsäure-3-[(5-chlor-pyridin-2-yl)-amid]-8-p-tolylamid
[88] 1-Oxa-2,8-diaza-spiro[4.5]dec-2-en-3,8-dicarbonsäure-3-(4-chlor-benzylamid)-8-[(3-ethyl-phenyl)-amid]
[89] 3-[4-(3-Chlor-pyridin-2-yl)-piperazin-1-carbonyl]-1-oxa-2,8-diaza-spiro[4.5]dec-2-en-8-carbonsäure-(3-ethyl-phenyl)-amid
[90] 1-Oxa-2,8-diaza-spiro[4.5]dec-2-en-3,8-dicarbonsäure-3-[(5-chlor-pyridin-2-yl)-amid]-8-[(3-ethyl-phenyl)-amid]
[91] 1-Oxa-2,8-diaza-spiro[4.5]dec-2-en-3,8-dicarbonsäure-3-(3,4-dimethoxy-benzylamid)-8-[(2-propyl-phenyl)-amid]
[92] 1-Oxa-2,8-diaza-spiro[4.5]dec-2-en-3,8-dicarbonsäure-3-(4-chlor-benzylamid)-8-[(2-propyl-phenyl)-amid]
[93] 1-Oxa-2,8-diaza-spiro[4.5]dec-2-en-3,8-dicarbonsäure-3-(4-chlor-benzylamid)-8-[(2-propyl-phenyl)-amid]
[94] 3-[4-(3-Chlor-pyridin-2-yl)-piperazin-1-carbonyl]-1-oxa-2,8-diaza-spiro[4.5]dec-2-en-8-carbonsäure-(2-propyl-phenyl)-amid
[95] 1-Oxa-2,8-diaza-spiro[4.5]dec-2-en-3,8-dicarbonsäure-3-[(5-chlor-pyridin-2-yl)-amid]-8-[(2-propyl-phenyl)-amid]
[96] 1-Oxa-2,8-diaza-spiro[4.5]dec-2-en-3,8-dicarbonsäure 8-[(2-bromo-phenyl)-amid]-3-(3,4-dimethoxy-benzylamid)
[97] 1-Oxa-2,8-diaza-spiro[4.5]dec-2-en-3,8-dicarbonsäure 8-[(2-bromo-phenyl)-amid]-3-(4-chlor-benzylamid)
[98] 3-[4-(3-Chlor-pyridin-2-yl)-piperazin-1-carbonyl]-1-oxa-2,8-diaza-spiro[4.5]dec-2-en-8-carbonsäure-(2-bromo-phenyl)-amid
[99] 3-[4-(3-Chlor-pyridin-2-yl)-piperazin-1-carbonyl]-1-oxa-2,8-diaza-spiro[4.5]dec-2-en-8-carbonsäure-(4-bromo-phenyl)-amid
[100] 3-[4-(3-Chlor-pyridin-2-yl)-piperazin-1-carbonyl]-1-oxa-2,8-diaza-spiro[4.5]dec-2-en-8-carbonsäure-biphenyl-4-ylamid
[101] 1-Oxa-2,8-diaza-spiro[4.5]dec-2-en-3,8-dicarbonsäure-3-(3,4-dimethoxy-benzylamid)-8-[(2-phenoxy-phenyl)-amid]
[102] 3-[4-(3-Chlor-pyridin-2-yl)-piperazin-1-carbonyl]-1-oxa-2,8-diaza-spiro[4.5]dec-2-en-8-carbonsäure-(2-phenoxy-phenyl)-amid
[103] 1-Oxa-2,8-diaza-spiro[4.5]dec-2-en-3,8-dicarbonsäure-3-[(5-chlor-pyridin-2-yl)-amid]-8-[(2-phenoxy-phenyl)-amid]
[104] 1-Oxa-2,8-diaza-spiro[4.5]dec-2-en-3,8-dicarbonsäure-3-(4-chlor-benzylamid)-8-[(2-trifluormethoxy-phenyl)-amid]
[105] 3-[4-(3-Chlor-pyridin-2-yl)-piperazin-1-carbonyl]-1-oxa-2,8-diaza-spiro[4.5]dec-2-en-8-carbonsäure-(2-trifluormethoxy-phenyl)-amid
[106] 1-Oxa-2,8-diaza-spiro[4.5]dec-2-en-3,8-dicarbonsäure-3-[(5-chlor-pyridin-2-yl)-amid]-8-[(2-trifluormethoxy-phenyl)-amid]
[107] 1-Oxa-2,8-diaza-spiro[4.5]dec-2-en-3,8-dicarbonsäure-3-(3,4-dimethoxy-benzylamid)-8-(4-methyl-benzylamid)
[108] 1-Oxa-2,8-diaza-spiro[4.5]dec-2-en-3,8-dicarbonsäure-3-(4-chlor-benzylamid)-8-(4-methyl-benzylamid)
[109] 3-[4-(3-Chlor-pyridin-2-yl)-piperazin-1-carbonyl]-1-oxa-2,8-diaza-spiro[4.5]dec-2-en-8-carbonsäure-4-methyl-benzylamid
[110] 1-Oxa-2,8-diaza-spiro[4.5]dec-2-en-3,8-dicarbonsäure-3-[(5-chlor-pyridin-2-yl)-amid]-8-(4-methyl-benzylamid)
[111] 1-Oxa-2,8-diaza-spiro[4.5]dec-2-en-3,8-dicarbonsäure-3-(3,4-dimethoxy-benzylamid)-8-(4-methoxy-benzylamid)
[112] 1-Oxa-2,8-diaza-spiro[4.5]dec-2-en-3,8-dicarbonsäure-3-(4-chlor-benzylamid)-8-(4-methoxy-benzylamid)
[113] 3-[4-(3-Chlor-pyridin-2-yl)-piperazin-1-carbonyl]-1-oxa-2,8-diaza-spiro[4.5]dec-2-en-8-carbonsäure-4-methoxy-benzylamid
[114] 1-Oxa-2,8-diaza-spiro[4.5]dec-2-en-3,8-dicarbonsäure 8-[(4-tert-butyl-phenyl)-amid]-3-(3,4-dimethoxy-benzylamid)
[115] 1-Oxa-2,8-diaza-spiro[4.5]dec-2-en-3,8-dicarbonsäure 8-[(4-tert-butyl-phenyl)-amid]-3-(4-chlor-benzylamid)
[116] 3-[4-(3-Chlor-pyridin-2-yl)-piperazin-1-carbonyl]-1-oxa-2,8-diaza-spiro[4.5]dec-2-en-8-carbonsäure-(4-tert-butyl-phenyl)-amid
[117] 1-Oxa-2,8-diaza-spiro[4.5]dec-2-en-3,8-dicarbonsäure-8-[(4-tert-butyl-phenyl)-amid]-3-[(5-chlor-pyridin-2-yl)-amid]
[118] 8-(2-Methoxy-phenylthiocarbamoyl)-1-oxa-2,8-diaza-spiro[4.5]dec-2-en-3-carbonsäure-3,4-dimethoxy-benzylamid
[119] 3-[4-(3-Chlor-pyridin-2-yl)-piperazin-1-carbonyl]-1-oxa-2,8-diaza-spiro[4.5]dec-2-en-8-carbonthiosäure-(2-methoxy-phenyl)-amid
[120] 8-(Cyclohexylmethyl-thiocarbamoyl)-1-oxa-2,8-diaza-spiro[4.5]dec-2-en-3-carbonsäure-3,4-dimethoxy-benzylamid
[121] 8-(Cyclohexylmethyl-thiocarbamoyl)-1-oxa-2,8-diaza-spiro[4.5]dec-2-en-3-carbonsäure-4-chlor-benzylamid
[122] 3-[4-(3-Chlor-pyridin-2-yl)-piperazin-1-carbonyl]-1-oxa-2,8-diaza-spiro[4.5]dec-2-en-8-carbonthiosäure cyclohexylmethyl-amid
[123] 8-Cyclooctylthiocarbamoyl-1-oxa-2,8-diaza-spiro[4.5]dec-2-en-3-carbonsäure-3,4-dimethoxy-benzylamid
[124] 8-Cyclooctylthiocarbamoyl-1-oxa-2,8-diaza-spiro[4.5]dec-2-en-3-carbonsäure-4-chlor-benzylamid
[125] 8-(3-Morpholin-4-yl-propylthiocarbamoyl)-1-oxa-2,8-diaza-spiro[4.5]dec-2-en-3-carbonsäure-4-chlor-benzylamid
[126] 8-(3-Morpholin-4-yl-propylthiocarbamoyl)-1-oxa-2,8-diaza-spiro[4.5]dec-2-en-3-carbonsäure-(5-chlor-pyridin-2-yl)-amid
[127] 8-p-Tolylthiocarbamoyl-1-oxa-2,8-diaza-spiro[4.5]dec-2-en-3-carbonsäure-4-chlor-benzylamid
[128] 8-Phenethylthiocarbamoyl-1-oxa-2,8-diaza-spiro[4.5]dec-2-en-3-carbonsäure-3,4-dimethoxy-benzylamid
[129] 8-Phenethylthiocarbamoyl-1-oxa-2,8-diaza-spiro[4.5]dec-2-en-3-carbonsäure-4-chlor-benzylamid
[130] 8-(3-Phenyl-propylthiocarbamoyl)-1-oxa-2,8-diaza-spiro[4.5]dec-2-en-3-carbonsäure-(5-chlor-pyridin-2-yl)-amid
[131] 8-(2-fluor-phenylthiocarbamoyl)-1-oxa-2,8-diaza-spiro[4.5]dec-2-en-3-carbonsäure-3,4-dimethoxy-benzylamid
[132] 8-(4-fluor-phenylthiocarbamoyl)-1-oxa-2,8-diaza-spiro[4.5]dec-2-en-3-carbonsäure-3,4-dimethoxy-benzylamid
[133] 8-(4-fluor-phenylthiocarbamoyl)-1-oxa-2,8-diaza-spiro[4.5]dec-2-en-3-carbonsäure-4-chlor-benzylamid
[134] 8-(2-chlor-phenylthiocarbamoyl)-1-oxa-2,8-diaza-spiro[4.5]dec-2-en-3-carbonsäure-3,4-dimethoxy-benzylamid
[135] 8-(2-chlor-phenylthiocarbamoyl)-1-oxa-2,8-diaza-spiro[4.5]dec-2-en-3-carbonsäure-4-chlor-benzylamid
[136] 8-(3-Trifluormethyl-phenylthiocarbamoyl)-1-oxa-2,8-diaza-spiro[4.5]dec-2-en-3-carbonsäure-4-chlor-benzylamid
[137] 8-(Naphthalen-1-ylthiocarbamoyl)-1-oxa-2,8-diaza-spiro[4.5]dec-2-en-3-carbonsäure-3,4-dimethoxy-benzylamid
[138] 8-(Naphthalen-1-ylthiocarbamoyl)-1-oxa-2,8-diaza-spiro[4.5]dec-2-en-3-carbonsäure-3,4-dimethoxy-benzylamid
[139] 8-Cyclopentylthiocarbamoyl-1-oxa-2,8-diaza-spiro[4.5]dec-2-en-3-carbonsäure-3,4-dimethoxy-benzylamid
[140] 3-[4-(3-Chlor-pyridin-2-yl)-piperazin-1-carbonyl]-1-oxa-2,8-diaza-spiro[4.5]dec-2-en-8-carbonthiosäure-cyclopentylamid
[141] 8-(2-Morpholin-4-yl-ethylthiocarbamoyl)-1-oxa-2,8-diaza-spiro[4.5]dec-2-en-3-carbonsäure-3,4-dimethoxy-benzylamid
[142] 8-(2-Morpholin-4-yl-ethylthiocarbamoyl)-1-oxa-2,8-diaza-spiro[4.5]dec-2-en-3-carbonsäure-4-chlor-benzylamid
[143] 8-(3-chlor-phenylthiocarbamoyl)-1-oxa-2,8-diaza-spiro[4.5]dec-2-en-3-carbonsäure-3,4-dimethoxy-benzylamid
[144] 3-[4-(3-Chlor-pyridin-2-yl)-piperazin-1-carbonyl]-1-oxa-2,8-diaza-spiro[4.5]dec-2-en-8-carbonthiosäure-(4-trifluormethyl-phenyl)-amid
[145] 8-(2-Methylsulfanyl-phenylthiocarbamoyl)-1-oxa-2,8-diaza-spiro[4.5]dec-2-en-3-carbonsäure-3,4-dimethoxy-benzylamid
[146] 8-(2-Methylsulfanyl-phenylthiocarbamoyl)-1-oxa-2,8-diaza-spiro[4.5]dec-2-en-3-carbonsäure-4-chlor-benzylamid
[147] 3-[4-(3-Chlor-pyridin-2-yl)-piperazin-1-carbonyl]-1-oxa-2,8-diaza-spiro[4.5]dec-2-en-8-carbonthiosäure (2-methylsulfanyl-phenyl)-amid
[148] 8-(4-Isopropyl-phenylthiocarbamoyl)-1-oxa-2,8-diaza-spiro[4.5]dec-2-en-3-carbonsäure-3,4-dimethoxy-benzylamid
[149] 8-(4-Isopropyl-phenylthiocarbamoyl)-1-oxa-2,8-diaza-spiro[4.5]dec-2-en-3-carbonsäure-4-chlor-benzylamid
[150] 8-(2-Iodo-phenylthiocarbamoyl)-1-oxa-2,8-diaza-spiro[4.5]dec-2-en-3-carbonsäure-3,4-dimethoxy-benzylamid
[151] 8-(2-Iodo-phenylthiocarbamoyl)-1-oxa-2,8-diaza-spiro[4.5]dec-2-en-3-carbonsäure-4-chlor-benzylamid
[152] 8-(2-Trifluormethyl-phenylthiocarbamoyl)-1-oxa-2,8-diaza-spiro[4.5]dec-2-en-3-carbonsäure-3,4-dimethoxy-benzylamid
[153] 8-(2-Trifluormethyl-phenylthiocarbamoyl)-1-oxa-2,8-diaza-spiro[4.5]dec-2-en-3-carbonsäure-4-chlor-benzylamid
[154] 3-[4-(3-Chlor-pyridin-2-yl)-piperazin-1-carbonyl]-1-oxa-2,8-diaza-spiro[4.5]dec-2-en-8-carbonthiosäure-(2-trifluormethyl-phenyl)-amid
[155] 8-[(Benzo[1,3]dioxol-5-ylmethyl)-thiocarbamoyl]-1-oxa-2,8-diaza-spiro[4.5]dec-2-en-3-carbonsäure-3,4-dimethoxy-benzylamid
[156] 8-[(Benzo[1,3]dioxol-5-ylmethyl)-thiocarbamoyl]-1-oxa-2,8-diaza-spiro[4.5]dec-2-en-3-carbonsäure-4-chlor-benzylamid
[157] 3-[4-(3-Chlor-pyridin-2-yl)-piperazin-1-carbonyl]-1-oxa-2,8-diaza-spiro[4.5]dec-2-en-8-carbonthiosäure-(benzo[1,3]dioxol-5-ylmethyl)-amid
[158] 3-[4-(3-Chlor-pyridin-2-yl)-piperazin-1-carbonyl]-1-oxa-2,8-diaza-spiro[4.5]dec-2-en-8-carbonsäure-(3-cyano-phenyl)-amid
[159] 1-Oxa-2,8-diaza-spiro[4.5]dec-2-en-3,8-dicarbonsäure-3-(3,4-dimethoxy-benzylamid)-8-[(2,5-dimethoxy-phenyl)-amid]
[160] 1-Oxa-2,8-diaza-spiro[4.5]dec-2-en-3,8-dicarbonsäure-3-(4-chlor-benzylamid)-8-[(2,5-dimethoxy-phenyl)-amid]
[161] 3-[4-(3-Chlor-pyridin-2-yl)-piperazin-1-carbonyl]-1-oxa-2,8-diaza-spiro[4.5]dec-2-en-8-carbonsäure-(2,5-dimethoxy-phenyl)-amid
[162] 1-Oxa-2,8-diaza-spiro[4.5]dec-2-en-3,8-dicarbonsäure-3-(3,4-dimethoxy-benzylamid)-8-[(2,4-dimethyl-phenyl)-amid]
[163] 1-Oxa-2,8-diaza-spiro[4.5]dec-2-en-3,8-dicarbonsäure-3-(4-chlor-benzylamid)-8-[(2,4-dimethyl-phenyl)-amid]
[164] 3-[4-(3-Chlor-pyridin-2-yl)-piperazin-1-carbonyl]-1-oxa-2,8-diaza-spiro[4.5]dec-2-en-8-carbonsäure-(2,4-dimethyl-phenyl)-amid
[165] 1-Oxa-2,8-diaza-spiro[4.5]dec-2-en-3,8-dicarbonsäure-8-butylamid 3-(3,4-dimethoxy-benzylamid)
[166] 1-Oxa-2,8-diaza-spiro[4.5]dec-2-en-3,8-dicarbonsäure-8-butylamid 3-(4-chlor-benzylamid)
[167] 3-[4-(3-Chlor-pyridin-2-yl)-piperazin-1-carbonyl]-1-oxa-2,8-diaza-spiro[4.5]dec-2-en-8-carbonsäurebutylamid
[168] 1-Oxa-2,8-diaza-spiro[4.5]dec-2-en-3,8-dicarbonsäure-3-(3,4-dimethoxy-benzylamid)-8-pentylamid
[169] 1-Oxa-2,8-diaza-spiro[4.5]dec-2-en-3,8-dicarbonsäure-3-(4-chlor-benzylamid)-8-pentylamid
[170] 3-[4-(3-Chlor-pyridin-2-yl)-piperazin-1-carbonyl]-1-oxa-2,8-diaza-spiro[4.5]dec-2-en-8-carbonsäurepentylamid
[171] 1-Oxa-2,8-diaza-spiro[4.5]dec-2-en-3,8-dicarbonsäure-3-(3,4-dimethoxy-benzylamid)-8-[(2-ethoxy-phenyl)-amid]
[172] 1-Oxa-2,8-diaza-spiro[4.5]dec-2-en-3,8-dicarbonsäure-3-(4-chlor-benzylamid)-8-[(2-ethoxy-phenyl)-amid]
[173] 1-Oxa-2,8-diaza-spiro[4.5]dec-2-en-3,8-dicarbonsäure-3-(3,4-dimethoxy-benzylamid)-8-[(4-ethoxy-phenyl)-amid]
[174] 1-Oxa-2,8-diaza-spiro[4.5]dec-2-en-3,8-dicarbonsäure 8-[(2,4-difluorphenyl)-amid]-3-(3,4-dimethoxy-benzylamid)
[175] 1-Oxa-2,8-diaza-spiro[4.5]dec-2-en-3,8-dicarbonsäure-3-(4-chlor-benzylamid)-8-[(2,4-difluor-phenyl)-amid]
[176] 1-Oxa-2,8-diaza-spiro[4.5]dec-2-en-3,8-dicarbonsäure 8-[(3-chlor-2-methyl-phenyl)-amid]-3-(3,4-dimethoxy-benzylamid)
[177] 1-Oxa-2,8-diaza-spiro[4.5]dec-2-en-3,8-dicarbonsäure-3-(4-chlor-benzylamid)-8-[(3-chlor-2-methyl-phenyl)-amid]
[178] 1-Oxa-2,8-diaza-spiro[4.5]dec-2-en-3,8-dicarbonsäure 8-[(4-chlor-2-methyl-phenyl)-amid]-3-(3,4-dimethoxy-benzylamid)
[179] 1-Oxa-2,8-diaza-spiro[4.5]dec-2-en-3,8-dicarbonsäure-3-(4-chlor-benzylamid)-8-[(4-chlor-2-methyl-phenyl)-amid]
[180] 1-Oxa-2,8-diaza-spiro[4.5]dec-2-en-3,8-dicarbonsäure 8-[(3-chlor-4-methyl-phenyl)-amid]-3-(3,4-dimethoxy-benzylamid)
[181] 1-Oxa-2,8-diaza-spiro[4.5]dec-2-en-3,8-dicarbonsäure-3-(4-chlor-benzylamid)-8-[(3-chlor-4-methyl-phenyl)-amid]
[182] 1-Oxa-2,8-diaza-spiro[4.5]dec-2-en-3,8-dicarbonsäure 8-[(3-chlor-4-fluor-phenyl)-amid]-3-(3,4-dimethoxy-benzylamid)
[183] 1-Oxa-2,8-diaza-spiro[4.5]dec-2-en-3,8-dicarbonsäure-3-(4-chlor-benzylamid)-8-[(3-chlor-4-fluor-phenyl)-amid]
[184] 1-Oxa-2,8-diaza-spiro[4.5]dec-2-en-3,8-dicarbonsäure 8-[(2,6-diisopropyl-phenyl)-amid]-3-(3,4-dimethoxy-benzylamid)
[185] 1-Oxa-2,8-diaza-spiro[4.5]dec-2-en-3,8-dicarbonsäure-3-(4-chlor-benzylamid)-8-[(2,6-diisopropyl-phenyl)-amid]
[186] 1-Oxa-2,8-diaza-spiro[4.5]dec-2-en-3,8-dicarbonsäure 8-[(5-chlor-2-methoxy-phenyl)-amid]-3-(3,4-dimethoxy-benzylamid)
[187] 1-Oxa-2,8-diaza-spiro[4.5]dec-2-en-3,8-dicarbonsäure-3-(4-chlor-benzylamid)-8-[(5-chlor-2-methoxy-phenyl)-amid]
[188] 1-Oxa-2,8-diaza-spiro[4.5]dec-2-en-3,8-dicarbonsäure-3-(4-chlor-benzylamid)-8-[(3,4,5-trimethoxy-phenyl)-amid]
[189] 1-Oxa-2,8-diaza-spiro[4.5]dec-2-en-3,8-dicarbonsäure-3-(3,4-dimethoxy-benzylamid)-8-[(3,5-dimethyl-phenyl)-amid]
[190] 1-Oxa-2,8-diaza-spiro[4.5]dec-2-en-3,8-dicarbonsäure-3-(4-chlor-benzylamid)-8-[(3,5-dimethyl-phenyl)-amid]
[191] 1-Oxa-2,8-diaza-spiro[4.5]dec-2-en-3,8-dicarbonsäure-3-(3,4-dimethoxy-benzylamid)-8-[(2,6-dimethyl-phenyl)-amid]
[192] 1-Oxa-2,8-diaza-spiro[4.5]dec-2-en-3,8-dicarbonsäure-3-(4-chlor-benzylamid)-8-[(2,6-dimethyl-phenyl)-amid]
[193] 1-Oxa-2,8-diaza-spiro[4.5]dec-2-en-3,8-dicarbonsäure-3-(3,4-dimethoxy-benzylamid)-8-[(3,4-dimethyl-phenyl)-amid]
[194] 1-Oxa-2,8-diaza-spiro[4.5]dec-2-en-3,8-dicarbonsäure-3-(3,4-dimethoxy-benzylamid)-8-[(2,5-dimethyl-phenyl)-amid]
[195] 1-Oxa-2,8-diaza-spiro[4.5]dec-2-en-3,8-dicarbonsäure-3-(4-chlor-benzylamid)-8-[(2,5-dimethyl-phenyl)-amid]
[196] 1-Oxa-2,8-diaza-spiro[4.5]dec-2-en-3,8-dicarbonsäure-3-(3,4-dimethoxy-benzylamid)-8-[(2-ethyl-6-methyl-phenyl)-amid]
[197] 1-Oxa-2,8-diaza-spiro[4.5]dec-2-en-3,8-dicarbonsäure-3-(4-chlor-benzylamid)-8-[(2-ethyl-6-methyl-phenyl)-amid]
[198] 1-Oxa-2,8-diaza-spiro[4.5]dec-2-en-3,8-dicarbonsäure-3-(3,4-dimethoxy-benzylamid)-8-[(4-methoxy-2-methyl-phenyl)-amid]
[199] 1-Oxa-2,8-diaza-spiro[4.5]dec-2-en-3,8-dicarbonsäure-3-(4-chlor-benzylamid)-8-[(4-methoxy-2-methyl-phenyl)-amid]
[200] 1-Oxa-2,8-diaza-spiro[4.5]dec-2-en-3,8-dicarbonsäure-3-(3,4-dimethoxy-benzylamid)-8-[(2-methoxy-5-methyl-phenyl)-amid]
[201] 1-Oxa-2,8-diaza-spiro[4.5]dec-2-en-3,8-dicarbonsäure-3-(4-chlor-benzylamid)-8-[(2-methoxy-5-methyl-phenyl)-amid]
[202] 1-Oxa-2,8-diaza-spiro[4.5]dec-2-en-3,8-dicarbonsäure-3-(3,4-dimethoxy-benzylamid)-8-[(2,4,5-trimethyl-phenyl)-amid]
[203] 1-Oxa-2,8-diaza-spiro[4.5]dec-2-en-3,8-dicarbonsäure-3-(4-chlor-benzylamid)-8-[(2,4,5-trimethyl-phenyl)-amid]
[204] 1-Oxa-2,8-diaza-spiro[4.5]dec-2-en-3,8-dicarbonsäure-3-(3,4-dimethoxy-benzylamid)-8-[(2,4,6-trimethyl-phenyl)-amid]
[205] 1-Oxa-2,8-diaza-spiro[4.5]dec-2-en-3,8-dicarbonsäure-3-(4-chlor-benzylamid)-8-[(2,4,6-trimethyl-phenyl)-amid]
[206] 1-Oxa-2,8-diaza-spiro[4.5]dec-2-en-3,8-dicarbonsäure-8-[(2-bromo-ethyl)-amid]-3-(3,4-dimethoxy-benzylamid)
[207] 1-Oxa-2,8-diaza-spiro[4.5]dec-2-en-3,8-dicarbonsäure-8-[(2-bromo-ethyl)-amid]-3-(4-chlor-benzylamid)
[208] 1-Oxa-2,8-diaza-spiro[4.5]dec-2-en-3,8-dicarbonsäure-8-[(4-butylphenyl)-amid]-3-(3,4-dimethoxy-benzylamid)
[209] 1-Oxa-2,8-diaza-spiro[4.5]dec-2-en-3,8-dicarbonsäure-8-[(4-butyl-phenyl)-amid]-3-(4-chlor-benzylamid)
[210] 2-{[3-(4-Chlor-benzylcarbamoyl)-1-oxa-2,8-diaza-spiro[4.5]dec-2-en-8-carbonyl]-amino}-benzoesäuremethylester
[211] 1-Oxa-2,8-diaza-spiro[4.5]dec-2-en-3,8-dicarbonsäure-8-biphenyl-2-ylamid 3-(3,4-dimethoxy-benzylamid)
[212] 1-Oxa-2,8-diaza-spiro[4.5]dec-2-en-3,8-dicarbonsäure-8-biphenyl-2-ylamid 3-(4-chlor-benzylamid)
[213] 1-Oxa-2,8-diaza-spiro[4.5]dec-2-en-3,8-dicarbonsäure-8-[(2,6-dichlorphenyl)-amid]-3-(3,4-dimethoxy-benzylamid)
[214] 1-Oxa-2,8-diaza-spiro[4.5]dec-2-en-3,8-dicarbonsäure-3-(4-chlor-benzylamid)-8-[(2,6-dichlor-phenyl)-amid]
[215] 3-{[3-(3,4-Dimethoxy-benzylcarbamoyl)-1-oxa-2,8-diaza-spiro[4.5]dec-2-en-8-carbonyl]-amino}-benzoesäureethylester
[216] 3-{[3-(4-Chlor-benzylcarbamoyl)-1-oxa-2,8-diaza-spiro[4.5]dec-2-en-8-carbonyl]-amino}-benzoesäureethylester
[217] 4-{[3-(3,4-Dimethoxy-benzylcarbamoyl)-1-oxa-2,8-diaza-spiro[4.5]dec-2-en-8-carbonyl]-amino}-benzoesäureethylester
[218] 4-{[3-(4-Chlor-benzylcarbamoyl)-1-oxa-2,8-diaza-spiro[4.5]dec-2-en-8-carbonyl]-amino}-benzoesäureethylester
[219] 1-Oxa-2,8-diaza-spiro[4.5]dec-2-en-3,8-dicarbonsäure-3-(4-chlor-benzylamid) 8-naphthalen-1-ylamid
[220] 3-[4-(3-Chlor-pyridin-2-yl)-piperazin-1-carbonyl]-1-oxa-2,8-diaza-spiro[4.5]dec-2-en-8-carbonthiosäure-(2-chlor-5-trifluormethyl-phenyl)-amid
[221] 8-(4-Chlor-3-trifluormethyl-phenylthiocarbamoyl)-1-oxa-2,8-diaza-spiro[4.5]dec-2-en-3-carbonsäure-3,4-dimethoxy-benzylamid
[222] 8-(3,5-Bis-trifluormethyl-phenylthiocarbamoyl)-1-oxa-2,8-diaza-spiro[4.5]dec-2-en-3-carbonsäure-3,4-dimethoxy-benzylamid
[223] 8-(3,5-Bis-trifluormethyl-phenylthiocarbamoyl)-1-oxa-2,8-diaza-spiro[4.5]dec-2-en-3-carbonsäure-4-chlor-benzylamid
[224] 3-[4-(3-Chlor-pyridin-2-yl)-piperazin-1-carbonyl]-1-oxa-2,8-diaza-spiro[4.5]dec-2-en-8-carbonthiosäure-(3,5-bis-trifluormethyl-phenyl)-amid
[225] 8-Cyclododecylthiocarbamoyl-1-oxa-2,8-diaza-spiro[4.5]dec-2-en-3-carbonsäure-3,4-dimethoxy-benzylamid
[226] 8-Benzylthiocarbamoyl-1-oxa-2,8-diaza-spiro[4.5]dec-2-en-3-carbonsäure-3,4-dimethoxy-benzylamid
[227] 8-Benzylthiocarbamoyl-1-oxa-2,8-diaza-spiro[4.5]dec-2-en-3-carbonsäure-4-chlor-benzylamid
[228] 3-[4-(3-Trifluoromethyl-pyridin-2-yl)-piperazin-1-carbonyl]-1-oxa-2,8-diaza-spiro[4.5]dec-2-en-8-carbonsäure-(4-tert-butyl-phenyl)-amid
[229] 3-[4-(3-Chlor-pyridin-2-yl)-piperazin-1-carbonyl]-1-oxa-2,8-diaza-spiro[4.5]dec-2-en-8-carbonsäure-(4-pentafluorsulfanyl-phenyl)-amid,
[230] N3-((5-Methylfuran-2-yl)methyl)-N8-(4-(trifluormethyl)phenyl)-1-oxa-2,8-diazaspiro[4.5]dec-2-en-3,8-dicarboxamid,
[231] N8-(4-Methoxyphenyl)-N3-((5-methylfuran-2-yl)methyl)-1-oxa-2,8-diazaspiro[4.5]dec-2-en-3,8-dicarboxamid,
[232] N8-(4-Chlorphenyl)-N3-((5-methylfuran-2-yl)methyl)-1-oxa-2,8-diazaspiro[4.5]dec-2-en-3,8-dicarboxamid,
[233] N3-(4-(3-Chlorpyridin-2-yl)piperazin-1-yl)-N8-(3,4-dichlorphenyl)-1-oxa-2,8-diazaspiro[4.5]dec-2-en-3,8-dicarboxamid,
[234] N3-(4-(3-Chlorpyridin-2-yl)piperazin-1-yl)-N8-(4-methoxyphenyl)-1-oxa-2,8-diazaspiro[4.5]dec-2-en-3,8-dicarboxamid,
[235] N8-(4-Chlorbenzyl)-N3-((5-methylfuran-2-yl)methyl)-1-oxa-2,8-diazaspiro[4.5]dec-2-en-3,8-dicarboxamid,
[236] N8-(3,4-Dichlorbenzyl)-N3-((5-methylfuran-2-yl)methyl)-1-oxa-2,8-diazaspiro[4.5]dec-2-en-3,8-dicarboxamid,
[237] N3-(4-Chlorbenzyl)-N8-(3,4-dichlorbenzyl)-1-oxa-2,8-diazaspiro[4.5]dec-2-en-3,8-dicarboxamid,
[238] N8-(3,4-Dichlorbenzyl)-N3-(4-hydroxy-3-methoxybenzyl)-1-oxa-2,8-diazaspiro[4.5]dec-2-en-3,8-dicarboxamid,
[239] N3-(4-tert-Butylbenzyl)-N8-(3,4-dichlorbenzyl)-1-oxa-2,8-diazaspiro[4.5]dec-2-en-3,8-dicarboxamid,
[240] N8-(3,4-Dichlorbenzyl)-N3-(4-(trifluormethyl)benzyl)-1-oxa-2,8-diazaspiro[4.5]dec-2-en-3,8-dicarboxamid,
[241] 3-(4-(3-Chlorpyridin-2-yl)piperazin-1-carbonyl)-N-(3,4-dichlorbenzyl)-1-oxa-2,8-diazaspiro[4.5]dec-2-en-8-carboxamid,
[242] N3-(4-(3-Chlorpyridin-2-yl)piperazin-1-yl)-N8-(3,4-dichlorbenzyl)-1-oxa-2,8-diazaspiro[4.5]dec-2-en-3,8-dicarboxamid,
[243] N8-(3,4-Dichlorphenyl)-N3-((5-methylfuran-2-yl)methyl)-1-oxa-2,8-diazaspiro[4.5]dec-2-en-3,8-dicarboxamid,
[244] N8-(4-Chlorphenyl)-N3-(4-hydroxy-3-methoxybenzyl)-1-oxa-2,8-diazaspiro[4.5]dec-2-en-3,8-dicarboxamid,
[245] N8-(3,4-Dichlorphenyl)-N3-(4-hydroxy-3-methoxybenzyl)-1-oxa-2,8-diazaspiro[4.5]dec-2-en-3,8-dicarboxamid,
[246] N3-(4-Chlorbenzyl)-N8-(3,4-dichlorphenyl)-1-oxa-2,8-diazaspiro[4.5]dec-2-en-3,8-dicarboxamid,
[247] N3-(4-Chlorbenzyl)-N8-(4-chlorphenyl)-1-oxa-2,8-diazaspiro[4.5]dec-2-en-3,8-dicarboxamid,
[248] N3-(4-Hydroxy-3-methoxybenzyl)-N8-(4-(trifluormethyl)phenyl)-1-oxa-2,8-diazaspiro[4.5]dec-2-en-3,8-dicarboxamid,
[249] N3-(4-Hydroxy-3-methoxybenzyl)-N8-(4-methoxyphenyl)-1-oxa-2,8-diazaspiro[4.5]dec-2-en-3,8-dicarboxamid,
[250] N3-(4-Chlorbenzyl)-N8-(4-(trifluormethyl)phenyl)-1-oxa-2,8-diazaspiro[4.5]dec-2-en-3,8-dicarboxamid,
[251] N8-(3,4-Dichlorbenzyl)-N3-(3,4-dimethoxybenzyl)-1-oxa-2,8-diazaspiro[4.5]dec-2-en-3,8-dicarboxamid,
[252] N3-(3,4-Dimethoxybenzyl)-N8-(4-(trifluormethyl)phenyl)-1-oxa-2,8-diazaspiro[4.5]dec-2-en-3,8-dicarboxamid,
[253] 3-[4-(3-Trifluormethyl-pyridin-2-yl)-piperazin-1-carbonyl]-1-oxa-2,8-diaza-spiro[4.5]dec-2-en-8-carbonsäure-(4-pentafluorsulfanyl-phenyl)-amid,
[254] N-(4-tert-Butylphenyl)-3-(4-(3-chlorpyridin-2-yl)-2-methylpiperazin-1-carbonyl)-1-oxa-2,8-diazaspiro[4.5]dec-2-en-8-carboxamid und
[255] N-(4-tert-Butylbenzyl)-3-(4-(3-chlorpyridin-2-yl)piperazin-1-carbonyl)-1-oxa-2,8-diazaspiro[4.5]dec-2-en-8-carboxamid.

## Claims

1. Substituted spiro compounds of the general formula I, 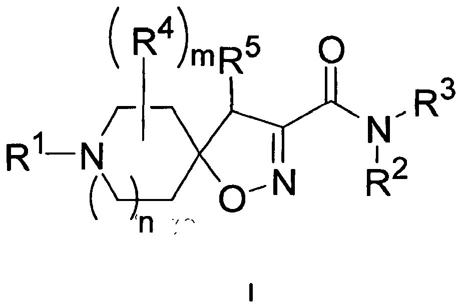 wherein
m is equal to 0, 1, 2, 3 or 4,
n is equal to 1;
R¹ represents a -C(=S)-NR⁸R⁹ group; or
if m is unequal to 0 and/or R⁵ is unequal to H, may represent a -C(=O)-NR⁶R⁷ group in addition;
R² represents a linear or branched, saturated or unsaturated, unsubstituted or at least monosubstituted aliphatic radical optionally including at least one heteroatom as a chain member,
where the substituents of the aliphatic radical may be selected independently from the group consisting of F, Cl, Br, I, -CN, -NO₂, -OH, - SH and -NH₂;
an unsubstituted or at least monosubstituted, unsaturated or saturated cycloaliphatic radical optionally including at least one heteroatom as ring member which can be bonded via a linear or branched,
unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
R³ represents a hydrogen radical;
a linear or branched, saturated or unsaturated, unsubstituted or at least monosubstituted aliphatic radical optionally including at least one heteroatom as a chain member,
where the substituents of the aliphatic radical may be selected independently from the group consisting of F, Cl, Br, I, -CN, -NO₂, -OH, - SH and -NH₂;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or
R² and R³, together with the nitrogen atom joining them, form a unsubstituted or at least monosubstituted, unsaturated or saturated cycloaliphatic radical optionally including at least one further heteroatom as ring member which can be condensed with an unsubstituted or at least monosubstituted monocyclic or polycyclic ring system;
R⁴ represents a halogen radical, a nitro group, a cyano group, an amino group, a hydroxy group, a thiol group, a carboxy group, a formyl group, a -NH-C(=O)-H group, an oxo group (=O), a -NH-R¹⁰ group, a -NR¹¹R¹² group, a -C(=O)-R¹³ group, a -C(=O)-O-R¹⁴ group, an -O-C(=O)-R¹⁵ group, a -NH-C(=O)-R¹⁶ group, a -NR¹⁷-C(=O)-R¹⁸ group, a -C(=O)-NH₂ group, a -C(=O)-NH-R¹⁹ group, a -C(=O)-NR²⁰R²¹ group, an -O-R²² group, an -S-R²³ group, or a linear or branched, saturated or unsaturated, unsubstituted or at least monosubstituted aliphatic radical;
R⁵ represents a hydrogen radical, a halogen radical, a nitro group, a cyano group, an amino group, a hydroxy group, a thiol group, a carboxy group, a formyl group, a -NH-C(=O)-H group, an oxo group (=O), a -NH-R¹⁰ group, a -NR¹¹R¹² group, a -C(=O)-R¹³ group, a -C(=O)-O-R¹⁴ group, an -O-C(=O)-R¹⁵ group, a -NH-C(=O)-R¹⁶ group, a -NR¹⁷-C(=O)-R¹⁸ group, a -C(=O)-NH₂ group, a -C(=O)-NH-R¹⁹ group, a -C(=O)-NR²⁰R²¹ group, an -O-R²² group, a -S-R²³ group, a linear or branched, saturated or unsaturated, unsubstituted or at least monosubstituted aliphatic radical,
or an unsubstituted or at least monosubstituted aryl or heteroaryl radical which can be bonded via a linear or branched, unsubstituted or at least monosubstituted alkylene, alkenylene or alkinylene group;
R⁶ and R⁸, independently of one another, each represents
a linear or branched, saturated or unsaturated, unsubstituted or at least monosubstituted aliphatic radical optionally including at least one heteroatom as a chain member;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system and/or can be bridged with at least a linear or branched, unsubstituted or at least monosubstituted alkylene group;
an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
a -C(=O)-R²⁴ group which can be bonded via a linear or branched, unsubstituted or at least monosubstituted alkylene group;
or a -C(=O)-O-R²⁵ group which can be bonded via a linear or branched, unsubstituted or at least monosubstituted alkylene group;
R⁷ and R⁹, independently of one another, each represents
a hydrogen radical,
a linear or branched, saturated or unsaturated, unsubstituted or at least monosubstituted aliphatic radical optionally including at least one heteroatom as a chain member;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system, and/or can be bridged with at least a linear or branched, unsubstituted or at least monosubstituted alkylene group;
an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
a -C(=O)-R²⁴ group which can be bonded via a linear or branched, unsubstituted or at least monosubstituted alkylene group;
or a -C(=O)-O-R²⁵ group which can be bonded via a linear or branched, unsubstituted or at least monosubstituted alkylene group;
and
R¹⁰ to R²⁵, independently of one another, each represents
a linear or branched, saturated or unsaturated, unsubstituted or at least monosubstituted aliphatic radical optionally including at least one heteroatom as a chain member;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates.

2. The compounds according to claim 1, **characterized in that**
m is equal to 0, 1, 2, 3 or 4;
n is equal to 0, 1 or 2;
R¹ represents a -C(=S)-NR⁸R⁹ group; or
if m is unequal to 0 and/or R⁵ is unequal to H, may represent a -C(=O)-NR⁶R⁷ group in addition;
R² represents a linear or branched, saturated or unsaturated, optionally substituted C₁₋₁₀ aliphatic radical;
an unsaturated or saturated, optionally substituted 3-, 4-, 5-, 6-, 7-, 8- or 9-membered cycloaliphatic radical which can be condensed with a saturated, unsaturated or aromatic, optionally substituted monocyclic or polycyclic ring system;
an optionally substituted 5- to 14-membered aryl or heteroaryl radical which can be condensed with a saturated or unsaturated, optionally substituted monocyclic or polycyclic ring system;
or -(CHR³⁰)-Xᵥ-(CHR³¹)_{w}-Yₓ((CHR³²)_{y}-Z_{z}-R³³ with v = 0 or 1, w = 0 or 1, x = 0 or 1, y = 0 or 1, z = 0 or 1, wherein X, Y and Z each independently represents O, S, NH, N(CH₃), N(C₂H₅) or N[CH(CH₃)₂];
R³ represents a hydrogen radical;
a linear or branched, saturated or unsaturated, optionally unsubstituted C₁₋₁₀ aliphatic radical;
an unsaturated or saturated, optionally substituted 3-, 4-, 5-, 6-, 7-, 8- or 9-membered cycloaliphatic radical which can be condensed with a saturated, unsaturated or aromatic, optionally substituted monocyclic or polycyclic ring system;
an optionally substituted 5- to 14-membered aryl or heteroaryl radical which can be condensed with a saturated or unsaturated, optionally substituted monocyclic or polycyclic ring system;
or -(CHR³⁰)-Xᵥ-(CHR³¹)_{w}-Yₓ-(CHR³²)_{y}-Z_{z}-R³³ mit v = 0 or 1, w = 0 or 1, x = 0 or 1, y = 0 or 1, z = 0 or 1, wherein X, Y and Z each independently represents O, S, NH, N(CH₃), N(C₂H₅) or N[CH(CH₃)₂];
or
R² and R³, together with the nitrogen atom joining them as ring member, form a saturated or unsaturated, optionally substituted 4-, 5-, 6-, 7-, 8- or 9-membered heterocycloaliphatic radical which can be condensed with a saturated, unsaturated or aromatic, optionally substituted monocyclic or polycyclic ring system;
R⁴ represents a halogen radical, a nitro group, a cyano group, an amino group, a hydroxy group, a thiol group, a carboxy group, a formyl group, a -NH-C(=O)-H group, an oxo group (=O), a -NH-R¹⁰ group, a -NR¹¹R¹² group, a -C(=O)-R¹³ group, a -C(=O)-O-R¹⁴ group, an -O-C(=O)-R¹⁵ group, a -NH-C(=O)-R¹⁶ group, a -NR¹⁷-C(=O)-R¹⁸ group, a -C(=O)-NH₂ group, a -C(=O)-NH-R¹⁹ group, a -C(=O)-NR²⁰R²¹ group, an -O-R²² group, an -S-R²³ group, or a linear or branched, saturated or unsaturated, optionally substituted C₁₋₁₀ aliphatic radical;
R⁵ represents a hydrogen radical, a halogen radical, a nitro group, a cyano group, an amino group, a hydroxy group, a thiol group, a carboxy group, a formyl group, a -NH-C(=O)-H group, an oxo group (=O), a -NH-R¹⁰ group, a -NR¹¹R¹² group, a -C(=O)-R¹³ group, a -C(=O)-O-R¹⁴ group, an -O-C(=O)-R¹⁵ group, a -NH-C(=O)-R¹⁶ group, a -NR¹⁷-C(=O)-R¹⁸ group, a -C(=O)-NH₂ group, a -C(=O)-NH-R¹⁹ group, a -C(=O)-NR²⁰R²¹ group, an -O-R²² group, a -S-R²³ group, a linear or branched, saturated or unsaturated, optionally substituted C₁₋₁₀ aliphatic radical;
an optionally substituted 5- to 14-membered aryl or heteroaryl radical;
or -(CH2)ₐₐ-R³⁴ with aa = 1, 2, 3 or 4;
R⁶ and R⁸, independently of one another, each represents
a linear or branched, saturated or unsaturated, optionally substituted C₁₂₀ aliphatic radical;
an unsaturated or saturated, optionally substituted 3-, 4-, 5-, 6-, 7-, 8-, 9-, 10-, 11- or 12-membered cycloaliphatic radical which can be condensed with a saturated, unsaturated or aromatic, optionally substituted monocyclic or polycyclic ring system and/or can be bridged with one or two linear or branched, optionally substituted C₁₋₅-alkylene groups;
an optionally substituted 5- to 14-membered aryl or heteroaryl radical which can be condensed with a saturated or unsaturated, optionally substituted monocyclic or polycyclic ring system;
-(CHR³⁵)-(CH₂)_{bb}-(CH₂)_{cc}-C(=O)-R²⁴ with bb = 0 or 1 and cc = 0 or 1;
-(CHR³⁶)-(CH₂)_{dd}-(CH₂)ₑₑ-C(=O)-O-R²⁵ with dd = 0 or 1 and ee = 0 or 1;
or -(CR³⁷R³⁸)-X_{ff}(CHR³⁹)_{gg}-Yₕₕ-(CHR⁴⁰)ⱼⱼ-Zₖₖ-R⁴¹ with ff = 0 or 1, gg = 0 or 1, hh = 0 or 1, jj = 0 or 1, kk = 0 or 1, wherein X, Y and Z each independently represents O, S, NH, N(CH₃), N(C₂H₅) or N[CH(CH₃)₂];
R⁷ and R⁹, independently of one another, each represents
a hydrogen radical;
a linear or branched, saturated or unsaturated, optionally substituted C₁₂₀ aliphatic radical;
an unsaturated or saturated, optionally substituted 3-, 4-, 5-, 6-, 7-, 8-, 9-, 10-, 11- or 12-membered cycloaliphatic radical which can be condensed with a saturated, unsaturated or aromatic, optionally substituted monocyclic or polycyclic ring system and/or can be bridged with one or two linear or branched, optionally substituted C₁₋₅-alkylene groups;
an optionally substituted 5- to 14-membered aryl or heteroaryl radical which can be condensed with a saturated or unsaturated, optionally substituted monocyclic or polycyclic ring system;
-(CHR³⁵)-(CH₂)_{bb}-(CH₂)_{cc}-C(=O)-R²⁴ with bb = 0 or 1 and cc = 0 or 1;
-(CHR³⁶)-(CH₂)_{dd}-(CH₂)ₑₑ-C(=O)-O-R²⁵ with dd = 0 or 1 and ee = 0 or 1;
or -(CR³⁷R³⁸)-X_{ff}(CHR³⁹)-Yₕₕ-(CHR⁴⁰)ⱼⱼ-Zₖₖ-R⁴¹ with ff = 0 or 1, gg = 0 or 1, hh = 0 or 1, jj = 0 or 1, kk = 0 or 1, wherein X, Y and Z each independently represents O, S, NH, N(CH₃), N(C₂H₅) or N[CH(CH₃)₂₁;
R¹⁰ to R²⁵, independently of one another, each represents
a linear or branched, saturated or unsaturated, optionally substituted C₁₁₀ aliphatic radical;
an unsaturated or saturated, optionally substituted 3-, 4-, 5-, 6-, 7-, 8- or 9-membered cycloaliphatic radical which can be condensed with a saturated, unsaturated or aromatic, optionally substituted monocyclic or polycyclic ring system;
an optionally substituted 5- to 14-membered aryl or heteroaryl radical which can be condensed with a saturated or unsaturated, optionally substituted monocyclic or polycyclic ring system;
or -(CH₂)ₘₘ-R⁴² with mm equal to 1, 2 or 3;
R²⁶, R²⁷, R²⁸, R³⁰, R³¹, R³², R³⁵, R³⁶, R³⁷, R³⁸, R³⁹ and R⁴⁰, independently of one another, each represents
a hydrogen radical;
a linear or branched, saturated or unsaturated, optionally unsubstituted C₁₋₁₀ aliphatic radical;
or an optionally substituted 5- to 14-membered aryl or heteroaryl radical which can be condensed with a saturated or unsaturated, optionally substituted monocyclic or polycyclic ring system;
R²⁹, R³³ and R⁴¹, independently of one another, each represents
a linear or branched, saturated or unsaturated, optionally unsubstituted C₁₋₁₀ aliphatic radical;
an unsaturated or saturated, optionally substituted 3-, 4-, 5-, 6-, 7-, 8- or 9-membered cycloaliphatic radical which can be bridged with 1, 2, 3, 4 or 5 linear or branched, optionally substituted C₁₋₅ alkylene groups and/or can be condensed with a saturated, unsaturated or aromatic, optionally substituted monocyclic or polycyclic ring system;
or an optionally substituted 5- to 14-membered aryl or heteroaryl radical which can be condensed with a saturated or unsaturated, optionally substituted monocyclic or polycyclic ring system;
and
R³⁴ and R⁴², independently of one another, each represents
an optionally substituted 5- to 14-membered aryl or heteroaryl radical;
wherein
each of the above-mentioned C₁₋₁₀ aliphatic radicals and C₁₋₂₀ aliphatic radicals can optionally be substituted with 1, 2, 3, 4, 5, 6, 7, 8 or 9 substituents selected independently from the group consisting of F, Cl, Br, I, -CN, -NO₂, - OH, -SH and -NH₂;
each of the above-mentioned cycloaliphatic radicals and heterocyclo-aliphatic radicals can optionally be substituted with 1, 2, 3, 4 or 5 substituents selected
independently from the group consisting of oxo (=O), thioxo (=S), F, Cl, Br, I, - CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, - C(=O)-NH₂, -C(=O)-NH-C₁₋₅ alkyl, C(=O)-N-(C₁₋₅ alkyl)₂, -S(=O)₂-C₁₋₅ alkyl, - S(=O)₂ phenyl, -NH-S(=O)₂-C₁₋₅ alkyl, -S(=O)₂-NH-C₁₋₅ alkyl, cyclohexyl, cyclopentyl, pyridinyl, pyridazinyl, -(CH₂) benzo[b]furanyl, -O-phenyl, -O benzyl, phenyl and benzyl, wherein in each case the cyclic portion of the radicals pyridinyl, cyclopentyl, cyclohexyl, pyridazinyl, -S(=O)₂ phenyl, -0-phenyl, -O benzyl, phenyl, -(CH₂) benzo[b]furanyl and benzyl can be substituted with 1, 2, 3, 4 or 5 substituents selected independently from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl;
and each of the above-mentioned cycloaliphatic radicals can optionally have 1, 2, 3 4, or 5 heteroatoms as ring members selected independently from the group consisting of oxygen, nitrogen and sulphur;
each of the above-mentioned heterocycloaliphatic radicals can optionally have 1, 2 or 3 additional heteroatoms as ring members selected independently from the group consisting of oxygen, nitrogen and sulphur;
each of the rings of the above-mentioned monocyclic or polycyclic ring systems can optionally be substituted with 1, 2, 3, 4 or 5 substituents selected independently from the group consisting of oxo (=O), thioxo (=S), F, Cl, Br, I, - CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, - C(=O)-NH₂, -C(=O)-NH-C₁₋₅ alkyl, C(=O)-N-(C₁₋₅ alkyl)₂, -S(=O)₂-C₁₋₅ alkyl, - S(=O)₂ phenyl, -NH-S(=O)₂-C₁₋₅ alkyl, -S(=O)₂-NH-C₁₋₅ alkyl, cyclohexyl, cyclopentyl, pyridinyl, pyridazinyl, -(CH₂) benzo[b]furanyl, -O-phenyl, -0-benzyl, phenyl and benzyl, wherein in each case the cyclic portion of the radicals pyridinyl, cyclopentyl, cyclohexyl, pyridazinyl, -S(=O)₂ phenyl, -0-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl;
and each of the rings of the above-mentioned monocyclic or polycyclic ring systems can optionally have 5, 6 or 7 members and can optionally have 1, 2, 3, 4 or 5 heteroatoms as ring members selected independently from the group consisting of oxygen, nitrogen and sulphur;
and each of the above-mentioned aryl or heteroaryl radicals can optionally be substituted with 1, 2, 3, 4 or 5 substituents selected independently from the group consisting of F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, - C(=O)-H, -C(=O)-C₁₋₅ alkyl, -C(=O)-NH₂, -C(=O)-NH-C₁₋₅ alkyl, C(=O)-N-(C₁₋₅ alkyl)₂, -S(=O)₂-C₁₋₅ alkyl, -S(=O)₂ phenyl, -NH-S(=O)₂-C₁₋₅ alkyl, -S(=O)₂-NH-C₁₋₅ alkyl, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl; cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl;
each of the above-mentioned heteroaryl radicals can optionally have 1, 2, 3, 4 or 5 heteroatoms as ring members selected independently from the group consisting of oxygen, nitrogen and sulphur;
and
each of the above-mentioned C₁₋₅ alkyl groups can optionally be substituted with 1, 2, 3, 4 or 5 substituents selected independently from the group consisting of F, Cl, Br, -OH, -SH, -NH₂, -CN and NO₂;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates.

3. The compounds according to claims 1 or 2, **characterized in that**
R² represents a radical selected from the group consisting of methyl, ethyl, n-propyl, isopropyl, n-butyl, sec-butyl, isobutyl, tert-butyl, n-pentyl, sec-pentyl, - (CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl, wherein in each case the radical can optionally be substituted with 1, 2, 3, 4 or 5 substituents selected independently from the group consisting of F, Cl, Br, I, -CN, -NO₂, -OH, -SH and -NH₂;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl and (1,2,3,4)-tetrahydroquinazolinyl, wherein in each case the radical can optionally be substituted with 1, 2, 3, 4 or 5 substituents selected independently from the group consisting of oxo (=O), thioxo (=S), F, Cl, Br, I, - CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
a radical selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, wherein in each case the radical can optionally be substituted with 1, 2, 3, 4 or 5 substituents selected independently from the group consisting of F, Cl, Br, I, -CN, -CF₃, -SF₅, -OH, - O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂-phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or -(CHR³⁰)-R³³; -(CHR³⁰)-(CHR³¹)-R³³; -(CHR³⁰)-(CHR³¹)-O-R³³; -(CHR³⁰)-(CHR³¹)-(CHR³²)-R³³; -(CHR³⁰)-(CHR³¹)-S-(CHR³²)-R³³; -(CHR³⁰)-(CHR³¹)-(CHR³²)-N(CH₃)-R³³.

4. The compounds according to one or more of claims 1 to 3, **characterized in that**
R³ represents a hydrogen radical.

5. The compounds according to one or more of claims 1 to 4, **characterized in that**
R² and R³ form, together with the nitrogen atom joining them as ring member, a radical selected from the group consisting of pyrrolidinyl, piperidinyl, (1,3,4,5)-tetrahydropyrido[4,3-b]indolyl, (3,4)-dihydro-1 H-isoquinolinyl, (1,3,4,9)-tetrahydro-[b]-carbolinyl, imidazolidinyl, (1,3)-thiazolidinyl, piperazinyl, morpholinyl, azepanyl, diazepanyl and thiomorpholinyl, wherein in each case the radical can be substituted with 1, 2, 3, 4 or 5 substituents selected independently from the group consisting of oxo (=O), thioxo (=S), F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂,-C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -0-benzyl, phenyl and benzyl, wherein in each case the cyclic portion of the radicals pyridinyl, cyclopentyl, cyclohexyl, pyridazinyl, -S(=O)₂ phenyl, -0-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl.

6. The compounds according to one or more of claims 1 to 5, **characterized in that**
R⁴ represents a radical selected from the group consisting of methyl, ethyl, n-propyl, isopropyl, n-butyl, sec-butyl, isobutyl, tert-butyl, n-pentyl, sec-pentyl, n-hexyl and n-heptyl, wherein in each case the radical can optionally be substituted with 1, 2, 3, 4 or 5 substituents selected independently from the group consisting of F, Cl, Br, I, -CN, -NO₂, -OH, -SH and -NH₂.

7. The compounds according to one or more of claims 1 to 6, **characterized in that**
R⁵ represents a hydrogen radical;
a radical selected from the group consisting of methyl, ethyl, n-propyl, isopropyl, n-butyl, sec-butyl, isobutyl, tert-butyl, n-pentyl, sec-pentyl, n-hexyl and n-heptyl, wherein in each case the radical can optionally be substituted with 1, 2, 3, 4 or 5 substituents selected independently from the group consisting of F, Cl, Br, I, -CN, -NO₂, -OH, -SH and -NH₂;
a radical selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂,-O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, -N(C₂H₅)₂, -NH-CH₃, -NH-C₂H₅, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂ and -C(=O)-N-(C₂H₅)₂;
or -(CH₂)-R³⁴, -(CH₂)-(CH₂)-R³⁴ or -(CH₂)-(CH₂)-(CH₂)-R³⁴.

8. The compounds according to one or more of claims 1 to 7, **characterized in that**
R⁶ and R⁸, independently of one another, each represents a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1-butinyl, 2-butinyl and 3-butinyl, wherein in each case the radical can optionally be substituted with 1, 2, 3, 4 or 5 substituents selected independently from the group consisting of F, Cl, Br, I,-CN, -NO₂, -OH, -SH and -NH₂;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein in each case the radical can optionally be substituted with 1, 2, 3, 4 or 5 substituents selected independently from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃,-SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH⁻CH3, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃,-C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂-phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, - OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -0-benzyl;
a radical selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, wherein in each case the radical can optionally be substituted with 1, 2, 3, 4 or 5 substituents selected independently from the group consisting of F, Cl, Br, I, -CN, -CF₃, -SF₅, -OH,-O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH,-C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂-phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
-(CHR³⁵)-C(=O)-R²⁴ or -(CHR³⁵)-(CH₂)-C(=O)-R²⁴;
-(CHR³⁶)-C(=O)-O-R²⁵ or -(CHR³⁶)-(CH₂)-C(=O)-O-R²⁵;
or -(CR³⁷R³⁸)-R⁴¹, -(CR³⁷R³⁸)-(CHR³⁹)-R⁴¹, -(CR³⁷R³⁸)-(CHR³⁹)-O-R⁴¹,-(CR³⁷R³⁸)-(CHR³⁹)-(CHR⁴⁰)-R⁴¹, -(CR³⁷R³⁸)-(CHR³⁹)-(CHR⁴⁰)-O-R⁴¹, - (CR³⁷R³⁸)-(CHR³⁹)-(CHR⁴⁰)-N(CH₃)-R⁴¹ or -(CR³⁷R³⁸)-(CHR³⁹)-(CHR⁴⁰)-N(C₂H₅)-R⁴¹.

9. The compounds according to one or more of claims 1 to 8, **characterized in that**
R⁷ and R⁹ each represents a hydrogen radical.

10. The compounds according to one or more of claims 1 to 9, **characterized in that**
R¹⁰ bis R²⁵, independently of one another, each represents a radical selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein in each case the radical can be substituted with 1 or 2 substituents selected independently from the group consisting of -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅,-S(=O)₂-phenyl, pyridinyl, phenyl and benzyl, wherein in each case the cyclic portion of the radicals pyridinyl, -S(=O)₂-phenyl, phenyl and benzyl can be substituted with 1, 2, 3, 4 or 5 substituents selected independently from the group consisting of F, Cl, Br, methyl, ethyl, n-propyl, isopropyl, n-butyl, sec-butyl, isobutyl, tert-butyl, -O-CH₃ and -O-C₂H₅;
a radical selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl and pyrimidinyl, wherein in each case the radical can optionally be substituted with 1, 2, 3, 4 or 5 substituents selected independently from the group consisting of F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, -O-benzyl and phenyl;
or -(CH₂)-R⁴² or -(CH₂)-(CH₂)-R⁴².

11. The compounds according to one or more of claims 1 to 10, **characterized in that**
R²⁶, R²⁷, R²⁸, R³⁰, R³¹, R³², R³⁵, R³⁶, R³⁷, R³⁸, R³⁹ and R⁴⁰, independently of one another, each represents
a hydrogen radical;
a radical selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl;
or a phenyl radical which can be substituted with 1, 2, 3, 4 or 5 substituents selected independently from the group consisting of F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, -O-benzyl and phenyl.

12. The compounds according to one or more of claims 1 to 11, **characterized in that**
R²⁹, R³³ and R⁴¹, independently of one another, each represents a radical selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl, wherein in each case the radical can optionally be substituted with 1, 2, 3, 4 or 5 substituents selected independently from the group consisting of F, Cl, Br, I, -CN, -NO₂, -OH, -SH and -NH₂;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl and (1,2,3,4)-tetrahydroquinazolinyl, wherein in each case the radical can optionally be substituted with 1, 2, 3, 4 or 5 substituents selected independently from the group consisting of oxo (=O), thioxo (=S), F, Cl, Br, I, - CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂,-C(=O)-NH-CH₃, -C(=O)-NH-C₂H₅, -C(=O)-N-(CH₃)₂, -C(=O)-N-(C₂H₅)₂,-S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂-phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
a radical selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, wherein in each case the radical can optionally be substituted with 1, 2, 3, 4 or 5 substituents selected independently from the group consisting of F, Cl, Br, I, -CN, -CF₃, -SF₅, -OH,-O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH,-C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂-phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl.

13. The compounds according to one or more of claims 1 to 12, **characterized in that**
R³⁴ and R⁴², independently of one another, each represents a radical selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl and pyrimidinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃.

14. The compounds according to one or more of claims 1 to 13, **characterized in that**
m is equal to 0.

15. The compounds according to one or more of claims 1 to 14, **characterized in that**
m is equal to 0, 1, 2, 3 or 4;
n is equal to 1;
R¹ represents a -C(=S)-NR⁸R⁹ group; or
if m is unequal to 0 and/or R⁵ is unequal to H, may represent a -C(=O)-NR⁶R⁷ group in addition;
R² represents a radical selected from the group consisting of methyl, ethyl, n-propyl, isopropyl, n-butyl, sec-butyl, isobutyl, tert-butyl, n-pentyl, sec-pentyl, n-hexyl and n-heptyl;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, and thiomorpholinyl, wherein in each case the radical can be substituted with 1 or 2 substituents selected independently from the group consisting of pyridinyl, pyridazinyl, phenyl and benzyl, wherein in each case the cyclic portion of the radicals pyridinyl, pyridazinyl, phenyl and benzyl can be substituted with 1, 2, 3, 4 or 5 substituents selected independently from the group consisting of -CF₃, F, Cl, Br, methyl, ethyl, n-propyl, isopropyl, n-butyl, sec-butyl, isobutyl, tert-butyl, -O-CH₃ and-O-C₂H₅;
a radical selected from the group consisting of phenyl, naphthyl, thiophenyl, furanyl, pyrrolyl, pyrazolyl, pyrazinyl and pyridinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, ₋S(=O)₂₋CH₃, -S(=O)₂-C₂H₅, -NH-S(=O)₂-CH₃, -NH-S(=O)₂-C₂H₅, -S(=O)₂-NH-CH₃ and -S(=O)₂-NH-C₂H₅;
or -(CHR³⁰)-R³³; -(CHR³⁰)-(CHR³¹)-R³³ or -(CHR³⁰)-(CHR³¹)-(CHR³²)-R³³;
R³ represents a hydrogen radical;
or
R² and R³ form, together with the nitrogen atom joining them as ring member, a radical selected from the group consisting of pyrrolidinyl, piperidinyl, piperazinyl, 3-methyl-piperazinyl, 2-methyl-piperazinyl, (3,5)-dimethylpiperazinyl, (2,6)-dimethylpiperazinyl, morpholinyl and thiomorpholinyl, wherein in each case the radical can be substituted with 1 or 2 substituents selected independently from the group consisting of -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅,-C(=O)-N-(CH₃)₂, -C(=O)-N-(C₂H₅)₂, -S(=O)₂-CH₃, -S(=O)₂-C₂H₅,-S(=O)₂ phenyl, pyridinyl, pyridazinyl, phenyl and benzyl, wherein in each case the cyclic portion of the radicals pyridinyl, -S(=O)₂ phenyl, pyridazinyl, phenyl and benzyl can be substituted with 1, 2, 3, 4 or 5 substituents selected independently from the group consisting of -CF₃, F, Cl, Br, methyl, ethyl, n-propyl, isopropyl, n-butyl, sec-butyl, isobutyl, tert-butyl, -O-CH₃ and -O-C₂H₅;
R⁴ represents a radical selected from the group consisting of methyl, ethyl, n-propyl, isopropyl, n-butyl, sec-butyl, isobutyl, tert-butyl, n-pentyl, sec-pentyl, n-hexyl and n-heptyl;
R⁵ represents a hydrogen radical;
a radical selected from the group consisting of methyl, ethyl, n-propyl, isopropyl, n-butyl, sec-butyl, isobutyl, tert-butyl, n-pentyl, sec-pentyl, n-hexyl and n-heptyl;
a phenyl radical, which can in each case be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃,-S-CF₃, -SH, -S-CH₃, -S-C₂H₅, methyl, ethyl, n-propyl, isopropyl, n-butyl, sec-butyl, isobutyl and tert-butyl;
or -(CH₂)-R³⁴;
R⁶ and R⁸, independently of one another, each represents a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1-butinyl, 2-butinyl and 3-butinyl, wherein in each case the radical can optionally be substituted with 1, 2, 3, 4 or 5 substituents selected independently from the group consisting of F, Cl and Br;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl;
a radical selected from the group consisting of phenyl, naphthyl, thiophenyl, furanyl, pyrrolyl, pyrazolyl, pyranyl, triazolyl, pyridinyl, imidazolyl, thiazolyl, oxazolyl, isoxazolyl, pyridazinyl, pyrazinyl and pyrimidinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH,-S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃,-C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂,-C(=O)-N-(C₂H₅)₂, -S(=O)₂-CH₃, -S(=O)₂-C₂H₅, -S(=O)₂ phenyl,-NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl,-O-benzyl, phenyl, -(CH₂)-benzo[b]furanyl and benzyl can be substituted with 1, 2, 3, 4 or 5 substituents selected independently from the group consisting of F, Cl, Br, -OH, -CF₃,-SF₅, -CN, -NO₂, methyl, wthyl, n-propyl, isopropyl, n-butyl, sec-butyl, isobutyl, tert-butyl, -O-CH₃, -O-C₂H₅, -O-CF₃, -S-CF₃, phenyl and -O-benzyl;
-(CHR³⁶)-C(=O)-O-R²⁵ or -(CHR³⁶)-(CH₂)-C(=O)-O-R²⁵;
or -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ or-(CR³⁷R³⁸)-(CHR³⁹)-(CHR⁴⁰)-N(C₂H₅)-R⁴¹;
R⁷ and R⁹ each represents a hydrogen radical;
R²⁵ represents a radical selected from the group consisting of methyl, ethyl, n-propyl, isopropyl, n-butyl, sec-butyl, isobutyl, and tert-butyl;
R²⁶, R²⁷, R²⁸, R³⁰, R³¹, R³², R³⁶, R³⁷, R³⁸, R³⁹ and R⁴⁰, independently of one another, each represents
a hydrogen radical;
a radical selected from the group consisting of methyl, ethyl, n-propyl, isopropyl, n-butyl, sec-butyl, isobutyl, and tert-butyl;
or a phenyl radical which can be substituted with 1, 2, 3, 4 or 5 substituents selected independently from the group consisting of F, Cl, Br, I, -CN, -CF₃, -O-CH₃, -O-C₂H₅, -NO₂, -O-CF₃, methyl, ethyl, n-propyl, isopropyl, n-butyl, sec-butyl, isobutyl and tert-butyl;
R²⁹ and R³³, independently of one another, each represents
a radical selected from the group consisting of phenyl, naphthyl, thiophenyl, furanyl, pyrrolyl, pyrazolyl, pyrazinyl and pyridinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ and -S(=O)₂-NH-C₂H₅;
R³⁴ represents a phenyl radical which can be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl and tert-butyl;
and
R⁴¹ represents a radical selected from the group consisting of methyl, ethyl, n-propyl, isopropyl, n-butyl, sec-butyl, isobutyl and tert-butyl;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl;
or a radical selected from the group consisting of phenyl, naphthyl, (1,3)-benzodioxolyl, (1,4)-benzodioxanyl, thiophenyl and furanyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃,-NH-C(=O)-O-C₂H₅, -NH-C(=O)-O-C(CH₃)₃, -C(=O)-H, -C(=O)-CH₃,-C(=O)-C₂H₅ and -C(=O)-C(CH₃)₃;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates.

16. The compounds according to one or more of claims 1 to 15, **characterized in that**
m is equal to 0;
n is equal to 1;
R¹ represents a -C(=S)-NR8R9 group;
R² represents a radical selected from the group consisting of phenyl, naphthyl and pyridinyl, wherein in each case the radical can be substituted with 1, 2 or 3 substituents selected independently from the group consisting of F, Cl, Br, -OH, -O-CH₃, -O-C₂H₅, -NH-S(=O)₂-CH₃ and -NH-S(=O)₂-C₂H₅;
a piperazinyl radical which can be substituted on a nitrogen atom with a substituent selected from the group consisting of pyridinyl, pyridazinyl, phenyl and benzyl, wherein in each case the cyclic portion of the substituents pyridinyl, pyridazinyl, phenyl and benzyl can be substituted with 1, 2 or 3 substituents selected independently from the group consisting of -CF₃, F, Cl, Br, methyl, ethyl, n-propyl, isopropyl, n-butyl, sec-butyl, isobutyl and tert-butyl;
or -(CHR³⁰)-R³³; -(CHR³⁰)-(CHR³¹)-R³³ or -(CHR³⁰)-(CHR³¹)-(CHR³²)-R³³;
R³ represents a hydrogen radical;
or
R² and R³ form, together with the nitrogen atom joining them as ring member, a radical selected from the group consisting of 3-methyl-piperazinyl, 2-methyl-piperazinyl, (3,5)-dimethylpiperazinyl, (2,6)-dimethylpiperazinyl and piperazinyl, which can be substituted on a nitrogen atom with a substituent selected from the group consisting of pyridinyl, pyridazinyl, phenyl and benzyl, wherein in each case the cyclic portion of the substituents pyridinyl, pyridazinyl, phenyl and benzyl can be substituted with 1, 2 or 3 substituents selected independently from the group consisting of -CF₃, F, Cl, Br, methyl, ethyl, n-propyl, isopropyl, n-butyl, sec-butyl, isobutyl and tert-butyl;
R⁵ represents a hydrogen radical;
R⁶ and R⁸, independently of one another, each represents a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, vinyl, 1-propenyl, 2-propenyl, 1-butenyl, 2-butenyl, 3-butenyl and 2-methyl-1-propenyl, wherein in each case the radical can optionally be substituted with 1, 2, 3, 4 or 5 substituents selected independently from the group consisting of F, Cl and Br;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl and adamantyl;
a radical selected from the group consisting of phenyl, naphthyl and pyridinyl, wherein in each case the radical can optionally be substituted with 1, 2, 3, 4 or 5 substituents selected independently from the group consisting of -SF₅, F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl,-C(=O)-OH, -C(=O)-O-CH₃, -C(=O)-O-C₂H₅, -C(=O)-O-C(CH₃)₃,-O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -C(=O)-CH₃, -C(=O)-C₂H₅,-C(=O)-C(CH₃)₃, cyclohexyl, cyclopentyl, -O-phenyl, -O-benzyl and phenyl, wherein in each case the cyclic portion of the radicals cyclopentyl, cyclohexyl, -O-phenyl, -O-benzyl and phenyl can be substituted with 1, 2, 3, 4 or 5 substituents selected independently from the group consisting of F, Cl, Br, methyl, ethyl, n-propyl, isopropyl, n-butyl, sec-butyl, isobutyl and tert-butyl;
-(CHR³⁶)-C(=O)-O-R²⁵ or -(CHR³⁶)-(CH₂)-C(=O)-O-R²⁵;
or -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ or-(CR³⁷R³⁸)-(CHR³⁹)-(CHR⁴⁰)-N(C₂H₅)-R⁴¹;
R⁷ and R⁹ each represents a hydrogen radical;
R²⁵ each represents a radical selected from the group consisting of methyl, ethyl, n-propyl, isopropyl, n-butyl, sec-butyl, isobutyl, and tert-butyl;
R³⁰, R³¹, R³² , R³⁹ and R⁴⁰, each represents a hydrogen radical;
R³³ represents a radical selected from the group consisting of phenyl, naphthyl, thiophenyl and furanyl, wherein in each case the radical can optionally be substituted with 1, 2, 3, 4 or 5 substituents selected independently from the group consisting of -CF₃, F, Cl, Br, -OH, -O-CH₃, -O-C₂H₅, methyl, ethyl, n-propyl, isopropyl, n-butyl, sec-butyl, isobutyl, tert-butyl, -NH-S(=O)₂-CH₃ and -NH-S(=O)₂-C₂H₅;
R³⁶ represents a hydrogen radical;
or a radical selected from the group consisting of methyl, ethyl, n-propyl, isopropyl, n-butyl, sec-butyl, isobutyl and tert-butyl;
R³⁷ and R³⁸, independently of one another, each represents
a hydrogen radical;
a radical selected from the group consisting of methyl, ethyl, n-propyl, isopropyl, n-butyl, sec-butyl, isobutyl and tert-butyl;
or a phenyl radical.
R⁴¹ represents a radical selected from the group consisting of methyl, ethyl, n-propyl, isopropyl, n-butyl, sec-butyl, isobutyl and tert-butyl;
a radical selected from the group consisting of tetrahydrofuranyl, tetrahydrothiophenyl, pyrrolidinyl, morpholinyl, piperidinyl and piperazinyl;
or a radical selected from the group consisting of phenyl, naphthyl, (1,3)-benzodioxolyl, (1,4)-benzodioxanyl, thiophenyl and furanyl, wherein in each case the radical can optionally be substituted with 1, 2, 3, 4 or 5 substituents selected independently from the group consisting of F, Cl, Br, -OH, -O-CH₃, -O-C₂H₅, methyl, ethyl, n-propyl, isopropyl, n-butyl, sec-butyl, isobutyl and tert-butyl;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates.

17. The compounds according to one or more of claims 1 to 16, **characterized in that** in a FLIPR assay in a concentration of 10 µM the compounds display inhibition of the Ca²⁺ ion inflow in dorsal root ganglia of rats of at least 10 %, preferably of at least 30 %, particularly preferably of at least 50 %, most particularly preferably of at least 70 %, even more preferably of at least 90 %, compared to the maximum achievable inhibition of the Ca²⁺ ion inflow with capsaicin in a concentration of 10 µM.

18. The method for preparing substituted spiro compounds of the general formula I according to one or more of claims 1 to 17, **characterized in that** at least one compound of the general formula II, 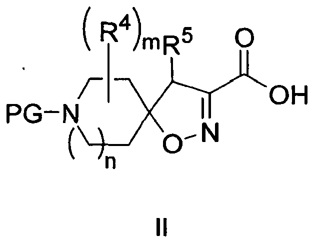 wherein R⁴, R⁵, m and n are as defined in one or more of claims 1 to 17 and PG represents a protective group, preferably a benzyloxycarbonyl or tert-butyloxycarbonyl group, is reacted in a reaction medium in the presence of at least one coupling reagent, optionally in the presence of at least one base, with a compound of the general formula HNR²R³, wherein R² and R³ are as defined in one or more of claims 1 to 17, to form at least one compound of the general formula III, 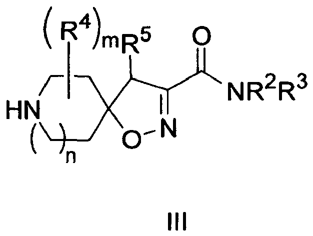 wherein R², R³, R⁴, R⁵, m, n and PG are as defined above, and at least one compound of the general formula III is optionally purified and/or isolated,
and
at least one compound of the general formula III is reacted in a reaction medium in the presence of at least one acid, preferably in the presence of at least one inorganic or organic acid selected from the group consisting of hydrochloric acid, sulphuric acid, acetic acid and trifluoroacetic acid, or in the
presence of hydrogen and a catalyst, preferably a catalyst based on palladium or platinum, to form at least one compound of the general formula IV, 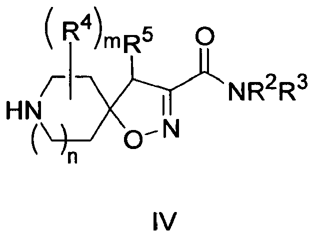 wherein R², R³, R⁴, R⁵, m and n are as defined above, and at least one compound of the general formula I is optionally purified and/or isolated,
and
at least one compound of the general formula IV is reacted in a reaction medium with at least one isocyanate of the general formula R⁶-N=C=O, wherein R⁶ is as defined in one or more of claims 1 to 17, optionally in the presence of at least one base, preferably in the presence of at least one base selected from the group consisting of triethylamine, 4,4-dimethylaminopyridine and diisopropylethylamine, to form at least one compound of the general formula I, wherein R², R³, R⁴, R⁵, m and n are as defined above and R¹ represents -C(=O)-NR⁶R, wherein R⁶ is as defined above and R⁷ represents a hydrogen radical, and at least one compound of the general formula I is optionally purified and/or isolated,
or
at least one compound of the general formula IV is reacted in a reaction medium with at least one isothiocyanate of the general formula S=C=N-R⁸, wherein R⁸ is as defined in one or more of claims 1 to 17, optionally in the presence of at least one base, preferably in the presence of at least one base selected from the group consisting of triethylamine, 4,4-dimethylaminopyridine and diisopropylethylamine, to form at least one compound of the general formula I, wherein R², R³, R⁴, R⁵, m and n are as defined above and R¹ represents -C(=S)-N-R⁸R⁹, wherein R⁸ is as defined above and R⁹ represents a hydrogen radical, and the at least one compound of the general formula I is optionally purified and/or isolated
and optionally at least one compound of the general formula I, wherein R², R³, R⁴, R⁵, m and n are as defined above and R¹ represents -C(=O)-NR⁶R⁷ or - C(=S)-N-R⁸R⁹, wherein R⁷ and R⁹ each represent a hydrogen radical, is reacted in a reaction medium, in the presence of at least one base, preferably in the presence of at least one metal hydride salt or a metal alcoholate salt, particularly preferably in the presence of a metal hydride salt or a metal alcoholate salt selected from the group consisting of sodium hydride, potassium hydride, potassium tert-butanolate, sodium tert-butanolate, potassium methanolate, sodium methanolate, sodium ethanolate and potassium ethanolate, with at least one compound of the general formula LG-R⁷ or of the general formula LG-R⁹, wherein LG represents a leaving group, preferably a halogen atom, particularly preferably a chlorine atom, and R⁷ and R⁹ are as defined above except for hydrogen, to form at least one compound of the general formula I, wherein R¹ to R⁵, m and n are as defined above, and the at least one compound of the general formula I is optionally purified and/or isolated.

19. Medicament comprising at least one compound according to one or more of claims 1 to 17 and optionally one or more physiologically compatible adjuvants.

20. The medicament according to claim 19 for the prophylaxis and/or treatment of pain, preferably of pain selected from the group consisting of acute pain, chronic pain, neuropathic pain and visceral pain.

21. The medicament according to claim 20 for the prophylaxis and/or treatment of one or more diseases selected from the group consisting of pain, preferably of pain selected from the group consisting of acute pain, chronic pain, neuropathic pain and visceral pain; arthralgia; migraine; depression; neuropathy; nerve injuries; neurodegenerative diseases, preferably selected from the group consisting of multiple sclerosis, Alzheimer's disease, Parkinson's disease and Huntington's disease; cognitive dysfunctions, preferably cognitive deficiencies, particularly preferably paramnesia; epilepsy; respiratory diseases, preferably selected from the group consisting of asthma and pneumonia; coughing; urinary incontinence; OAB (overactive bladder); stomach ulcers; irritable bowel syndrome; strokes; irritations of the eyes; irritations of the skin; neurotic skin diseases; inflammatory diseases, preferably intestinal inflammations; diarrhoea; pruritus; eating disorders, preferably selected from the group consisting of bulimia, cachexia, anorexia and obesity; medication dependency; medication abuse; withdrawal symptoms in medication dependency; development of tolerance to medications, preferably to natural or synthetic opioids; drug addiction; drug abuse; withdrawal symptoms in drug addiction; alcohol addiction; alcohol abuse and withdrawal symptoms in alcohol addiction; for diuresis; for antinatriuresis; for influencing the cardiovascular system; for increasing vigilance; for increasing libido; for modulating motor activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil (DA-5018).

22. The use of at least one compound according to one or more of claims 1 to 17 for producing a medicament for the prophylaxis and/or treatment of pain, preferably of pain selected from the group consisting of acute pain, chronic pain and neuropathic pain.

23. The use of at least one compound according to one or more of claims 1 to 17 for producing a medicament for the prophylaxis and/or treatment of one or more diseases selected from the group consisting of pain, preferably of pain selected from the group consisting of acute pain, chronic pain, neuropathic pain and visceral pain; arthralgia; migraine; depression; neuropathy; nerve injuries; neurodegenerative diseases, preferably selected from the group consisting of multiple sclerosis, Alzheimer's disease, Parkinson's disease and Huntington's disease; cognitive dysfunctions, preferably cognitive deficiencies, particularly preferably paramnesia; epilepsy; respiratory diseases, preferably selected from the group consisting of asthma and pneumonia; coughing; urinary incontinence; OAB (overactive bladder); stomach ulcers; irritable bowel syndrome; strokes; irritations of the eyes; irritations of the skin; neurotic skin diseases; inflammatory diseases, preferably intestinal inflammations; diarrhoea; pruritus; eating disorders, preferably selected from the group consisting of bulimia, cachexia, anorexia and obesity; medication dependency; medication abuse; withdrawal symptoms in medication dependency; development of tolerance to medication, preferably to natural or synthetic opioids; drug addiction; drug abuse; withdrawal symptoms in drug addiction; alcohol addiction; alcohol abuse and withdrawal symptoms in alcohol addiction; for diuresis; for antinatriuresis; for influencing the cardiovascular system; for increasing vigilance; for increasing libido; for modulating motor activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil (DA-5018).

24. The use of at least one of the substituted spiro compounds of the general formula I, 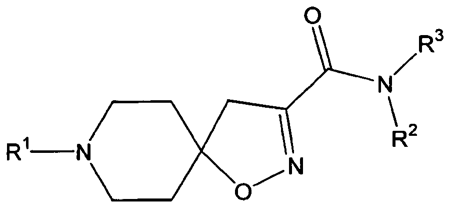 wherein
R¹ represents a -C(=O)-NR⁶R⁷ group;
R² represents a linear or branched, saturated or unsaturated, unsubstituted or at least monosubstituted aliphatic radical optionally including at least one heteroatom as a chain member,
where the substituents of the aliphatic radical may be selected independently from the group consisting of F, Cl, Br, I,-CN,-NO_{2,} -OH, - SH and -NH₂;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
R³ represents a hydrogen radical;
a linear or branched, saturated or unsaturated, unsubstituted or at least monosubstituted aliphatic radical optionally including at least one heteroatom as a chain member;
where the substituents of the aliphatic radical may be selected independently from the group consisting of F, Cl, Br, I,-CN,-NO₂, -OH, - SH and -NH₂;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or
R² and R³, together with the nitrogen atom joining them, form a unsubstituted or at least monosubstituted, unsaturated or saturated cycloaliphatic radical optionally including at least one further heteroatom as ring member which can be condensed with an unsubstituted or at least monosubstituted monocyclic or polycyclic ring system;
R⁶ represents a linear or branched, saturated or unsaturated, unsubstituted or at least monosubstituted aliphatic radical optionally including at least one heteroatom as a chain member;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system and/or can be bridged with at least a linear or branched, unsubstituted or at least monosubstituted alkylene group;
an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
a -C(=O)-R²⁴ group which can be bonded via a linear or branched, unsubstituted or at least monosubstituted alkylene group;
or a -C(=O)-O-R²⁵ group which can be bonded via a linear or branched, unsubstituted or at least monosubstituted alkylene group;
R⁷ represents hydrogen radical;
a linear or branched, saturated or unsaturated, unsubstituted or at least monosubstituted aliphatic radical optionally including at least one heteroatom as a chain member;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system, and/or can be bridged with at least a linear or branched, unsubstituted or at least monosubstituted alkylene group;
an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
a -C(=O)-R²⁴ group which can be bonded visa, a linear or branched, unsubstituted or at least monosubstituted alkylene group;
or a -C(=O)-O-R²⁵ group which can be bonded via a linear or branched, unsubstituted or at least monosubstituted alkylene group;
and
R²⁴ and R²⁵, independently of one another, each represents
a linear or branched, saturated or unsaturated, unsubstituted or at least monosubstituted aliphatic radical optionally including at least one heteroatom as a chain member;
an unsubstituted or at least monosubstituted, unsaturated or saturated cycloaliphatic radical optionally including at least one heteroatom as ring member which can be bonded via a linear or branched, unsubstituted or at least monosubstituted alkylene, alkenylene or alkinylene group optionally including at least one heteroatom as a chain member and/or can be condensed with an unsubstituted or at least monosubstituted monocyclic or polycyclic ring system;
or an unsubstituted or at least monosubstituted aryl or heteroaryl radical which can be bonded via a linear or branched, unsubstituted or at least monosubstituted alkylene, alkenylene or alkinylene group optionally including at least one heteroatom as a chain member;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates;
for the production of a medicament for the prophylaxis and/or treatment of disorders or diseases which are transmitted, at least in some cases, by vanilloid receptors 1 (VR1/TRPV1).

25. The use according to claim 24 for the treatment and/or prophylaxis of one or more diseases selected from the group consisting of acute pain; visceral pain; arthralgia; cognitive dysfunctions, preferably cognitive deficiencies, particularly preferably paramnesia; pneumonia; coughing; OAB (overactive bladder); irritable bowel syndrome; irritations of the eyes; irritations of the skin; neurotic skin diseases; intestinal inflammations; development of tolerance to medications, preferably to natural or synthetic opioids; and/or for diuresis or for antinatriuresis and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

26. The use according to claim 24 or 25, **characterized in that**
R¹ represents a -C(=O)-NR⁶R⁷ group;
R² represents a linear or branched, saturated or unsaturated, optionally substituted C₁₋₁₀ aliphatic radical;
an unsaturated or saturated, optionally substituted 3-, 4-, 5-, 6-, 7-, 8- or 9-membered cycloaliphatic radical which can be condensed with a saturated, unsaturated or aromatic, optionally substituted monocyclic or polycyclic ring system;
an optionally substituted 5- to 14-membered aryl or heteroaryl radical which can be condensed with a saturated or unsaturated, optionally substituted monocyclic or polycyclic ring system;
or -(CHR³⁰)-Xᵥ-(CHR³¹)_{w}-Yₓ-(CHR³²)y-Z_{z}-R³³ with v = 0 or 1, w = 0 or 1, x = 0 or 1, y = 0 or 1, z = 0 or 1, wherein X, Y and Z each independently represents O, S, NH, N(CH₃), N(C₂H₅) or N[CH(CH₃)₂];
R³ represents a hydrogen radical;
a linear or branched, saturated or unsaturated, optionally unsubstituted C₁-₁₀ aliphatic radical;
an unsaturated or saturated, optionally substituted 3-, 4-, 5-, 6-, 7-, 8- or 9-membered cycloaliphatic radical which can be condensed with a saturated, unsaturated or aromatic, optionally substituted monocyclic or polycyclic ring system;
an optionally substituted 5- to 14-membered aryl or heteroaryl radical which can be condensed with a saturated or unsaturated, optionally substituted monocyclic or polycyclic ring system;
or -(CHR³⁰)-Xᵥ-(CHR³¹)_{w}-Yₓ-(CHR³² )_{y}-Z_{z}-R³³ mit v = 0 or 1, w = 0 or 1, x = 0 or 1, y = 0 or 1, z = 0 or 1, wherein X, Y and Z each independently represents O, S, NH, N(CH₃), N(C₂H₅) or N[CH(CH₃)₂];
or
R² and R³, together with the nitrogen atom joining them as ring member, form a saturated or unsaturated, optionally substituted 4-, 5-, 6-, 7-, 8- or 9-membered heterocycloaliphatic radical which can be condensed with a saturated, unsaturated or aromatic, optionally substituted monocyclic or polycyclic ring system;
R⁶ represents a linear or branched, saturated or unsaturated, optionally substituted C₁₋₂₀ aliphatic radical;
an unsaturated or saturated, optionally substituted 3-, 4-, 5-, 6-, 7-, 8-, 9-, 10-, 11- or 12-membered cycloaliphatic radical which can be condensed with a saturated, unsaturated or aromatic, optionally substituted monocyclic or polycyclic ring system and/or can be bridged with one or two linear or branched, optionally substituted C₁₋₅-alkylene groups;
an optionally substituted 5- to 14-membered aryl or heteroaryl radical which can be condensed with a saturated or unsaturated, optionally substituted monocyclic or polycyclic ring system;
-(CHR³⁵)-(CH₂)_{bb}-(CH₂)_{cc}-C(=O)-R²⁴ with bb = 0 or 1 and cc = 0 or 1;
-(CHR³⁶)-(CH₂)_{dd}-(CH₂)ₑₑ-C(=O)-O-R²⁵ with dd = 0 or 1 and ee = 0 or 1;
or -(CR³⁷R³⁸)-X_{ff}-(CHR³⁹)_{gg}-Yₕₕ-(CHR⁴¹)ⱼⱼ-Zₖₖ-R⁴¹ with ff = 0 or 1, gg = 0 or 1, hh = 0 or 1, jj = 0 or 1, kk = 0 or 1, wherein X, Y and Z each independently represents O, S, NH, N(CH₃), N(C₂H₅) or N[CH(CH₃)₂];
R⁷ represents a hydrogen radical;
a linear or branched, saturated or unsaturated, optionally substituted C₁₋₂₀ aliphatic radical;
an unsaturated or saturated, optionally substituted 3-, 4-, 5-, 6-, 7-, 8-, 9-, 10-, 11- or 12-membered cycloaliphatic radical which can be condensed with a saturated, unsaturated or aromatic, optionally substituted monocyclic or polycyclic ring system and/or can be bridged with one or two linear or branched, optionally substituted C1-5-alkylene groups;
an optionally substituted 5- to 14-membered aryl or heteroaryl radical which can be condensed with a saturated or unsaturated, optionally substituted monocyclic or polycyclic ring system;
-(CHR³⁵)-(CH₂)_{bb}-(CH₂)_{cc}-C(=O)-R²⁴ with bb = 0 or 1 and cc = 0 or 1;
-(CHR³⁶)-(CH₂)_{dd}-(CH₂)ₑₑ-C(=O)-O-R²⁵ with dd = 0 or 1 and ee = 0 or 1;
or (CR³⁷R³⁸)-X_{ff}CHR³⁹)_{gg}-Yₕₕ-(CHR⁴⁰)ⱼⱼ-Zₖₖ-R⁴¹ with ff = 0 or 1, gg = 0 or 1, hh = 0 or 1, jj = 0 or 1, kk = 0 or 1, wherein X, Y and Z each independently represents O, S, NH, N(CH₃), N(C₂H₅) or N[CH(CH₃)₂];
R²⁴ and R²⁵, independently of one another, each represents
a linear or branched, saturated or unsaturated, optionally substituted C₁₋₁₀ aliphatic radical;
an unsaturated or saturated, optionally substituted 3-, 4-, 5-, 6-, 7-, 8- or 9-membered cycloaliphatic radical which can be condensed with a saturated, unsaturated or aromatic, optionally substituted monocyclic or polycyclic ring system;
an optionally substituted 5- to 14-membered aryl or heteroaryl radical which can be condensed with a saturated or unsaturated, optionally substituted monocyclic or polycyclic ring system;
or -(CH₂)ₘₘ-R⁴² with mm equal to 1, 2 or 3;
R³⁰, R³¹, R³², R³⁵, R³⁶, R³⁷, R³⁸, R³⁹ and R⁴⁰, independently of one another, each represents
a hydrogen radical;
a linear or branched, saturated or unsaturated, optionally unsubstituted C₁₋₁₀ aliphatic radical;
or an optionally substituted 5- to 14-membered aryl or heteroaryl radical which can be condensed with a saturated or unsaturated, optionally substituted monocyclic or polycyclic ring system;
R³³ and R⁴¹, independently of one another, each represents
a linear or branched, saturated or unsaturated, optionally unsubstituted C₁₋₁₀ aliphatic radical;
an unsaturated or saturated, optionally substituted 3-, 4-, 5-, 6-, 7-, 8- or 9-membered cycloaliphatic radical which can be bridged with 1, 2, 3, 4 or 5 linear or branched, optionally substituted C₁₋₅ alkylene groups and/or can be condensed with a saturated, unsaturated or aromatic, optionally substituted monocyclic or polycyclic ring system;
or an optionally substituted 5- to 14-membered aryl or heteroaryl radical which can be condensed with a saturated or unsaturated, optionally substituted monocyclic or polycyclic ring system;
and
R³⁴ and R⁴², independently of one another, each represents
an optionally substituted 5- to 14-membered aryl or heteroaryl radical;
wherein
each of the above-mentioned C₁₋₁₀ aliphatic radicals and C₁₋₂₀ aliphatic radicals can optionally be substituted with 1, 2, 3, 4, 5, 6, 7, 8 or 9 substituents selected independently from the group consisting of F, Cl, Br, I, -CN, -NO₂, - OH, -SH and -NH₂;
each of the above-mentioned cycloaliphatic radicals and heterocyclo-aliphatic radicals can optionally be substituted with 1, 2, 3, 4 or 5 substituents selected independently from the group consisting of oxo (=O), thioxo (=S), F, Cl, Br, I, - CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, - C(=O)-NH₂, -C(=O)-NH-C₁₋₅ alkyl, C(=O)-N-(C₁₋₅ alkyl)₂, -S(=O)₂-C₁₋₅ alkyl, - S(=O)₂ phenyl, -NH-S(=O)₂-C₁₋₅ alkyl, -S(=O)₂-NH-C₁₋₅ alkyl, cyclohexyl, cyclopentyl, pyridinyl, pyridazinyl, -(CH₂) benzo[b]furanyl, -O-phenyl, -O benzyl, phenyl and benzyl, wherein in each case the cyclic portion of the radicals pyridinyl, cyclopentyl, cyclohexyl, pyridazinyl, -S(=O)₂ phenyl, -O-phenyl, -O benzyl, phenyl, -(CH₂) benzo[b]furanyl and benzyl can be substituted with 1, 2, 3, 4 or 5 substituents selected independently from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl;
and each of the above-mentioned cycloaliphatic radicals can optionally have 1, 2, 3 4, or 5 heteroatoms as ring members selected independently from the group consisting of oxygen, nitrogen and sulphur;
each of the above-mentioned heterocycloaliphatic radicals can optionally have 1, 2 or 3 additional heteroatoms as ring members selected independently from the group consisting of oxygen, nitrogen and sulphur;;
each of the rings of the above-mentioned monocyclic or polycyclic ring systems can optionally be substituted with 1, 2, 3, 4 or 5 substituents selected independently from the group consisting of oxo (=O), thioxo (=S), F, Cl, Br, I, - CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, - C(=O)-NH₂, -C(=O)-NH-C₁₋₅ alkyl, C(=O)-N-(C₁₋₅ alkyl)₂, -S(=O)₂-C₁₋₅ alkyl, - S(=O)₂ phenyl, -NH-S(=O)₂-C₁₋₅ alkyl, -S(=O)₂-NH-C₁₋₅ alkyl, cyclohexyl, cyclopentyl, pyridinyl, pyridazinyl, -(CH₂) benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl;
and each of the rings of the above-mentioned monocyclic or polycyclic ring systems can optionally have 5, 6 or 7 members and can optionally have 1, 2, 3, 4 or 5 heteroatoms as ring members selected independently from the group consisting of oxygen, nitrogen and sulphur;
and each of the above-mentioned aryl or heteroaryl radicals can optionally be substituted with 1, 2, 3, 4 or 5 substituents selected independently from the group consisting of F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, - C(=O)-H, -C(=O)-C₁₋₅ alkyl, -C(=O)-NH₂, -C(=O)-NH-C₁₋₅ alkyl, C(=O)-N-(C₁₋₅ alkyl)₂, -S(=O)₂-C₁₋₅ alkyl, -S(=O)₂ phenyl, -NH-S(=O)₂-C₁₋₅ alkyl, -S(=O)₂-NH-C₁₋₅ alkyl, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl;
each of the above-mentioned heteroaryl radicals can optionally have 1, 2, 3, 4 or 5 heteroatoms as ring members selected independently from the group consisting of oxygen, nitrogen and sulphur;
and
each of the above-mentioned C₁₋₅ alkyl groups can optionally be substituted with 1, 2, 3, 4 or 5 substituents selected independently from the group consisting of F, Cl, Br, -OH, -SH, -NH₂, -CN and NO₂;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates.

27. The use according to one or more of claims 24-26, **characterized in that**
R² represents a radical selected from the group consisting of methyl, ethyl, n-propyl, isopropyl, n-butyl, sec-butyl, isobutyl, tert-butyl, n-pentyl, sec-pentyl,-(CH₂)-(CH₂)-(C(CH₃)₃), n-hexyl, n-heptyl, n-octyl,-(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl, wherein in each case the radical can optionally be substituted with 1, 2, 3, 4 or 5 substituents selected independently from the group consisting of F, Cl, Br, I, -CN, -NO₂, -OH, -SH and -NH₂;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-nenzodioxanyl, (1,2,3,4)-tetrahydronaphthyl, (1,2,3,4)-tetrahydrochinolinyl and (1,2,3,4)-tetrahydrochinazolinyl, wherein in each case the radical can optionally be substituted with 1, 2, 3, 4 or 5 substituents selected independently from the group consisting of oxo (=O), thioxo (=S), F, Cl, Br, I, - CN, -CF₃, -SFₛ, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SFs, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
a radical selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, chinazolinyl, chinolinyl, isochinolinyl, benzimidazolinyl, benzoxazolyl, benzisoxazolyl and benzothiazolyl, wherein in each case the radical can optionally be substituted with 1, 2, 3, 4 or 5 substituents selected independently from the group consisting of F, Cl, Br, I, -CN, -CF₃, -SF₅, -OH, - O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, - O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or -(CHR³⁰)-R³³; -(CHR³⁰)-(CHR³¹)-R³³; -(CHR³⁰)-(CHR³¹)-O-R³³; -(CHR³⁰)-(CHR³¹)-(CHR³²)-R³³; -(CHR³⁰)-(CHR³¹)-S-(CHR³²)-R³³; -(CHR³⁰)-(CHR³¹)-(CHR³²)-N(CH₃)-R³³_{.}

28. The use according to one or more of claims 24-27, **characterized in that**
R³ represents a hydrogen radical.

29. The use according to one or more of claims 24-28, **characterized in that**
R² and R³ form, together with the nitrogen atom joining them as ring member, a radical selected from the group consisting of pyrrolidinyl, piperidinyl, (1,3,4,5)-tetrahydropyrido[4,3-b]indolyl, (3,4)-dihydro-1 H-isochinolinyl, (1,3,4,9)-tetrahydro-[b]-carbolinyl, imidazolidinyl, (1,3)-thiazolidinyl, piperazinyl, morpholinyl, azepanyl, diazepanyl and thiomorpholinyl bilden, wherein in each case the radical can be substituted with 1, 2, 3, 4 or 5 substituents selected independently from the group consisting of oxo (=O), thioxo (=S), F, Cl, Br, I, -CN, -CF₃, -SF₅, -OH, -O-CH₃, - O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂Hₛ, -C(=O)-O-C(CH₃)₃, -O-C(=O)-CH₃, -O-C(=O)-C₂H₅, -0-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, - O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl.

30. The use according to one or more of claims 24-29, **characterized in that**
R⁶ represents a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1-butinyl, 2-butinyl and 3-butinyl, wherein in each case the radicals can optionally be substituted with 1, 2, 3, 4 or 5 substituents selected independently from the group consisting of F, Cl, Br, I, -CN, -NO₂, -OH, -SH and -NH₂;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein in each case the radical can optionally be substituted with 1, 2, 3, 4 or 5 substituents selected independently from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, - SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, - C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, - OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -0-benzyl;
a radical selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[blfuranyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, wherein in each case the radical can optionally be substituted with 1, 2, 3, 4 or 5 substituents selected independently from the group consisting of F, Cl, Br, I, -CN, -CF₃, -SF₅, -OH, - O-CH₃, -O-C₂Hₛ, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH3, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, - O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[blfuranyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
-(CHR³⁵)-C(=O)-R²⁴ or -(CHR³⁵)-(CH₂)-C(=O)-R²⁴;
-(CHR³⁶)-C(=O)-O-R²⁵ or -(CHR³⁶)-(CH₂)-C(=O)-O-R²⁵;
or -(CR³⁷R³⁸)-R⁴¹, -(CR³⁷R³⁸)-(CHR³⁹)-R⁴¹, -(CR³⁷R³⁸)-(CHR³⁹)-O-R⁴¹,- (CR³⁷R³⁸)-(CHR³⁹)-(CHR⁴⁰)-R⁴¹, -(CR³⁷R³⁸)-(CHR³⁹)-(CHR⁴⁰)-O-R⁴¹, - (CR³⁷R³⁸)-(CHR³⁹)-(CHR⁴⁰)-N(CH₃)-R⁴¹ or -(CR³⁷R³⁸)-(CHR³⁹)-(CHR⁴⁰)-N(C₂H₅)-R⁴¹.

31. The use according to one or more of claims 24-30, **characterized in that**
R⁷ represents a hydrogen radical.

32. The use according to one or more of claims 24-31, **characterized in that**
R²⁴ and R²⁵, independently of one another, each represents a radical selected from the group consisting of methyl, ethyl, n-propyl, isopropyl, n-butyl, sec-butyl, isobutyl, tert-butyl, n-pentyl, sec-pentyl,-(CH₂)-(CH₂)-(C(CH₃)₃), n-hexyl, n-heptyl, n-octyl,-(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein in each case the radical can be substituted with 1 er 2 substituents selected independently from the group consisting of -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₅)₂, -C(=O)-N-(C₂H₅)₂, -S(=O)₂-CH₃, -S(=O)₂-C₂H₅, - S(=O)₂ phenyl, pyridinyl, phenyl and benzyl, wherein in each case the cyclic portion of the radicals pyridinyl, -S(=O)₂ phenyl, phenyl and benzyl can be substituted with 1, 2, 3, 4 or 5 substituents selected independently from the group consisting of F, Cl, Br, methyl, ethyl, n-propyl, isopropyl, n-butyl, sec-butyl, isobutyl, tert-butyl, -O-CH₃ and -O-C₂H₅;
a radical selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl and pyrimidinyl, wherein in each case the radical can optionally be substituted with 1, 2, 3, 4 or 5 substituents selected independently from the group consisting of F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, O-benzyl and phenyl,
or -(CH₂)-R⁴² or -(CH₂)-(CH₂)-R⁴².

33. The use according to one or more of claims 24-32, **characterized in that**
R³⁰, R³¹ , R³² , R³⁵, R³⁶, R³⁷, R³⁸ , R³⁹ and R⁴⁰, independently of one another, each represents
a hydrogen radical;
a radical selected from the group consisting of methyl, ethyl, n-propyl, isopropyl, n-butyl, sec-butyl, isobutyl, tert-butyl, n-pentyl, sec-pentyl,-(CH₂)-(CH₂)-(C(CH₃)₃), n-hexyl, n-heptyl, n-octyl,-(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl;
or a phenyl radical, which can be substituted with 1, 2, 3, 4 or 5 substituents selected independently from the group consisting of F, Cl, Br, I, -CN, -CF₃, -O-CH₃, -O-C₂H₅, -NO₂, -O-CF₃, -S-CH₃, -S-C₂H₅, methyl,ethyl, n-propyl, isopropyl, n-butyl, sec-butyl, isobutyl, tert-butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, -O-benzyl and phenyl.

34. The use according to one or more of claims 24-33, **characterized in that**
R³³ and R⁴¹, independently of one another, each represents a radical selected from the group consisting of methyl, ethyl, n-propyl, isopropyl, n-butyl, sec-butyl, Isobutyl, tert-butyl, n-pentyl, sec-pentyl,-(CH₂)-(CH₂)-(C(CH₃)₃), n-hexyl, n-heptyl, n-octy),-(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl, wherein in each case the radical can optionally be substituted with 1, 2, 3, 4 or 5 substituents selected independently from the group consisting F, Cl, Br, I, -CN, -NO₂, -OH, -SH and -NH₂;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydrochiriolinyl and (1,2,3,4)-tetrahydrochinazolinyl, wherein in each case the radical can optionally be substituted with 1, 2, 3, 4 or 5 substituents selected independently from the group consisting of oxo (=O), thioxo (=S), F, Cl, Br, I, - CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituent selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
a radical selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, chinazolinyl, chinolinyl, isochinolinyl, benzimidazolinyl, benzoxazolyl, benzisoxazolyl and benzothiazolyl, wherein in each case the radical can optionally be substituted with 1, 2, 3, 4 or 5 substituents selected independently from the group consisting of F, Cl, Br, I, -CN, -CF₃, -SF₅, -OH, - O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-bbtyl, sec-butyl, isobutyl, tert-butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, - O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl.

35. The use according to one or more of claims 24-34, **characterized in that**
R³⁴ and R⁴², independently of one another, each represents a radical selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl and pyrimidinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃.

36. The use according to one or more of claims 24-35, **characterized in that**
R¹ represents a -C(=O)-NR⁶R⁷ group;
R² represents a radical selected from the group consisting of methyl, ethyl, n-propyl, isopropyl, n-butyl, sec-butyl, isobutyl, tert-butyl, n-pentyl, sec-pentyl, n-hexyl and n-heptyl;
a radical selected from the group consisting of cyclopropyl, cyclobutyl, cyclopentyl, cyclohexyl, cycloheptyl, cyclopentenyl, cyclohexenyl, cycloheptenyl, imidazolidinyl; tetrahydrofuranyl, tetrahydrothiophenyl, pyrrolidinyl, piperidinyl, morpholinyl, piperazinyl and thiomorpholinyl, wherein in each case the radical can be substituted with 1 or 2 substituents selected independently from the group consisting of pyridinyl, pyridazinyl, phenyl and benzyl, wherein in each case the cyclic portion of the radicals pyridinyl, pyridazinyl, phenyl and benzyl can be substituted with 1, 2, 3, 4 or 5 substituents selected independently from the group consisting of -CF₃, F, Cl, Br, methyl, ethyl, n-propyl, isopropyl, n-butyl, sec-butyl, isobutyl, tert-butyl, -O-CH₃ and - O-C₂H₅;
a radical selected from the group consisting of phenyl, naphthyl, thiophenyl, furanyl, pyrrolyl, pyrazolyl, pyrazinyl and pyridinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ and -S(=O)₂-NH-C₂H₅;
or -(CHR³⁰)-R³³; -(CHR³⁰)-(CHR³¹)-R³³ or -(CHR³⁰)-(CHR³¹)-(CHR³²)-R³³;
R³ represents a hydrogen radical;
or
R² and R³ form, together with the nitrogen atom joining them as ring member, a radical selected from the group consisting of pyrrolidinyl, piperidinyl, piperazinyl, 3-methyl-piperazinyl, 2-methyl-piperazinyl, (3,5)-dimethylpiperazinyl, (2,6)-dimethylpiperazinyl, morpholinyl and thiomorpholinyl, wherein in each case the radical can be substituted with 1 or 2 substituents selected independently from the group consisting of -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, - S(=O)₂ phenyl, pyridinyl, pyridazinyl, phenyl and benzyl, wherein in each case the cyclic portion of the radicals pyridinyl, -S(=O)₂ phenyl, pyridazinyl, phenyl and benzyl can be substituted with 1, 2, 3, 4 or 5 substituents selected independently from the group consisting of -CF₃, F, Cl, Br, methyl, ethyl, n-propyl, isopropyl, n-butyl, sec-butyl, isobutyl, tert-butyl, -O-CH₃ and -O-C₂H₅;
R⁶ represents a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1-butinyl, 2-butinyl and 3-butinyl, wherein in each case the radical can optionally be substituted with 1, 2, 3, 4 or 5 substituents selected independently from the group consisting of F, Cl and Br;
a radical selected from the group consisting of cyclopropyl, cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl;
a radical selected from the group consisting of phenyl, naphthyl, thiophenyl, furanyl, pyrrolyl, pyrazolyl, pyranyl, triazolyl, pyridinyl, Imidazolyl, thiazolyl, oxazolyl, isoxazolyl, pyridazinyl, pyrazinyl and pyrimidinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, - S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, - C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂ phenyl, - NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein in each case the cyclic portion of the radicals pyridinyl, cyclopentyl, cyclohexyl, pyridazinyl, -S(=O)₂ phenyl, -O-phenyl, - O-benzyl, phenyl, -(CH₂)-benzo[b]furanyl and benzyl can be substituted with 1, 2, 3, 4 or 5 substituents selected independently from the group consisting of F, Cl, Br, -OH, -CF₃, - SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
-(CHR³⁶)-C(=O)-O-R²⁵ or -(CHR³⁶)-(CH₂)-C(=O)-O-R²⁵;
or-(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ or - (CR³⁷R³⁸)-(CHR³⁹)-(CHR⁴⁰)-N(C₂H₅)-R⁴¹;
R⁷ represents a hydrogen radical;
R²⁵ represents a radical selected from the group consisting of methyl, ethyl, n-propyl, isopropyl, n-butyl, sec-butyl, isobutyl, and tert-butyl
R³⁰, R³¹, R³², R³⁶, R³⁷, R³⁸, R³⁹ and R⁴⁰, independently of one another, each represents a hydrogen radical;
a radical selected from the group consisting of methyl, ethyl, n-propyl, isopropyl, n-butyl, sec-butyl, isobutyl, and tert-butyl;
or a phenyl radical which can be substituted with 1, 2, 3, 4 or 5 substituents selected independently from the group consisting of F, Cl, Br, I, -CN, -CF₃, -O-CH₃, -O-C₂H₅, -NO₂, -O-CF₃, methyl, ethyl, n-propyl, isopropyl, n-butyl, sec-butyl, isobutyl and tert-butyl;
R³³ represents a radical selected from the group consisting of phenyl, naphthyl, thiophenyl, furanyl, pyrrolyl, pyrazolyl, pyrazinyl and pyridinyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(-O)₂-CH₃, -S(=O)₂-C₂H₅, -NH-S(=O)₂-CH₃, -NH-S(=O)₂-C₂H₅, -S(=O)₂-NH-CH₃ and -S(=O)₂-NH-C₂H₅;
and
R⁴¹ represents a radical selected from the group consisting of methyl, ethyl, n-propyl, isopropyl, n-butyl, sec-butyl, isobutyl and tert-butyl;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl;
or a radical selected from the group consisting of phenyl, naphthyl, (1,3)-benzodioxolyl, (1,4)-benzodioxanyl, thiophenyl and furanyl, wherein in each case the radical can optionally be substituted with 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, - C(=O)-C₂H₅ and -C(=O)-C(CH₃)₃;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates.

37. The use according to one or more of claims 24-36, **characterized in that**
R¹ represents a -C(=O)-NR⁶R⁷ group;
R² represents a radical selected from the group consisting of phenyl, naphthyl and pyridinyl, wherein in each case the radical can be substituted with 1, 2 or 3 substituents selected independently from the group consisting of F, Cl, Br, -OH, -O-CH₃, -O-C₂H₅, -NH-S(=O)₂-CH₃ and -NH-S(=O)₂-C₂H₅;
a piperazinyl radical which can be substituted on a nitrogen atom with a substituent selected from the group consisting of pyridinyl, pyridazinyl, phenyl and benzyl, wherein in each case the cyclic portion of the substituents pyridinyl, pyridazinyl, phenyl and benzyl can be substituted with 1, 2 or 3 substituents selected independently from the group consisting of -CF₃, F, Cl, Br, methyl, ethyl, n-propyl, isopropyl, n-butyl, sec-butyl, isobutyl and tert-butyl;
or -(CHR³⁰)-R³³; -(CHR³⁰)-(CHR³¹)-R³³ or -(CHR³⁰)-(CHR³¹)-(CHR³²)-R³³; R³ represents a hydrogen radical;
or
R² and R³ form, together with the nitrogen atom joining them as ring member, a radical selected from the group consisting of 3-methyl-piperazinyl, 2-methyl-piperazinyl, (3,5)-dimethylpiperazinyl, (2,6)-dimethylpiperazinyl and piperazinyl, which can be substituted on a nitrogen atom with a substituent selected from the group consisting of pyridinyl, pyridazinyl, phenyl and benzyl, wherein in each case the cyclic portion of the substituents pyridinyl, pyridazinyl, phenyl and benzyl can be substituted with 1, 2 or 3 substituents selected independently from the group consisting of -CF₃, F, Cl, Br, methyl, ethyl, n-propyl, isopropyl, n-butyl, sec-butyl, isobutyl and tert-butyl;
R⁶ represents a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, vinyl, 1-propenyl, 2-propenyl, 1-butenyl, 2-butenyl, 3-butenyl and 2-methyl-1-propenyl, wherein in each case the radical can optionally be substituted with 1, 2, 3, 4 or 5 substituents selected independently from the group consisting of F, Cl and Br;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl and adamantyl;
a radical selected from the group consisting of phenyl, naphthyl and pyridinyl, wherein in each case the radical can optionally be substituted with 1, 2, 3, 4 or 5 substituents selected independently from the group consisting of -SF₅, F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, - C(=O)-OH, -C(=O)-O-CH₃, -C(=O)-O-C₂H₅, -C(=O)-O-C(CH₃)₃, - O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -C(=O)-CH₃, -C(=O)-C₂H₅, - C(=O)-C(CH₃)₃, cyclohexyl, cyclopentyl, -O-phenyl, -O-benzyl and phenyl, wherein in each case the cyclic portion of the radicals cyclopentyl, cyclohexyl, -O-phenyl, -O-benzyl and phenyl can be substituted with 1, 2, 3, 4 or 5 substituents selected independently from the group consisting of F, Cl, Br, methyl, ethyl, n-propyl, isopropyl, n-butyl, sec-butyl, isobutyl and tert-butyl;
-(CHR³⁶)-C(=O)-O-R²⁵ or -(CHR³⁶)-(CH₂)-C(=O)-O-R²⁵;
or -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ or - (CR³⁷R³⁸)-(CHR³⁹)-(CHR⁴⁰)-N(C₂H₅)-R⁴¹;
R⁷ represents a hydrogen radical;
R²⁵ each represents a radical selected from the group consisting of methyl, ethyl, n-propyl, isopropyl, n-butyl, sec-butyl, isobutyl, and tert-butyl;
R³⁰ R³¹, R³², R³⁹ and R⁴⁰, each represents a hydrogen radical;
R³³ represents a radical selected from the group consisting of phenyl, naphthyl, thiophenyl and furanyl, wherein in each case the radical can optionally be substituted with 1, 2, 3, 4 or 5 substituents selected independently from the group consisting of -CF₃, F, Cl, Br, -OH, -O-CH₃, -O-C₂H₅, methyl, ethyl, n-propyl, isopropyl, n-butyl, sec-butyl, isobutyl, tert-butyl, -NH-S(=O)₂-CH₃ and -NH-S(=O)₂-C₂H₅;
R³⁶ represents a hydrogen radical;
or a radical selected from the group consisting of methyl, ethyl, n-propyl, isopropyl, n-butyl, sec-butyl, isobutyl and tert-butyl;
R³⁷ and R³⁸, independently of one another, each represents
a hydrogen radical;
a radical selected from the group consisting of methyl, ethyl, n-propyl, isopropyl, n-butyl, sec-butyl, isobutyl and tert-butyl;
or a phenyl radical.
R⁴¹ represents a radical selected from the group consisting of methyl, ethyl, n-propyl, isopropyl, n-butyl, sec-butyl, isobutyl and tert-butyl;
a radical selected from the group consisting of tetrahydrofuranyl, tetrahydrothiophenyl, pyrrolidinyl, morpholinyl, piperidinyl and piperazinyl;
or a radical selected from the group consisting of phenyl, naphthyl, (1,3)-benzodioxolyl, (1,4)-benzodioxanyl, thiophenyl and furanyl, wherein in each case the radical can optionally be substituted with 1, 2, 3, 4 or 5 substituents selected independently from the group consisting of F, Cl, Br, -OH, -O-CH₃, -O-C₂H₅, methyl, ethyl, n-propyl, isopropyl, n-butyl, sec-butyl, isobutyl and tert-butyl;
in each case optionally in the form of one of their pure stereoisomers, particularly enantiomers or diastereomers, racemates thereof or in the form of a mixture of stereoisomers, particularly enantiomers and/or diasatereomers, in any mixing ratio, or in the form of respective corresponding salts, or respectively in the form of corresponding solvates.

38. The use according to one or more of claims 24 to 37, **characterized in that** the spiro compounds is selected from the group consisting of
[1] 3-[4-(3-chloropyridin-2-yl)-piperazin-1-carbonyl]-1-oxa-2,8-diazaspiro[4.5]dec-2-en-8-carbonyl acid-(2-fluor-phenyl)-amide
[2] 1-oxa-2,8-diaza-spiro[4.5]dec-2-en-3,8-dicarbonyl acid-3-[(5-chloropyridin-2-yl)-amide]-8-[(2-fluor-phenyl)-amide]
[3] 1-oxa-2,8-diaza-spiro[4.5]dec-2-en-3,8-dicarbonyl acid-3-(4-chlorobenzylamide)-8-[(3-fluor-phenyl)-amide]
[4] 3-[4-(3-chloropyridin-2-yl)-piperazin-1-carbonyl]-1-oxa-2,8-diazaspiro[4.5]dec-2-en-8-carbonyl acid-(3-fluor-phenyl)-arnide
[5] 1-oxa-2,8-diaza-spiro[4.5]dec-2-en-3,8-dicarbonyl acid-3-[(5-chloropyridin-2-yl)-amide]-8-[(3-fluor-phenyl)-amide]
[6] 1-oxa-2,8-diaza-spiro[4.5]dec-2-en-3,8-dicarbonyl acid 8-[(2-chlorophenyl)-amide]-3-(3,4-dimethoxy-benzylamide)
[7] 1-oxa-2,8-diaza-spiro[4.5]dec-2-en-3,8-dicarbonyl acid-3-(4-chlorobenzylamide)-8-[(2-chlorophenyl)-amide]
[8] 3-[4-(3-chloropyridin-2-yl)-piperazin-1-carbonyl]-1-oxa-2,8-diazaspiro[4.5]dec-2-en-8-carbonyl acid-(2-chlorophenyl)-amide
[9] 1-oxa-2,8-diaza-spiro[4.5]dec-2-en-3,8-dicarbonyl acid 8-[(2-chlorophenyl)-amide]-3-[(5-chloropyridin-2-yl)-amide]
[10] 1-oxa-2,8-diaza-spiro[4.5]dec-2-en-3,8-dicarbonyl acid 8-[(3-bromophenyl)-amide]-3-(3,4-dimethoxy-benzylamide)
[11] 3-[4-(3-chloropyridin-2-yl)-piperazin-1-carbonyl]-1-oxa-2,8-diazaspiro[4.5]dec-2-en-8-carbonyl acid-(3-bromo-phenyl)-amide
[12] 1-oxa-2,8-diaza-spiro[4.5]dec-2-en-3,8-dicarbonyl acid 8-[(3-bromophenyl)-amide]-3-[(5-chloropyridin-2-yl)-amide]
[13] 1-oxa-2,8-diaza-spiro[4.5]dec-2-en-3,8-dicarbonyl acid-3-(3,4-dimethoxy-benzylamide)-8-[(2-methoxy-phenyl)-amide]
[14] 1-oxa-2,8-diaza-spiro[4.5]dec-2-en-3,8-dicarbonyl acid-3-(4-chlorobenzylamide)-8-[(2-methoxy-phenyl)-amide]
[15] 3-[4-(3-chloropyridin-2-yl)-piperazin-1-carbonyl]-1-oxa-2,8-diazaspiro[4.5]dec-2-en-8-carbonyl acid-(2-methoxy-phenyl)-amide
[16] 1-oxa-2,8-diaza-spiro[4.5]dec-2-en-3,8-dicarbonyl acid-3-(3,4-dimethoxy-benzylamide)-8-[(3-methoxy-phenyl)-amide]
[17] 3-[4-(3-chloropyridin-2-yi)-piperazin-l-carbonyl)-l-oxa-2,8-diazaspiro[4.5]dec-2-en-8-carbonyl acid-(3-methoxy-phenyl)-amide
[18] 1-oxa-2,8-diaza-spiro(4.5]dec-2-en-3,8-dicarbonyl acid-3-[(5-chloropyridin-2-yl)-amide]-8-[(3-methoxy-phenyl)-amide]
[19] 3-[4-(3-chloropyridin-2-yl)-piperazin-1-carbonyl]-1-oxa-2,8-diazaspiro[4.5]dec-2-en-8-carbonyl acid-(4-phenoxy-phenyl)-amide
[20] 1-oxa-2,8-diaza-spiro[4.5]dec-2-en-3,8-dicarbonyl acid 8-[(2-chloro-5-trifluormethyl-phenyl)-amide]-3-(3,4-dimethoxy-benzylamide)
[21] 1-oxa-2,8-diaza-spiro[4.5]dec-2-en-3,8-dicarbonyl acid-3-(4-chloro-benzylamide)-8-[(2-chloro-5-trifluormethyl-phenyl)-amide]
[22] 3-[4-(3-chloropyridin-2-yl)-piperazin-1-carbonyl]-1-oxa-2,8-diazaspiro[4.5]dec-2-en-8-carbonyl acid-(2-chloro-5-trifluormethyl-phenyl)-amide
[23] 1-oxa-2,8-diaza-spiro[4.5]dec-2-en-3,8-dicarbonyl acid-3-[(5-chloropyridin-2-yl)-amide]-8-[(2-chloro-5-trifluormethyl-phenyl)-amide]
[24] 1-oxa-2,8-diaza-spiro[4.5]dec-2-en-3,8-dicarbonyl acid 8-[(4-chloro-2-trifluormethyl-phenyl)-amide]-3-(3,4-dimethoxy-benzylamide)
[25] 3-[4-(3-chloropyridin-2-yl)-piperazin-1-carbonyl]-1-oxa-2,8-diazaspiro[4.5]dec-2-en-8-carbonyl acid-(4-chloro-2-trifluormethyl-phenyl)-amide
[26] 1-oxa-2,8-diaza-spiro[4.5]dec-2-en-3,8-dicarbonyl acid-3-[(5-chloropyridin-2-yl)-amide]-8-[(4-chloro-2-trifluormethyl-phenyl)-amide]
[27] 1-oxa-2,8-diaza-spiro[4.5]dec-2-en-3,8-dicarbonyl acid 8-[(4-chloro-3-trifluormethyl-phenyl)-amide]-3-(3,4-dimethoxy-benzylamide)
[28] 3-[4-(3-chloropyridin-2-yl)-piperazin-1-carbonyl]-1-oxa-2,8-diazaspiro[4.5]dec-2-en-8-carbonyl acid-(4-chloro-3-trifluormethyl-phenyl)-amide
[29] 1-oxa-2,8-diaza-spiro[4.5]dec-2-en-3,8-dicarbonyl acid-3-[(5-chloropyridin-2-yl)-amide]-8-[(4-chloro-3-trifluormethyl-phenyl)-amide]
[30] 1-oxa-2,8-diaza-spiro[4.5]dec-2-en-3,8-dicarbonyl acid-3-[(2-tert-butyl-6-methyl-phenyl)-amide]-8-(4-chloro-benzylamide)
[31] 3-[4-(3-chloropyridin-2-yl)-piperazin-1-carbonyl]-1-oxa-2,8-diazaspiro[4.5]dec-2-en-8-carbonyl acid-(2-tert-butyl-6-methyl-phenyl)-amide
[32] 1-oxa-2,8-diaza-spiro[4.5]dec-2-en-3,8-dicarbonyl acid-3-(3,4-dimethoxy-benzylamide)-8-[(4-trifluormethoxy-phenyl)-amide]
[33] 1-oxa-2,8-diaza-spiro[4.5]dec-2-en-3,8-dicarbonyl acid-3-(4-chlorobenzylamide)-8-[(4-trifluormethoxy-phenyl)-amide]
[34] 3-[4-(3-chloropyridin-2-yl)-piperazin-1-carbonyl]-1-oxa-2,8-diazaspiro[4.5]dec-2-en-8-carbonyl acid-(4-trifluormethoxy-phenyl)-amide
[35] 1-oxa-2,8-diaza-spiro[4.5]dec-2-en-3,8-dicarbonyl acid-3-[(5-chloropyridin-2-yl)-amide]-8-[(4-trifluormethoxy-phenyl)-amide]
[36] 1-oxa-2,8-diaza-spiro[4.5]dec-2-en-3,8-dicarbonyl acid-3-(3,4-dimethoxy-benzylamide)-8-{phenethyl-amide)
[37] 1-oxa-2,8-diaza-spiro[4.5]dec-2-en-3,8-dicarbonyl acid-3-(4-chloro-benzylamide)-8-(phenethyl-amide)
[38] 3-[4-(3-chloropyridin-2-yl)-piperazin-1-carbonyl]-1-oxa-2,8-diazaspiro[4.5]dec-2-en-8-carbonyl acid ethyl-amide
[39] 1-oxa-2,8-diaza-spiro[4.5]dec-2-en-3,8-dicarbonyl acid-3-(3,4-dimethoxy-benzylamide)-8-phenylamide
[40] 3-[4-(3-chloropyridin-2-yl)-piperazin-1-carbonyl]-1-oxa-2,8-diazaspiro[4.5]dec-2-en-8-carbonyl acid phenylamide
[41] 1-oxa-2,8-diaza-spiro[4.5]dec-2-en-3,8-dicarbonyl acid-3-[(5-chloropyridin-2-yl)-amide]-8-phenylamide
[42] 1-oxa-2,8-diaza-spiro[4.5]dec-2-en-3,8-dicarbonyl acid-3-(4-chlorobenzylamide)-8-m-tolylamide
[43] 3-[4-(3-chloropyridin-2-yl)-piperazin-1-carbonyl]-1-oxa-2,8-diazaspiro[4.5]dec-2-en-8-carbonyl acid-m-tolylamide
[44] 1-oxa-2,8-diaza-spiro[4.5]dec-2-en-3,8-dicarbonyl acid-3-(3,4-dimethoxy-benzylamide)-8-[(4-fluor-phenyl)-amide]
[45] 3-[4-(3-chloropyridin-2-yl)-piperazin-1-carbonyl]-1-oxa-2,8-diazaspiro[4.5]dec-2-en-8-carbonyl acid-(4-fluor-phenyl)-amide
[46] 1-oxa-2,8-diaza-spiro[4.5]dec-2-en-3,8-dicarbonyl acid 8-[(3-chlorophenyl)-amide]-3-(3,4-dimethoxy-benzylamide)
[47] 3-[4-(3-chloropyridin-2-yl)-piperazin-1-carbonyl]-1-oxa-2,8-diazaspiro[4.5]dec-2-en-8-carbonyl acid-(3-chlorophenyl)-amide
[48] 1-oxa-2,8-diaza-spiro[4.5]dec-2-en-3,8-dicarbonyl acid 8-[(3-chlorophenyl)-amide]-3-[(5-chloropyridin-2-yl)-amide]
[49] 1-oxa-2,8-diaza-spiro[4.5]dec-2-en-3,8-dicarbonyl acid 8-[(4-chlorophenyl)-amide]-3-(3,4-dimethoxy-benzylamide)
[50] 1-oxa-2,8-diaza-spiro[4.5]dec-2-en-3,8-dicarbonyl acid-3-(4-chloro-benzylamide)-8-[(4-chlorophenyl)-amide]
[51] 3-[4-(3-chloropyridin-2-yl)-piperazin-1-carbonyl]-1-oxa-2,8-diazaspiro[4.5]dec-2-en-8-carbonyl acid-(4-chlorophenyl)-amide
[52] 3-[4-(3-chloropyridin-2-yl)-piperazin-1-carbonyl]-1-oxa-2,8-diazaspiro[4.5]dec-2-en-8-carbonyl acid-(4-methoxy-phenyl)-amide
[53] 1-oxa-2,8-diaza-spiro[4.5]dec-2-en-3,8-dicarbonyl acid-3-(3,4-dimethoxy-benzylamide)-8-[(2-methylsulfanyl-phenyl)-amide]
[54] 3-[4-(3-chloropyridin-2-yl)-piperazin-1-carbonyl]-1-oxa-2,8-diazaspiro[4.5]dec-2-en-8-carbonyl acid-(2-methylsulfanyl-phenyl)-amide
[55] 1-oxa-2,8-diaza-spiro[4.5]dec-2-en-3,8-dicarbonyl acid-3-(3,4-dimethoxy-benzylamide)-8-[(3-methylsulfanyl-phenyl)-amide]
[56] 3-[4-(3-chloropyridin-2-yl)-piperazin-1-carbonyl]-1-oxa-2,8-diazaspiro[4.5]dec-2-en-8-carbonyl acid-(3-methylsulfanyl-phenyl)-amide
[57] 1-oxa-2,8-d iaza-spiro[4. 5]dec-2-en-3, 8-dicarbonyl acid-3-(3,4-dimethoxy-benzylamide)-8-[(4-methylsulfanyl-phenyl)-amide]
[58] 3-[4-(3-chloropyridin-2-yl)-piperazin-1-carbonyl]-1-oxa-2,8-diazaspiro[4.5]dec-2-en-8-carbonyl acid-(4-methylsulfanyl-phenyl)-amide
[59] 1-oxa-2,8-diaza-spiro[4.5]dec-2-en-3,8-dicarbonyl acid-3-[(5-chloropyridin-2-yl)-amide]-8-[(4-methylsulfanyl-phenyl)-amide]
[60] 1-oxa-2,8-diaza-spiro[4.5]dec-2-en-3,8-dicarbonyl acid-3-(3,4-dimethoxy-benzylamide)-8-[(2-isopropyl-phenyl)-amide]
[61] 3-[4-(3-chloropyridin-2-yl)-piperazin-1-carbonyl]-1-oxa-2,8-diazaspiro[4.5]dec-2-en-8-carbonyl acid-(2-isopropyl-phenyl)-amide
[62] 1-oxa-2,8-diaza-spiro[4.5]dec-2-en-3,8-dicarbonyl acid-3-[(5-chloropyridin-2-yl)-amide]-8-[(2-isopropyl-phenyl)-amide]
[63] 3-[4-(3-chloropyridin-2-yl)-piperazin-1-carbonyl]-1-oxa-2,8-diazaspiro[4.5]dec-2-en-8-carbonyl acid-(4-isopropyl-phenyl)-amide
[64] 1-oxa-2,8-diaza-spiro[4.5]dec-2-en-3,8-dicarbonyl acid-3-(3,4-dimethoxy-benzylamide)-8-[(2-trifluormethyl-phenyl)-amide]
[65] 3-[4-(3-chloropyridin-2-yl)-piperazin-1-carbonyl]-1-oxa-2,8-diazaspiro[4.5]dec-2-en-8-carbonyl acid-(2-trifluormethyl-phenyl)-amide
[66] 1-oxa-2,8-diaza-spiro[4.5]dec-2-en-3,8-dicarbonyl acid-3-[(5-chloropyridin-2-yl)-amide]-8-[(2-trifluormethyl-phenyl)-amide]
[67] 1-oxa-2,8-diaza-spiro[4.5]dec-2-en-3,8-dicarbonyl acid-3-(3,4-dimethoxy-benzylamide)-8-[(3-trifluormethyl-phenyl)-amide]
[68] 3-[4-(3-chloropyridin-2-yl)-piperazin-1-carbonyl]-1-oxa-2,8-diazaspiro[4.5]dec-2-en-8-carbonyl acid-(3-trifluormethyl-phenyl)-amide
[69] 1-oxa-2,8-diaza-spiro[4.5]dec-2-en-3,8-dicarbonyl acid-3-[(5-chloropyridin-2-yl)-amide]-8-[(3-trifluormethyl-phenyl)-amide]
[70] 1-oxa-2,8-diaza-spiro[4.5]dec-2-en-3,8-dicarbonyl acid-3-(3,4-dimethoxy-benzylamide)-8-[(4-trifluormethyl-phenyl)-amide]
[71] 3-[4-(3-chloropyridin-2-yl)-piperazin-1-carbonyl]-1-oxa-2,8-diazaspiro[4.5]dec-2-en-8-carbonyl acid-(4-trifluormethyl-phenyl)-amide
[72] 1-oxa-2,8-diaza-spiro[4.5]dec-2-en-3,8-dicarbonyl acid-8-cyclohexylamide-3-(3,4-dimethoxy-benzylamide)
[73] 1-oxa-2,8-diaza-spiro[4.5]dec-2-en-3,8-dicarbonyl acid-3-(4-chloro-benzylamide)-8-cyclohexylamide
[74] 1-oxa-2,8-diaza-spiro[4.5]dec-2-en-3,8-dicarbonyl acid-8-benzylamide-3-(3,4-dimethoxy-benzylamide)
[75] 1-oxa-2,8-diaza-spiro[4.5]dec-2-en-3,8-dicarbonyl acid-3-(3,4-dimethoxy-benzylamide)-8-o-tolylamide
[76] 1-oxa-2,8-diaza-spiro[4.5]dec-2-en-3,8-dicarbonyl acid-3-(4-chloro-benzylamide)-8-o-tolylamide
[77] 1-oxa-2,8-diaza-spiro[4.5]dec-2-en-3,8-dicarbonyl acid-3-[(5-chloropyridin-2-yl)-amide]-8-o-tolylamide
[78] 1-oxa-2,8-diaza-spiro[4.5]dec-2-en-3,8-dicarbonyl acid-3-(3,4-dimethoxy-benzylamide)-8-[(2-ethyl-phenyl)-amide]
[79] 3-[4-(3-chloropyridin-2-yl)-piperazin-1-carbonyl]-1-oxa-2,8-diazaspiro[4.5]dec-2-en-8-carbonyl acid-(2-ethyl-phenyl)-amide
[80] 1-oxa-2,8-diaza-spiro[4.5]dec-2-en-3,8-dicarbonyl acid-3-[(5-chloropyridin-2-yl)-amide]=8-[(2-ethyl-phenyl)-amide]
[81] 1-oxa-2,8-diaza-spiro[4.5]dec-2-en-3,8-dicarbonyl acid-3-(4-chloro-benzylamide)-8-[(4-ethyl-phenyl)-amide]
[82] 1-oxa-2,8-diaza-spiro[4.5]dec-2-en-3,8-dicarbonyl acid-3-[(5-chloropyridin-2-yl)-amide]-8-[(4-ethyl-phenyl)-amide]
[83] 1-oxa-2,8-diaza-spiro[4.5]dec-2-en-3,8-dicarbonyl acid-3-(3,4-dimethoxy-benzylamide)-8-[(2-fluor-phenyl)-amide]
[84] 1-oxa-2,8-diaza-spiro[4.5]dec-2-en-3,8-dicarbonyl acid-3-(3,4-dimethoxy-benzylamide)-8-p-tolylamide
[85] 1-oxa-2,8-diaza-spiro[4.5]dec-2-en-3,8-dicarbonyl acid-3-(4-chlorobenzylamide)-8-p-tolylamide
[86] 3-[4-(3-chloropyridin-2-yl)-piperazin-1-carbonyl]-1-oxa-2,8-diazaspiro[4.5]dec-2-en-8-carbonyl acid-p-tolylamide
[87] 1-oxa-2,8-diaza-spiro[4.5]dec-2-en-3,8-dicarbonyl acid-3-[(5-chloropyridin-2-yl)-amide]-8-p-tolylamide
[88] 1-oxa-2,8-diaza-spiro[4.5]dec-2-en-3,8-dicarbonyl acid-3-(4-chlorobenzylarriide)-8-[(3-ethyl-phenyl)-amide]
[89] 3-[4-(3-chloropyridin-2-yl)-piperazin-1-carbonyl]-1-oxa-2,8-diazaspiro[4.5]dec-2-en-8-carbonyl acid-(3-ethyl-phenyl)-amide
[90] 1-oxa-2,8-diaza-spiro[4.5]dec-2-en-3,8-dicarbonyl acid-3-[(5-chloropyridin-2-yl)-amide]-8-[(3-ethyl-phenyl)-amide]
[91] 1-oxa-2,8-diaza-spiro[4.5]dec-2-en-3,8-dicarbonyl acid-3-(3,4-dimethoxy-benzytamide)-8-[(2-propyl-phenyl)-amide]
[92] 1-oxa-2,8-diaza-spiro[4.5]dec-2-en-3,8-dicarbonyl acid-3-(4-chloro-benzylamide)-8-[(2-propyl-phenyl)-amide]
[93] 1-oxa-2,8-diaza-spiro[4.5]dec-2-en-3,8-dicarbonyl acid-3-(4-chloro-benzylamide)-8-[(2-propyl-phenyl)-amide]
[94] 3-[4-(3-chloropyridin-2-yl)-piperazin-1-carbonyl]-1-oxa-2,8-diazaspiro[4.5]dec-2-en-8-carbonyl acid-(2-propyl-phenyl)-amide
[95] 1-oxa-2,8-diaza-spiro[4.5]dec-2-en-3,8-dicarbonyl acid-3-[(5-chloropyridin-2-yl)-amide]-8-[(2-propyl-phenyl)-amide]
[96] 1-oxa-2,8-diaza-spiro[4.5]dec-2-en-3,8-dicarbonyl acid 8-[(2-bromophenyl)-amide]-3-(3,4-dimethoxy-benzylamide)
[97] 1-oxa-2,8-diaza-spiro[4.5]dec-2-en-3,8-dicarbonyl acid 8-[(2-bromophenyl)-amide]-3-(4-chloro-benzylamide)
[98] 3-[4-(3-chloropyddin-2-yl)-piperazin-l-carbonyl]-l-oxa-2,8-diazaspiro[4.5]dec-2-en-8-carbonyl acid-(2-bromo-phenyl)-amide
[99] 3-[4-(3-chloropyridin-2-yl)-piperazin-1-carbonyl]-1-oxa-2,8-diazaspiro[4.5]dec-2-en-8-carbonyl acid-(4-bromo-phenyl)-amide
[100] 3-[4-(3-chloropyridin-2-yl)-piperazin-1-carbonyl]-1-oxa-2,8-diazaspiro[4.5]dec-2-en-8-carbonyl acid-biphenyl-4-ylamide
[101] 1-oxa-2,8-diaza-spiro[4.5]dec-2-en-3,8-dicarbonyl acid-3-(3,4-dimethoxy-benzylamide)-8-[(2-phenoxy-phenyl)-amide]
[102] 3-[4-(3-chloropyridin-2-yl)-piperazin-1-carbonyl]-1-oxa-2,8-diazaspiro[4.5]dec-2-en-8-carbonyl acid-(2-phenoxy-phenyl)-amide
[103] 1-oxa-2,8-diaza-spiro[4.5]dec-2-en-3,8-dicarbonyl acid-3-[(5-chloropyridin-2-yl)-amide]-8-[(2-phenoxy-phenyl)-amide]
[104] 1-oxa-2,8-diaza-spiro[4.5]dec-2-en-3,8-dicarbonyl acid-3-(4-chlorobenzylamide)-8-[(2-trifluormethoxy-phenyl)-amide]
[105] 3-[4-(3-chloropyridin-2-yl)-piperazin-1-carbonyl]-1-oxa-2,8-diazaspiro[4.5]dec-2-en-8-carbonyl acid-(2-trifluormethoxy-phenyl)-amide
[106] 1-oxa-2,8-diaza-spiro[4.5]dec-2-en-3,8-dicarbonyl acid-3-[(5-chloropyridin-2-yl)-amide]-8-[(2-trifluorrnethoxy-phenyl)-amide]
[107] 1-oxa-2,8-diaza-spiro[4.5]dec-2-en-3,8-dicarbonyl acid-3-(3,4-dimethoxy-benzylamide)-8-(4-methyl-benzylamide)
[108] 1-oxa-2,8-diaza-spiro[4.5]dec-2-en-3,8-dicarbonyl acid-3-(4-chloro-benzylamide)-8-(4-methyl-benzylamide)
[109] 3-[4-(3-chloropyridin-2-yl)-piperazin-1-carbonyl]-1-oxa-2,8-diazaspiro[4.5]dec-2-en-8-carbonyl acid-4-methyl-benzylamide
[110] 1-oxa-2,8-diaza-spiro[4.5]dec-2-en-3,8-dicarbonyl acid-3-[(5-chloropyridin-2-yl)-amide]-8-(4-methyl-benzylamide)
[111] 1-oxa-2,8-diaza-spiro[4.5]dec-2-en-3,8-dicarbonyl acid-3-(3,4-dimethoxy-benzylamide)-8-(4-methoxy-benzylamide)
[112] 1-oxa-2,8-diaza-spiro[4.5]dec-2-en-3,8-dicarbonyl acid-3-(4-chloro-benzylamide)-8-(4-methoxy-benzylamide)
[113] 3-[4-(3-chloropyridin-2-yl)-piperazin-1-carbonyl]-1-oxa-2,8-diazaspiro[4.5]dec-2-en-8-carbonyl acid-4-methoxy-benzylamide
[114] 1-oxa-2,8-diaza-spiro[4.5]dec-2-en-3,8-dicarbonyl acid 8-[(4-tert-butyl-phenyl)-amide]-3-(3,4-dimethoxy-benzylamide)
[115] 1-oxa-2,8-diaza-spiro[4.5]dec-2-en-3,8-dicarbonyl acid 8-[(4-tert-butyl-phenyl)-amide]-3-(4-chloro-benzylamide)
[116] 3-[4-(3-chloropyridin-2-yl)-piperazin-1-carbonyl]-1-oxa-2,8-diazaspiro[4.5]dec-2-en-8-carbonyl acid-(4-tert-butyl-phenyl)-amide
[117] 1-oxa-2,8-diaza-spiro[4.5]dec-2-en-3,8-dicarbonyl acid-8-[(4-tert-butylphenyl)-amide]-3-[(5-chloropyridin-2-yl)-amide]
[118] 8-(2-Methoxy-phenylthiocarbamoyl)-1-oxa-2,8-diaza-spiro[4.5]dec-2-en-3-carbonyl acid-3,4-dimethoxy-benzylamide
[119] 3-[4-(3-chloropyridin-2-yl)-piperazin-1-carbonyl]-1-oxa-2,8-diazaspiro[4.5]dec-2-en-8-carbonyl thioacid-(2-methoxy-phenyl)-amide
[120] 8-(cyclohexylmethyl-thiocarbamoyl)-1-oxa-2,8-diaza-spiro[4.5]dec-2-en-3-carbonyl acid-3,4-dimethoxy-benzylamide
[121] 8-(cyclohexylmethyl-thiocarbamoyl)-1-oxa-2,8-diaza-spiro[4.5]dec-2-en-3-carbonyl acid-4-chloro-benzylamide
[122] 3-[4-(3-chloropyridin-2-yl)-piperazin-1-carbonyl]-1-oxa-2,8-diazaspiro[4.5]dec-2-en-8-carbonyl thioacid cyclohexylmethyl-amide
[123] 8-cyclooctylthiocarbamoyl-1-oxa-2,8-diaza-spiro[4.5]dec-2-en-3-carbonyl acid-3,4-dimethoxy-benzylamide
[124] 8-cyclooctytthiocarbamoyl-1-oxa-2,8-diaza-spiro[4.5]dec-2-en-3-carbonyl acid-4-chloro-benzylamide
[125] 8-(3-morpholin-4-yl-propylthiocarbamoyl)-1-oxa-2,8-diaza-spiro[4.5]dec-2-en-3-carbonyl acid-4-chloro-benzylamide
[126] 8-(3-morpholin-4-yl-propylthiocarbamoyl)-1-oxa-2,8-diaza-spiro[4.5]dec-2-en-3-carbonyl acid-(5-chloropyridin-2-yl)-amide
[127] 8-p-Tolylthiocarbamoyl-1-oxa-2,8-diaza-spiro[4.5]dec-2-en-3-carbonyl acid-4-chloro-benzylamide
[128] 8-phenethylthiocarbamoyl-1-oxa-2,8-diaza-spiro[4.5]dec-2-en-3-carbonyl acid-3,4-dimethoxy-benzylamide
[129] 8-phenethylthiocarbamoyl-1-oxa-2,8-diaza-spiro[4.5]dec-2-en-3-carbonyl acid-4-chloro-benzylamide
[130] 8-(3 phenyl-propylthiocarbamoyl)-1-oxa-2,8-diaza-spiro[4.5]dec-2-en-3-carbonyl acid-(5-chloropyridin-2-yl)-amide
[131] 8-(2-fluor-phenylthiocarbamoyl)-1-oxa-2,8-diaza-spiro[4.5]dec-2-en-3-carbonyl acid-3,4-dimethoxy-benzylamide
[132] 8-(4-fluor-phenylthiocarbamoyl)-1-oxa-2,8-diaza-spiro[4.5]dec-2-en-3-carbonyl acid-3,4-dimethoxy-benzylamide
[133] 8-(4-fluor-phenylthiocarbamoyl)-1-oxa-2,8-diaza-spiro[4.5]dec-2-en-3-carbonyl acid-4-chloro-benzylamide
[134] 8-(2-chlorophenylthiocarbamoyl)-1-oxa-2,8-diaza-spiro[4.5]dec-2-en-3-carbonyl acid-3,4-dimethoxy-benzylamide
[135] 8-(2-chlorophenylthiocarbamoyl)-1-oxa-2,8-diaza-spiro[4.5]dec-2-en-3-carbonyl acid-4-chloro-benzylamide
[136] 8-(3-trifluormethyl-phenylthiocarbamoyl)-1-oxa-2,8-diaza-spiro[4.5]dec-2-en-3-carbonyl acid-4-chloro-benzylamide
[137] 8-(naphthalen-1-ylthiocarbamoyl)-1-oxa-2,8-diaza-spiro[4.5]dec-2-en-3-carbonyl acid-3,4-dimethoxy-benzylamide
[138] 8-(naphthalen-1-ylthiocarbamoyl)-1-oxa-2,8-diaza-spiro[4.5]dec-2-en-3-carbonyl acid-3,4-dimethoxy-benzylamide
[139] 8-cyclopentylthiocarbamoyl-1-oxa-2,8-diaza-spiro[4.5]dec-2-en-3-carbonyl acid-3,4-dimethoxy-benzylamide
[140] 3-[4-(3-chloropyridin-2-yl)-piperazin-1-carbonyl]-1-oxa-2,8-diazaspiro[4.5]dec-2-en-8-carbonyl thioacid-cyclopentylamide
[141] 8-(2-morpholin-4-yl-ethylthiocarbamoyl)-l-oxa-2,8-diaza-spiro[4.5]dec-2-en-3-carbonyl acid-3,4-dimethoxy-benzylamide
[142] 8-(2-morpholin-4-yl-ethylthiocarbamoyl)-1-oxa-2,8-diaza-spiro[4.5]dec-2-en-3-carbonyl acid-4-chloro-benzylamide
[143] 8-(3-chlorophenylthiocarbamoyl)-1-oxa-2,8-diaza-spiro[4.5]dec-2-en-3-carbonyl acid-3,4-dimethoxy-benzylamide
[144] 3-[4-(3-chloropyridin-2-yl)-piperazin-1-carbonyl]-1-oxa-2,8-diazaspiro[4.5]dec-2-en-8-carbonyl thioacid-(4-trifluormethyl-phenyl)-amide
[145] 8-(2-methylsulfanyl-phenylthiocarbamoyl)-1-oxa-2,8-diazaspiro[4.5]dec-2-en-3-carbonyl acid-3,4-dimethoxy-benzylamide
[146] 8-(2-methylsulfanyl-phenylthiocarbamoyl)-1-oxa-2,8-diazaspiro[4.5]dec-2-en-3-carbonyl acid-4-chloro-benzylamide
[147] 3-[4-(3-chloropyridin-2-yl)-piperazin-1-carbonyl]-1-oxa-2,8-diazaspiro[4.5]dec-2-en-8-carbonyl thioacid (2-methylsulfanyl-phenyl)-amide
[148] 8-(4-isopropyl-phenylthiocarbamoyl)-1-oxa-2,8-diaza-spiro[4.5]dec-2-en-3-carbonyl acid-3,4-dimethoxy-benzylamide
[149] 8-(4-isopropyl-phenylthiocarbamoyl)-1-oxa-2,8-diaza-spiro[4.5]dec-2-en-3-carbonyl acid-4-chloro-benzylamide
[150] 8-(2-Iodo-phenylthiocarbamoyl)-1-oxa-2,8-diaza-spiro[4.5]dec-2-en-3-carbonyl acid-3,4-dimethoxy-benzylamide
[151] 8-(2-lodo-phenylthiocarbamoyl)-1-oxa-2,8-diaza-spiro[4.5]dec-2-en-3-carbonyl acid-4-chloro-benzylamide
[152] 8-(2-trifluormethyl-phenylthiocarbamoyl)-1-oxa-2,8-diaza-spiro[4.5]dec-2-en-3-carbonyl acid-3,4-dimethoxy-benzylamide
[153] 8-(2-trifluormethyl-phenylthiocarbamoyl)-1-oxa-2,8-diaza-spiro[4.5]dec-2-en-3-carbonyl acid-4-chloro-benzylamide
[154] 3-[4-(3-chloropyridin-2-yl)-piperazin-1-carbonyl]-1-oxa-2,8-diazaspiro[4.5]dec-2-en-8-carbonyl thioacid-(2-trifluormethyl-phenyl)-amide
[155] 8-[(benzo[1,3]dioxol-5-ylmethyl)-thiocarbamoyl]-1-oxa-2,8-diazaspiro[4.5]dec-2-en-3-carbonyl acid-3,4-dimethoxy-benzylamide
[156] 8-[(benzo[1,3]dioxol-5-ylmethyl)-thiocarbamoyl]-1-oxa-2,8-diazaspiro[4.5]dec-2-en-3-carbonyl acid-4-chloro-benzylamide
[157] 3-[4-(3-chloropyridin-2-yl)-piperazin-1-carbonyl]-1-oxa-2,8-diazaspiro[4.5]dec-2-en-8-carbonyl thioacid-(benzo[1,3]dioxol-5-ylmethyl)-amide
[158] 3-[4-(3-chloropyridin-2-yl)-piperazin-1-carbonyl]-1-oxa-2,8-diazaspiro[4.5]dec-2-en-8-carbonyl acid-(3-cyano-phenyl)-amide
[159] 1-oxa-2,8-diaza-spiro[4.5]dec-2-en-3,8-dicarbonyl acid-3-(3,4-dimethoxy-benzylamide)-8-[(2,5-dimethoxy-phenyl)-amide]
[160] 1-oxa-2,8-diaza-spiro[4.5]dec-2-en-3,8-dicarbonyl acid-3-(4-chloro-benzylamide)-8-[(2,5-dimethoxy-phenyl)-amide]
[161] 3-[4-(3-chloropyridin-2-yl)-piperazin-1-carbonyl]-1-oxa-2,8-diazaspiro[4.5]dec-2-en-8-carbonyl acid-(2,5-dimethoxy-phenyl)-amide
[162] 1-oxa-2,8-diaza-spiro[4.5]dec-2-en-3,8-dicarbonyl acid-3-(3,4-dimethoxy-benzylamide)-8-[(2,4-dimethyl-phenyl)-amide]
[163] 1-oxa-2,8-diaza-spiro[4.5]dec-2-en-3,8-dicarbonyl acid-3-(4-chlorobenzylamide)-8-[(2,4-dimethyl-phenyl)-amide]
[164] 3-[4-(3-chloropyridin-2-yl)-piperazin-1-carbonyl]-1-oxa-2,8-diazaspiro[4.5]dec-2-en-8-carbonyl acid-(2,4-dimethyl-phenyl)-amide
[165] 1-oxa-2,8-diaza-spiro[4.5]dec-2-en-3,8-dicarbonyl acid-8-butylamide 3-(3,4-dimethoxy-benzylamide)
[166] 1-oxa-2,8-diaza-spiro[4.5]dec-2-en-3,8-dicarbonyl acid-8-butylamide 3-(4-chloro-benzylamide)
[167] 3-[4-(3-chloropyridin-2-yl)-piperazin-1-carbonyl]-1-oxa-2,8-diazaspiro[4.5]dec-2-en-8-carbonyl acid butylamide
[168] 1-oxa-2,8-diaza-spiro[4.5]dec-2-en-3,8-dicarbonyl acid-3-(3,4-dimethoxy-benzylamide)-8-pentylamide
[169] 1-oxa-2,8-diaza-spiro[4.5]dec-2-en-3,8-dicarbonyl acid-3-(4-chlorobenzylamide)-8-pentylamide
[170] 3-[4-(3-chloropyridin-2-yl)-piperazin-1-carbonyl]-1-oxa-2,8-diazaspiro[4.5]dec-2-en-8-carbonyl acid pentylamide
[171] 1-oxa-2,8-diaza-spiro[4.5]dec-2-en-3,8-dicarbonyl acid-3-(3,4-dimethoxy-benzylamide)-8-[(2-ethoxy-phenyl)-amide]
[172] 1-oxa-2,8-diaza-spiro[4.5]dec-2-en-3,8-dicarbonyl acid-3-(4-chlorobenzylamide)-8-[(2-ethoxy-phenyl)-amide]
[173] 1-oxa-2,8-diaza-spiro[4.5]dec-2-en-3,8-dicarbonyl acid-3-(3,4-dimethoxy-benzylamide)-8-[(4-ethoxy-phenyl)-amide]
[174] 1-oxa-2,8-diaza-spiro[4.5]dec-2-en-3,8-dicarbonyl acid 8-[(2,4-difluorphenyl)-amide]-3-(3,4-dimethoxy-benzylamide)
[175] 1-oxa-2,8-diaza-spiro[4.5]dec-2-en-3,8-dicarbonyl acid-3-(4-chlorobenzylamide)-8-[(2,4-difluor-phenyl)-amide]
[176] 1-oxa-2,8-diaza-spiro[4.5]dec-2-en-3,8-dicarbonyl acid 8-[(3-chloro-2-methyl-phenyl)-amide]-3-(3,4-dimethoxy-benzylamide)
[177] 1-oxa-2,8-diaza-spiro[4.5]dec-2-en-3,8-dicarbonyl acid-3-(4-chlorobenzylamide)-8-[(3-chloro-2-methyl-phenyl)-amide]
[178] 1-oxa-2,8-diaza-spiro[4.5]dec-2-en-3,8-dicarbonyl acid 8-[(4-chloro-2-methyl-phenyl)-amide]-3-(3,4-dimethoxy-benzylamide)
[179] 1-oxa-2,8-diaza-spiro[4.5]dec-2-en-3,8-dicarbonyl acid-3-(4-chlorobenzylamide)-8-[(4-chloro-2-methyl-phenyl)-amide]
[180] 1-oxa-2,8-diaza-spiro[4.5]dec-2-en-3,8-dicarbonyl acid 8-[(3-chloro-4-methyl-phenyl)-amide]-3-(3,4-dimethoxy-benzylamide)
[181] 1-oxa-2,8-diaza-spiro(4.5]dec-2-en-3,8-dicarbonyl acid-3-(4-chloro-benzylamide)-8-[(3-chloro-4-methyl-phenyl)-amide]
[182] 1-oxa-2,8-diaza-spiro[4.5]dec-2-en-3,8-dicarbonyl acid 8-[(3-chloro-4-fluor-phenyl)-amide]-3-(3,4-dimethoxy-benzylamide)
[183] 1-oxa-2,8-diaza-spiro[4.5]dec-2-en-3,8-dicarbonyl acid-3-(4-chlorobenzylamide)-8-[(3-chloro-4-fluor-phenyl)-amide]
[184] 1-oxa-2,8-diaza-spiro[4.5]dec-2-en-3,8-dicarbonyl acid 8-[(2,6-diisopropyl-phenyl)-amide]-3-(3,4-dimethoxy-benzylamide)
[185] 1-oxa-2,8-diaza-spiro[4.5]dec-2-en-3,8-dicarbonyl acid-3-(4-chlorobenzylamide)-8-[(2,6-diisopropyl-phenyl)-amide]
[186] 1-oxa-2,8-diaza-spiro[4.5]dec-2-en-3,8-dicarbonyl acid 8-[(5-chloro-2-methoxy-phenyl)-amide]-3-(3,4-dimethoxy-benzylamide)
[187] 1-oxa-2,8-diaza-spiro[4.5]dec-2-en-3,8-dicarbonyl acid-3-(4-chloro-benzylamide)-8-[(5-chloro-2-methoxy-phenyl)-amide]
[188] 1-oxa-2,8-diaza-spiro[4.5]dec-2-en-3,8-dicarbonyl acid-3-(4-chloro-benzylamide)-8-[(3,4,5-trimethoxy-phenyl)-amide]
[189] 1-oxa-2,8-diaza-spiro[4.5]dec-2-en-3,8-dicarbonyl acid-3-(3,4-dimethoxy-benzylamide)-8-[(3,5-dimethyl-phenyl)-amide]
[190] 1-oxa-2,8-diaza-spiro[4.5]dec-2-en-3,8-dicarbonyl acid-3-(4-chlorobenzylamide)-8-((3,5-dimethyl-phenyl)-amide]
[191] 1-oxa-2,8-diaza-spiro[4.5]dec-2-en-3,8-dicarbonyl acid-3-(3,4-dimethoxy-benzylamide)-8-[(2,6-dimethyl-phenyl)-amide]
[192] 1-oxa-2,8-diaza-spiro[4.5]dec-2-en-3,8-dicarbonyl acid-3-(4-chlorobenzylamide)-8-[(2,6-dimethyl-phenyl)-amide]
[193] 1-oxa-2,8-diaza-spiro[4.5]dec-2-en-3,8-dicarbonyl acid-3-(3,4-dimethoxy-benzylamide)-8-[(3,4-dimethyl-phenyl)-amide]
[194] 1-oxa-2,8-diaza-spiro[4.5]dec-2-en-3,8-dicarbonyl acid-3-(3,4-dimethoxy-benzylamide)-8-[(2,5-dimethyl-phenyl)-amide]
[195] 1-oxa-2,8-diaza-spiro[4.5]dec-2-en-3,8-dicarbonyl acid-3-(4-chlorobenzylamide)-8-[(2,5-dimethyl-phenyl)-amide]
[196] 1-oxa-2,8-diaza-spiro[4.5]dec-2-en-3,8-dicarbonyl acid-3-(3,4-dimethoxy-benzylamide)-8-[(2-ethyl-6-methyl-phenyl)-amide]
[197] 1-oxa-2,8-diaza-spiro[4.5]dec-2-en-3,8-dicarbonyl acid-3-(4-chlorobenzylamide)-8-[(2-ethyl-6-methyl-phenyl)-amide]
[198] 1-oxa-2,8-diaza-spiro[4.5]dec-2-en-3,8-dicarbonyl acid-3-(3,4-dimethoxy-benzylamide)-8-[(4-methoxy-2-methyl-phenyl)-amide]
[199] 1-oxa-2,8-diaza-spiro[4.5]dec-2-en-3,8-dicarbonyl acid-3-(4-chlorobenzylamide)-8-[(4-methoxy-2-methyl-phenyl)-amide]
[200] 1-oxa-2,8-diaza-spiro[4.5]dec-2-en-3,8-dicarbonyl acid-3-(3,4-dimethoxy-benzylamide)-8-[(2-methoxy-5-methyl-phenyl)-amide]
[201] 1-oxa-2,8-diaza-spiro[4.5]dec-2-en-3,8-dicarbonyl acid-3-(4-chlorobenzylamide)-8-[(2-methoxy-5-methyl-phenyl)-amide]
[202] 1-oxa-2,8-diaza-spiro[4.5]dec-2-en-3,8-dicarbonyl acid-3-(3,4-dimethoxy-benzylamide)-8-[(2,4,5-trimethyl-phenyl)-amide]
[203] 1-oxa-2,8-diaza-spiro[4.5]dec-2-en-3,8-dicarbonyl acid-3-(4-chlorobenzylamide)-8-[(2,4,5-trimethyl-phenyl)-amide]
[204] 1-oxa-2,8-diaza-spiro[4.5]dec-2-en-3,8-dicarbonyl acid-3-(3,4-dimethoxy-benzylamide)-8-[(2,4,6-trimethyl-phenyl)-amide]
[205] 1-oxa-2,8-diaza-spiro[4.5]dec-2-en-3,8-dicarbonyl acid-3-(4-chlorobenzylamide)-8-[(2,4,6-trimethyl-phenyl)-amide]
[206] 1-oxa-2,8-diaza-spiro[4.5]dec-2-en-3,8-dicarbonyl acid-8-[(2-bromoethyl)-amide]-3-(3,4-dimethoxy-benzylamide)
[207] 1-oxa-2,8-diaza-spiro[4.5]dec-2-en-3,8-dicarbonyl acid-8-[(2-bromoethyl)-amide]-3-(4-chloro-benzylamide)
[208] 1-oxa-2,8-diaza-spiro[4.5]dec-2-en-3,8-dicarbonyl acid-8-[(4-butylphenyl)-amide]-3-(3,4-dimethoxy-benzylamide)
[209] 1-oxa-2,8-diaza-spiro[4.5]dec-2-en-3,8-dicarbonyl acid-8-[(4-butylphenyl)-amide]-3-(4-chloro-benzylamide)
[210] 2-{[3-(4-chloro-benzylcarbamoyl)-1-oxa-2,8-diaza-spiro[4.5]dec-2-en-8-carbonyl]-amino}-benzoic acid methylester
[211] 1-oxa-2,8-diaza-spiro[4.5]dec-2-en-3,8-dicarbonyl acid-8-biphenyl-2-ylamide 3-(3,4-dimethoxy-benzylamide)
[212] 1-oxa-2,8-diaza-spiro[4.5]dec-2-en-3,8-dicarbonyl acid-8-biphenyl-2-ylamide 3-(4-chloro-benzylamide)
[213] 1-oxa-2,8-diaza-spiro[4.5]dec-2-en-3,8-dicarbonyl acid-8-[(2,6-dichlorophenyl)-amide]-3-(3,4-dimethoxy-benzylamide)
[214] 1-oxa-2,8-diaza-spiro[4.5]dec-2-en-3,8-dicarbonyl acid-3-(4-chlorobenzylamide)-8-[(2,6-dichlorophenyl)-amide]
[215] 3-{[3-(3,4-dimethoxy-benzylcarbamoyl)-1-oxa-2,8-diaza-spiro[4.5]dec-2-en-8-carbonyl]-amino)-benzoic acid ethylester
[216] 3-{[3-(4-chloro-benzylcarbamoyl)-1-oxa-2,8-diaza-spiro[4.5]dec-2-en-8-carbonyl]-amino)-benzoic acid ethylester
[217] 4-{[3-(3,4-dimethoxy-benzylcarbamoyl)-1-oxa-2,8-diaza-spiro[4.5]dec-2-en-8-carbonyl]-amino)-benzoic acid ethylester
[218] 4-{[3-(4-chloro-benzylcarbamoyl)-1-oxa-2,8-diaza-spiro[4.5]dec-2-en-8-carbonyl]-amino}-benzoic acid ethylester
[219] 1-oxa-2,8-diaza-spiro[4.5]dec-2-en-3,8-dicarbonyl acid-3-(4-chloro-benzylamide) 8-naphthalen-1-ylamide
[220] 3-[4-(3-chloropyridin-2-yl)-piperazin-1-carbonyl]-1-oxa-2,8-diazaspiro[4.5]dec-2-en-8-carbonyl thioacid-(2-chloro-5-trifluormethylphenyl)-amide
[221] 8-(4-chloro-3-trifluormethyl-phenylthiocarbamoyl)-1-oxa-2,8-diaza-spiro[4.5]dec-2-en-3-carbonyl acid-3,4-dimethoxy-benzylamide
[222] 8-(3,5-bis-trifluormethyl-phenylthiocarbamoyl)-1-oxa-2,8-diaza-spiro[4.5]dec-2-en-3-carbonyl acid-3,4-dimethoxy-benzylamide
[223] 8-(3,5-bis-trifluormethyl-phenylthiocarbamoyl)-1-oxa-2,8-diaza-spiro[4.5]dec-2-en-3-carbonyl acid-4-chloro-benzylamide
[224] 3-[4-(3-chloropyridin-2-yl)-piperazin-1-carbonyl]-1-oxa-2,8-diaza-spiro[4.5]dec-2-en-8-carbonyl thioacid-(3,5-bis-trifluormethyl-phenyl)-amide
[225] 8-cyclododecylthiocarbamoyl-1-oxa-2,8-diaza-spiro[4.5]dec-2-en-3-carbonyl acid-3,4-dimethoxy-benzylamide
[226] 8-benzylthiocarbamoyl-1-oxa-2,8-diaza-spiro[4.5]dec-2-en-3-carbonyl acid-3,4-dimethoxy-benzylamide
[227] 8-benzylthiocarbamoyl-1-oxa-2,8-diaza-spiro[4.5]dec-2-en-3-carbonyl acid-4-chloro-benzylamide
[228] 3-[4-(3-trifluoromethyl-pyridin-2-yl)-piperazin-1-carbonyl]-1-oxa-2,8-diaza-spiro[4.5]dec-2-en-8-carbonyl acid-(4-tert-butyl-phenyl)-amide
[229] 3-[4-(3-chloropyridin-2-yl)-piperazin-1-carbonyl]-1-oxa-2,8-diaza-spiro[4.5]dec-2-en-8-carbonyl acid-(4-pentafluorsulfanyl-phenyl)-amide,
[230] N3-((5-methylfuran-2-yl)methyl)-N8-(4-(trifluormethyl)phenyl)-1-oxa-2,8-diazaspiro[4.5]dec-2-en-3,8-dicarboxamide,
[231] N8-(4-Methoxyphenyl)-N3-((5-methylfuran-2-yl)methyl)-1-oxa-2,8-diazaspiro[4.5]dec-2-en-3,8-dicarboxamide,
[232] N8-(4-chlorophenyl)-N3-((5-methylfuran-2-yl)methyl)-1-oxa-2,8-diazaspiro[4.5]dec-2-en-3,8-dicarboxamide,
[233] N3-(4-(3-chloropyridin-2-yl)piperazin-1-yl)-N8-(3,4-dichlorophenyl)-1-oxa-2,8-diazaspiro[4.5]dec-2-en-3,8-dicarboxamide,
[234] N3-(4-(3-chloropyridin-2-yl)piperazin-1-yl)-N8-(4-methoxyphenyl)-1-oxa-2,8-diazaspiro[4.5]dec-2-en-3,8-dicarboxamide,
[235] N8-(4-chlorobenzyl)-N3-((5-methylfuran-2-yl)methyl)-1-oxa-2,8-diazaspiro[4.5]dec-2-en-3,8-dicarboxamide,
[236] N8-(3,4-dichlorbenzyl)-N3-((5-methylfuran-2-yl)methyl)-1-oxa-2,8-diazaspiro[4.5]dec-2-en-3,8-dicarboxamide,
[237] N3-(4-chlorobenzyl)-N8-(3,4-dichlorobenzyl)-1-oxa-2,8-diazaspiro[4.5]dec-2-en-3,8-dicarboxamide,
[238] N8-(3,4-dichlorbenzyl)-N3-(4-hydroxy-3-methoxybenzyl)-1-oxa-2,8-diazaspiro[4.5]dec-2-en-3,8-dicarboxamide,
[239] N3-(4-tert-butylbenzyl)-N8-(3,4-dichlorobenzyl)-1-oxa-2,8-diazaspiro[4.5]dec-2-en-3,8-dicarboxamide,
[240] N8-(3,4-dichlorbenzyl)-N3-(4-(trifluormethyl)benzyl)-1-oxa-2,8-diazaspiro[4.5]dec-2-en-3,8-dicarboxamide,
[241] 3-(4-(3-chloropyridin-2-yl)piperazin-1-carbonyl)-N-(3,4-dichlorobenzyl)-1-oxa-2,8-diazaspiro[4.5]dec-2-en-8-carboxamide,
[242] N3-(4-(3-chloropyridin-2-yl)piperazin-1-yl)-N8-(3,4-dichlorobenzyl)-1-oxa-2,8-diazaspiro[4.5]dec-2-en-3,8-dicarboxamide,
[243] N8-(3,4-dichlorophenyl)-N3-((5-methylfuran-2-yl)methyl)-1-oxa-2,8-diazaspiro[4.5]dec-2-en-3,8-dicarboxamide,
[244] N8-(4-chlorophenyl)-N3-(4-hydroxy-3-methoxybenzyl)-1-oxa-2,8-diazaspiro[4.5]dec-2-en-3,8-dicarboxamide,
[245] N8-(3,4-dichlorophenyl)-N3-(4-hydroxy-3-methoxybenzyl)-1-oxa-2,8-diazaspiro[4.5]dec-2-en-3,8-dicarboxamide,
[246] N3-(4-chlorobenzyl)-N8-(3,4-dichlorophenyl)-1-oxa-2,8-diazaspiro[4.5]dec-2-en-3,8-dicarboxamide,
[247] N3-(4-chlorobenzyl)-N8-(4-chlorphenyl)-1-oxa-2,8-diazaspiro[4.5]dec-2-en-3,8-dicarboxamide,
[248] N3-(4-hydroxy-3-methoxybenzyl)-N8-(4-(trifluormethyl)phenyl)-1-oxa-2,8-diazaspiro[4.5]dec-2-en-3,8-dicarboxamide,
[249] N3-(4-hydroxy-3-methoxybenzyl)-N8-(4-methoxyphenyl)-1-oxa-2,8-diazaspiro[4.5]dec-2-en-3,8-dicarboxamide,
[250] N3-(4-chlorobenzyl)-N8-(4-(trifluormethyl)phenyl)-1-oxa-2,8-diazaspiro[4.5]dec-2-en-3,8-dicarboxamide,
[251] N8-(3,4-dichlorbenzyl)-N3-(3,4-dimethoxybenzyl)-1-oxa-2,8-diazaspiro[4.5]dec-2-en-3,8-dicarboxamide,
[252] N3-(3,4-dimethoxybenzyl)-N8-(4-(trifluormethyl)phenyl)-1-oxa-2,8-diazaspiro[4.5]dec-2-en-3,8-dicarboxamide,
[253] 3-[4-(3-trifluormethyl-pyridin-2-yl)-piperazin-1-carbonyl]-1-oxa-2,8-diaza-spiro[4.5]dec-2-en-8-carbonyl acid-(4-pentafluorsulfanyl-phenyl)-amide,
[254] N-(4-tert-butylphenyl)-3-(4-(3-chloropyridin-2-yl)-2-methylpiperazin-1-carbonyl)-1-oxa-2,8-diazaspiro[4.5]dec-2-en-8-carboxamide and
[255] N-(4-tert-butylbenzyl)-3-(4-(3-chloropyridin-2-yl)piperazin-1-carbonyl)-1-oxa-2,8-diazaspiro[4.5]dec-2-en-8-carboxamide.

## Revendications

1. Composés spiro substitués de formule générale I 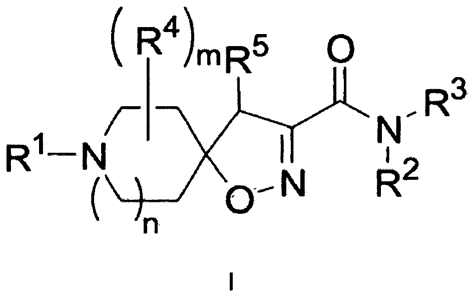 dans laquelle
m représente 0, 1, 2, 3 ou 4,
n représente 1,
R¹ représente un groupe -C(=S)-NR⁸R⁹ ou
si m est différent de 0 et/ou R⁵ est différent de H, peut également représenter un groupe -C(=O)-NR⁶R⁷,
R² représente un radical aliphatique linéaire ou ramifié, saturé ou insaturé, non substitué ou substitué au moins une fois, comprenant éventuellement au moins un hétéroatome en tant qu'élément de chaîne ;
les substituants du radical aliphatique pouvant être choisis indépendamment les uns des autres dans le groupe constitué par F, CI, Br, I, -CN, -NO₂, -OH, -SH et -NH₂ ;
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois ;
R³ représente un radical hydrogène,
un radical aliphatique linéaire ou ramifié, saturé ou insaturé, non substitué ou substitué au moins une fois, comprenant éventuellement au moins un hétéroatome en tant qu'élément de chaîne ;
les substituants du radical aliphatique pouvant être choisis indépendamment les uns des autres dans le groupe constitué par F, CI, Br, I, -CN, -NO₂, -OH, -SH et -NH₂ ;
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois ;
ou
R² et R³ forment ensemble avec l'atome d'azote qui les relie un radical cycloaliphatique non substitué ou substitué au moins une fois, insaturé ou saturé, comprenant éventuellement au moins un hétéroatome supplémentaire en tant qu'élément de cycle, qui peut être condensé avec un système cyclique mono- ou polycyclique non substitué ou substitué au moins une fois ;
R⁴ représente un radical halogène, un groupe nitro, un groupe cyano, un groupe amino, un groupe hydroxy, un groupe thiol, un groupe carboxy, un groupe formyle, un groupe -NH-C(=O)-H, un groupe oxo (=O), un groupe -NH-R¹⁰, un groupe -NR¹¹R¹², un groupe -C(=O)-R¹³, un groupe -C(=O)-O-R¹⁴, un groupe -O-C(=O)-R¹⁵, un groupe -NH-C(=O)-R¹⁶, un groupe -NR¹⁷-C(=O)-R¹⁸, un groupe -C(=O)-NH₂, un groupe -C(=O)-NH-R¹⁹, un groupe -C(=O)-NR²⁰R²¹, un groupe -O-R²², un groupe -S-R²³ ou un radical aliphatique linéaire ou ramifié, saturé ou insaturé, non substitué ou substitué au moins une fois ;
R⁵ représente un représente un radical hydrogène, un radical halogène, un groupe nitro, un groupe cyano, un groupe amino, un groupe hydroxy, un groupe thiol, un groupe carboxy, un groupe formyle, un groupe -NH-C(=O)-H, un groupe oxo (=O), un groupe -NH-R¹⁰, un groupe -NR¹¹R¹², un groupe -C(=O)-R¹³, un groupe -C(=O)-O-R¹⁴, un groupe -O-C(=O)-R¹⁵, un groupe -NH-C(=O)-R¹⁶, un groupe -NR¹⁷-C(=O)-R¹⁸, un groupe -C(=O)-NH₂, un groupe -C(=O)-NH-R¹⁹, un groupe -C(=O)-NR²⁰R²¹, un groupe -O-R²², un groupe -S-R²³ ou un radical aliphatique linéaire ou ramifié, saturé ou insaturé, non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois ;
R⁶ et R⁸ représentent chacun indépendamment l'un de l'autre un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois, et/ou
ponté avec au moins un groupe alkylène linéaire ou ramifié, non substitué ou substitué au moins une fois,
un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
un groupe -C(=O)-R²⁴, qui peut être relié par un groupe alkylène linéaire ou ramifié, non substitué ou substitué au moins une fois,
ou un groupe -C(=O)-O-R²⁵, qui peut être relié par un groupe alkylène linéaire ou ramifié, non substitué ou substitué au moins une fois ;
R⁷ et R⁹ représentent chacun indépendamment l'un de l'autre un radical hydrogène,
un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois, et/ou ponté avec au moins un groupe alkylène linéaire ou ramifié, non substitué ou substitué au moins une fois, un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
un groupe -C(=O)-R²⁴, qui peut être relié par un groupe alkylène linéaire ou ramifié, non substitué ou substitué au moins une fois,
ou un groupe -C(=O)-O-R²⁵, qui peut être relié par un groupe alkylène linéaire ou ramifié, non substitué ou substitué au moins une fois ;
et
R¹⁰ à R²⁵ représentent chacun indépendamment les uns des autres un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne,
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants.

2. Composés selon la revendication 1, **caractérisés en ce que**
m représente 0, 1, 2, 3 ou 4,
n représente 1,
R¹ représente un groupe -C(=S)-NR⁸R⁹ ou
si m est différent de 0 et/ou R⁵ est différent de H, peut également représenter un groupe -C(=O)-NR⁶R⁷,
R² représente un radical aliphatique en C₁-₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué, qui peut être condensé avec un système cyclique mono-ou polycyclique saturé ou insaturé, éventuellement substitué,
ou -(CHR³⁰)-Xᵥ-(CHR³¹)_{w-}Yₓ-(CHR³²)_{y}-Z_{z}-R³³ avec v = 0 ou 1, w = 0 ou 1, x = 0 ou 1, y = 0 ou 1, z = 0 ou 1, X, Y et Z représentant chacun indépendamment les uns des autres O, S, NH, N(CH₃), N(C₂H₅) ou N[CH(CH₃)₂] ;
R³ représente un radical hydrogène,
un radical aliphatique en C₁₋₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué, qui peut être condensé avec un système cyclique mono-ou polycyclique saturé ou insaturé, éventuellement substitué,
ou -(CHR³⁰)-Xᵥ-(CHR³¹)_{w}-Yₓ-CHR³²)_{y}-Z_{z}-R³³ avec v = 0 ou 1, w = 0 ou 1, x = 0 ou 1, y = 0 ou 1, z = 0 ou 1, X, Y et Z représentant chacun indépendamment les uns des autres O, S, NH, N(CH₃), N(C₂H₅) ou N[CH(CH₃)₂] ;
ou
R² et R³ forment ensemble avec l'atome d'azote qui les relie en tant qu'élément de cycle un radical hétérocycloaliphatique à 4, 5, 6, 7, 8 ou 9 éléments, saturé ou insaturé, éventuellement substitué, qui peut être condensé avec un système cyclique mono- ou polycyclique saturé, insaturé ou aromatique, éventuellement substitué,
R⁴ représente un radical halogène, un groupe nitro, un groupe cyano, un groupe amino, un groupe hydroxy, un groupe thiol, un groupe carboxy, un groupe formyle, un groupe -NH-C(=O)-H, un groupe oxo (=O), un groupe -NH-R¹⁰, un groupe -NR¹¹R¹², un groupe -C(=O)-R¹³, un groupe -C(=O)-O-R¹⁴, un groupe -O-C(=O)-R¹⁵, un groupe -NH-C(=O)-R¹⁶, un groupe -NR¹⁷-C(=O)-R¹⁸, un groupe -C(=O)-NH₂, un groupe -C(=O)-NH-R¹⁹, un groupe -C(=O)-NR²⁰R²¹, un groupe -O-R²², un groupe -S-R²³ ou un radical aliphatique en C₁₋₁₀ linéaire ou ramifié, saturé ou insaturé, éventuellement substitué ;
R⁵ représente un représente un radical hydrogène, un radical halogène, un groupe nitro, un groupe cyano, un groupe amino, un groupe hydroxy, un groupe thiol, un groupe carboxy, un groupe formyle, un groupe -NH-C(=O)-H, un groupe oxo (=O), un groupe -NH-R¹⁰, un groupe -NR¹¹R¹², un groupe -C(=O)-R¹³, un groupe -C(=O)-O-R¹⁴, un groupe -O-C(=O)-R¹⁵, un groupe -NH-C(=O)-R¹⁶, un groupe -NR¹⁷-C(=O)-R¹⁸, un groupe -C(=O)-NH₂, un groupe -C(=O)-NH-R¹⁹, un groupe -C(=O)-NR²⁰R²¹, un groupe -O-R²², un groupe -S-R²³ ou un radical aliphatique en C₁₋₁₀ linéaire ou ramifié, saturé ou insaturé, éventuellement substitué,
un radical aryle ou hétéroaryle de 5 à 14 éléments, éventuellement substitué,
ou -(CH₂)ₐₐ-R³⁴ avec aa = 1, 2, 3 ou 4 ;
R⁶ et R⁸ représentent chacun indépendamment l'un de l'autre un radical aliphatique en C₁₋₂₀ linéaire ou ramifié, saturé ou insaturé, éventuellement substitué,
un radical cycloaliphatique à 3, 4, 5, 6, 7, 8, 9, 10, 11 ou 12 éléments, insaturé ou saturé, éventuellement substitué, qui peut être condensé avec un système cyclique mono- ou polycyclique saturé, insaturé ou aromatique, éventuellement substitué, et/ou ponté avec un ou deux groupes alkylène en C₁₋₅ linéaires ou ramifiés, éventuellement substitués ;
un radical aryle ou hétéroaryle de 5 à 14 éléments, éventuellement substitué, qui peut être condensé avec un système cyclique mono-ou polycyclique saturé ou insaturé, éventuellement substitué ;
(CHR³⁵)-(CH₂)_{bb}-(CH₂)_{cc}-C(=O)-R²⁴ avec bb = 0 ou 1 et cc = 0 ou 1 ; -(CHR³⁶)-(CH₂)_{dd}-(CH₂)ₑₑ-C(=O)-O-R²⁵ avec dd = 0 ou 1 et ee = 0 ou 1 ;
ou -(CR³⁷R³⁸)-X_{ff}-(CHR³⁹)_{gg}-Yₕₕ-(CHR⁴⁰)ⱼⱼ-Zₖₖ-R⁴¹ avec ff = 0 ou 1, gg = 0 ou 1, hh = 0 ou 1, jj = 0 ou 1, kk = 0 ou 1, X, Y et Z, représentant chacun indépendamment les uns des autres O, S, NH, N(CH₃), N(C₂H₅) ou N[CH(CH₃)₂] ;
R⁷ et R⁹ représentent chacun indépendamment l'un de l'autre un radical hydrogène,
un radical aliphatique en C₁₋₂₀ linéaire ou ramifié, saturé ou insaturé, éventuellement substitué,
un radical cycloaliphatique à 3, 4, 5, 6, 7, 8, 9, 10, 11 ou 12 éléments, insaturé ou saturé, éventuellement substitué, qui peut être condensé avec un système cyclique mono- ou polycyclique saturé, insaturé ou aromatique, éventuellement substitué, et/ou ponté avec un ou deux groupes alkylène en C₁₋₅ linéaires ou ramifiés, éventuellement substitués ;
un radical aryle ou hétéroaryle de 5 à 14 éléments, éventuellement substitué, qui peut être condensé avec un système cyclique mono-ou polycyclique saturé ou insaturé, éventuellement substitué ;
(CHR³⁵)-(CH₂)_{bb}-(CH₂)_{cc}-C(=O)-R²⁴ avec bb = 0 ou 1 et cc = 0 ou 1 ; -(CHR³⁶)-(CH₂)_{dd}-(CH₂)ₑₑ-C(=O)-O-R²⁵ avec dd = 0 ou 1 et ee = 0 ou 1 ;
ou -(CR³⁷R³⁸)-X_{ff}-(CHR³⁹)_{gg}-Yₕₕ-(CHR⁴⁰)ⱼⱼ-Zₖₖ-R⁴¹ avec ff = 0 ou 1, gg = 0 ou 1, hh = 0 ou 1, jj = 0 ou 1, kk = 0 ou 1, X, Y et Z, représentant chacun indépendamment les uns des autres O, S, NH, N(CH₃), N(C₂H₅) ou N[CH(CH₃)₂] ;
R¹⁰ à R²⁵ représentent chacun indépendamment les uns des autres
un radical aliphatique en C₁₋₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué, qui peut être condensé avec un système cyclique mono-ou polycyclique saturé ou insaturé, éventuellement substitué,
ou -(CH₂)ₘₘ-R⁴², avec mm représentant 1, 2 ou 3 ;
R²⁶, R²⁷, R²⁸, R³⁰, R³¹, R³², R³⁵, R³⁶, R³⁷, R³⁸, R³⁹ et R⁴⁰ représentent chacun indépendamment les uns des autres
un radical hydrogène ;
un radical aliphatique en C₁₋₁₀ linéaire ou ramifié, saturé ou insaturé, éventuellement substitué ;
ou un radical aryle ou hétéroaryle de 5 à 14 éléments, éventuellement substitué, qui peut être condensé avec un système cyclique mono- ou polycyclique saturé ou insaturé, éventuellement substitué ;
R²⁹, R³³ et R⁴¹ représentent chacun indépendamment les uns des autres
un radical aliphatique en C₁₋₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être ponté avec 1, 2, 3, 4 ou 5 groupes alkylène en C₁₋₅ linéaires ou ramifiés, éventuellement substitués, et/ou condensé avec un système cyclique mono- ou polycyclique, saturé, insaturé ou aromatique, éventuellement substitué ;
ou un radical aryle ou hétéroaryle de 5 à 14 éléments, éventuellement substitué, qui peut être condensé avec un système cyclique mono- ou polycyclique saturé ou insaturé, éventuellement substitué ;
et
R³⁴ et R⁴² représentent chacun indépendamment l'un de l'autre un radical aryle ou hétéroaryle de 5 à 14 éléments, éventuellement substitué ;
les radicaux aliphatiques en C₁₋₁₀ et les radicaux aliphatiques en C₁₋₂₀ mentionnés précédemment pouvant chacun éventuellement être substitués avec 1, 2, 3, 4, 5, 6, 7, 8 ou 9 substituants choisis indépendamment les uns des autres dans le groupe constitué par F, CI, Br, I, -CN, -NO₂, -OH, -SH et -NH₂ ;
les radicaux cycloaliphatiques et les radicaux hétérocycloaliphatiques mentionnés précédemment pouvant chacun éventuellement être substitués avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -OC(=O)-alkyle en C₁₋₅, -NH-alkyle en C₁₋₅, -N(alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -S(=O)₂-NH-alkyle en C₁₋₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, -alkyle en C₁₋₅, -O-alkyle en C₁₋₅, -O-CF₃, -S-CF₃, phényle et -O-benzyle,
et les radicaux cycloaliphatiques mentionnés précédemment pouvant chacun éventuellement comprendre 1, 2, 3, 4 ou 5 hétéroatomes choisis indépendamment les uns des autres dans le groupe constitué par l'oxygène, l'azote et le soufre en tant qu'éléments de cycle ;
les radicaux hétérocycloaliphatiques mentionnés précédemment pouvant chacun éventuellement comprendre 1, 2 ou 3 hétéroatomes supplémentaires choisis indépendamment les uns des autres dans le groupe constitué par l'oxygène, l'azote et le soufre en tant qu'éléments de cycle ;
les cycles des systèmes cycliques mono- ou polycycliques mentionnés précédemment pouvant chacun éventuellement être substitués avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -O-C(=O)-alkyle en C₁₅, -NH-alkyle en C₁₋₅, -N(alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -S(=O)₂-NH-alkyle en C₁₋₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, -alkyle en C₁₋₅, -O-alkyle en C₁₋₅, -O-CF₃, -S-CF₃, phényle et -O-benzyle,
et les cycles des systèmes cycliques mono- ou polycycliques mentionnés précédemment étant chacun à 5, 6 ou 7 éléments et pouvant chacun éventuellement comprendre 1, 2, 3, 4 ou 5 hétéroatomes en tant qu'éléments de cycle, choisis indépendamment les uns des autres dans le groupe constitué par l'oxygène, l'azote et le soufre ;
et les radicaux aryle ou hétéroaryle mentionnés précédemment pouvant chacun éventuellement être substitués avec 1, 2, 3, 4 ou 5 substituants choisis indépendamment les uns des autres dans le groupe constitué par F, CI, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -NH-alkyle en C₁₋₅, -N(alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -S(=O)₂-NH-alkyle en C₁₋₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -0-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, -alkyle en C₁₋₅, -O-alkyle en C₁₋₅, -O-CF₃, -S-CF₃, phényle et -O-benzyle,
les radicaux hétéroaryle mentionnés précédemment pouvant chacun éventuellement comprendre 1, 2, 3, 4 ou 5 hétéroatomes choisis indépendamment les uns des autres dans le groupe constitué par l'oxygène, l'azote et le soufre en tant qu'éléments de cycle ;
et
les groupes alkylène en C₁₋₅ mentionnés précédemment pouvant chacun éventuellement être substitués avec 1, 2, 3, 4 ou 5 substituants choisis indépendamment les uns des autres dans le groupe constitué par F, CI, Br, -OH, -SH, -NH₂, -CN et NO₂ ;
à chaque fois éventuellement sous la forme d'un de leurs stéréoisomères purs, notamment énantiomères ou diastéréomères, de leurs racémats ou
sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants.

3. Composés selon la revendication 1 ou 2, **caractérisés en ce que**
R² représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, CI, Br, I, -CN, -NO₂, -OH, -SH, et -NH₂ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical pouvant à chaque fois éventuellement être substitué avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ; un radical choisi dans le groupe constitué par phényle, naphtyle, (1,3)-benzodioxolyle, (1,4)-benzodioxanyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(₌O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle,
ou -(CHR³⁰)-R³³ ; -(CHR³⁰)-(CHR³¹)-R³³ ; -(CHR³⁰)-(CHR³¹)-O-R³³; -(CHR³⁰)-(CHR³¹)-(CHR³²)-R³³ ; -(CHR³⁰)-(CHR³¹)-S-(CHR³²)-R³³ ; -(CHR³⁰)-(CHR³¹)-(CHR³²)-N(CH³²)-R³³_{.}

4. Composés selon une ou plusieurs des revendications 1 à 3, **caractérisés en ce que**
R³ représente un radical hydrogène.

5. Composés selon une ou plusieurs des revendications 1 à 4, **caractérisés en ce que**
R² et R³ forment ensemble avec l'atome d'azote qui les relie en tant qu'élément de cycle un radical choisi dans le groupe constitué par pyrrolidinyle, pipéridinyle, (1,3,4,5)-tétrahydropyrido[4,3-b]indolyle, (3,4)-dihydro-1H-isoquinolinyle, (1,3,4,9)-tétrahydro-[b]-carbolinyle, imidazolidinyle, (1,3)-thiazolidinyle, pipérazinyle, morpholinyle, azépanyle, diazépanyle et thiomorpholinyle, le radical pouvant à chaque fois être substitué avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(₌O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O=CF3, -S-CF₃, phényle et -O-benzyle.

6. Composés selon une ou plusieurs des revendications 1 à 5, **caractérisés en ce que**
R⁴ représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle, le radical pouvant à chaque fois éventuellement être substitué avec 1, 2, 3, 4 ou 5 substituants choisis indépendamment les uns des autres dans le groupe constitué par F, CI, Br, I, -CN, -NO₂, -OH, -SH et -NH₂.

7. Composés selon une ou plusieurs des revendications 1 à 6, **caractérisés en ce que**
R⁵ représente un radical hydrogène,
un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et -NH₂ ;
un radical choisi dans le groupe constitué par phényle, naphtyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(₌O)-C₂H₅, -O-C(-O)-C(CH₃)₃, -N(CH₃)₂, -N(C₂H₅)₂, -NH-CH₃, -NH-C₂H₅, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂ et -C(=O)-N-(C₂H₅)₂ ;
ou -(CH₂)-R34, -(CH₂)-(CH₂)-R³⁴ ou -(CH₂)-(CH₂)-(CH₂)-R³⁴.

8. Composés selon une ou plusieurs des revendications 1 à 7, **caractérisés en ce que**
R⁶ et R⁸ représentent chacun indépendamment l'un de l'autre un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, CI, Br, I, -CN, -NO₂, -OH, -SH et -NH₂ ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois éventuellement être substitué avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(₌O)-N-(C₂H₅)₂, -S(₌O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF_{S}, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinotinyle, isoquinolinyle, benzimidazolinyle, benzoxazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
-(CHR³⁵)-C(=O)-R^{2a} ou -(CHR³⁵)-(CH₂)-C(=O)-R²⁴ ;
-(CHR³⁶)-C(=O)-O-R²⁵ ou -(CHR³⁶)-(CH₂)-C(=O)-O-R²⁵ ;
ou -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-CHR⁴⁰)-N(CH₃)-R⁴¹ ou -(CR³⁷R³⁸)-(CHR³⁹)-(CHR⁴⁰)-N(C₂H₅)-R⁴¹.

9. Composés selon une ou plusieurs des revendications 1 à 8, **caractérisés en ce que**
R⁷ et R⁹ représentent chacun un radical hydrogène.

10. Composés selon une ou plusieurs des revendications 1 à 9, **caractérisés en ce que**
R¹⁰ à R²⁵ représentent chacun indépendamment les uns des autres un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle,
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois être substitué avec 1 ou 2 substituants choisis indépendamment l'un de l'autre dans le groupe constitué par -C(=O)-O-CH₃, -C(=O)-O-C₂H₅, -C(=O)-O-C(CH₃)₃, -C(₌O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, pyridinyle, phényle et benzyle, la partie cyclique des radicaux pyridinyle, -S(=O)₂-phényle, phényle et benzyle pouvant à chaque fois être substituée avec 1, 2, 3, 4 ou 5 substituants choisis indépendamment les uns des autres dans le groupe constitué par F, CI, Br, méthyle, éthyle, n-propyle, isopropyle, n-butyle, sec-butyle, isobutyle, tert-butyle, -O-CH₃ et -O-C₂H₅ ;
un radical choisi dans le groupe constitué par phényle, naphtyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle et pyrimidinyle, le radical pouvant à chaque fois éventuellement être substitué avec 1, 2, 3, 4 ou 5 substituants choisis indépendamment les uns des autres dans le groupe constitué par F, CI, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -O-phényle, -O-benzyle et phényle,
ou -(CH₂)-R⁴² ou -(CH₂)-(CH₂)-_{R}⁴².

11. Composés selon une ou plusieurs des revendications 1 à 10, **caractérisés en ce que**
R²⁶, R²⁷, R²⁸, R³⁰, R³¹, R³², R³⁵, R³⁶, R³⁷, R³⁸, R³⁹ et R⁴⁰ représentent chacun indépendamment les uns des autres un radical hydrogène ;
un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle, ou un radical phényle, qui peut être substitué avec 1, 2, 3, 4 ou 5 substituants choisis indépendamment les uns des autres dans le groupe constitué par F, CI, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -O-phényle, -O-benzyle et phényle.

12. Composés selon une ou plusieurs des revendications 1 à 11, **caractérisés en ce que**
R²⁹, R³³ et R⁴¹ représentent chacun indépendamment les uns des autres un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, CI, Br, I, -CN, -NO₂, -OH, -SH et -NH₂ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical pouvant à chaque fois éventuellement être substitué avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle.

13. Composés selon une ou plusieurs des revendications 1 à 12, **caractérisés en ce que**
R³⁴ et R⁴² représentent chacun indépendamment l'un de l'autre un radical choisi dans le groupe constitué par phényle, naphtyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle et pyrimidinyle, le radical pouvant à chaque fois éventuellement être substitué avec 1, 2, 3, 4 ou 5 substituants choisis indépendamment les uns des autres dans le groupe constitué par F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃.

14. Composés selon une ou plusieurs des revendications 1 à 13, **caractérisés en ce que**
m représente 0.

15. Composés selon une ou plusieurs des revendications 1 à 14, **caractérisés en ce que**
m représente 0, 1, 2, 3 ou 4 ;
n représente 1 ;
R¹ représente un groupe -C(=S)-NR⁸R⁹ ou
si m est différent de 0 et/ou n est différent de 1 et/ou R⁵ est différent de H, peut également représenter un groupe -C(=O)-NR⁶R⁷,
R² représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle et thiomorpholinyle, le radical pouvant à chaque fois être substitué avec 1 ou 2 substituants choisis indépendamment l'un de l'autre dans le groupe constitué par pyridinyle, pyridazinyle, phényle et benzyle, la partie cyclique des radicaux pyridinyle, pyridazinyle, phényle et benzyle pouvant à chaque fois être substituée avec 1, 2, 3, 4 ou 5 substituants choisis indépendamment les uns des autres dans le groupe constitué par -CF₃, F, CI, Br, méthyle, éthyle, n-propyle, isopropyle, n-butyle, sec-butyle, isobutyle, tert-butyle, -O-CH₃ et -O-C₂H₅ ;
un radical choisi dans le groupe constitué par phényle, naphtyle, thiophényle, furanyle, pyrrolyle, pyrazolyle, pyrazinyle et pyridinyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ et -S(=O)₂-NH-C₂H₅,
ou -(CHR³⁰)-R³³; -(CHR³⁰)-(CHR³¹)-R³³ ou -(CHR³⁰)-(CHR³¹)-(CHR³²)-R³³ ;
R³ représente un radical hydrogène ;
ou
R² et R³ forment ensemble avec l'atome d'azote qui les relie en tant qu'élément de cycle un radical choisi dans le groupe constitué par pyrrolidinyle, pipéridinyle, pipérazinyle, 3-méthyl-pipérazinyle, 2-méthyl-pipérazinyle, (3,5)-diméthylpipérazinyle, (2,6)-diméthylpipérazinyle, morpholinyle et thiomorpholinyle, le radical pouvant à chaque fois être substitué avec 1 ou 2 substituants choisis indépendamment l'un de l'autre dans le groupe constitué par -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, pyridinyle, pyridazinyle, phényle et benzyle, la partie cyclique des radicaux pyridinyle, -S(=O)₂-phényle, pyridazinyle, phényle et benzyle pouvant à chaque fois être substituée avec 1, 2, 3, 4 ou 5 substituants choisis indépendamment les uns des autres dans le groupe constitué par -CF₃, F, CI, Br, méthyle, éthyle, n-propyle, isopropyle, n-butyle, sec-butyle, isobutyle, tert-butyle, -O-CH₃ et -O-C₂H₅ ;
R⁴ représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle,
R⁵ représente un radical hydrogène,
un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle,
un radical phényle, qui peut à chaque fois être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle et tert-butyle ; ou -(CH₂)-R³⁴ ;
R⁶ et R⁸ représentent chacun indépendamment l'un de l'autre un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl et Br ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle ;
un radical choisi dans le groupe constitué par phényle, naphtyle, thiophényle, furanyle, pyrrolyle, pyrazolyle, pyranyle, triazolyle, pyridinyle, imidazolyle, thiazolyle, oxazolyle, isoxazolyle, pyridazinyle, pyrazinyle et pyrimidinyle, le radical pouvant à chaque fois éventuellement être substitué avec 1, 2, 3, 4 ou 5 substituants choisis indépendamment les uns des autres dans le groupe constitué par F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
-(CHR³⁶)-C(=O)-O-R²⁵ ou -(CHR³⁶)-(CH₂)-C(=O)-O-R²⁵ ;
ou -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹HCHR⁴⁰)-N(CH₃)-R⁴¹ ou -(CR³⁷R³⁸)-(CHR³⁹)-(CHR⁴⁰)-N(C₂H₅)-R⁴¹ ;
R⁷ et R⁹ représentent chacun un radical hydrogène ;
R²⁵ représente un radical choisi dans le groupe constitué par méthyle, éthyle, n-propyle, isopropyle, n-butyle, sec-butyle, isobutyle et tert-butyle ;
R²⁶, R²⁷, R²⁸, R³⁰, R³¹, R³², R³⁶, R³⁷, R³⁸, R³⁹ et R⁴⁰ représentent chacun indépendamment les uns des autres
un radical hydrogène ;
un radical choisi dans le groupe constitué par méthyle, éthyle, n-propyle, isopropyle, n-butyle, sec-butyle, isobutyle et tert-butyle ;
ou un radical phényle, qui peut être substitué avec 1, 2, 3, 4 ou 5 substituants choisis indépendamment les uns des autres dans le groupe constitué par F, CI, Br, I, -CN, -CF₃, -O-CH₃, -O-C₂H₅, -NO₂, -O-CF₃, méthyle, éthyle, n-propyle, isopropyle, n-butyle, sec-butyle, isobutyle et tert-butyle ;
R²⁹ et R³³ représentent chacun indépendamment l'un de l'autre
un radical choisi dans le groupe constitué par phényle, naphtyle, thiophényle, furanyle, pyrrolyle, pyrazolyle, pyrazinyle et pyridinyle, le radical pouvant à chaque fois éventuellement être substitué avec 1, 2, 3, 4 ou 5 substituants choisis indépendamment les uns des autres dans le groupe constitué par F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ et -S(=O)₂-NH-C₂H₅ ;
R³⁴ représente un radical phényle, qui peut être substitué avec 1, 2, 3, 4 ou 5 substituants choisis indépendamment les uns des autres dans le groupe constitué par F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle et tert-butyle ;
et
R⁴¹ représente un radical choisi dans le groupe constitué par méthyle, éthyle, n-propyle, isopropyle, n-butyle, sec-butyle, isobutyle et tert-butyle ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle et dithiolanyle ;
ou un radical choisi dans le groupe constitué par phényle, naphtyle, (1,3)-benzodioxolyle, (1,4)-benzodioxanyle, thiophényle et furanyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅ et -C(=O)-C(CH₃)₃ ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants.

16. Composés selon une ou plusieurs des revendications 1 à 15, **caractérisés en ce que**
m représente 0 ;
n représente 1 ;
R¹ représente un groupe -C(=S)-NR⁸R⁹ ;
R² représente un radical choisi dans le groupe constitué par phényle, naphtyle et pyridinyle, le radical pouvant à chaque fois être substitué avec 1, 2 ou 3 substituants choisis indépendamment les uns des autres dans le groupe constitué par F, CI, Br, -OH, -O-CH₃, -O-C₂H₅, -NH-S(=O)₂-CH₃ et -NH-S(=O)₂-C₂H₅ ;
un radical pipérazinyle, qui peut être substitué sur un atome d'azote avec un substituant choisi dans le groupe constitué par pyridinyle, pyridazinyle, phényle et benzyle, la partie cyclique des substituants pyridinyle, pyridazinyle, phényle et benzyle pouvant à chaque fois être substituée avec 1, 2 ou 3 substituants choisis indépendamment les uns des autres dans le groupe constitué par -CF₃, F, CI, Br, méthyle, éthyle, n-propyle, isopropyle, n-butyle, sec-butyle, isobutyle et tert-butyle ;
ou -(CHR³⁰)-R³³ ; -(CHR³⁰)-(CHR³¹)-R³³ ou -(CHR³⁰)-(CHR³¹)-(CHR³²)-R³³ ;
R³ représente un radical hydrogène ;
ou
R² et R³ forment ensemble avec l'atome d'azote qui les relie en tant qu'élément de cycle un radical choisi dans le groupe constitué par 3-méthyl-pipérazinyle, 2-méthyl-pipérazinyle, (3,5)-diméthylpipérazinyle, (2,6)-diméthylpipérazinyle et pipérazinyle, qui peut être substitué sur un atome d'azote avec un substituant choisi dans le groupe constitué par pyridinyle, pyridazinyle, phényle et benzyle, la partie cyclique des radicaux pyridinyle, pyridazinyle, phényle et benzyle pouvant à chaque fois être substituée avec 1, 2 ou 3 substituants choisis indépendamment les uns des autres dans le groupe constitué par -CF₃, F, CI, Br, méthyle, éthyle, n-propyle, isopropyle, n-butyle, sec-butyle, isobutyle et tert-butyle ;
R⁵ représente un radical hydrogène,
R⁶ et R⁸ représentent chacun indépendamment les uns des autres un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle et 2-méthyl-1-propényle, le radical pouvant à chaque fois éventuellement être substitué avec 1, 2, 3, 4 ou 5 substituants choisis indépendamment les uns des autres dans le groupe constitué par F, CI et Br ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle et adamantyle ;
un radical choisi dans le groupe constitué par phényle, naphtyle et pyridinyle, le radical pouvant à chaque fois éventuellement être substitué avec 1, 2, 3, 4 ou 5 substituants choisis indépendamment les uns des autres dans le groupe constitué par -SF₅, F, CI, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -O-phényle, -O-benzyle et phényle, la partie cyclique des radicaux cyclopentyle, cyclohexyle, -O-phényle, -O-benzyle et phényle pouvant à chaque fois être substituée avec 1, 2, 3, 4 ou 5 substituants choisis indépendamment les uns des autres dans le groupe constitué par F, CI, Br, méthyle, éthyle, n-propyle, isopropyle, n-butyle, sec-butyle, isobutyle et tert-butyle ;
-(CHR³⁶)-C(=O)-O-R²⁵ ou -(CHR³⁶)-(CH₂)-C(=O)-O-R²⁵ ;
ou -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ ou -(CR³⁷R³⁸)-(CHR³⁹)-(CHR⁴⁰)-N(C₂H₅)-R⁴¹;
R⁷ et R⁹ représentent chacun un radical hydrogène ;
R²⁵ représente un radical choisi dans le groupe constitué par méthyle, éthyle, n-propyle, isopropyle, n-butyle, sec-butyle, isobutyle et tert-butyle ;
R³⁰, R³¹, R³², R³⁹ et R⁴⁰ représentent chacun un radical hydrogène ;
R³³ représente un radical choisi dans le groupe constitué par phényle, naphtyle, thiophényle et furanyle, le radical pouvant à chaque fois éventuellement être substitué avec 1, 2, 3, 4 ou 5 substituants choisis indépendamment les uns des autres dans le groupe constitué par -CF₃, F, CI, Br, -OH, -O-CH₃, -O-C₂H₅, méthyle, éthyle, n-propyle, isopropyle, n-butyle, sec-butyle, isobutyle, tert-butyle, -NH-S(=O)₂-CH₃ et -NH-S(=O)₂-C₂H₅ ;
R³⁶ représente un radical hydrogène
ou un radical choisi dans le groupe constitué par méthyle, éthyle, n-propyle, isopropyle, n-butyle, sec-butyle, isobutyle et tert-butyle ;
R³⁷ et R³⁸ représentent chacun indépendamment l'un de l'autre un radical hydrogène ;
ou un radical choisi dans le groupe constitué par méthyle, éthyle, n-propyle, isopropyle, n-butyle, sec-butyle, isobutyle et tert-butyle ;
ou un radical phényle ;
R⁴¹ représente un radical choisi dans le groupe constitué par méthyle, éthyle, n-propyle, isopropyle, n-butyle, sec-butyle, isobutyle et tert-butyle ;
un radical choisi dans le groupe constitué par tétrahydrofuranyle, tétrahydrothiophényle, pyrrolidinyle, morpholinyle, pipéridinyle et pipérazinyle ;
ou un radical choisi dans le groupe constitué par phényle, naphtyle, (1,3)-benzodioxolyle, (1,4)-benzodioxanyle, thiophényle et furanyle, le radical pouvant à chaque fois éventuellement être substitué avec 1, 2, 3, 4 ou 5 substituants choisis indépendamment les uns des autres dans le groupe constitué par F, CI, Br, -OH, -O-CH₃, -O-C₂H₅, méthyle, éthyle, n-propyle, isopropyle, n-butyle, sec-butyle, isobutyle et tert-butyle ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants.

17. Composés selon une ou plusieurs des revendications 1 à 16, **caractérisés en ce que** les composés présentent lors de l'essai FLIPR en une concentration de 10 µM une inhibition de l'afflux d'ions Ca²⁺ dans les ganglions rachidiens de rats d'au moins 10 %, de préférence d'au moins 30 %, de manière particulièrement préférée d'au moins 50 %, de manière tout particulièrement préférée d'au moins 70 %, de manière encore davantage préférée d'au moins 90 %, en comparaison de l'inhibition maximale atteignable de l'afflux d'ions Ca²⁺ avec la capsaïcine en une concentration de 10 µM.

18. Procédé de fabrication de composés spiro substitués de formule générale I selon une ou plusieurs des revendications 1 à 17, **caractérisé en ce qu'**au moins un composé de formule générale II 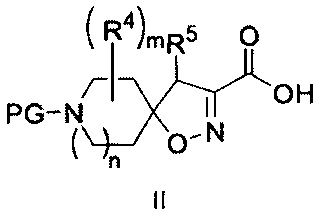 dans laquelle R⁴, R⁵, m et n ont la signification selon une ou plusieurs des revendications 1 à 17 et PG représente un groupe protecteur, de préférence un groupe benzyloxycarbonyle ou tert-butyloxycarbonyle, est mis en réaction dans un milieu réactionnel en présence d'au moins un réactif de couplage, éventuellement en présence d'au moins une base, avec un composé de formule générale HNR²R³, R² et R³ ayant la signification selon une ou plusieurs des revendications 1 à 17, pour former au moins un composé de formule générale III 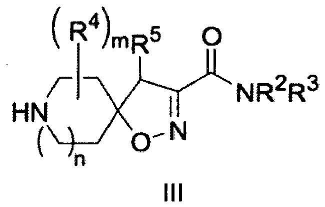 dans laquelle R², R³, R⁴, R⁵, m, n et PG ont la signification indiquée précédemment, et celui-ci est éventuellement purifié et/ou isolé
et
au moins un composé de formule générale III est mis en réaction dans un milieu réactionnel en présence d'au moins un acide, de préférence en présence d'au moins un acide inorganique ou organique choisi dans le groupe constitué par l'acide chlorhydrique, l'acide sulfurique, l'acide acétique et l'acide trifluoroacétique, ou en présence d'hydrogène et d'un catalyseur, de préférence d'un catalyseur à base de palladium ou de platine, pour former au moins un composé de formule générale IV 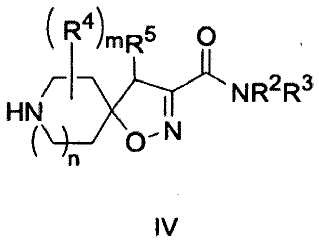 dans laquelle R², R³, R⁴, R⁵, m et n ont la signification indiquée précédemment, et celui-ci est éventuellement purifié et/ou isolé
et
au moins un composé de formule générale IV est mis en réaction dans un milieu réactionnel avec au moins un isocyanate de formule générale R⁶-N=C=O, R⁶ ayant la signification selon une ou plusieurs des revendications 1 à 17, éventuellement en présence d'au moins une base, de préférence en présence d'au moins une base choisie dans le groupe constitué par la triéthylamine, la 4,4-diméthylaminopyridine et la diisopropyléthylamine, pour former au moins un composé de formule générale I, dans laquelle R², R³, R⁴, R⁵, m et n ont la signification indiquée précédemment et R¹ représente -C(=O)-NR⁶R⁷, R⁶ ayant la signification indiquée précédemment et R⁷ représentant un radical hydrogène, et celui-ci est éventuellement purifié et/ou isolé
ou
au moins un composé de formule générale IV est mis en réaction dans un milieu réactionnel avec au moins un isothiocyanate de formule générale S=C=N-R⁸, R⁸ ayant la signification selon une ou plusieurs des revendications 1 à 17, éventuellement en présence d'au moins une base, de préférence en présence d'au moins une base choisie dans le groupe constitué par la triéthylamine, la 4,4-diméthylaminopyridine et la diisopropyléthylamine, pour former au moins un composé de formule générale I, dans laquelle R², R³, R⁴, R⁵, m et n ont la signification indiquée précédemment et R¹ représente -C(=S)-N-R⁸R⁹, R⁸ ayant la signification indiquée précédemment et R⁹ représentant un radical hydrogène, et celui-ci est éventuellement purifié et/ou isolé
et éventuellement au moins un composé de formule générale I, dans laquelle R², R³, R⁴, R⁵, m et n ont la signification indiquée précédemment et R¹ représente -C(=O)-NR⁶R⁷ ou -C(=S)-N-R⁸R⁹, R⁷ et R⁹ représentant chacun un radical hydrogène, est mis en réaction dans un milieu réactionnel, en présence d'au moins une base, de préférence en présence d'au moins un sel d'hydrure métallique ou d'un sel d'alcoolate métallique, de manière particulièrement préférée en présence d'un sel d'hydrure métallique ou d'un sel d'alcoolate métallique choisi dans le groupe constitué par l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R⁷ ou de formule générale LG-R⁹, LG représentant un groupe partant, de préférence un atome d'halogène, de manière particulièrement préférée un atome de chlore, et R⁷ et R⁹ ayant la signification indiquée précédemment à l'exception de l'hydrogène, pour former au moins un composé de formule générale I, dans laquelle R¹ à R⁵, m et n ont la signification indiquée précédemment, et celui-ci est éventuellement purifié et/ou isolé.

19. Médicament contenant au moins un composé selon une ou plusieurs des revendications 1 à 17 et éventuellement un ou plusieurs adjuvants physiologiquement compatibles.

20. Médicament selon la revendication 19, pour la prophylaxie et/ou le traitement de la douleur, de préférence de la douleur choisie dans le groupe constitué par la douleur aiguë, la douleur chronique, la douleur neuropathique et la douleur viscérale.

21. Médicament selon la revendication 20, pour le traitement et/ou la prophylaxie d'une ou de plusieurs maladies choisies dans le groupe constitué par la douleur, de préférence la douleur choisie dans le groupe constitué par la douleur aiguë, la douleur chronique, la douleur neuropathique et la douleur viscérale ; la douleur articulaire ; les migraines; les dépressions ; les neuropathies ; les lésions nerveuses ; les maladies neurodégénératives, de préférence choisies dans le groupe constitué par la sclérose en plaques, la maladie d'Alzheimer, la maladie de Parkinson et la maladie d'Huntington ; les dysfonctionnements cognitifs, de préférence les déficiences cognitives, de manière particulièrement préférée les troubles de la mémoire ; l'épilepsie ; les maladies des voies respiratoires, de préférence choisies dans le groupe constitué par l'asthme et l'inflammation pulmonaire ; la toux ; l'incontinence urinaire ; une vessie hyperactive (overactive bladder, OAB) ; les ulcères gastriques ; le syndrome du côlon irritable ; les accidents vasculaires cérébraux ; les irritations oculaires ; les irritations cutanées ; les maladies cutanées neurotiques ; les maladies inflammatoires, de préférence les inflammations de l'intestin ; la diarrhée ; le prurit ; les troubles de l'alimentation, de préférence choisis dans le groupe constitué par la boulimie, la cachexie, l'anorexie et l'obésité ; la dépendance à des médicaments ; l'abus de médicaments ; les phénomènes de sevrage en cas de dépendance à des médicaments ; le développement de tolérance à des médicaments, de préférence aux opioïdes naturels ou synthétiques ; la dépendance à des drogues ; l'abus de drogues ; les phénomènes de sevrage en cas de dépendance à des drogues ; la dépendance à l'alcool ; l'abus d'alcool et les phénomènes de sevrage en cas de dépendance à l'alcool ; pour la diurèse ; pour l'antinatriurèse ; pour la régulation du système cardiovasculaire ; pour l'augmentation de la vigilance ; pour l'augmentation de la libido ; pour la modulation de l'activité physique ; pour l'anxiolyse ; pour l'anesthésie locale et/ou pour l'inhibition des effets secondaires indésirables, de préférence choisis dans le groupe constitué par l'hyperthermie, l'hypertension et le rétrécissement des bronches déclenché par l'administration d'agonistes des récepteurs vanilloïdes 1 (récepteurs de VR1/TRPV1), de préférence choisis dans le groupe constitué par la capsaïcine, la résinifératoxine, l'olvanil, l'arvanil, SDZ-249665, SDZ-249482, le nuvanil et le capsavanil (DA-5018).

22. Utilisation d'au moins un composé selon une ou plusieurs des revendications 1 à 17 pour la fabrication d'un médicament pour la prophylaxie et/ou le traitement de la douleur, de préférence de la douleur choisie dans le groupe constitué par la douleur aiguë, la douleur chronique et la douleur neuropathique.

23. Utilisation d'au moins un composé selon une ou plusieurs des revendications 1 à 17 pour la fabrication d'un médicament pour la prophylaxie et/ou le traitement d'une ou de plusieurs maladies choisies dans le groupe constitué par la douleur, de préférence la douleur choisie dans le groupe constitué par la douleur aiguë, la douleur chronique, la douleur neuropathique et la douleur viscérale ; la douleur articulaire ; les migraines ; les dépressions ; les neuropathies ; les lésions nerveuses ; les maladies neurodégénératives, de préférence choisies dans le groupe constitué par la sclérose en plaques, la maladie d'Alzheimer, la maladie de Parkinson et la maladie d'Huntington ; les dysfonctionnements cognitifs, de préférence les déficiences cognitives, de manière particulièrement préférée les troubles de la mémoire ; l'épilepsie ; les maladies des voies respiratoires, de préférence choisies dans le groupe constitué par l'asthme et l'inflammation pulmonaire ; la toux ; l'incontinence urinaire ; une vessie hyperactive (overactive bladder, OAB) ; les ulcères gastriques ; le syndrome du côlon irritable ; les accidents vasculaires cérébraux ; les irritations oculaires ; les irritations cutanées ; les maladies cutanées neurotiques ; les maladies inflammatoires, de préférence les inflammations de l'intestin ; la diarrhée ; le prurit ; les troubles de l'alimentation, de préférence choisis dans le groupe constitué par la boulimie, la cachexie, l'anorexie et l'obésité ; la dépendance à des médicaments ; l'abus de médicaments ; les phénomènes de sevrage en cas de dépendance à des médicaments ; le développement de tolérance à des médicaments, de préférence aux opioïdes naturels ou synthétiques ; la dépendance à des drogues ; l'abus de drogues ; les phénomènes de sevrage en cas de dépendance à des drogues ; la dépendance à l'alcool ; l'abus d'alcool et les phénomènes de sevrage en cas de dépendance à l'alcool ; pour la diurèse ; pour l'antinatriurèse , pour la régulation du système cardiovasculaire ; pour l'augmentation de la vigilance ; pour l'augmentation de la libido ; pour la modulation de l'activité physique ; pour l'anxiolyse ; pour l'anesthésie locale et/ou pour l'inhibition des effets secondaires indésirables, de préférence choisis dans le groupe constitué par l'hyperthermie, l'hypertension et le rétrécissement des bronches déclenché par l'administration d'agonistes du récepteur vanilloïdes 1 (récepteurs de VR1/TRPV1), de préférence choisis dans le groupe constitué par la capsaïcine, la résinifératoxine, l'olvanil, l'arvanil, SDZ-249665, SDZ-249482, le nuvanil et le capsavanil (DA-5018).

24. Utilisation d'au moins un composé spiro substitué de formule générale I 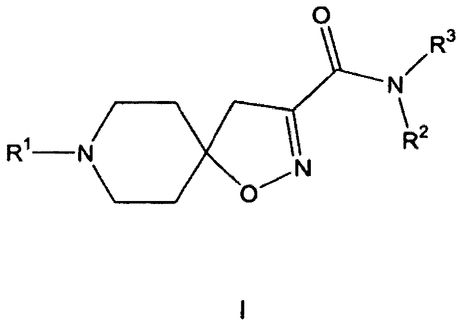 dans laquelle
R¹ représente un groupe -C(=O)-NR⁶R⁷,
R² représente un radical aliphatique linéaire ou ramifié, saturé ou insaturé, non substitué ou substitué au moins une fois, comprenant éventuellement au moins un hétéroatome en tant qu'élément de chaîne ;
les substituants du radical aliphatique pouvant être choisis indépendamment les uns des autres dans le groupe constitué par F, CI, Br, I, -CN, -NO₂, -OH, -SH et -NH₂ ;
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois ;
R³ représente un radical hydrogène,
un radical aliphatique linéaire ou ramifié, saturé ou insaturé, non substitué ou substitué au moins une fois, comprenant éventuellement au moins un hétéroatome en tant qu'élément de chaîne ;
les substituants du radical aliphatique pouvant être choisis indépendamment les uns des autres dans le groupe constitué par F, Cl, Br, I, -CN, -NO₂, -OH, -SH et -NH₂ ;
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois ;
ou
R² et R³ forment ensemble avec l'atome d'azote qui les relie un radical cycloaliphatique non substitué ou substitué au moins une fois, insaturé ou saturé, comprenant éventuellement au moins un hétéroatome supplémentaire en tant qu'élément de cycle, qui peut être condensé avec un système cyclique mono- ou polycyclique non substitué ou substitué au moins une fois ;
R⁶ représente un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois, et/ou ponté avec au moins un groupe alkylène linéaire ou ramifié, non substitué ou substitué au moins une fois,
un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
un groupe -C(=O)-R²⁴, qui peut être relié par un groupe alkylène linéaire ou ramifié, non substitué ou substitué au moins une fois, ou un groupe -C(=O)-O-R²⁵, qui peut être relié par un groupe alkylène linéaire ou ramifié, non substitué ou substitué au moins une fois ;
R⁷ représente un radical hydrogène,
un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois, et/ou ponté avec au moins un groupe alkylène linéaire ou ramifié, non substitué ou substitué au moins une fois,
un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
un groupe -C(=O)-R²⁴, qui peut être relié par un groupe alkylène linéaire ou ramifié, non substitué ou substitué au moins une fois, ou un groupe -C(=O)-O-R²⁵, qui peut être relié par un groupe alkylène linéaire ou ramifié, non substitué ou substitué au moins une fois ;
et
R²⁴ et R²⁵ représentent chacun indépendamment l'un de l'autre
un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne,
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants,
pour la fabrication d'un médicament pour la prophylaxie et/ou le traitement de troubles ou de maladies, qui sont médiés au moins en partie par les récepteurs vanilloïdes 1 (VR1/TRPV1).

25. Utilisation selon la revendication 24 pour le traitement et/ou la prophylaxie d'une ou de plusieurs maladies choisies dans le groupe constitué par la douleur aiguë ; la douleur viscérale ; la douleur articulaire; les dysfonctionnements cognitifs, de préférence les déficiences cognitives, de manière particulièrement préférée les troubles de la mémoire ; l'inflammation pulmonaire ; la toux ; une vessie hyperactive (overactive bladder, OAB) ; le syndrome du côlon irritable (irritable bowel syndrom) ; les irritations oculaires ; les irritations cutanées ; les maladies cutanées neurotiques ; les inflammations de l'intestin ; le développement de tolérance à des médicaments, de préférence aux opioïdes naturels ou synthétiques ; et/ou pour la diurèse ou pour l'antinatriurèse et/ou pour l'inhibition des effets secondaires indésirables, de préférence choisis dans le groupe constitué par l'hyperthermie, l'hypertension et le rétrécissement des bronches déclenché par l'administration d'agonistes des récepteurs vanilloïdes 1 (récepteurs de VR1/TRPV1), de préférence choisis dans le groupe constitué par la capsaïcine, la résinifératoxine, l'olvanil, l'arvanil, SDZ-249665, SDZ-249482, le nuvanil et le capsavanil.

26. Utilisation selon la revendication 24 ou 25, **caractérisée en ce que**
R¹ représente un groupe -C(=O)-NR⁶R⁷,
R² représente un radical aliphatique en C₁₋₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué, qui peut être condensé avec un système cyclique mono- ou polycyclique saturé ou insaturé, éventuellement substitué,
ou -(CHR³⁰)-Xᵥ-(CHR³¹)_{w}-Yₓ-(CHR³²)_{y}-Z_{z}-R³³ avec v = 0 ou 1, w = 0 ou 1, x = 0 ou 1, y = 0 ou 1, z = 0 ou 1, X, Y et Z représentant chacun indépendamment les uns des autres O, S, NH, N(CH₃), N(C₂H₅) ou N[CH(CH₃)₂] ;
R³ représente un radical hydrogène,
un radical aliphatique en C₁₋₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué, qui peut être condensé avec un système cyclique mono- ou polycyclique saturé ou insaturé, éventuellement substitué,
ou -(CHR³⁰)-Xᵥ-(CHR³¹)_{w}-Yₓ-(CHR³²)_{y}-Z_{z}-R³³ avec v = 0 ou 1, w = 0 ou 1, x = 0 ou 1, y = 0 ou 1, z = 0 ou 1, X, Y et Z représentant chacun indépendamment les uns des autres O, S, NH, N(CH₃), N(C₂H₅) ou N[CH(CH₃)₂] ;
ou
R² et R³ forment ensemble avec l'atome d'azote qui les relie en tant qu'élément de cycle un radical hétérocycloaliphatique à 4, 5, 6, 7, 8 ou 9 éléments, saturé ou insaturé, éventuellement substitué, qui peut être condensé avec un système cyclique mono- ou polycyclique saturé, insaturé ou aromatique, éventuellement substitué,
R⁶ représente un radical aliphatique en C₁₋₂₀ linéaire ou ramifié, saturé ou insaturé, éventuellement substitué,
un radical cycloaliphatique à 3, 4, 5, 6, 7, 8, 9, 10, 11 ou 12 éléments, insaturé ou saturé, éventuellement substitué, qui peut être condensé avec un système cyclique mono- ou polycyclique saturé, insaturé ou aromatique, éventuellement substitué, et/ou ponté avec un ou deux groupes alkylène en C₁₋₅ linéaires ou ramifiés, éventuellement substitués ;
un radical aryle ou hétéroaryle de 5 à 14 éléments, éventuellement substitué, qui peut être condensé avec un système cyclique mono- ou polycyclique saturé ou insaturé, éventuellement substitué ;
(CHR³⁵)-(CH₂)_{bb}-(CH₂)_{cc}-C(=O)-R²⁴ avec bb = 0 ou 1 et cc = 0 ou 1 ; -(CHR³⁶)-(CH₂)_{dd}-(CH₂)ₑₑ-C(=O)-O-R²⁵ avec dd = 0 ou 1 et ee = 0 ou 1 ;
ou -(CR³⁷R³⁸)-X_{ff}-(CHR³⁹)_{gg}-Yₕₕ-(CHR⁴⁰)ⱼⱼZₖₖ-R⁴¹ avec ff = 0 ou 1, gg = 0 ou 1, hh = 0 ou 1, jj = 0 ou 1, kk = 0 ou 1, X, Y et Z, représentant chacun indépendamment les uns des autres O, S, NH, N(CH₃), N(C₂H₅) ou N[CH(CH₃)₂] ;
R⁷ représente un radical hydrogène,
un radical aliphatique en C₁₋₂₀ linéaire ou ramifié, saturé ou insaturé, éventuellement substitué,
un radical cycloaliphatique à 3, 4, 5, 6, 7, 8, 9, 10, 11 ou 12 éléments, insaturé ou saturé, éventuellement substitué, qui peut être condensé avec un système cyclique mono- ou polycyclique saturé, insaturé ou aromatique, éventuellement substitué, et/ou ponté avec un ou deux groupes alkylène en C₁₋₅ linéaires ou ramifiés, éventuellement substitués ;
un radical aryle ou hétéroaryle de 5 à 14 éléments, éventuellement substitué, qui peut être condensé avec un système cyclique mono- ou polycyclique saturé ou insaturé, éventuellement substitué ;
(CHR³⁵)-(CH₂)_{bb}-(CH₂)_{cc}-C(=O)-R²⁴ avec bb = 0 ou 1 et cc = 0 ou 1 ; -(CHR³⁶)-(CH₂)_{dd}-(CH₂)ₑₑ-C(=O)-O-R²⁵ avec dd = 0 ou 1 et ee = 0 ou 1 ;
ou -(CR³⁷R³⁸)-X_{ff}-(CHR³⁹)_{gg}-Yₕₕ-(CHR⁴⁰)ⱼⱼ-Zₖₖ-R⁴¹ avec ff = 0 ou 1, gg = 0 ou 1, hh = 0 ou 1, jj = 0 ou 1, kk = 0 ou 1, X, Y et Z, représentant chacun indépendamment les uns des autres O, S, NH, N(CH₃), N(C₂H₅) ou N[CH(CH₃)₂] ;
R²⁴ et R²⁵ représentent chacun indépendamment l'un de l'autre un radical aliphatique en C₁₋₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué, qui peut être condensé avec un système cyclique mono- ou polycyclique saturé ou insaturé, éventuellement substitué,
ou -(CH₂)ₘₘ-R⁴², avec mm représentant 1, 2 ou 3 ;
R³⁰, R³¹, R³², R³⁵, R³⁶, R³⁷, R³⁸, R³⁹ et R⁴⁰ représentent chacun indépendamment les uns des autres
un radical hydrogène ;
un radical aliphatique en C₁₋₁₀ linéaire ou ramifié, saturé ou insaturé, éventuellement substitué ;
ou un radical aryle ou hétéroaryle de 5 à 14 éléments, éventuellement substitué, qui peut être condensé avec un système cyclique mono- ou polycyclique saturé ou insaturé, éventuellement substitué ;
R³³ et R⁴¹ représentent chacun indépendamment l'un de l'autre
un radical aliphatique en C₁₋₁₀ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être ponté avec 1, 2, 3, 4 ou 5 groupes alkylène en C₁₋₅ linéaires ou ramifiés, éventuellement substitués, et/ou condensé avec un système cyclique mono- ou polycyclique, saturé, insaturé ou aromatique, éventuellement substitué ;
ou un radical aryle ou hétéroaryle de 5 à 14 éléments, éventuellement substitué, qui peut être condensé avec un système cyclique mono- ou polycyclique saturé ou insaturé, éventuellement substitué ;
et
R³⁴ et R⁴² représentent chacun indépendamment l'un de l'autre
un radical aryle ou hétéroaryle de 5 à 14 éléments, éventuellement substitué ;
les radicaux aliphatiques en C₁₋₁₀ et les radicaux aliphatiques en C₁₂₀ mentionnés précédemment pouvant chacun éventuellement être substitués avec 1, 2, 3, 4, 5, 6, 7, 8 ou 9 substituants choisis indépendamment les uns des autres dans le groupe constitué par F, CI, Br, I, -CN, -NO₂, -OH, -SH et -NH₂ ;
les radicaux cycloaliphatiques et les radicaux hétérocycloaliphatiques mentionnés précédemment pouvant chacun éventuellement être substitués avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -NH-alkyle en C₁₋₅, -N(alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -S(=O)₂-NH-alkyle en C₁₋₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, CI, Br, -OH, -CF₃, -SF₅, -CN, -NO₂, -alkyle en C₁₋₅, -O-alkyle en C₁₋₅, -O-CF₃, -S-CF₃, phényle et -O-benzyle,
et les radicaux cycloaliphatiques mentionnés précédemment pouvant chacun éventuellement comprendre 1, 2, 3, 4 ou 5 hétéroatomes choisis indépendamment les uns des autres dans le groupe constitué par l'oxygène, l'azote et le soufre en tant qu'éléments de cycle ;
les radicaux hétérocycloaliphatiques mentionnés précédemment pouvant chacun éventuellement comprendre 1, 2 ou 3 hétéroatomes supplémentaires choisis indépendamment les uns des autres dans le groupe constitué par l'oxygène, l'azote et le soufre en tant qu'éléments de cycle ;
les cycles des systèmes cycliques mono- ou polycycliques mentionnés précédemment pouvant chacun éventuellement être substitués avec 1, 2, 3, 4 ou 5 substituants choisis indépendamment les uns des autres dans le groupe constitué par oxo (=O), thioxo (=S), F, CI, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -NH-alkyle en C₁₋₅, -N(alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -S(=O)₂-NH-alkyle en C₁₋₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, -alkyle en C₁₋₅, -O-alkyle en C₁₋₅, -O-CF₃, -S-CF₃, phényle et -O-benzyle,
et les cycles des systèmes cycliques mono- ou polycycliques mentionnés précédemment étant chacun à 5, 6 ou 7 éléments et pouvant chacun éventuellement comprendre 1, 2, 3, 4 ou 5 hétéroatomes en tant qu'éléments de cycle, choisis indépendamment les uns des autres dans le groupe constitué par l'oxygène, l'azote et le soufre ;
et les radicaux aryle ou hétéroaryle mentionnés précédemment pouvant chacun éventuellement être substitués avec 1, 2, 3, 4 ou 5 substituants choisis indépendamment les uns des autres dans le groupe constitué par F, Cl, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -NH-alkyle en C₁₋₅, -N(alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -S(=O)₂-NH-alkyle en C₁₋₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, -alkyle en C₁₋₅, -O-alkyle en C₁₋₅, -O-CF₃, -S-CF₃, phényle et -O-benzyle,
les radicaux hétéroaryle mentionnés précédemment pouvant chacun éventuellement comprendre 1, 2, 3, 4 ou 5 hétéroatomes choisis indépendamment les uns des autres dans le groupe constitué par l'oxygène, l'azote et le soufre en tant qu'éléments de cycle ;
et
les groupes alkylène en C₁₋₅ mentionnés précédemment pouvant chacun éventuellement être substitués avec 1, 2, 3, 4 ou 5 substituants choisis indépendamment les uns des autres dans le groupe constitué par F, Cl, Br, -OH, -SH, -NH₂, -CN et NO₂ ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants.

27. Utilisation selon une ou plusieurs des revendications 24 à 26, **caractérisée en ce que**
R² représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH, et -NH₂ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ; un radical choisi dans le groupe constitué par phényle, naphtyle, (1,3)-benzodioxolyle, (1,4)-benzodioxanyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle,
ou -(CHR³⁰)-R³³; -(CHR³⁰)-(CHR³⁰)-R³³ ; -(CHR³⁰)-(CHR³⁰)-O-R³³; -(CHR³⁰)-(CHR³¹)-(CHR³²)-R³³) ;-(CHR³⁰)-(CHR³¹)-S-(CHR³²)-R³³; -(CHR³⁰)-(CHR³¹)-(CHR³²)-N(CH₃)-R³³.

28. Utilisation selon une ou plusieurs des revendications 24 à 27, **caractérisée en ce que**
R³ représente un radical hydrogène.

29. Utilisation selon une ou plusieurs des revendications 24 à 28, **caractérisée en ce que**
R² et R³ forment ensemble avec l'atome d'azote qui les relie en tant qu'élément de cycle un radical choisi dans le groupe constitué par pyrrolidinyle, pipéridinyle, (1,3,4,5)-tétrahydropyrido[4,3-b]indolyle, (3,4)-dihydro-1H-isoquinolinyle, (1,3,4,9)-tétrahydro-[b]-carbolinyle, imidazolidinyle, (1,3)-thiazolidinyle, pipérazinyle, morpholinyle, azépanyle, diazépanyle et thiomorpholinyle, le radical pouvant à chaque fois être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle.

30. Utilisation selon une ou plusieurs des revendications 24 à 29, **caractérisée en ce que**
R⁶ représente un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et -NH₂ ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ; un radical choisi dans le groupe constitué par phényle, naphtyle, (1,3)-benzodioxolyle, (1,4)-benzodioxanyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(-O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
-(CHR³⁵)-C(=O)-R²⁴ ou -(CHR³⁵)-(CH₂)-C(=O)-R²⁴ ;
-(CHR³⁶)-C(=O)-O-R²⁵ ou -(CHR³⁶)-(CH₂)-C(=O)-O-R²⁵ ;
ou -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-CHR⁴⁰)-N(CH₃)-R⁴¹ ou -(CR³⁷R³⁸)-(CHR³⁹)-(CHR⁴⁰)-N(C₂H₅)-R⁴¹.

31. Utilisation selon une ou plusieurs des revendications 24 à 30, **caractérisée en ce que**
R⁷ représente un radical hydrogène.

32. Utilisation selon une ou plusieurs des revendications 24 à 31, **caractérisée en ce que**
R²⁴ et R²⁵ représentent chacun indépendamment l'un de l'autre un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle,
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois être substitué avec 1 ou 2 substituants choisis indépendamment l'un de l'autre dans le groupe constitué par -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, pyridinyle, phényle et benzyle, la partie cyclique des radicaux pyridinyle, -S(=O)₂-phényle, phényle et benzyle pouvant à chaque fois être substituée avec 1, 2, 3, 4 ou 5 substituants choisis indépendamment les uns des autres dans le groupe constitué par F, Cl, Br, méthyle, éthyle, n-propyle, isopropyle, n-butyle, sec-butyle, isobutyle, tert-butyle, -O-CH₃ et -O-C₂H₅ ;
un radical choisi dans le groupe constitué par phényle, naphtyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle et pyrimidinyle, le radical pouvant à chaque fois éventuellement être substitué avec 1, 2, 3, 4 ou 5 substituants choisis indépendamment les uns des autres dans le groupe constitué par F, Cl, Br, l, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -O-phényle, -O-benzyle et phényle,
ou -(CH₂)-R⁴² ou -(CH₂)-(CH₂)-R⁴².

33. Utilisation selon une ou plusieurs des revendications 24 à 32, **caractérisée en ce que**
R³⁰, R³¹, R³², R³⁵, R³⁶, R³⁷, R³⁸, R³⁹ et R⁴⁰ représentent chacun indépendamment les uns des autres
un radical hydrogène ;
un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle ;
ou un radical phényle, qui peut être substitué avec 1, 2, 3, 4 ou 5 substituants choisis indépendamment les uns des autres dans le groupe constitué par F, Cl, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -O-phényle, -O-benzyle et phényle.

34. Utilisation selon une ou plusieurs des revendications 24 à 33, **caractérisée en ce que**
R³³ et R⁴¹ représentent chacun indépendamment l'un de l'autre un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et -NH₂ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ; un radical choisi dans le groupe constitué par phényle, naphtyle, (1,3)-benzodioxolyle, (1,4)-benzodioxanyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=OC(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle.

35. Utilisation selon une ou plusieurs des revendications 24 à 34, **caractérisée en ce que**
R³⁴ et R⁴² représentent chacun indépendamment l'un de l'autre un radical choisi dans le groupe constitué par phényle, naphtyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle et pyrimidinyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃.

36. Utilisation selon une ou plusieurs des revendications 24 à 35, **caractérisée en ce que**
R¹ représente un groupe -C(=O)-NR⁶R⁷,
R² représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle et thiomorpholinyle, le radical pouvant à chaque fois être substitué avec 1 ou 2 substituants choisis indépendamment l'un de l'autre dans le groupe constitué par pyridinyle, pyridazinyle, phényle et benzyle, la partie cyclique des radicaux pyridinyle, pyridazinyle, phényle et benzyle pouvant à chaque fois être substituée avec 1, 2, 3, 4 ou 5 substituants choisis indépendamment les uns des autres dans le groupe constitué par -CF₃, F, CI, Br, méthyle, éthyle, n-propyle, isopropyle, n-butyle, sec-butyle, isobutyle, tert-butyle, -O-CH₃ et -O-C₂H₅ ;
un radical choisi dans le groupe constitué par phényle, naphtyle, thiophényle, furanyle, pyrrolyle, pyrazolyle, pyrazinyle et pyridinyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ et -S(=O)₂-NH-C₂H₅,
ou -(CHR³⁰)-R³³; -(CHR³⁰)-(CHR³¹)-R³³ ou -(CHR³⁰)-(CHR³¹)-(CHR³²)-R³³;
R³ représente un radical hydrogène ;
ou
R² et R³ forment ensemble avec l'atome d'azote qui les relie en tant qu'élément de cycle un radical choisi dans le groupe constitué par pyrrolidinyle, pipéridinyle, pipérazinyle, 3-méthyl-pipérazinyle, 2-méthyl-pipérazinyle, (3,5)-diméthylpipérazinyle, (2,6)-diméthylpipérazinyle, morpholinyle et thiomorpholinyle, le radical pouvant à chaque fois être substitué avec 1 ou 2 substituants choisis indépendamment l'un de l'autre dans le groupe constitué par -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(-O)₂-CH₃, -S(=O)₂-C₂H₅, -S(=O)₂-phényle, pyridinyle, pyridazinyle, phényle et benzyle, la partie cyclique des radicaux pyridinyle, -S(=O)₂-phényle, pyridazinyle, phényle et benzyle pouvant à chaque fois être substituée avec 1, 2, 3, 4 ou 5 substituants choisis indépendamment les uns des autres dans le groupe constitué par -CF₃, F, Cl, Br, méthyle, éthyle, n-propyle, isopropyle, n-butyle, sec-butyle, isobutyle, tert-butyle, -O-CH₃ et -O-C₂H₅ ;
R⁶ représente un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, CI et Br ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle ;
un radical choisi dans le groupe constitué par phényle, naphtyle, thiophényle, furanyle, pyrrolyle, pyrazolyle, pyranyle, triazolyle, pyridinyle, imidazolyle, thiazolyle, oxazolyle, isoxazolyle, pyridazinyle, pyrazinyle et pyrimidinyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
-(CHR³⁶)-C(=O)-O-R²⁵ ou -(CHR³⁶)-(CH₂)-C(=O)-O-R²⁵;
ou -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹HCHR⁴⁰)-N(CH₃)-R⁴¹ ou -(CR³⁷R³⁸)-(CHR³⁹)-(CHR⁴⁰)-N(C₂H₅)-R⁴¹;
R⁷ représente un radical hydrogène ;
R²⁵ représente un radical choisi dans le groupe constitué par méthyle, éthyle, n-propyle, isopropyle, n-butyle, sec-butyle, isobutyle et tert-butyle ;
R³⁰, R³¹, R³², R³⁶, R³⁷, R³⁸, R³⁹ et R⁴⁰ représentent chacun indépendamment les uns des autres
un radical hydrogène ;
un radical choisi dans le groupe constitué par méthyle, éthyle, n-propyle, isopropyle, n-butyle, sec-butyle, isobutyle et tert-butyle ;
ou un radical phényle, qui peut être substitué avec 1, 2, 3, 4 ou 5 substituants choisis indépendamment les uns des autres dans le groupe constitué par F, Cl, Br, I, -CN, -CF₃, -O-CH₃, -O-C₂H₅, -NO₂, -O-CF₃, méthyle, éthyle, n-propyle, isopropyle, n-butyle, sec-butyle, isobutyle et tert-butyle ;
R³³ représente un radical choisi dans le groupe constitué par phényle, naphtyle, thiophényle, furanyle, pyrrolyle, pyrazolyle, pyrazinyle et pyridinyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ et -S(=O)₂-NH-C₂H₅ ;
et
R⁴¹ représente un radical choisi dans le groupe constitué par méthyle, éthyle, n-propyle, isopropyle, n-butyle, sec-butyle, isobutyle et tert-butyle ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle et dithiolanyle ;
ou un radical choisi dans le groupe constitué par phényle, naphtyle, (1,3)-benzodioxolyle, (1,4)-benzodioxanyle, thiophényle et furanyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅ et -C(=O)-C(CH₃)₃ ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants.

37. Utilisation selon une ou plusieurs des revendications 24 à 36, **caractérisée en ce que**
R¹ représente un groupe -C(=O)-NR⁶R⁷;
R² représente un radical choisi dans le groupe constitué par phényle, naphtyle et pyridinyle, le radical pouvant à chaque fois être substitué avec 1, 2 ou 3 substituants choisis indépendamment les uns des autres dans le groupe constitué par F, CI, Br, -OH, -O-CH₃, -O-C₂H₅, -NH-S(=O)₂-CH₃ et -NH-S(=O)₂-C₂H₅ ;
un radical pipérazinyle, qui peut être substitué sur un atome d'azote avec un substituant choisi dans le groupe constitué par pyridinyle, pyridazinyle, phényle et benzyle, la partie cyclique des substituants pyridinyle, pyridazinyle, phényle et benzyle pouvant à chaque fois être substituée avec 1, 2 ou 3 substituants choisis indépendamment les uns des autres dans le groupe constitué par -CF₃, F, CI, Br, méthyle, éthyle, n-propyle, isopropyle, n-butyle, sec-butyle, isobutyle et tert-butyle ;
ou -(CHR³⁰)-R³³ ; -(CHR³⁰)-(CHR³¹)-R³³ ou -(CHR³⁰)-(CHR³¹)-(CHR³²)-R³³ ;
R³ représente un radical hydrogène ;
ou
R² et R³ forment ensemble avec l'atome d'azote qui les relie en tant qu'élément de cycle un radical choisi dans le groupe constitué par 3-méthyl-pipérazinyle, 2-méthyl-pipérazinyle, (3,5)-diméthylpipérazinyle, (2,6)-diméthylpipérazinyle et pipérazinyle, qui peut être substitué sur un atome d'azote avec un substituant choisi dans le groupe constitué par pyridinyle, pyridazinyle, phényle et benzyle, la partie cyclique des radicaux pyridinyle, pyridazinyle, phényle et benzyle pouvant à chaque fois être substituée avec 1, 2 ou 3 substituants choisis indépendamment les uns des autres dans le groupe constitué par -CF₃, F, Cl, Br, méthyle, éthyle, n-propyle, isopropyle, n-butyle, sec-butyle, isobutyle et tert-butyle ;
R⁶ représente un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle et 2-méthyl-1-propényle, le radical pouvant à chaque fois éventuellement être substitué avec 1, 2, 3, 4 ou 5 substituants choisis indépendamment les uns des autres dans le groupe constitué par F, CI et Br ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle et adamantyle ;
un radical choisi dans le groupe constitué par phényle, naphtyle et pyridinyle, le radical pouvant à chaque fois éventuellement être substitué avec 1, 2, 3, 4 ou 5 substituants choisis indépendamment les uns des autres dans le groupe constitué par -SF₅, F, Cl, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -O-phényle, -O-benzyle et phényle, la partie cyclique des radicaux cyclopentyle, cyclohexyle, -O-phényle, -O-benzyle et phényle pouvant à chaque fois être substituée avec 1, 2, 3, 4 ou 5 substituants choisis indépendamment les uns des autres dans le groupe constitué par F, CI, Br, méthyle, éthyle, n-propyle, isopropyle, n-butyle, sec-butyle, isobutyle et tert-butyle ;
-(CHR³⁶)-C(=O)-O-R²⁵ ou -(CHR³⁶)-(CH₂)-C(=O)-O-R²⁵ ;
ou -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ ou -(CR³⁷R³⁸)-(CHR³⁹)-(CHR⁴⁰)-N(C₂H₅)-R⁴¹ ;
R⁷ représente un radical hydrogène ;
R²⁵ représente un radical choisi dans le groupe constitué par méthyle, éthyle, n-propyle, isopropyle, n-butyle, sec-butyle, isobutyle et tert-butyle ;
R³⁰, R³¹, R³² , R³⁹ et R⁴⁰ représentent chacun un radical hydrogène ;
R³³ représente un radical choisi dans le groupe constitué par phényle, naphtyle, thiophényle et furanyle, le radical pouvant à chaque fois éventuellement être substitué avec 1, 2, 3, 4 ou 5 substituants choisis indépendamment les uns des autres dans le groupe constitué par -CF₃, F, CI, Br, -OH, -O-CH₃, -O-C₂H₅, méthyle, éthyle, n-propyle, isopropyle, n-butyle, sec-butyle, isobutyle, tert-butyle, -NH-S(=O)₂-CH₃ et -NH-S(=O)₂-C₂H₅ ;
R³⁶ représente un radical hydrogène
ou un radical choisi dans le groupe constitué par méthyle, éthyle, n-propyle, isopropyle, n-butyle, sec-butyle, isobutyle et tert-butyle ;
R³⁷ et R³⁸ représentent chacun indépendamment l'un de l'autre un radical hydrogène
un radical choisi dans le groupe constitué par méthyle, éthyle, n-propyle, isopropyle, n-butyle, sec-butyle, isobutyle et tert-butyle ;
ou un radical phényle ;
R⁴¹ représente un radical choisi dans le groupe constitué par méthyle, éthyle, n-propyle, isopropyle, n-butyle, sec-butyle, isobutyle et tert-butyle ;
un radical choisi dans le groupe constitué par tétrahydrofuranyle, tétrahydrothiophényle, pyrrolidinyle, morpholinyle, pipéridinyle et pipérazinyle ;
ou un radical choisi dans le groupe constitué par phényle, naphtyle, (1,3)-benzodioxolyle, (1,4)-benzodioxanyle, thiophényle et furanyle, le radical pouvant à chaque fois éventuellement être substitué avec 1, 2, 3, 4 ou 5 substituants choisis indépendamment les uns des autres dans le groupe constitué par F, CI, Br, -OH, -O-CH₃, -O-C₂H₅, méthyle, éthyle, n-propyle, isopropyle, n-butyle, sec-butyle, isobutyle et tert-butyle ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou des diastéréomères, en un rapport de mélange quelconque, ou à chaque fois sous la forme des sels correspondants, ou à chaque fois sous la forme des solvates correspondants.

38. Utilisation selon une ou plusieurs des revendications 24 à 37, **caractérisée en ce que** le composé spiro est choisi dans le groupe constitué par
[1] (2-fluoro-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[2] 3-[(5-chloro-pyridin-2-yl)-amide]-8-[(2-fluoro-phényl)-amide] de l'acide 1-oxa-2,8-diaza-spiro[4.5]déc-2-ène-3,8-dicarboxylique
[3] 3-(4-chloro-benzylamide)-8-[(3-fluoro-phényl)-amide] de l'acide 1-oxa-2,8-diaza-spiro[4.5]déc-2-ène-3,8-dicarboxylique
[4] (3-fluoro-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[5] 3-[(5-chloro-pyridin-2-yl)-amide]-8-[(3-fluoro-phényl)-amide] de l'acide 1-oxa-2,8-diaza-spiro[4.5]déc-2-ène-3,8-dicarboxylique
[6] 8-[(2-chloro-phényl)-amide]-3-(3,4-diméthoxy-benzylamide) de l'acide 1-oxa-2,8-diaza-spiro[4.5]déc-2-ène-3,8-dicarboxylique
[7] 3-(4-chloro-benzylamide)-8-[(2-chloro-phényl)-amide] de l'acide 1-oxa-2,8-diaza-spiro[4.5]déc-2-ène-3,8-dicarboxylique
[8] (2-chloro-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[9] 8-[(2-chloro-phényl)-amide]-3-[(5-chloro-pyridin-2-yl)-amide] de l'acide 1-oxa-2,8-diaza-spiro[4.5]déc-2-ène-3,8-dicarboxylique
[10] 8-[(3-bromo-phényl)-amide]-3-(3,4-diméthoxy-benzylamide) de l'acide 1-oxa-2,8-diaza-spiro[4.5]déc-2-ène-3,8-dicarboxylique
[11] (3-bromo-phényl)-amide de l'acide 3-[4-(3-chloro-pyridin-2-yl)-pipérazine-1-carbonyl]-1-oxa-2,8-d iaza-spiro[4.5]déc-2-ène-8-carboxylique
[12] 8-[(3-bromo-phényl)-amide]-3-[(5-chloro-pyridin-2-yl)-amide] de l'acide 1-oxa-2,8-diaza-spiro[4.5]déc-2-ène-3,8-dicarboxylique
[13] 3-(3,4-diméthoxy-benzylamide)-8-[(2-méthoxy-phényl)-amide] de l'acide 1-oxa-2,8-diaza-spiro[4.5]déc-2-ène-3,8-dicarboxylique
[14] 3-(4-chloro-benzylamide)-8-[(2-méthoxy-phényl)-amide] de l'acide 1-oxa-2,8-diaza-spiro[4.5]déc-2-ène-3,8-dicarboxylique
[15] (2-méthoxy-phényl)-amide de l'acide 3-[4-(3-chloro-pyridin-2-yl)-pipérazine-1-carbonyl]-1-oxa-2, 8-diaza-spiro[4.5]déc-2-ène-8-carboxylique
[16] 3-(3,4-diméthoxy-benzylamide)-8-[(3-méthoxy-phényl)-amide] de l'acide 1-oxa-2,8-diaza-spiro[4.5]déc-2-ène-3,8-dicarboxylique
[17] (3-méthoxy-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[18] 3-[(5-chloro-pyridin-2-yl)-amide]-8-[(3-méthoxy-phényl)-amide] de l'acide 1-oxa-2,8-diaza-spiro[4.5]déc-2-ène-3,8-dicarboxylique
[19] (4-phénoxy-phényl)-amide de l'acide 3-[4-(3-chloro-pyridin-2-yl)-pipérazine-1-carbonyl]-1-oxa-2,8-diaza-spiro[4.5]déc-2-ène-8-carboxyliq ue
[20] 8-[(2-chloro-5-trifluorométhyl-phényl)-amide]-3-(3,4-diméthoxy-benzylamide) de l'acide 1-oxa-2,8-diaza-spiro[4.5]déc-2-ène-3,8-dicarboxylique
[21] 3-(4-chloro-benzylamide)-8-[(2-chloro-5-trifluorométhyl-phényl)-amide] de l'acide 1-oxa-2,8-diaza-spiro[4.5]déc-2-ène-3,8-dicarboxylique
[22] (2-chloro-5-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[23] 3-[(5-chloro-pyridin-2-yl)-amide]-8-[(2-chloro-5-trifluorométhyl-phényl)-amide] de l'acide 1-oxa-2,8-diaza-spiro[4.5]déc-2-ène-3,8-dicarboxylique
[24] 8-[(4-chloro-2-trifluorométhyl-phényl)-amide]-3-(3,4-diméthoxy-benzylamide) de l'acide 1-oxa-2,8-diaza-spiro[4.5]déc-2-ène-3,8-dicarboxylique
[25] (4-chloro-2-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[26] 3-[(5-chloro-pyridin-2-yl)-amide]-8-[(4-chloro-2-trifluorométhyl-phényl)-amide] de l'acide 1-oxa-2,8-diaza-spiro[4.5]déc-2-ène-3,8-dicarboxylique
[27] 8-[(4-chloro-3-trifluorométhyl-phényl)-amide]-3-(3,4-diméthoxy-benzylamide) de l'acide 1-oxa-2,8-diaza-spiro[4.5]déc-2-ène-3,8-dicarboxylique
[28] (4-chloro-3-trifluorométhyl-phényl)-amide de l'acide 3-[4-(3-chloro-pyrid in-2-yl)-pipérazine-1-carbonyl]-1-oxa-2, 8-d iaza-spiro[4.5]déc-2-ène-8-carboxylique
[29] 3-[(5-chloro-pyridin-2-yl)-amide]-8-[(4-chloro-3-trifluorométhyl-phényl)-amide] de l'acide 1-oxa-2,8-diaza-spiro[4.5]déc-2-ène-3,8-dicarboxylique
[30] 3-[(2-tert-butyl-6-méthyl-phényl)-amide]-8-(4-chloro-benzylamide) de l'acide 1-oxa-2,8-diaza-spiro[4.5]déc-2-ène-3,8-dicarboxylique
[31] (2-tert-butyl-6-méthyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[32] 3-(3,4-diméthoxy-benzylamide)-8-[(4-trifluorométhoxy-phényl)-amide] de l'acide 1-oxa-2,8-diaza-spiro[4.5]déc-2-ène-3,8-dicarboxylique
[33] 3-(4-chloro-benzylamide)-8-[(4-trifluorométhoxy-phényl)-amide] de l'acide 1-oxa-2,8-diaza-spiro[4.5]déc-2-ène-3,8-dicarboxylique
[34] (4-trifluorométhoxy-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[35] 3-[(5-chloro-pyridin-2-yl)-amide]-8-[(4-trifluorométhoxy-phényl)-amide] de l'acide 1-oxa-2,8-diaza-spiro[4.5]déc-2-ène-3,8-dicarboxylique
[36] 3-(3,4-diméthoxy-benzylamide)-8-(phénéthyl-amide) de l'acide 1-oxa-2,8-diaza-spiro[4.5]déc-2-ène-3,8-dicarboxylique
[37] 3-(4-chloro-benzylamide)-8-(phénéthyl-amide) de l'acide 1-oxa-2,8-diaza-spiro[4.5]déc-2-ène-3,8-dicarboxylique
[38] phénéthyl-amide de l'acide 3-[4-(3-chloro-pyridin-2-yl)-pipérazine-1-carbonyl]-1-oxa-2,8-diaza-spiro[4.5]déc-2-ène-8-carboxylique
[39] 3-(3,4-diméthoxy-benzylamide)-8-phénylamide de l'acide 1-oxa-2,8-diaza-spiro[4.5]déc-2-ène-3,8-dicarboxylique
[40] phénylamide de l'acide 3-[4-(3-chloro-pyridin-2-yl)-pipérazine-1-carbonyl]-1-oxa-2,8-diaza-spiro[4.5]déc-2-ène-8-carboxylique
[41] 3-[(5-chloro-pyridin-2-yl)-amide]-8-phénylamide de l'acide 1-oxa-2,8-diaza-spiro[4.5]déc-2-ène-3,8-dicarboxylique
[42] 3-(4-chloro-benzylamide)-8-m-tolylamide de l'acide 1-oxa-2,8-diaza-spiro[4.5]déc-2-ène-3,8-dicarboxylique
[43] m-tolylamide de l'acide 3-[4-(3-chloro-pyridin-2-yl)-pipérazine-1-carbonyl]-1-oxa-2,8-diaza-spiro[4.5]déc-2-ène-8-carboxylique
[44] 3-(3,4-diméthoxy-benzylamide)-8-[(4-fluoro-phényl)-amide] de l'acide 1-oxa-2,8-diaza-spiro[4.5]déc-2-ène-3,8-dicarboxylique
[45] (4-fluoro-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[46] 8-[(3-chloro-phényl)-amide]-3-(3,4-diméthoxy-benzylamide) de l'acide 1-oxa-2,8-diaza-spiro[4.5]déc-2-ène-3,8-dicarboxylique
[47] (3-chloro-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[48] 8-[(3-chloro-phényl)-amide]-3-[(5-chloro-pyridin-2-yl)-amide] de l'acide 1-oxa-2,8-diaza-spiro[4.5]déc-2-ène-3,8-dicarboxylique
[49] 8-[(4-chloro-phényl)-amide]-3-(3,4-diméthoxy-benzylamide) de l'acide 1-oxa-2,8-diaza-spiro[4.5]déc-2-ène-3,8-dicarboxylique
[50] 3-(4-chloro-benzylamide)-8-[(4-chloro-phényl)-amide] de l'acide 1-oxa-2,8-diaza-spiro[4.5]déc-2-ène-3,8-dicarboxylique
[51] (4-chloro-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[52] (4-méthoxy-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[53] 3-(3,4-diméthoxy-benzylamide)-8-[(2-méthylsulfanyl-phényl)-amide] de l'acide 1-oxa-2,8-diaza-spiro[4.5]déc-2-ène-3,8-dicarboxylique
[54] (2-méthylsulfanyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[55] 3-(3,4-diméthoxy-benzylamide)-8-[(3-méthylsulfanyl-phényl)-amide] de l'acide 1-oxa-2,8-diaza-spiro[4.5]déc-2-ène-3,8-dicarboxylique
[56] (3-méthylsulfanyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[57] 3-(3,4-diméthoxy-benzylamide)-8-[(4-méthylsulfanyl-phényl)-amide] de l'acide 1-oxa-2,8-diaza-spiro[4.5]déc-2-ène-3,8-dicarboxylique
[58] (4-méthylsulfanyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[59] 3-[(5-chloro-pyridin-2-yl)-amide]-8-[(4-méthylsulfanyl-phényl)-amide] de l'acide 1-oxa-2,8-diaza-spiro[4.5]déc-2-ène-3,8-dicarboxylique
[60] 3-(3,4-diméthoxy-benzylamide)-8-[(2-isopropyl-phényl)-amide] de l'acide 1-oxa-2,8-diaza-spiro[4.5]déc-2-ène-3,8-dicarboxylique
[61] (2-isopropyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[62] 3-[(5-chloro-pyridin-2-yl)-amide]-8-[(2-isopropyl-phényl)-amide] de l'acide 1-oxa-2,8-diaza-spiro[4.5]déc-2-ène-3,8-dicarboxylique
[63] (4-isopropyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[64] 3-(3,4-diméthoxy-benzylamide)-8-[(2-trifluorométhyl-phényl)-amide] de l'acide 1-oxa-2,8-diaza-spiro[4.5]déc-2-ène-3,8-dicarboxylique
[65] (2-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[66] 3-[(5-chloro-pyridin-2-yl)-amide]-8-[(2-trifluorométhyl-phényl)-amide] de l'acide 1-oxa-2,8-diaza-spiro[4.5]déc-2-ène-3,8-dicarboxylique
[67] 3-(3,4-diméthoxy-benzylamide)-8-[(3-trifluorométhyl-phényl)-amide] de l'acide 1-oxa-2,8-diaza-spiro[4.5]déc-2-ène-3,8-dicarboxylique
[68] (3-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[69] 3-[(5-chloro-pyridin-2-yl)-amide]-8-[(3-trifluorométhyl-phényl)-amide] de l'acide 1-oxa-2,8-diaza-spiro[4.5]déc-2-ène-3,8-dicarboxylique
[70] 3-(3,4-diméthoxy-benzylamide)-8-[(4-trifluorométhyl-phényl)-amide] de l'acide 1-oxa-2,8-diaza-spiro[4.5]déc-2-ène-3,8-dicarboxylique
[71] (4-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[72] 8-cyclohexylamide-3-(3,4-diméthoxy-benzylamide) de l'acide 1-oxa-2,8-diaza-spiro[4.5]déc-2-ène-3,8-dicarboxylique
[73] 3-(4-chloro-benzylamide)-8-cyclohexylamide de l'acide 1-oxa-2,8-diaza-spiro[4.5]déc-2-ène-3,8-dicarboxylique
[74] 8-benzylamide-3-(3,4-diméthoxy-benzylamide) de l'acide 1-oxa-2,8-diaza-spiro[4.5]déc-2-ène-3,8-dicarboxylique
[75] 3-(3,4-diméthoxy-benzylamide)-8-o-tolylamide de l'acide 1-oxa-2,8-diaza-spiro[4.5]déc-2-ène-3,8-dicarboxylique
[76] 3-(4-chloro-benzylamide)-8-o-tolylamide de l'acide 1-oxa-2,8-diaza-spiro[4.5]déc-2-ène-3,8-dicarboxylique
[77] 3-[(5-chloro-pyridin-2-yl)-amide]-8-o-tolylamide de l'acide 1-oxa-2,8-diaza-spiro[4.5]déc-2-ène-3,8-dicarboxylique
[78] 3-(3,4-diméthoxy-benzylamide)-8-[(2-éthyl-phényl)-amide] de l'acide 1-oxa-2,8-diaza-spiro[4.5]déc-2-ène-3,8-dicarboxylique
[79] (2-éthyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[80] 3-[(5-chloro-pyridin-2-yl)-amide]-8-[(2-éthyl-phényl)-amide] de l'acide 1-oxa-2,8-diaza-spiro[4.5]déc-2-ène-3,8-dicarboxylique
[81] 3-(4-chloro-benzylamide)-8-[(4-éthyl-phényl)-amide] de l'acide 1-oxa-2,8-diaza-spiro[4.5]déc-2-ène-3,8-dicarboxylique
[82] 3-[(5-chloro-pyridin-2-yl)-amide]-8-[(4-éthyl-phényl)-amide] de l'acide 1-oxa-2,8-diaza-spiro[4.5]déc-2-ène-3,8-dicarboxylique
[83] 3-(3,4-diméthoxy-benzylamide)-8-[(2-fluoro-phényl)-amide] de l'acide 1-oxa-2,8-diaza-spiro[4.5]déc-2-ène-3,8-dicarboxylique
[84] 3-(3,4-diméthoxy-benzylamide)-8-p-tolylamide de l'acide 1-oxa-2,8-diaza-spiro[4.5]déc-2-ène-3,8-dicarboxylique
[85] 3-(4-chloro-benzylamide)-8-p-tolylamide de l'acide 1-oxa-2,8-diaza-spiro[4.5]déc-2-ène-3,8-dicarboxylique
[86] p-tolylamide de l'acide 3-[4-(3-chloro-pyridin-2-yl)-pipérazine-1-carbonyl]-1-oxa-2,8-diaza-spiro[4.5]déc-2-ène-8-carboxylique
[87] 3-[(5-chloro-pyridin-2-yl)-amide]-8-p-tolylamide de l'acide 1-oxa-2,8-diaza-spiro[4.5]déc-2-ène-3,8-dicarboxylique
[88] 3-(4-chloro-benzylamide)-8-[(3-éthyl-phényl)-amide] de l'acide 1-oxa-2,8-diaza-spiro[4.5]déc-2-ène-3,8-dicarboxylique
[89] (3-éthyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[90] 3-[(5-chloro-pyridin-2-yl)-amide]-8-[(3-éthyl-phényl)-amide] de l'acide 1-oxa-2,8-diaza-spiro[4.5]déc-2-ène-3,8-dicarboxylique
[91] 3-(3,4-diméthoxy-benzylamide)-8-[(2-propyl-phényl)-amide] de l'acide 1-oxa-2,8-diaza-spiro[4.5]déc-2-ène-3,8-dicarboxylique
[92] 3-(4-chloro-benzylamide)-8-[(2-propyl-phényl)-amide] de l'acide 1-oxa-2,8-diaza-spiro[4.5]déc-2-ène-3,8-dicarboxylique
[93] 3-(4-chloro-benzylamide)-8-[(2-propyl-phényl)-amide] de l'acide 1-oxa-2,8-diaza-spiro[4.5]déc-2-ène-3,8-dicarboxylique
[94] (2-propyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[95] 3-[(5-chloro-pyridin-2-yl)-amide]-8-[(2-propyl-phényl)-amide] de l'acide 1-oxa-2,8-diaza-spiro[4.5]déc-2-ène-3,8-dicarboxylique
[96] 8-[(2-bromo-phényl)-amide]-3-(3,4-diméthoxy-benzylamide) de l'acide 1-oxa-2,8-diaza-spiro[4.5]déc-2-ène-3,8-dicarboxylique
[97] 8-[(2-bromo-phényl)-amide]-3-(4-chloro-benzylamide) de l'acide 1-oxa-2,8-diaza-spiro[4.5]déc-2-ène-3,8-dicarboxylique
[98] (2-bromo-phényl)-amide de l'acide 3-[4-(3-chloro-pyridin-2-yl)-pipérazine-1-carbonyl]-1-oxa-2,8-d iaza-spiro[4.5]déc-2-ène-8-carboxylique
[99] (4-bromo-phényl)-amide de l'acide 3-[4-(3-chloro-pyridin-2-yl)-pipérazine-1-carbonyl]-1-oxa-2,8-d iaza-spiro[4.5]déc-2-ène-8-carboxylique
[100] biphényl-4-ylamide de l'acide 3-[4-(3-chloro-pyridin-2-yl)-pipérazine-1-carbonyl]-1-oxa-2,8-diaza-spiro[4.5]déc-2-ène-8-carboxylique
[101] 3-(3,4-diméthoxy-benzylamide)-8-[(2-phénoxy-phényl)-amide] de l'acide 1-oxa-2,8-diaza-spiro[4.5]déc-2-ène-3,8-dicarboxylique
[102] (2-phénoxy-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[103] 3-[(5-chloro-pyridin-2-yl)-amide]-8-[(2-phénoxy-phényl)-amide] de l'acide 1-oxa-2,8-diaza-spiro[4.5]déc-2-ène-3,8-dicarboxylique
[104] 3-(4-chloro-benzylamide)-8-[(2-trifluorométhoxy-phényl)-amide] de l'acide 1-oxa-2,8-diaza-spiro[4.5]déc-2-ène-3,8-dicarboxylique
[105] (2-trifluorométhoxy-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[106] 3-[(5-chloro-pyridin-2-yl)-amide]-8-[(2-trifluorométhoxy-phényl)-amide] de l'acide 1-oxa-2,8-diaza-spiro[4.5]déc-2-ène-3,8-dicarboxylique
[107] 3-(3,4-diméthoxy-benzylamide)-8-(4-méthyl-benzylamide) de l'acide 1-oxa-2,8-diaza-spiro[4.5]déc-2-ène-3,8-dicarboxylique
[108] 3-(4-chloro-benzylamide)-8-(4-méthyl-benzylamide) de l'acide 1-oxa-2,8-diaza-spiro[4.5]déc-2-ène-3,8-dicarboxylique
[109] 4-méthyl-benzylamide de l'acide 3-[4-(3-chloro-pyridin-2-yl)-pipérazine-1-carbonyl]-1-oxa-2,8-diaza-spiro[4.5]déc-2-ène-8-carboxylique
[110] 3-[(5-chloro-pyridin-2-yl)-amide]-8-(4-méthyl-benzylamide) de l'acide 1-oxa-2,8-diaza-spiro[4.5]déc-2-ène-3,8-dicarboxylique
[111] 3-(3,4-diméthoxy-benzylamide)-8-(4-méthoxy-benzylamide) de l'acide 1-oxa-2,8-diaza-spiro[4.5]déc-2-ène-3,8-dicarboxylique
[112] 3-(4-chloro-benzylamide)-8-(4-méthoxy-benzylamide) de l'acide 1-oxa-2,8-diaza-spiro[4.5]déc-2-ène-3,8-dicarboxylique
[113] 4-méthoxy-benzylamide de l'acide 3-[4-(3-chloro-pyridin-2-yl)-pipérazine-1-carbonyl]-1-oxa-2,8-diaza-spiro[4.5]déc-2-ène-8-carboxylique
[114] 8-[(4-tert-butyl-phényl)-amide]-3-(3,4-diméthoxy-benzylamide) de l'acide 1-oxa-2,8-diaza-spiro[4.5]déc-2-ène-3,8-dicarboxylique
[115] 8-[(4-tert-butyl-phényl)-amide]-3-(4-chloro-benzylamide) de l'acide 1-oxa-2,8-diaza-spiro[4.5]déc-2-ène-3,8-dicarboxylique
[116] (4-tert-butyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[117] 8-[(4-tert-butyl-phényl)-amide]-3-[(5-chloro-pyridin-2-yl)-amide] de l'acide 1-oxa-2,8-diaza-spiro[4.5]déc-2-ène-3,8-dicarboxylique
[118] 3,4-diméthoxy-benzylamide de l'acide 8-(2-méthoxy-phénylthiocarbamoyl)-1-oxa-2,8-diaza-spiro[4.5]déc-2-ène-3-carboxylique
[119] (2-méthoxy-phényl)-amide de l'acide 3-[4-(3-chloro-pyridin-2-yl)-pipérazine-1-carbonyl]-1-oxa-2,8-diaza-spiro[4.5]déc-2-ène-8-thiocarboxylique
[120] 3,4-diméthoxy-benzylamide de l'acide 8-(cyclohexylméthyl-thiocarbamoyl)-1-oxa-2,8-diaza-spiro[4.5]déc-2-ène-3-carboxylique
[121] 4-chloro-benzylamide de l'acide 8-(cyclohexylméthyl-thiocarbamoyl)-1-oxa-2,8-diaza-spiro[4.5]déc-2-ène-3-carboxylique
[122] cyclohexylméthyl-amide de l'acide 3-[4-(3-chloro-pyridin-2-yl)-pipérazine-1-carbonyl]-1-oxa-2,8-diaza-spiro[4.5]déc-2-ène-8-thiocarboxylique
[123] 3,4-diméthoxy-benzylamide de l'acide 8-cyclooctylthiocarbamoyl-1-oxa-2,8-diaza-spiro[4.5]déc-2-ène-3-carboxylique
[124] 4-chloro-benzylamide de l'acide 8-cyclooctylthiocarbamoyl-1-oxa-2,8-diaza-spiro[4.5]déc-2-ène-3-carboxylique
[125] 4-chloro-benzylamide de l'acide 8-(3-morpholin-4-yl-propylthiocarbamoyl)-1-oxa-2,8-diaza-spiro[4.5]déc-2-ène-3-carboxylique
[126] (5-chloro-pyridin-2-yl)-amide de l'acide 8-(3-morpholin-4-yl-propylthiocarbamoyl)-1-oxa-2,8-diaza-spiro[4.5]déc-2-ène-3-carboxylique
[127] 4-chloro-benzylamide de l'acide 8-p-tolylthiocarbamoyl-1-oxa-2,8-diaza-spiro[4.5]déc-2-ène-3-carboxylique
[128] 3,4-diméthoxy-benzylamide de l'acide 8-phénéthylthiocarbamoyl-1-oxa-2,8-diaza-spiro[4.5]déc-2-ène-3-carboxylique
[129] 4-chloro-benzylamide de l'acide 8-phénéthylthiocarbamoyl-1-oxa-2,8-diaza-spiro[4.5]déc-2-ène-3-carboxylique
[130] (5-chloro-pyridin-2-yl)-amide de l'acide 8-(3-phényl-propylthiocarbamoyl)-1-oxa-2,8-diaza-spiro[4.5]déc-2-ène-3-carboxylique
[131] 3,4-diméthoxy-benzylamide de l'acide 8-(2-fluoro-phénylthiocarbamoyl)-1-oxa-2,8-diaza-spiro[4.5]déc-2-ène-3-carboxylique
[132] 3,4-diméthoxy-benzylamide de l'acide 8-(4-fluoro-phénylthiocarbamoyl)-1-oxa-2,8-diaza-spiro[4.5]déc-2-ène-3-carboxylique
[133] 4-chloro-benzylamide de l'acide 8-(4-fluoro-phénylthiocarbamoyl)-1-oxa-2,8-diaza-spiro[4.5]déc-2-ène-3-carboxylique
[134] 3,4-diméthoxy-benzylamide de l'acide 8-(2-chloro-phénylthiocarbamoyl)-1-oxa-2,8-diaza-spiro[4.5]déc-2-ène-3-carboxylique
[135] 4-chloro-benzylamide de l'acide 8-(2-chloro-phénylthiocarbamoyl)-1-oxa-2,8-diaza-spiro[4.5]déc-2-ène-3-carboxylique
[136] 4-chloro-benzylamide de l'acide 8-(3-trifluorométhyl-phénylthiocarbamoyl)-1-oxa-2,8-diaza-spiro[4.5]déc-2-ène-3-carboxylique
[137] 3,4-diméthoxy-benzylamide de l'acide 8-(naphtalén-1-ylthiocarbamoyl)-1-oxa-2,8-diaza-spiro[4.5]déc-2-ène-3-carboxylique
[138] 3,4-diméthoxy-benzylamide de l'acide 8-(naphtalén-1-ylthiocarbamoyl)-1-oxa-2,8-diaza-spiro[4.5]déc-2-ène-3-carboxylique
[139] 3,4-diméthoxy-benzylamide de l'acide 8-cyclopentylthiocarbamoyl-1-oxa-2,8-diaza-spiro[4.5]déc-2-ène-3-carboxylique
[140] cyclopentylamide de l'acide 3-[4-(3-chloro-pyridin-2-yl)-pipérazine-1-carbonyl]-1-oxa-2,8-diaza-spiro[4.5]déc-2-ène-8-thiocarboxylique
[141] 3,4-diméthoxy-benzylamide de l'acide 8-(2-morpholin-4-yl-éthylthiocarbamoyl)-1-oxa-2,8-diaza-spiro[4.5]déc-2-ène-3-carboxylique
[142] 4-chloro-benzylamide de l'acide 8-(2-morpholin-4-yl-éthylthiocarbamoyl)-1-oxa-2,8-diaza-spiro[4.5]déc-2-ène-3-carboxylique
[143] 3,4-diméthoxy-benzylamide de l'acide 8-(3-chloro-phénylthiocarbamoyl)-1-oxa-2,8-diaza-spiro[4.5]déc-2-ène-3-carboxylique
[144] (4-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-thiocarboxylique
[145] 3,4-diméthoxy-benzylamide de l'acide 8-(2-méthylsulfanyl-phénylthiocarbamoyl)-1-oxa-2,8-diaza-spiro[4.5]déc-2-ène-3-carboxylique
[146] 4-chloro-benzylamide de l'acide 8-(2-méthylsulfanyl-phénylthiocarbamoyl)-1-oxa-2,8-diaza-spiro[4.5]déc-2-ène-3-carboxylique
[147] (2-méthylsulfanyl-phényl)-amide de l'acide 3-[4-(3-chloro-pyridin-2-yl)-pipérazine-1-carbonyl]-1-oxa-2,8-diaza-spiro[4.5]déc-2-ène-8-thiocarboxylique
[148] 3,4-diméthoxy-benzylamide de l'acide 8-(4-Isopropyl-phénylthiocarbamoyl)-1-oxa-2,8-diaza-spiro[4.5]déc-2-ène-3-carboxylique
[149] 4-chloro-benzylamide de l'acide 8-(4-isopropyl-phénylthiocarbamoyl)-1-oxa-2,8-diaza-spiro[4.5]déc-2-ène-3-carboxylique
[150] 3,4-diméthoxy-benzylamide de l'acide 8-(2-iodo-phénylthiocarbamoyl)-1-oxa-2,8-diaza-spiro[4.5]déc-2-ène-3-carboxylique
[151] 4-chloro-benzylamide de l'acide 8-(2-iodo-phénylthiocarbamoyl)-1-oxa-2,8-diaza-spiro[4.5]déc-2-ène-3-carboxylique
[152] 3,4-diméthoxy-benzylamide de l'acide 8-(2-trifluorométhyl-phénylthiocarbamoyl)-1-oxa-2,8-diaza-spiro[4.5]déc-2-ène-3-carboxylique
[153] 4-chloro-benzylamide de l'acide 8-(2-trifluorométhyl-phénylthiocarbamoyl)-1-oxa-2,8-diaza-spiro[4.5]déc-2-ène-3-carboxylique
[154] (2-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-thiocarboxylique
[155] 3,4-diméthoxy-benzylamide de l'acide 8-[(benzo[1,3]dioxol-5-ylméthyl)-thiocarbamoyl]-1-oxa-2,8-diaza-spiro[4.5]déc-2-ène-3-carboxylique
[156] 4-chloro-benzylamide de l'acide 8-[(benzo[1,3]dioxol-5-ylméthyl)-thiocarbamoyl]-1-oxa-2,8-diaza-spiro[4.5]déc-2-ène-3-carboxylique
[157] (benzo[1,3]dioxol-5-ylméthyl)-amide de l'acide 3-[4-(3-chloro-pyridin-2-yl)-pipérazine-1-carbonyl]-1-oxa-2,8-diaza-spiro[4.5]déc-2-ène-8-thiocarboxylique
[158] (3-cyano-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[159] 3-(3,4-diméthoxy-benzylamide)-8-[(2,5-diméthoxy-phényl)-amide] de l'acide 1-oxa-2,8-diaza-spiro[4.5]déc-2-ène-3,8-dicarboxylique
[160] 3-(4-chloro-benzylamide)-8-[(2,5-diméthoxy-phényl)-amide] de l'acide 1-oxa-2,8-diaza-spiro[4.5]déc-2-ène-3,8-dicarboxylique
[161] (2,5-diméthoxy-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[162] 3-(3,4-diméthoxy-benzylamide)-8-[(2,4-diméthyl-phényl)-amide] de l'acide 1-oxa-2,8-diaza-spiro[4.5]déc-2-ène-3,8-dicarboxylique
[163] 3-(4-chloro-benzylamide)-8-[(2,4-diméthyl-phényl)-amide] de l'acide 1-oxa-2,8-diaza-spiro[4.5]déc-2-ène-3,8-dicarboxylique
[164] (2,4-diméthyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[165] 8-butylamide-3-(3,4-diméthoxy-benzylamide) de l'acide 1-oxa-2,8-diaza-spiro[4.5]déc-2-ène-3,8-dicarboxylique
[166] 8-butylamide-3-(4-chloro-benzylamide) de l'acide 1-oxa-2,8-diaza-spiro[4.5]déc-2-ène-3,8-dicarboxylique
[167] butylamide de l'acide 3-[4-(3-chloro-pyridin-2-yl)-pipérazine-1-carbonyl]-1-oxa-2,8-diaza-spiro[4.5]déc-2-ène-8-carboxylique
[168] 3-(3,4-diméthoxy-benzylamide)-8-pentylamide de l'acide 1-oxa-2,8-diaza-spiro[4.5]déc-2-ène-3,8-dicarboxylique
[169] 3-(4-chloro-benzylamide)-8-pentylamide de l'acide 1-oxa-2,8-diaza-spiro[4.5]déc-2-ène-3,8-dicarboxylique
[170] pentylamide de l'acide 3-[4-(3-chloro-pyridin-2-yl)-pipérazine-1-carbonyl]-1-oxa-2,8-diaza-spiro[4.5]déc-2-ène-8-carboxylique
[171] 3-(3,4-diméthoxy-benzylamide)-8-[(2-éthoxy-phényl)-amide] de l'acide 1-oxa-2,8-diaza-spiro[4.5]déc-2-ène-3,8-dicarboxylique
[172] 3-(4-chloro-benzylamide)-8-[(2-éthoxy-phényl)-amide] de l'acide 1-oxa-2,8-diaza-spiro[4.5]déc-2-ène-3,8-dicarboxylique
[173] 3-(3,4-diméthoxy-benzylamide)-8-[(4-éthoxy-phényl)-amide] de l'acide 1-oxa-2,8-diaza-spiro[4.5]déc-2-ène-3,8-dicarboxylique
[174] 8-[(2,4-difluoro-phényl)-amide]-3-(3,4-diméthoxy-benzylamide) de l'acide 1-oxa-2,8-diaza-spiro[4.5]déc-2-ène-3,8-dicarboxylique
[175] 3-(4-chloro-benzylamide)-8-[(2,4-difluoro-phényl)-amide] de l'acide 1-oxa-2,8-diaza-spiro[4.5]déc-2-ène-3,8-dicarboxylique
[176] 8-[(3-chloro-2-méthyl-phényl)-amide]-3-(3,4-diméthoxy-benzylamide) de l'acide 1-oxa-2,8-diaza-spiro[4.5]déc-2-ène-3,8-dicarboxylique
[177] 3-(4-chloro-benzylamide)-8-[(3-chloro-2-méthyl-phényl)-amide] de l'acide 1-oxa-2,8-diaza-spiro[4.5]déc-2-ène-3,8-dicarboxylique
[178] 8-[(4-chloro-2-méthyl-phényl)-amide]-3-(3,4-diméthoxy-benzylamide) de l'acide 1-oxa-2,8-diaza-spiro[4.5]déc-2-ène-3,8-dicarboxylique
[179] 3-(4-chloro-benzylamide)-8-[(4-chloro-2-méthyl-phényl)-amide] de l'acide 1-oxa-2,8-diaza-spiro[4.5]déc-2-ène-3,8-dicarboxylique
[180] 8-[(3-chloro-4-méthyl-phényl)-amide]-3-(3,4-diméthoxy-benzylamide) de l'acide 1-oxa-2,8-diaza-spiro[4.5]déc-2-ène-3,8-dicarboxylique
[181] 3-(4-chloro-benzylamide)-8-[(3-chloro-4-méthyl-phényl)-amide] de l'acide 1-oxa-2,8-diaza-spiro[4.5]déc-2-ène-3,8-dicarboxylique
[182] 8-[(3-chloro-4-fluoro-phényl)-amide]-3-(3,4-diméthoxy-benzylamide) de l'acide 1-oxa-2,8-diaza-spiro[4.5]déc-2-ène-3,8-dicarboxylique
[183] 3-(4-chloro-benzylamide)-8-[(3-chloro-4-fluoro-phényl)-amide] de l'acide 1-oxa-2,8-diaza-spiro[4.5]déc-2-ène-3,8-dicarboxylique
[184] 8-[(2,6-diisopropyl-phényl)-amide]-3-(3,4-diméthoxy-benzylamide) de l'acide 1-oxa-2,8-diaza-spiro[4.5]déc-2-ène-3,8-dicarboxylique
[185] 3-(4-chloro-benzylamide)-8-[(2,6-diisopropyl-phényl)-amide] de l'acide 1-oxa-2,8-diaza-spiro[4.5]déc-2-ène-3,8-dicarboxylique
[186] 8-[(5-chloro-2-méthoxy-phényl)-amide]-3-(3,4-diméthoxy-benzylamide) de l'acide 1-oxa-2,8-diaza-spiro[4.5]déc-2-ène-3,8-dicarboxylique
[187] 3-(4-chloro-benzylamide)-8-[(5-chloro-2-méthoxy-phényl)-amide] de l'acide 1-oxa-2,8-diaza-spiro[4.5]déc-2-ène-3,8-dicarboxylique
[188] 3-(4-chloro-benzylamide)-8-[(3,4,5-triméthoxy-phényl)-amide] de l'acide 1-oxa-2,8-diaza-spiro[4.5]déc-2-ène-3,8-dicarboxylique
[189] 3-(3,4-diméthoxy-benzylamide)-8-[(3,5-diméthyl-phényl)-amide] de l'acide 1-oxa-2,8-diaza-spiro[4.5]déc-2-ène-3,8-dicarboxylique
[190] 3-(4-chloro-benzylamide)-8-[(3,5-diméthyl-phényl)-amide] de l'acide 1-oxa-2,8-diaza-spiro[4.5]déc-2-ène-3,8-dicarboxylique
[191] 3-(3,4-diméthoxy-benzylamide)-8-[(2,6-diméthyl-phényl)-amide] de l'acide 1-oxa-2,8-diaza-spiro[4.5]déc-2-ène-3,8-dicarboxylique
[192] 3-(4-chloro-benzylamide)-8-[(2,6-diméthyl-phényl)-amide] de l'acide 1-oxa-2,8-diaza-spiro[4.5]déc-2-ène-3,8-dicarboxylique
[193] 3-(3,4-diméthoxy-benzylamide)-8-[(3,4-diméthyl-phényl)-amide] de l'acide 1-oxa-2,8-diaza-spiro[4.5]déc-2-ène-3,8-dicarboxylique
[194] 3-(3,4-diméthoxy-benzylamide)-8-[(2,5-diméthyl-phényl)-amide] de l'acide 1-oxa-2,8-diaza-spiro[4.5]déc-2-ène-3,8-dicarboxylique
[195] 3-(4-chloro-benzylamide)-8-[(2,5-diméthyl-phényl)-amide] de l'acide 1-oxa-2,8-diaza-spiro[4.5]déc-2-ène-3,8-dicarboxylique
[196] 3-(3,4-diméthoxy-benzylamide)-8-[(2-éthyl-6-méthyl-phényl)-amide] de l'acide 1-oxa-2,8-diaza-spiro[4.5]déc-2-ène-3,8-dicarboxylique
[197] 3-(4-chloro-benzylamide)-8-[(2-éthyl-6-méthyl-phényl)-amide] de l'acide 1-oxa-2,8-diaza-spiro[4.5]déc-2-ène-3,8-dicarboxylique
[198] 3-(3,4-diméthoxy-benzylamide)-8-[(4-méthoxy-2-méthyl-phényl)-amide] de l'acide 1-oxa-2,8-diaza-spiro[4.5]déc-2-ène-3,8-dicarboxylique
[199] 3-(4-chloro-benzylamide)-8-[(4-méthoxy-2-méthyl-phényl)-amide] de l'acide 1-oxa-2,8-diaza-spiro[4.5]déc-2-ène-3,8-dicarboxylique
[200] 3-(3,4-diméthoxy-benzylamide)-8-[(2-méthoxy-5-méthyl-phényl)-amide] de l'acide 1-oxa-2,8-diaza-spiro[4.5]déc-2-ène-3,8-dicarboxylique
[201] 3-(4-chloro-benzylamide)-8-[(2-méthoxy-5-méthyl-phényl)-amide] de l'acide 1-oxa-2,8-diaza-spiro[4.5]déc-2-ène-3,8-dicarboxylique
[202] 3-(3,4-diméthoxy-benzylamide)-8-[(2,4,5-triméthyl-phényl)-amide] de l'acide 1-oxa-2,8-diaza-spiro[4.5]déc-2-ène-3,8-dicarboxylique
[203] 3-(4-chloro-benzylamide)-8-[(2,4,5-triméthyl-phényl)-amide] de l'acide 1-oxa-2,8-diaza-spiro[4.5]déc-2-ène-3,8-dicarboxylique
[204] 3-(3,4-diméthoxy-benzylamide)-8-[(2,4,6-triméthyl-phényl)-amide] de l'acide 1-oxa-2,8-diaza-spiro[4.5]déc-2-ène-3,8-dicarboxylique
[205] 3-(4-chloro-benzylamide)-8-[(2,4,6-triméthyl-phényl)-amide] de l'acide 1-oxa-2,8-diaza-spiro[4.5]déc-2-ène-3,8-dicarboxylique
[206] 8-[(2-bromo-éthyl)-amide]-3-(3,4-diméthoxy-benzylamide) de l'acide 1-oxa-2,8-diaza-spiro[4.5]déc-2-ène-3,8-dicarboxylique
[207] 8-[(2-bromo-éthyl)-amide]-3-(4-chloro-benzylamide) de l'acide 1-oxa-2,8-diaza-spiro[4.5]déc-2-ène-3,8-dicarboxylique
[208] 8-[(4-butyl-phényl)-amide]-3-(3,4-diméthoxy-benzylamide) de l'acide 1-oxa-2,8-diaza-spiro[4.5]déc-2-ène-3,8-dicarboxylique
[209] 8-[(4-butyl-phényl)-amide]-3-(4-chloro-benzylamide) de l'acide 1-oxa-2,8-diaza-spiro[4.5]déc-2-ène-3,8-dicarboxylique
[210] ester méthylique de l'acide 2-{[3-(4-chloro-benzylcarbamoyl)-1-oxa-2,8-diaza-spiro[4.5]déc-2-ène-8-carbonyl]-amino}-benzoïque
[211] 8-biphényl-2-ylamide-3-(3,4-diméthoxy-benzylamide) de l'acide 1-oxa-2,8-diaza-spiro[4.5]déc-2-ène-3,8-dicarboxylique
[212] 8-biphényl-2-ylamide-3-(4-chloro-benzylamide) de l'acide 1-oxa-2,8-diaza-spiro[4.5]déc-2-ène-3,8-dicarboxylique
[213] 8-[(2,6-dichloro-phényl)-amide]-3-(3,4-diméthoxy-benzylamide) de l'acide 1-oxa-2,8-diaza-spiro[4.5]déc-2-ène-3,8-dicarboxylique
[214] 3-(4-chloro-benzylamide)-8-[(2,6-dichloro-phényl)-amide] de l'acide 1-oxa-2,8-diaza-spiro[4.5]déc-2-ène-3,8-dicarboxylique
[215] ester éthylique de l'acide 3-{[3-(3,4-diméthoxy-benzylcarbamoyl)-1-oxa-2,8-diaza-spiro[4.5]déc-2-ène-8-carbonyl]-amino}-benzoïque
[216] ester éthylique de l'acide 3-{[3-(4-chloro-benzylcarbamoyl)-1-oxa-2,8-diaza-spiro[4.5]déc-2-ène-8-carbonyl]-amino}-benzoïque
[217] ester éthylique de l'acide 4-{[3-(3,4-diméthoxy-benzylcarbamoyl)-1-oxa-2,8-diaza-spiro[4.5]déc-2-ène-8-carbonyl]-amino}-benzoïque
[218] ester éthylique de l'acide 4-{[3-(4-chloro-benzylcarbamoyl)-1-oxa-2,8-diaza-spiro[4.5]déc-2-ène-8-carbonyl]-amino}-benzoïque
[219] 3-(4-chloro-benzylamide)8-naphtalén-1-ylamide de l'acide 1-oxa-2,8-diaza-spiro[4.5]déc-2-ène-3,8-dicarboxylique
[220] (2-chloro-5-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-thiocarboxylique
[221] 3,4-diméthoxy-benzylamide de l'acide 8-(4-chloro-3-trifluorométhyl-phénylthiocarbamoyl)-1-oxa-2,8-diaza-spiro[4.5]déc-2-ène-3-carboxylique
[222] 3,4-diméthoxy-benzylamide de l'acide 8-(3,5-bis-trifluorométhyl-phénylthiocarbamoyl)-1-oxa-2,8-diaza-spiro[4.5]déc-2-ène-3-carboxylique
[223] 4-chloro-benzylamide de l'acide 8-(3,5-bis-trifluorométhyl-phénylthiocarbamoyl)-1-oxa-2,8-diaza-spiro[4.5]déc-2-ène-3-carboxylique
[224] (3,5-bis-trifluorométhyl-phényl)-amide de l'acide 3-[4-(3-chloro-pyridin-2-yl)-pipérazine-1-carbonyl]-1-oxa-2,8-diaza-spiro[4.5]déc-2-ène-8-thiocarboxylique
[225] 3,4-diméthoxy-benzylamide de l'acide 8-cyclododécylthiocarbamoyl-1-oxa-2,8-diaza-spiro[4.5]déc-2-ène-3-carboxylique
[226] 3,4-diméthoxy-benzylamide de l'acide 8-benzylthiocarbamoyl-1-oxa-2,8-diaza-spiro[4.5]déc-2-ène-3-carboxylique
[227] 4-chloro-benzylamide de l'acide 8-benzylthiocarbamoyl-1-oxa-2,8-diaza-spiro[4.5]déc-2-ène-3-carboxylique
[228] (4-tert-butyl-phényl)-amide de l'acide 3-[4-(3-trifluorométhyl-pyridin-2-yl)-pipérazine-1-carbonyl]-1-oxa-2,8-d iaza-spiro[4.5]déc-2-ène-8-carboxylique
[229] (4-pentafluorosulfanyl-phényl)-amide de l'acide 3-[4-(3-chloro-pyridin-2-yl)-pipérazine-1-carbonyl]-1-oxa-2,8-diaza-spiro[4.5]déc-2-ène-8-carboxylique
[230] N3-((5-méthylfuran-2-yl)méthyl)-N8-(4-(trifluorométhyl)phényl)-1-oxa-2,8-diazaspiro[4.5]déc-2-ène-3,8-dicarboxamide
[231] N8-(4-méthoxyphényl)-N3-((5-méthylfuran-2-yl)méthyl)-1-oxa-2,8-diazaspiro[4.5]déc-2-ène-3,8-dicarboxamide
[232] N8-(4-chlorophényl)-N3-((5-méthylfuran-2-yl)méthyl)-1-oxa-2,8-diazaspiro[4.5]déc-2-ène-3,8-dicarboxamide
[233] N3-(4-(3-chloropyridin-2-yl)pipérazine-1-yl)-N8-(3,4-dichlorophényl)-1-oxa-2,8-diazaspiro[4.5]déc-2-ène-3,8-dicarboxamide
[234] N3-(4-(3-chloropyridin-2-yl)pipérazine-1-yl)-N8-(4-méthoxyphényl)-1-oxa-2,8-diazaspiro[4.5]déc-2-ène-3,8-dicarboxamide
[235] N8-(4-chlorobenzyl)-N3-((5-méthylfuran-2-yl)méthyl)-1-oxa-2,8-diazaspiro[4.5]déc-2-ène-3,8-dicarboxamide
[236] N8-(3,4-dichlorobenzyl)-N3-((5-méthylfuran-2-yl)méthyl)-1-oxa-2,8-diazaspiro[4.5]déc-2-ène-3,8-dicarboxamide
[237] N3-(4-chlorobenzyl)-N8-(3,4-dichlorobenzyl)-1-oxa-2,8-diazaspiro[4.5]déc-2-ène-3,8-dicarboxamide
[238] N8-(3,4-dichlorobenzyl)-N3-(4-hydroxy-3-méthoxybenzyl)-1-oxa-2,8-diazaspiro[4.5]déc-2-ène-3,8-dicarboxamide
[239] N3-(4-tert-butylbenzyl)-N8-(3,4-dichlorobenzyl)-1-oxa-2,8-diazaspiro[4.5]déc-2-ène-3,8-dicarboxamide
[240] N8-(3,4-dichlorobenzyl)-N3-(4-(trifluorométhyl)benzyl)-1-oxa-2,8-diazaspiro[4.5]déc-2-ène-3,8-dicarboxamide
[241] 3-(4-(3-chloropyridin-2-yl)pipérazine-1-carbonyl)-N-(3,4-dichlorobenzyl)-1-oxa-2,8-diazaspiro[4.5]déc-2-ène-8-carboxamide
[242] N3-(4-(3-chloropyridin-2-yl)pipérazine-1-yl)-N8-(3,4-dichlorobenzyl)-1-oxa-2,8-diazaspiro[4.5]déc-2-ène-3,8-dicarboxamide
[243] N8-(3,4-dichlorophényl)-N3-((5-méthylfuran-2-yl)méthyl)-1-oxa-2,8-diazaspiro[4.5]déc-2-ène-3,8-dicarboxamide
[244] N8-(4-chlorophényl)-N3-(4-hydroxy-3-méthoxybenzyl)-1-oxa-2,8-diazaspiro[4.5]déc-2-ène-3,8-dicarboxamide
[245] N8-(3,4-dichlorophényl)-N3-(4-hydroxy-3-méthoxybenzyl)-1-oxa-2,8-diazaspiro[4.5]déc-2-ène-3,8-dicarboxamide
[246] N3-(4-chlorobenzyl)-N8-(3,4-dichlorophényl)-1-oxa-2,8-diazaspiro[4.5]déc-2-ène-3,8-dicarboxamide
[247] N3-(4-chlorobenzyl)-N8-(4-chlorophényl)-1-oxa-2,8-diazaspiro[4.5]déc-2-ène-3,8-dicarboxamide
[248] N3-(4-hydroxy-3-méthoxybenzyl)-N8-(4-(trifluorométhyl)phényl)-1-oxa-2,8-diazaspiro[4.5]déc-2-ène-3,8-dicarboxamide
[249] N3-(4-hydroxy-3-méthoxybenzyl)-N8-(4-méthoxyphényl)-1-oxa-2,8-diazaspiro[4.5]déc-2-ène-3,8-dicarboxamide
[250] N3-(4-chlorobenzyl)-N8-(4-(trifluorométhyl)phényl)-1-oxa-2,8-diazaspiro[4.5]déc-2-ène-3,8-dicarboxamide
[251] N8-(3,4-dichlorobenzyl)-N3-(3,4-diméthoxybenzyl)-1-oxa-2,8-diazaspiro[4.5]déc-2-ène-3,8-dicarboxamide
[252] N3-(3,4-diméthoxybenzyl)-N8-(4-(trifluorométhyl)phényl)-1-oxa-2,8-diazaspiro[4.5]déc-2-ène-3,8-dicarboxamide
[253] (4-pentafluorosulfanyl-phényl)-amide de l'acide 3-[4-(3-trifluorométhyl-pyridin-2-yl)-pipérazine-1-carbonyl]-1-oxa-2,8-diaza-spiro[4.5]déc-2-ène-8-carboxylique
[254] N-(4-tert-butylphényl)-3-(4-(3-chloropyridin-2-yl)-2-méthylpipérazine-1-carbonyl)-1-oxa-2,8-diazaspiro[4.5]déc-2-ène-8-carboxamide
et
[255] N-(4-tert-butylbenzyl)-3-(4-(3-chloropyridin-2-yl)pipérazine-1-carbonyl)-1-oxa-2,8-diazaspiro[4.5]déc-2-ène-8-carboxamide.
